# EUROPEAN PATENT APPLICATION

(11) **EP 1 577 290 A1**
(43) Date of publication of application: **21.09.2005**
(21) Application number: 03780917.5
(22) Date of filing: 19.12.2003
(51) Int. Cl.: C07C 235/34, C07C 327/44, C07C 69/734, C07C 59/64, A01N 37/38

(54) **AMIDE COMPOUND AND METHOD OF CONTROLLING PLANT DISEASE WITH THE SAME**

(30) Priority: 24.12.2002 JP 2002371737; 04.03.2003 JP 2003056810
(71) Applicant: Sumitomo Chemical Company, Limited, Tokyo 104-8260 (JP)
(72) Inventor: SAKAGUCHI, Hiroshi, Toyonaka-shi, Osaka 561-0802 (JP)
(74) Representative: Henkel, Feiler & Hänzel
(86) International application number: PCT/JP2003/016294
(87) International publication number: WO 2004/058685

(57) **Abstract**

A amid compound of the formula (1): 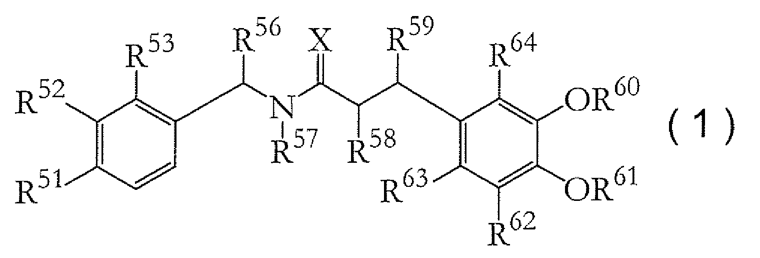 wherein, in the formula,
R⁵¹ represents a halogen atom, a C1-C6 alkyl group and the like;
R⁵² represents a hydrogen atom, a halogen atom, a C1-C6 alkyl group and the like; R⁵³ represents a halogen atom and the like;
R⁵⁶ represents a halogen atom and the like; R⁵⁷ represents a hydrogen atom and the like; R⁵⁸ and R⁵⁹ independently represent a hydrogen atom, a C1-C3 alkyl group and the like; R⁶⁰ represents a C1-C4 alkyl group, a C1-C4 haloalkyl group, a C3-C4 alkenyl group, or a C3-C6 alkynyl group; R⁶¹ represents a C1-C4 alkyl group, a C1-C4 haloalkyl group, a C3-C4 alkenyl group or a C3-C6 alkynyl group or a C2-C4 cyanoalkyl group; R⁶², R⁶³ and R⁶⁴ represent a hydrogen atom, a halogen atom and the like; X represents a oxygen atom or a sulfur atom;
has an excellent activity against plant diseases.

## Description

### Technical Field

The present invention relates amide compounds and a method for controlling plant diseases applying the same for plants or soils growing the plant.

### Background Art

The development of plant diseases controlling composition have been carried out, and many compounds which have a controlling activity against plant diseases was found out. However, sometimes their controlling activity are not always enough, and there have been studying to find out new compounds.

On the other hand, though the amide compound (A) shown by the following formula is disclosed in Journal of Chemical Society, Perkin Transactions I, 6, p.661 (1976), the concrete use of said compound is not disclosed in it.

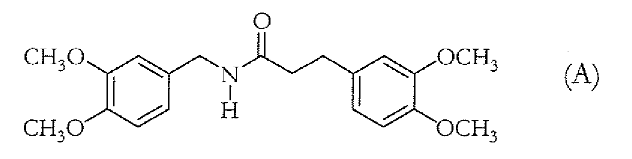

### Disclosure of Invention

The present inventors, after intensively studying to find out the compounds which have an excellent controlling activity against plant diseases, have found that an amide compound shown by the following formula (1) has an excellent controlling activity against plant diseases and accomplished the present invention.

Namely, the present invention provides an amide compound
(1) represented below (referred as "the compound of the present invention" hereinafter.): 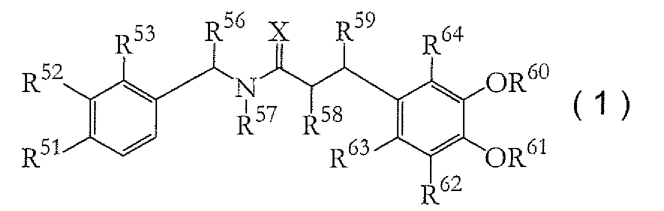 wherein, in the formula,
   R⁵¹ represents a halogen atom, a C1-C6 alkyl group, a C3-C6 cycloalkyl group, a C1-C6 haloalkyl group, a C2-C6 alkenyl group, a C2-C6 haloalkenyl group, a C2-C6 alkynyl group, a C2-C6 haloalkynyl group, a C1-C6 alkoxy group, a C3-C6 alkenyloxy group, a C3-C6 alkynyloxy group, a C1-C6 haloalkoxy group, a (C1-C6 alkoxy) C1-C6 alkyl group, a phenoxy C1-C6 alkyl group, a hydroxy C1-C6 alkyl group, a (C1-C6 alkyl sulfonyloxy) C1-C6 alkyl group, a C1-C6 alkylthio group, a C1-C6 haloalkylthio group, a C1-C6 alkylamino group, a di(C1-C6alkyl) amino group, a formyl group, a (C1-C6 alkyl) carbonyl group, a (C1-C6 alkoxy) carbonyl group, a (C1-C6 alkoxy) imino C1-C6 alkyl group, benzyloxyimino C1-C6 alkyl group, a di (C1-C6 alkylamino) imino C1-C6 alkyl group, a tri (C1-C6 alkyl) silyl group, a phenyl group, a phenoxy group, a cyano group or a nitro group; R⁵² represents a hydrogen atom, a halogen atom, a C1-C6 alkyl group, a C1-C6 haloalkyl group, a C2-C6 alkenyl group, a C2-C6 alkynyl group, a cyano group or a nitro group; or both of R⁵¹ and R⁵² are combined together to represent a C3-C6 alkylene group or a group of -CR⁶⁵=CR⁶⁶-CR⁶⁷=CR⁶⁸-R⁶⁵, R⁶⁶, R⁶⁷ and R⁶⁸ independently represent a hydrogen atom, a halogen atom, a C1-C3 alkyl group, a C1-C3 alkoxy group or a C1-C3 haloalkyl group);
   R⁵³ represents a hydrogen atom, a halogen atom, a C1-C3 alkyl group or a C1-C3 haloalkyl group;
   R⁵⁶ represents a hydrogen atom, a C1-C4 alkyl group, a C2-C4 alkenyl group or a C2-C4 alkynyl group;
   R⁵⁷ represents a hydrogen atom, a C1-C4 alkyl group, a C2-C4 alkenyl group or a C2-C4 alkynyl group;
   R⁵⁸ and R⁵⁹ independently represent a hydrogen atom, a halogen atom or a C1-C3 alkyl group;
   R⁶⁰ represents a C1-C4 alkyl group, a C1-C4 haloalkyl group, a C3-C4 alkenyl group or a C3-C6 alkynyl group;
   R⁶¹ represents a C1-C4 alkyl group, a C1-C4 haloalkyl group, a C3-C4 alkenyl group or a C3-C6 alkynyl group or a C2-C4 cyanoalkyl group;
   each of R⁶², R⁶³ and R⁶⁴ represents a hydrogen atom, a halogen atom or a C1-C2 alkyl group;
   X represents an oxygen atom or a sulfur atom;
   a plant diseases controlling composition comprising the compound of the present invention as an active ingredient; and
   a method for controlling plant diseases comprising a step applying an effective amount of the compound of the present invention to plants or soils growing the plant. The present invention also provides the compound (3) represented below (referred as "the intermediate 1 of the present invention" hereinafter.): 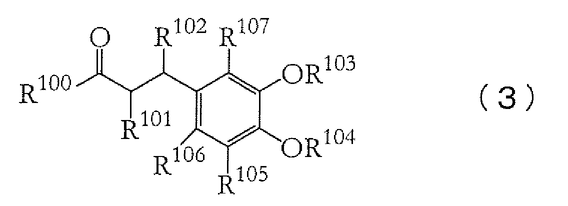 wherein, in the formula,
   R¹⁰⁰ represents a methoxy group, an ethoxy group, a propoxy group, an isopropoxy group, a butyloxy group, an isopropyloxy group, a tert-buthyloxy group, an OH group or a chlorine atom; R¹⁰¹ and
   R¹⁰² independently represent a hydrogen atom, a halogen atom or a C1-C3 alkyl group; R¹⁰³ represents a C1-C4 alkyl group; R¹⁰⁴ represents a C3-C6 alkynyl group; R¹⁰⁵, R¹⁰⁶ and R¹⁰⁷ independently represent a hydrogen atom, a halogen atom or a C1-C2 alkyl group; and the amide compound (4) represented below (referred as "the intermediate 2 of the present invention" hereinafter.): 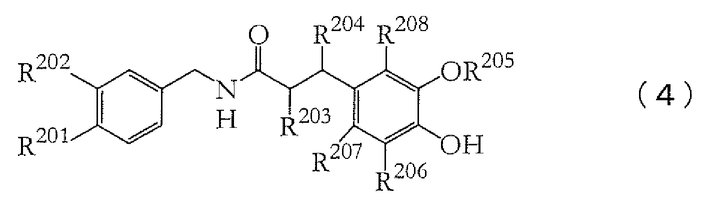 wherein, in the formula,
   R²⁰¹ represents a halogen atom, a C1-C4 alkyl group, a C1-C4 haloalkyl group, a C1-C4 alkoxy group, a C1-C4 haloalkoxy group, a di(C1-C4alkyl)amino group or a cyano group; R²⁰² represents a hydrogen atom, a halogen atom, a C1-C4 alkyl group or a C1-C4 haloalkyl group; or both of R²⁰¹ and R²⁰² are combined together to represent a C3-C5 alkylene group or a group of -CH=CH-CH=CH-; R²⁰³ and R²⁰⁴ independently represent a hydrogen atom, a halogen atom or a C1-C3 alkyl group; R²⁰⁵ represents a C1-C4 alkyl group, R²⁰⁶, R²⁰⁷ and R²⁰⁸ independently represent a hydrogen atom, a halogen atom or a C1-C2 alkyl group;
   which are important intermediates in production of the compound of the present invention.

The substituents in the amide compound (1), the compound (2), and the amide compound (4) of the present invention are concretely exampled below.

In the representation of R⁵¹,
the halogen atom includes a fluorine atom, a chlorine atom, a bromine atom, an iodine atom;
the C1-C6 alkyl group includes a C1-C4 alkyl group such as a methyl group, an ethyl group, a propyl group, an isopropyl group, a butyl group, an isobutyl group, a sec-butyl group, a tert-butyl group; a C5-C6 alkyl group such as a pentyl group, a 2-methylpentyl group, a 3-methylpentyl group, a 4-methylpentyl group, a hexyl group and the like;
the C3-C6 cycloalkyl group includes a cyclopropyl group, a cyclobutyl group, a cyclopentyl group and a cyclohexyl group; the C1-C6 haloalkyl group includes, for example, a C1-C4 haloalkyl group such as a fluoromethyl group, a difluoromethyl group, a trifluoromethyl group;
the C2-C6 alkenyl group includes, for example, a C2-C4 alkenyl group such as a vinyl group, a 1-methylvinyl group, a 1-propenyl group, a 2-propenyl group, a 1-methyl-2-propenyl group, a 2-methyl-2-propenyl group, a 2-butenyl group, a 3-butenyl group and the like; a C5-C6 alkenyl group such as a 1-pentenyl group, a 4-pentenyl group, a 1-hexenyl group, a 5-hexenyl group and the like;
the C2-C6 haloalkenyl group includes, for example, a 2-fluorovinyl group, a 2-chlorovinyl group, a 2-bromovinyl group, a 2,2-difluorovinyl group, a 2,2-dichlorovinyl group, a 2, 2-dibromovinyl group, a 1-methyl-2,2-dichlorovinyl group, a 1-methyl-2,2-dibromovinyl group; the C2-C6 alkynyl group includes, for example, a C2-C4 alkynyl group such as an ethynyl group, a 1-propynyl group, a 2-propynyl group, 1-methyl-2-propynyl group, a 1-butynyl group, a 2-butynyl group, a 3-butynyl group and the like; a C5-C6 alkenyl group such as a 3,3-dimethyl-1-butynyl group, a 1-pentynyl group, a 4-pentynyl group, a 1-hexynyl group, a 5-hexynyl group and the like;
the C2-C6 haloalkynyl group includes, for example, a chloroethynyl group, a bromoethynyl group, a iodoethynyl group; the C1-C6 alkoxy group includes, for example, a C1-C4 alkoxy group such as a methoxy group, an ethoxy group, a propoxy group, an isopropoxy group, a butoxy group, an isobutoxy group, a sec-butoxy group, a tert-butoxy group; a C5-C6 alkoxy group such as a pentyloxy group, a hexyloxy and the like;
the C3-C6 alkenyloxy group includes, for example, a 2-propenyloxy group, a 1-methyl-2-propenyloxy group, a2-methyl-2-propenyloxy group, a 2-butenyloxy group, a 3-butenyloxy group, a 4-pentenyloxy group, a 5-hexenyloxy group;
the C3-C6 alkynyloxy group includes, for example, a 2-propynyloxy group, a 1-methyl-2-propynyloxy group, a 2-butynyloxy group, a 3-butynyloxy group, a 3-pentynyloxy group, a 4-pentynyloxy group, a 5-hexynyloxy group;
the C1-C6 haloalkoxy group includes, for example, a C1-C4 haloalkoxy group such as a fluoromethoxy group, a difluoromethoxy group, a trifluoromethoxy group, a chloromethyl group, a dichloromethyl group, a trichloromethyl group, a 2,2,2-trifluoroethoxy group, a 1,1,2,2,-tetrafluoroethoxy group, a 2-fluoroethoxy group and the like;
the (C1-C6 alkoxy) C1-C6 alkyl group includes a methoxymethyl group, an ethoxymethyl group, a propyloxymethyl group, a 1-methoxyethyl group, a 2-methoxyethyl group, a 2-methoxypropyl group, a 2-methoxyisoprppyl group;
the phenoxy C1-C6 alkyl group includes a phenoxy methyl group, a1-phenoxyethyl group, a2 -phenoxyethyl group, a1 -phenoxypropyl group, a 3-phenoxypropyl group, a 4-phenoxybuthyl group; hydroxy C1-C6 alkyl group includes a hydroxymethyl group, a 1-hydroxyethyl group, a 2-hydroxyethyl group, a 1-hydroxypropyl group, a 1-hydroxybutyl group;
the (C1-C6 alkylslfonyloxy) C1-C6 alkyl group includes a methylsulfonyloxymethyl group, a ethylsulfonyloxymethyl group, a 1-methylsulfonyloxyethyl group, a 2-sulfonyloxyethyl group, a 1-methylsulfonyloxypropyl group;
the C1-C6 alkylthio group includes, for example, a methylthio group, a ethylthio group, a propylthio group, a isopropylthio group, a butylthio group, a isobutylthio group, a sec-butylthio group, a tert-butylthio group, a pentylthio group, a hexylthio group;
the C1-C6 haloalkylthio group includes, for example, a fluoromethylthio group, a difluoromethylthio group, a trifluoromethylthio group;
the C1-C6 alkylamino group includes, for example, a C1-C4 alkylamino group such as a methylamino group, an ethylamino group, a propylamino group, an isopropylamino group; a pentylamino group, a hexylamino;
the di(C1-C6 alkyl)amino group includes, for example, a C1-C4 dialkylamino group such as a dimethylamino group, a diethylamino group, a dipropylamino group; a dipentylamino group, a dihexylamino group;
the (C1-C6 alkyl) carbonyl group includes a methylcarbonyl group, an ethylcarbonyl group, a propylcarbonyl group, an isopropylcarbonyl group;
the (C1-C6 alkoxy) carbonyl group includes a methoxycarbonyl group, a ethoxycarbonyl group, a propoxycarbonyl group, a isopropoxycarbonyl group;
the (C1-C6 alkoxyimino) C1-C6 alkyl group includes a methoxyiminomethyl group, a 1-methoxyiminoethyl group, a 1-methoxyiminopropyl group, a 1-methoxyiminobutyl group, an ethoxyiminomethyl group, a 1-ethoxyiminoethyl group, a propoxyiminomethyl group, a 1-isopropoxyiminoethyl group, a butoxyiminomethyl group, a pentyloxyiminomethyl group, a 1-hexyloxyiminoethyl group;
the benzyloxyimino C1-C6 alkyl group includes a benzyloxyiminomethyl group, a 1-(benzyloxyimino)ethyl group, a 2-(benzyloxyimino)ethyl group, a 1-(benzyloxyimino)propyl group, a 1-(benzyloxyimino)butyl group;
the di(C1-C6 alkyl)aminoimino C1-C6 alkyl group includes a dimethylaminoiminomethyl group, a 1-(dimethylaminoimino) ethyl group, a diethylaminoiminomethyl group, a 1-(diethylaminoimino)ethyl group;
the tri (C1-C6 alkyl) silyl group includes a trimethyl silyl group, a triethyl silyl group, a tert-butyldimethyl silyl group;

In the representation of R⁵²,
the halogen atom includes a fluorine atom, a chlorine atom, a bromine atom, an iodine atom;
the C1-C6 alkyl group includes a C1-C4 alkyl group such as a methyl group, an ethyl group, a propyl group, an isopropyl group, a butyl group, an isobutyl group, sec-butyl group, tert-butyl group; a C5-C6 alkyl group such as a pentyl group, a 2-methylpentyl group, a 3-methylpentyl group, a 4-methylpentyl group, a hexyl group and the like;
the C1-C6 haloalkyl group includes, for example, a fluoromethyl group, a difluoromethyl group, a trifluoromethyl group;
the C2-C6 alkenyl group includes, for example, a C1-C4 alkenyl group such as a vinyl group, a 1-methylvinyl group, a 1-propenyl group, a 2-propenyl group, a 1-methyl-2-propenyl group, a 2-methyl-2-propenyl group, a 2-butenyl group, a 3-butenyl group and the like; a C5-C6 alkyl group such as a 1-pentenyl group, a 4-pentenyl group, a 1-hexenyl group, a 5-hexenyl group and the like;
the C2-C6 alkynyl group includes, for example, a C2-C4 alkynyl group such as an ethynyl group, a 1-propynyl group, a 2-propynyl group, a 1-methyl-2-propynyl group, a 1-butynyl group, a 2-butynyl group, a 3-butynyl group and the like; a C5-C6 alkynyl group such as a 1-pentynyl group, a 4-pentynyl group, a 1-hexynyl group, a 5-hexynyl group and the like;
the C3-C6 alkylene group represented by R⁵¹ and R⁵² are combined together includes, for example, a trimethylene group, a tetraethylene group, a pentamethylene group and hexamethylene group.

In the representation of R⁵³,
the halogen atom includes a fluorine atom, a chlorine atom, a bromine atom, an iodine atom;
the C1-C3 alkyl group includes a methyl group, an ethyl group, a propyl group, an isopropyl group;
the C1-C3 haloalkyl group includes, for example, a trifluoromethyl group.

In the representation of R⁵⁶,
the C1-C4 alkyl group includes a methyl group, an ethyl group, a propyl group, an isopropyl group, a butyl group, an isobutyl group, a sec-butyl group, a tert-butyl group;
the C2-C4 alkenyl group includes, for example, a vinyl group, a 1-methylvinyl group, a 1-propenyl group, a 2-propenyl group, a 1-methyl-2-propenyl group, a 2-methyl-2-propenyl group, a 2-butenyl group, a 3-butenyl group and the like;
the C2-C4 alkynyl group includes, for example, an ethynyl group, a 1-propynyl group, a 2-propynyl group, a 1-methyl-2-propynyl group, a 2-butynyl group, a 3-butynyl group and the like.

In the representation of R⁵⁷,
the C1-C4 alkyl group, for example, includes a methyl group, an ethyl group, a propyl group, a butyl group;
the C3-C4 alkenyl group includes, for example, a 2-propenyl group, a 1-methyl-2-propenyl group, a 2-methyl-2-propenyl group, a 2-butenyl group, a 3-butenyl group and the like;
the C3-C4 alkynyl group includes a 2-propynyl group, 1-methyl-2-propynyl group, a 2-butynyl group, a 3-butynyl group and the like.

In the representation of R⁵⁸ and R⁵⁹,
the halogen atom includes a fluorine atom, a chlorine atom, a bromine atom;
the C1-C3 alkyl group includes a methyl group, an ethyl group, a propyl group, an isopropyl group.

In the representation of R⁶⁰,
the C1-C4 alkyl group includes a C1-C2 alkyl group such as a methyl group, an ethyl group; a propyl group, an isopropyl group, a butyl group, an isobutyl group, a sec-butyl group, a tert-butyl group;
the C1-C4 haloalkyl group includes, for example, a fluoromethyl group, a difluoromethyl group, a trifluoromethyl group, a 2,2,2-trifluoroethyl group, a 1,1,2,2-tetrafluoroethyl group, a 2-fluoroethyl group;
the C3-C4 alkenyl group includes, for example, a 2-propenyl group, a 1-methyl-2-propenyl group, a 2-methyl-2-propenyl group, a 2-butenyl group, 3-butenyl group;
the C3-C6 alkynyl group includes, for example, a C3-C4 alkynyl group such as 2-propynyl group, a 1-methyl-2-propynyl group, a 2-butynyl group, a 3-butynyl group and the like; a C5-C6 alkynyl group such as a 1,1-dimethyl-2-propynyl group, a 1-methyl-2-butynyl group, a 2-pentynyl group, a 1-methyl-2-pentynyl group, a 4-methyl-2-pentynyl group, a 3-pentynyl group, a 4-pentynyl group, a 2-hexynyl group, a 3-hexynyl group and the like;

In the representation of R⁶¹,
the C1-C4 alkyl group includes a methyl group, an ethyl group, a propyl group, an isopropyl group, a butyl group, an isobutyl group, a sec-butyl group, a tert-butyl group;
the C1-C4 haloalkyl group includes, for example, a fluoromethyl group, a difluoromethyl group, a trifluoromethyl group, a 2,2,2-trifluoroethyl group, a 1,1,2,2-tetrafluoroethyl group, a 2-fluoroethyl group;
the C3-C4 alkenyl group includes, for example, a 2-propenyl group, a 1-methyl-2-propenyl group, a 2-methyl-2-propenyl group, a 2-butenyl group, a 3-butenyl group;
the C3-C6 alkynyl group includes, for example, a C3-C4 alkynyl group such as a 2-propynyl group, a 1-methyl-2-propynyl group, a 2-butynyl group, a 3-butynyl group; a C5-C6 alkynyl group such as a 1, 1-dimethyl-2-propynyl group, a 1-methyl-2-butynyl group, a 2-pentynyl group, a 1-methyl-2-pentynyl group, a 4-methyl-2-pentynyl group, a 3-pentynyl group, a 4-pentynyl group, a 2-hexynyl group, a 3-hexynyl group and the like;
the C2-C4 cyanoalkyl group includes, for example, a cyanomethyl group, a cyanoethyl group, a cyanopropyl group, a 1-methylcyanomethyl group, a 1,1-dimethylcyanomethyl group.

In the representation of R⁶², R⁶³ and R⁶⁴,
the halogen atom includes a fluorine atom, a chlorine atom, a bromine atom, an iodine atom;
the C1-C2 alkyl group includes a methyl group and an ethyl group.

In the representation of R⁶⁵, R⁶⁶, R⁶⁷ and R⁶⁸,
the halogen atom includes a fluorine atom, a chlorine atom, a bromine atom, an iodine atom;
the C1-C3 alkyl group includes a methyl group, an ethyl group, a propyl group, an isopropyl group;
the C1-C3 alkoxy group includes a methoxy group, an ethoxy group, a propyloxy group, an isopropyloxy group;
the C1-C3 haloalkyl group includes, for example, a trifluoromethyl group.

In the representation of R¹⁰¹ and R¹⁰²,
the halogen atom includes a fluorine atom, a chlorine atom, a bromine atom;
the C1-C3 alkyl group includes a methyl group, an ethyl group, a propyl group, an isopropyl group.

In the representation of R¹⁰³,
the C1-C4 alkyl group includes a C1-C2 alkyl group such as a methyl group, an ethyl group; a propyl group, an isopropyl group, a butyl group, an isobutyl group, a sec-butyl group, a tert-butyl group.

In the representation of R¹⁰⁴,
the C3-C6 alkynyl group includes, for example, a C3-C4 alkynyl group such as a 2-propynyl group, a 1-methyl-2-propynyl group, a 2-butynyl group, a 3-butynyl group; a C5-C6 alkynyl group such as a 1, 1-dimethyl-2-propynyl group, a I-methyl-2-butynyl group, a 2-pentynyl group, a 1-methyl-2-pentynyl group, a 4-methyl-2-pentynyl group, a 3-pentynyl group, a 4-pentynyl group, a 2-hexynyl group, 3-hexynyl group.

In the representation of R¹⁰⁵, R¹⁰⁶ and R¹⁰⁷,
the halogen atom includes a fluorine atom, a chlorine atom, a bromine atom, an iodine atom;
the C1-C2 alkyl group includes a methyl group, an ethyl group.

In the representation of R²⁰¹,
the halogen atom includes a fluorine atom, a chlorine atom, a bromine atom, an iodine atom;
the C1-C4 alkyl group includes a methyl group, an ethyl group, a propyl group, an isopropyl group, a butyl group, an isobutyl group, a sec-butyl group, a tert-butyl group;
the C1-C4 haloalkyl group includes, for example, a fluoromethyl group, a difluoromethyl group, a trifluoromethyl group;
the C1-C4 alkoxy group includes, for example, a methoxy group, an ethoxy group, a propoxy group, an isopropoxy group, a butoxy group, an isobutoxy group, a sec-butoxy group, a tert-butoxy group;
the C1-C4 haloalkoxy group includes, for example, a fluoromethoxy group, a difluoromethoxy group, a trifluoromethoxy group, a chloromethyl group, a dichloromethyl group, a trichloromethyl group, a 2,2,2-trifluoroethoxy group, a 1,1,2,2-tetrafluoroethoxy group, a 2-fluoroethoxy group;
the di(C1-C4alkyl)amino group includes, for example, a dimethylamino group, a diethylamino group, a dipropylamino group;

In the representation of R²⁰²,
the halogen atom includes a fluorine atom, a chlorine atom, a bromine atom, an iodine atom;
the C1-C4 alkyl group includes, for example, a methyl group, an ethyl group, a propyl group, an isopropyl group, a butyl group, an isobutyl group, a sec-butyl group, a tert-butyl group;
the C1-C6 haloalkyl group includes, for example, a fluoromethyl group, a difluoromethyl group, a trifluoromethyl group;
the C3-C5 alkylene group represented by R²⁰¹ and R²⁰² are combined together, for example, a trimethylene group, a tetraethylene group and a pentaethylene group.

In the representation R²⁰³ and R²⁰⁴,
the halogen atom includes a fluorine atom, a chlorine atom, a bromine atom;
the C1-C3 alkyl group includes a methyl group, an ethyl group, a propyl group and an isopropyl group.

In the representation of R²⁰⁵,
the C1-C4 alkyl group includes a C1-C2 alkyl group such as a methyl group, an ethyl group; a propyl group, an isopropyl group, a butyl group, an isobutyl group, a sec-butyl group, a tert-butyl group.

In the representation of R²⁰⁶, R²⁰⁷ and R²⁰⁸,
the halogen atom includes a fluorine atom, a chlorine atom, a bromine atom, an iodine atom;
the C1-C2 alkyl group includes a methyl group and an ethyl group.

The embodiment of the compound of the present invention includes, for example, the following compounds:
an amide compound represented by formula (1) wherein R⁵³ is a hydrogen atom;
an amide compound represented by formula (1) wherein R⁶², R⁶³ and R⁶⁴ are hydrogen atoms;
an amide compound represented by formula (1) wherein R⁵⁸ and R⁵⁹ are hydrogen atoms;
an amide compound represented by formula (1) wherein R⁵⁶ is a hydrogen atom;
an amide compound represented by formula (1) wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di(C1-C4 alkyl)amino group or a cyano group, R⁵² is a hydrogen atom, a halogen atom, a C1-C4 alkyl group, a C1-C4 haloalkyl group, a C2-C4 alkenyl group or a C2-C4 alkynyl group or both of R⁵¹ and
R⁵² are combined together to be a C3-C5 alkylene group or a group of -CH=CH-CH=CH-;
an amide compound represented by formula (1) wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di(C1-C4 alkyl)amino group or a cyano group;
an amide compound represented by formula (1) wherein R⁵² is a hydrogen atom, a halogen atom, a C1-C4 alkyl group, a C1-C4 haloalkyl group, a C2-C4 alkenyl group or a C2-C4 alkynyl group; an amide compound represented by formula (1) wherein R⁵⁷ is a hydrogen atom or a C1-C3 alkyl group;
an amide compound represented by formula (1) wherein R⁶⁰ is a C1-C4 alkyl group, a C1-C4 haloalkyl group, a C3-C4 alkenyl group or a C3-C4 alkynyl group;
an amide compound represented by formula (1) wherein R⁶¹ is a C1-C4 alkyl group, a C1-C4 haloalkyl group, a C3-C4 alkenyl group or a C3-C4 alkynyl group;
an amide compound represented by formula (1) wherein R⁵¹ is a halogen atom, a C1-C4 alkyl group, a C1-C4 haloalkyl group, a C1-C4 alkoxy group, a C1-C4 haloalkoxy group, a di(C1-C4 alkyl)amino group or a cyano group, R⁵² is a hydrogen atom, a halogen atom, a C1-C4 alkyl group, a C1-C4 haloalkyl group, a C2-C4 alkenyl group or a C2-C4 alkynyl group or both of R⁵¹ and
R⁵² are combined together to be a C3-C5 alkylene group or a group of -CH=CH-CH=CH-, R⁵³ is a hydrogen atom, R⁵⁶ is a hydrogen atom, R⁵⁷ is a hydrogen atom, X is an oxygen atom, R⁶⁰ is a C1-C4 alkyl group, R⁶¹ is a C3-C6 alkynyl group;
an amide compound represented by formula (1) wherein R⁵¹ is a halogen atom, a C1-C4 alkyl group, a C1-C4 haloalkyl group, a C1-C4 alkoxy group, a C1-C4 haloalkoxy group, a di(C1-C4 alkyl)amino group or a cyano group, R⁵² is a hydrogen atom, a halogen atom, a C1-C4 alkyl group, a C1-C4 haloalkyl group, a C2-C4 alkenyl group or a C2-C4 alkynyl group or both of R⁵¹ and
R⁵² are combined together to be a C3-C5 alkylene group or a group of -CH=CH-CH=CH-, R⁵³ is a hydrogen atom, R⁵⁶ is a hydrogen atom, R⁵⁷ is a hydrogen atom, X is an oxygen atom, each of R⁵⁸ and R⁵⁹ is a hydrogen atom, a fluorine atom or a methyl group, R⁶⁰ is a methyl group or an ethyl group, R⁶¹ is a C3-C6 alkynyl group, R⁶², R⁶³ and R⁶⁴ are hydrogen atoms;
an amide compound represented by formula (1) wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di (C1-C4 alkyl)amino group or a cyano group, R⁵² is a hydrogen atom, a halogen atom, a C1-C4 alkyl group, a C1-C4 haloalkyl group, a C2-C4 alkenyl group or a C2-C4 alkynyl group or both of R⁵¹ and R⁵² are combined together to be a C3-C6 alkylene group or a group of -CH=CH-CH=CH-, R⁵³ and R⁵⁶ are hydrogen atoms, R⁵⁷ is a hydrogen atom or a C1-C3 alkyl group, R⁵⁸ and R⁵⁹ are hydrogen atoms, R⁶⁰ is a C1-C4 alkyl group, a C1-C4 haloalkyl group, a C3-C4 alkenyl group or a C3-C4 alkynyl group, R⁶¹ is a C1-C4 alkyl group, a C1-C4 haloalkyl group, a C3-C4 alkenyl group or a C3-C4 alkynyl group, R⁶², R⁶³ and R⁶⁴ are hydrogenatoms, namely, the amide compound represented by formula (2): 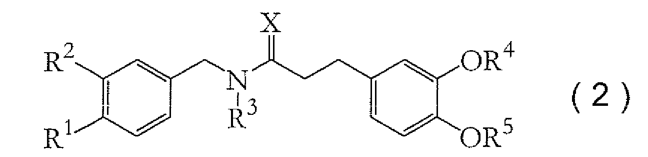
wherein,
R¹ represents a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di(C1-C4alkyl)amino group or a cyano group; R² represents a hydrogen atom, a halogen atom, a C1-C4 alkyl group, a C1-C4 haloalkyl group, a C2-C4 alkenyl group or a C2-C4 alkynyl group; or both of R¹ and R² are combined together to represent a C3-C5 alkylene group or a group of -CH=CH-CH=CH-;
R³ represents a hydrogen atom or a C1-C3 alkyl group;
R⁴ represents a C1-C4 alkyl group, a C1-C4 haloalkyl group, a C3-C4 alkenyl group or a C3-C4 alkynyl group;
R⁵ represents a C1-C4 alkyl group, a C1-C4 haloalkyl group, a C3-C4 alkenyl group or a C3-C4 alkynyl group;
X represents an oxygen atom or a sulfur atom.
an amide compound represented by formula (2) wherein R¹ is a halogen atom;
an amide compound represented by formula (2) wherein R¹ is a C1-C4 alkyl group;
an amide compound represented by formula (2) wherein R¹ is a C1-C4 haloalkyl group;
an amide compound represented by formula (2) wherein R¹ is a C1-C4 alkylamino group;
an amide compound represented by formula (2) wherein R¹ is a di(C1-C4 alkyl)amino group;
an amide compound represented by formula (2) wherein R¹ is a chlorine atom;
an amide compound represented by formula (2) wherein R¹ is a methyl group;
an amide compound represented by formula (2) wherein R¹ is a trifluoromethyl group;
an amide compound represented by formula (2) wherein R² is a hydrogen atom;
an amide compound represented by formula (2) wherein R² is a halogen atom;
an amide compound represented by formula (2) wherein R² is a C1-C4 haloalkyl group;
an amide compound represented by formula (2) wherein R³ is a hydrogen atom;
an amide compound represented by formula (2) wherein R⁴ is a C1-C4 alkyl group;
an amide compound represented by formula (2) wherein R⁴ is a C1-C2 alkyl group;
an amide compound represented by formula (2) wherein R⁵ is a C1-C4 alkyl group;
an amide compound represented by formula (2) wherein R⁵ is a C3-C4 alkynyl group;
an amide compound represented by formula (2) wherein R⁵ is a C1-C2 alkyl group;
an amide compound represented by formula (2) wherein R⁵ is a 2-propynyl group;
an amide compound represented by formula (2) wherein X is an oxygen atom;
an amide compound represented by formula (2) wherein R¹ is a halogen atom, R² is a hydrogen atom;
an amide compound represented by formula (2) wherein R¹ is a halogen atom, R² is a halogen atom;
an amide compound represented by formula (2) wherein R¹ is a halogen atom, R² is a C1-C4 haloalkyl group;
an amide compound represented by formula (2) wherein R¹ is a halogen atom, R³ is a hydrogen atom;
an amide compound represented by formula (2) wherein R¹ is a halogen atom, R⁴ is a C1-C4 alkyl group;
an amide compound represented by formula (2) wherein R¹ is a halogen atom, R⁴ is a C1-C2 alkyl group;
an amide compound represented by formula (2) wherein R¹ is a halogen atom, R⁵ is a C1-C4 alkyl group;
an amide compound represented by formula (2) wherein R¹ is a halogen atom, R⁵ is a C3-C4 alkynyl group;
an amide compound represented by formula (2) wherein R¹ is a halogen atom, R⁵ is a C1-C2 alkyl group;
an amide compound represented by formula (2) wherein R¹ is a halogen atom, R⁵ is a 2-propynyl group;
an amide compound represented by formula (2) wherein R¹ is a C1-C4 alkyl group, R² is a hydrogen atom;
an amide compound represented by formula (2) wherein R¹ is a C1-C4 alkyl group, R² is a halogen atom;
an amide compound represented by formula (2) wherein R¹ is a C1-C4 alkyl group, R² is a C1-C4 haloalkyl group;
an amide compound represented by formula (2) wherein R¹ is a C1-C4 alkyl group, R³ is a hydrogen atom;
an amide compound represented by formula (2) wherein R¹ is a C1-C4 alkyl group, R⁴ is a C1-C4 alkyl group;
an amide compound represented by formula (2) wherein R¹ is a C2-C4 alkyl group, R⁴ is a C1-C2 alkyl group;
an amide compound represented by formula (2) wherein R¹ is a C1-C4 alkyl group, R⁵ is a C1-C4 alkyl group;
an amide compound represented by formula (2) wherein R¹ is a C1-C4 alkyl group, R⁵ is a C3-C4 alkynyl group;
an amide compound represented by formula (2) wherein R¹ is a C1-C4 alkyl group, R⁵ is a C1-C2 alkyl group;
an amide compound represented by formula (2) wherein R¹ is a C1-C4 alkyl group, R⁵ is a 2-propynyl group;
an amide compound represented by formula (2) wherein R¹ is a C1-C4 haloalkyl group, R² is a hydrogen atom;
an amide compound represented by formula (2) wherein R¹ is a C1-C4 haloalkyl group, R² is a halogen atom;
an amide compound represented by formula (2) wherein R¹ is a C1-C4 haloalkyl group, R² is a C1-C4 haloalkyl group;
an amide compound represented by formula (2) wherein R¹ is a C1-C4 haloalkyl group, R³ is a hydrogen atom;
an amide compound represented by formula (2) wherein R¹ is a C1-C4 haloalkyl group, R⁴ is a C1-C4 alkyl group;
an amide compound represented by formula (2) wherein R¹ is a C1-C4 haloalkyl group, R⁴ is a C1-C2 alkyl group;
an amide compound represented by formula (2) wherein R¹ is a C1-C4 haloalkyl group, R⁵ is a C1-C4 alkyl group;
an amide compound represented by formula (2) wherein R¹ is a C1-C4 haloalkyl group, R⁵ is a C3-C4 alkynyl group;
an amide compound represented by formula (2) wherein R¹ is a C1-C4 haloalkyl group, R⁵ is a C1-C2 alkyl group;
an amide compound represented by formula (2) wherein R¹ is a C1-C4 haloalkyl group, R⁵ is a 2-propynyl group;
an amide compound represented by formula (2) wherein R¹ is a chlorine atom, R² is a hydrogen atom;
an amide compound represented by formula (2) wherein R¹ is a chlorine atom, R² is a halogen atom;
an amide compound represented by formula (2) wherein R¹ is a chlorine atom, R² is a C1-C4 haloalkyl group;
an amide compound represented by formula (2) wherein R¹ is a chlorine atom, R³ is a hydrogen atom;
an amide compound represented by formula (2) wherein R¹ is a chlorine atom, R⁴ is a C1-C4 alkyl group;
an amide compound represented by formula (2) wherein R¹ is a chlorine atom, R⁴ is a C1-C2 alkyl group;
an amide compound represented by formula (2) wherein R¹ is a chlorine atom, R⁵ is a C1-C4 alkyl group;
an amide compound represented by formula (2) wherein R¹ is a chlorine atom, R⁵ is a C3-C4 alkynyl group;
an amide compound represented by formula (2) wherein R¹ is a chlorine atom, R⁵ is a C1-C2 alkyl group;
an amide compound represented by formula (2) wherein R¹ is a chlorine atom, R⁵ is a 2-propynyl group;
an amide compound represented by formula (2) wherein R¹ is a methyl group, R² is a hydrogen atom;
an amide compound represented by formula (2) wherein R¹ is a methyl group, R² is a halogen atom;
an amide compound represented by formula (2) wherein R¹ is a methyl group, R² is a C1-C4 haloalkyl group;
an amide compound represented by formula (2) wherein R¹ is a methyl group, R³ is a hydrogen atom;
an amide compound represented by formula (2) wherein R¹ is a methyl group, R⁴ is a C1-C4 alkyl group;
an amide compound represented by formula (2) wherein R¹ is a methyl group, R⁴ is a C1-C2 alkyl group;
an amide compound represented by formula (2) wherein R¹ is a methyl group, R⁵ is a C1-C4 alkyl group;
an amide compound represented by formula (2) wherein R¹ is a methyl group, R⁵ is a C3-C4 alkynyl group;
an amide compound represented by formula (2) wherein R¹ is a methyl group, R⁵ is a C1-C2 alkyl group;
an amide compound represented by formula (2) wherein R¹ is a methyl group, R⁵ is a 2-propynyl group;
an amide compound represented by formula (2) wherein R¹ is a trifluoromethyl group, R² is a hydrogen atom;
an amide compound represented by formula (2) wherein R¹ is a trifluoromethyl group, R² is a halogen atom;
an amide compound represented by formula (2) wherein R¹ is a trifluoromethyl group, R² is a C1-C4 haloalkyl group;
an amide compound represented by formula (2) wherein R¹ is a trifluoromethyl group, R³ is a hydrogen atom;
an amide compound represented by formula (2) wherein R¹ is a trifluoromethyl group, R⁴ is a C1-C4 alkyl group;
an amide compound represented by formula (2) wherein R¹ is a trifluoromethyl group, R⁴ is a C1-C2 alkyl group;
an amide compound represented by formula (2) wherein R¹ is a trifluoromethyl group, R⁵ is a C1-C4 alkyl group;
an amide compound represented by formula (2) wherein R¹ is a trifluoromethyl group, R⁵ is a C3-C4 alkynyl group;
an amide compound represented by formula (2) wherein R¹ is a trifluoromethyl group, R⁵ is a C1-C2 alkyl group;
an amide compound represented by formula (2) wherein R¹ is a trifluoromethyl group, R⁵ is a 2-propynyl group;
an amide compound represented by formula (2) wherein R¹ is a di(C1-C4alkyl)amino group, R² is a hydrogen atom;
an amide compound represented by formula (2) wherein R¹ is a di(C1-C4alkyl)amino group, R² is a halogen atom;
an amide compound represented by formula (2) wherein R¹ is a di(C1-C4alkyl)amino group, R² is a C1-C4 haloalkyl group;
an amide compound represented by formula (2) wherein R¹ is a di(C1-C4alkyl)amino group, R³ is a hydrogen atom;
an amide compound represented by formula (2) wherein R¹ is a di(C1-C4alkyl)amino group, R⁴ is a C1-C4 alkyl group;
an amide compound represented by formula (2) wherein R¹ is a di(C1-C4alkyl)amino group, R⁴ is a C1-C2 alkyl group;
an amide compound represented by formula (2) wherein R¹ is a di(C1-C4alkyl)amino group, R⁵ is a C1-C4 alkyl group;
an amide compound represented by formula (2) wherein R¹ is a di(C1-C4alkyl)amino group, R⁵ is a C3-C4 alkynyl group;
an amide compound represented by formula (2) wherein R¹ is a di(C1-C4alkyl)amino group, R⁵ is a C1-C2 alkyl group;
an amide compound represented by formula (2) wherein R¹ is a di(C1-C4alkyl)amino group, R⁵ is a 2-propynyl group;
an amide compound representedby formula (2) wherein R¹ is a halogen atom, R² is a hydrogen atom, R³ is a hydrogen atom;
an amide compound represented by formula (2) wherein R¹ is a halogen atom, R² is a hydrogen atom, R⁴ is a C1-C4 alkyl group;
an amide compound represented by formula (2) wherein R¹ is a halogen atom, R² is a hydrogen atom, R⁴ is a C1-C2 alkyl group;
an amide compound represented by formula (2) wherein R¹ is a halogen atom, R² is a hydrogen atom, R⁵ is a C1-C4 alkyl group;
an amide compound represented by formula (2) wherein R¹ is a halogen atom, R² is a hydrogen atom, R⁵ is a C3-C4 alkynyl group;
an amide compound represented by formula (2) wherein R¹ is a halogen atom, R² is a hydrogen atom, R⁵ is a C1-C2 alkyl group;
an amide compound represented by formula (2) wherein R¹ is a halogen atom, R² is a hydrogen atom, R⁵ is a 2-propynyl group;
an amide compound represented by formula (2) wherein R¹ is a C1-C4 alkyl group, R² is a hydrogen atom, R³ is a hydrogen atom;
an amide compound represented by formula (2) wherein R¹ is a C1-C4 alkyl group, R² is a hydrogen atom, R⁴ is a C1-C4 alkyl group; an amide compound represented by formula (2) wherein R¹ is a C1-C4 alkyl group, R² is a hydrogen atom, R⁴ is a C1-C2 alkyl group; an amide compound represented by formula (2) wherein R¹ is a C1-C4 alkyl group, R² is a hydrogen atom, R⁵ is a C1-C4 alkyl group; an amide compound represented by formula (2) wherein R¹ is a C1-C4 alkyl group, R² is a hydrogen atom, R⁵ is a C3-C4 alkynyl group; an amide compound represented by formula (2) wherein R¹ is a C1-C4 alkyl group, R² is a hydrogen atom, R⁵ is a C1-C2 alkyl group; an amide compound represented by formula (2) wherein R¹ is a C1-C4 alkyl group, R² is a hydrogen atom, R⁵ is a 2-propynyl group; an amide compound represented by formula (2) wherein R¹ is a C1-C4 haloalkyl group, R² is a hydrogen atom, R³ is a hydrogen atom; an amide compound represented by formula (2) wherein R¹ is a C1-C4 haloalkyl group, R² is a hydrogen atom, R⁴ is a C1-C4 alkyl group; an amide compound represented by formula (2) wherein R¹ is a C1-C4 haloalkyl group, R² is a hydrogen atom, R⁴ is a C1-C2 alkyl group; an amide compound represented by formula (2) wherein R¹ is a C1-C4 haloalkyl group, R² is a hydrogen atom, R⁵ is a C1-C4 alkyl group; an amide compound represented by formula (2) wherein R¹ is a C1-C4 haloalkyl group, R² is a hydrogen atom, R⁵ is a C3-C4 alkynyl group;
an amide compound represented by formula (2) wherein R¹ is a C1-C4 haloalkyl group, R² is a hydrogen atom, R⁵ is a C1-C2 alkyl group; an amide compound represented by formula (2) wherein R¹ is a C1-C4 haloalkyl group, R² is a hydrogen atom, R⁵ is a 2-propynyl group; an amide compound represented by formula (2) wherein R¹ is a chlorine atom, R² is a hydrogen atom, R³ is a hydrogen atom; an amide compound represented by formula (2) wherein R¹ is a chlorine atom, R² is a hydrogen atom, R⁴ is a C1-C4 alkyl group; an amide compound represented by formula (2) wherein R¹ is a chlorine atom, R² is a hydrogen atom, R⁴ is a C1-C2 alkyl group; an amide compound represented by formula (2) wherein R¹ is a chlorine atom, R² is a hydrogen atom, R⁵ is a C1-C4 alkyl group; an amide compound represented by formula (2) wherein R¹ is a chlorine atom, R² is a hydrogen atom, R⁵ is a C3-C4 alkynyl group; an amide compound represented by formula (2) wherein R¹ is a chlorine atom, R² is a hydrogen atom, R⁵ is a C1-C2 alkyl group; an amide compound represented by formula (2) wherein R¹ is a chlorine atom, R² is a hydrogen atom, R⁵ is a 2-propynyl group; an amide compound represented by formula (2) wherein R¹ is a methyl group, R² is a hydrogen atom, R³ is a hydrogen atom;
an amide compound represented by formula (2) wherein R¹ is a methyl group, R² is a hydrogen atom, R⁴ is a C1-C4 alkyl group;
an amide compound represented by formula (2) wherein R¹ is a methyl group, R² is a hydrogen atom, R⁴ is a C1-C2 alkyl group;
an amide compound represented by formula (2) wherein R¹ is a methyl group, R² is a hydrogen atom, R⁵ is a C1-C4 alkyl group;
an amide compound represented by formula (2) wherein R¹ is a methyl group, R² is a hydrogen atom, R⁵ is a C3-C4 alkynyl group;
an amide compound represented by formula (2) wherein R¹ is a methyl group, R² is a hydrogen atom, R⁵ is a C1-C2 alkyl group;
an amide compound represented by formula (2) wherein R¹ is a methyl group, R² is a hydrogen atom, R⁵ is a 2-propynyl group; an amide compound represented by formula (2) wherein R¹ is a trifluoromethyl group, R² is a hydrogen atom, R³ is a hydrogen atom;
an amide compound represented by formula (2) wherein R¹ is a trifluoromethyl group, R² is a hydrogen atom, R⁴ is a C1-C4 alkyl group;
an amide compound represented by formula (2) wherein R¹ is a trifluoromethyl group, R² is a hydrogen atom, R⁴ is a C1-C2 alkyl group;
an amide compound represented by formula (2) wherein R¹ is a trifluoromethyl group, R² is a hydrogen atom, R⁵ is a C1-C4 alkyl group;
an amide compound represented by formula (2) wherein R¹ is a trifluoromethyl group, R² is a hydrogen atom, R⁵ is a C3-C4 alkynyl group;
an amide compound represented by formula (2) wherein R¹ is a trifluoromethyl group, R² is a hydrogen atom, R⁵ is a C1-C2 alkyl group;
an amide compound represented by formula (2) wherein R¹ is a trifluoromethyl group, R² is a hydrogen atom, R⁵ is a 2-propynyl group;
an amide compound represented by formula (2) wherein R¹ is a halogen atom, R² is a hydrogen atom, R³ is a hydrogen atom, R⁴ is a C1-C4 alkyl group;
an amide compound represented by formula (2) wherein R¹ is a halogen atom, R² is a hydrogen atom, R³ is a hydrogen atom, R⁴ is a C1-C2 alkyl group;
an amide compound represented by formula (2) wherein R¹ is a halogen atom, R² is a hydrogen atom, R³ is a hydrogen atom, R⁵ is a C1-C4 alkyl group;
an amide compound represented by formula (2) wherein R¹ is a halogen atom, R² is a hydrogen atom, R³ is a hydrogen atom, R⁵ is a C3-C4 alkynyl group;
an amide compound represented by formula (2) wherein R¹ is a halogen atom, R² is a hydrogen atom, R³ is a hydrogen atom, R⁵ is a C1-C2 alkyl group;
an amide compound represented by formula (2) wherein R¹ is a halogen atom, R² is a hydrogen atom, R³ is a hydrogen atom, R⁵ is a 2-propynyl group;
an amide compound represented by formula (2) wherein R¹ is a C1-C4 alkyl group, R² is a hydrogen atom, R³ is a hydrogen atom, R⁴ is a C1-C4 alkyl group;
an amide compound represented by formula (2) wherein R¹ is a C1-C4 alkyl group, R² is a hydrogen atom, R³ is a hydrogen atom, R⁴ is a C1-C2 alkyl group;
an amide compound represented by formula (2) wherein R¹ is a C1-C4 alkyl group, R² is a hydrogen atom, R³ is a hydrogen atom, R⁵ is a C1-C4 alkyl group;
an amide compound represented by formula (2) wherein R¹ is a C1-C4 alkyl group, R² is a hydrogen atom, R³ is a hydrogen atom, R⁵ is a C3-C4 alkynyl group;
an amide compound represented by formula (2) wherein R¹ is a C1-C4 alkyl group, R² is a hydrogen atom, R³ is a hydrogen atom, R⁵ is a C1-C2 alkyl group;
an amide compound represented by formula (2) wherein R¹ is a C1-C4 alkyl group, R² is a hydrogen atom, R³ is a hydrogen atom, R⁵ is a 2-propynyl group;
an amide compound represented by formula (2) wherein R¹ is a C1-C4 haloalkyl group, R² is a hydrogen atom, R³ is a hydrogen atom, R⁴ is a C1-C4 alkyl group;
an amide compound represented by formula (2) wherein R¹ is a C1-C4 haloalkyl group, R² is a hydrogen atom, R³ is a hydrogen atom, R⁴ is a C1-C2 alkyl group;
an amide compound represented by formula (2) wherein R¹ is a C1-C4 haloalkyl group, R² is a hydrogen atom, R³ is a hydrogen atom, R⁵ is a C1-C4 alkyl group;
an amide compound represented by formula (2) wherein R¹ is a C1-C4 haloalkyl group, R² is a hydrogen atom, R³ is a hydrogen atom, R⁵ is a C3-C4 alkynyl group;
an amide compound represented by formula (2) wherein R¹ is a C1-C4 haloalkyl group, R² is a hydrogen atom, R³ is a hydrogen atom, R⁵ is a C1-C2 alkyl group;
an amide compound represented by formula (2) wherein R¹ is a C1-C4 haloalkyl group, R² is a hydrogen atom, R³ is a hydrogen atom, R⁵ is a 2-propynyl group;
an amide compound represented by formula (2) wherein R¹ is a chlorine atom, R² is a hydrogen atom, R³ is a hydrogen atom, R⁴ is a C1-C4 alkyl group;
an amide compound represented by formula (2) wherein R¹ is a chlorine atom, R² is a hydrogen atom, R³ is a hydrogen atom, R⁴ is a C1-C2 alkyl group;
an amide compound represented by formula (2) wherein R¹ is a chlorine atom, R² is a hydrogen atom, R³ is a hydrogen atom, R⁵ is a C1-C4 alkyl group;
an amide compound represented by formula (2) wherein R¹ is a chlorine atom, R² is a hydrogen atom, R³ is a hydrogen atom, R⁵ is a C3-C4 alkynyl group;
an amide compound represented by formula (2) wherein R¹ is a chlorine atom, R² is a hydrogen atom, R³ is a hydrogen atom, R⁵ is a C1-C2 alkyl group;
an amide compound represented by formula (2) wherein R¹ is a chlorine atom, R² is a hydrogen atom, R³ is a hydrogen atom, R⁵ is a 2-propynyl group;
an amide compound represented by formula (2) wherein R¹ is a methyl group, R² is a hydrogen atom, R³ is a hydrogen atom, R⁴ is a C1-C4 alkyl group;
an amide compound represented by formula (2) wherein R¹ is a methyl group, R² is a hydrogen atom, R³ is a hydrogen atom, R⁴ is a C1-C2 alkyl group;
an amide compound represented by formula (2) wherein R¹ is a methyl group, R² is a hydrogen atom, R³ is a hydrogen atom, R⁵ is a C1-C4 alkyl group;
an amide compound represented by formula (2) wherein R¹ is a methyl group, R² is a hydrogen atom, R³ is a hydrogen atom, R⁵ is a C3-C4 alkynyl group;
an amide compound represented by formula (2) wherein R¹ is a methyl group, R² is a hydrogen atom, R³ is a hydrogen atom, R⁵ is a C1-C2 alkyl group;
an amide compound represented by formula (2) wherein R¹ is a methyl group, R² is a hydrogen atom, R³ is a hydrogen atom, R⁵ is a 2-propynyl group;
an amide compound represented by formula (2) wherein R¹ is a trifluoromethyl group, R² is a hydrogen atom, R³ is a hydrogen atom, R⁴ is a C1-C4 alkyl group;
an amide compound represented by formula (2) wherein R¹ is a trifluoromethyl group, R² is a hydrogen atom, R³ is a hydrogen atom, R⁴ is a C1-C2 alkyl group;
an amide compound represented by formula (2) wherein R¹ is a trifluoromethyl group, R² is a hydrogen atom, R³ is a hydrogen atom, R⁵ is a C1-C4 alkyl group;
an amide compound represented by formula (2) wherein R¹ is a trifluoromethyl group, R² is a hydrogen atom, R³ is a hydrogen atom, R⁵ is a C3-C4 alkynyl group;
an amide compound represented by formula (2) wherein R¹ is a trifluoromethyl group, R² is a hydrogen atom, R³ is a hydrogen atom, R⁵ is a C1-C2 alkyl group;
an amide compound represented by formula (2) wherein R¹ is a trifluoromethyl group, R² is a hydrogen atom, R³ is a hydrogen atom, R⁵ is a 2-propynyl group;
an amide compound represented by formula (2) wherein R¹ is a halogen atom, R² is a hydrogen atom, R³ is a hydrogen atom, R⁴ is a C1-C4 alkyl group, R⁵ is a C1-C4 alkyl group;
an amide compound represented by formula (2) wherein R¹ is a halogen atom, R² is a hydrogen atom, R³ is a hydrogen atom, R⁴ is a C1-C4 alkyl group, R⁵ is a C1-C2 alkyl group;
an amide compound represented by formula (2) wherein R¹ is a halogen atom, R² is a hydrogen atom, R³ is a hydrogen atom, R⁴ is a C1-C4 alkyl group, R⁵ is a C1-C4 alkyl group;
an amide compound represented by formula (2) wherein R¹ is a halogen atom, R² is a hydrogen atom, R³ is a hydrogen atom, R⁴ is a C1-C4 alkyl group, R⁵ is a C3-C4 alkynyl group;
an amide compound represented by formula (2) wherein R¹ is a halogen atom, R² is a hydrogen atom, R³ is a hydrogen atom, R⁴ is a C1-C4 alkyl group, R⁵ is a C1-C2 alkyl group;
an amide compound represented by formula (2) wherein R¹ is a halogen atom, R² is a hydrogen atom, R³ is a hydrogen atom, R⁴ is a C1-C4 alkyl group, R⁵ is a 2-propynyl group;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a C1 -C4 alkyl group, R² is a hydrogen atom, R³ is a hydrogen atom, R⁴ is a C1-C4 alkyl group, R³ is a C1-C4 alkyl group;
an amide compound represented by formula (2) wherein R¹ is a C1-C4 alkyl group, R² is a hydrogen atom, R³ is a hydrogen atom, R⁴ is a C1-C4 alkyl group, R⁵ is a C3-C4 alkynyl group;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a C1-C4 alkyl group, R² is a hydrogen atom, R³ is a hydrogen atom, R⁴ is a C1-C4 alkyl group, R⁵ is a 2-propynyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C3-C4 alkynyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a C1-C4 haloalkyl group, R² is a hydrogen atom, R³ is a hydrogen atom, R⁴ is a C1-C4 alkyl group, R⁵ is a 2-propynyl group;
an amide compound represented by formula (2) wherein R¹ is a chlorine atom, R² is a hydrogen atom, R³ is a hydrogen atom, R⁴ is a C1-C4 alkyl group, R⁵ is a C1-C4 alkyl group;
an amide compound represented by formula (2) wherein R¹ is a chlorine atom, R² is a hydrogen atom, R³ is a hydrogen atom, R⁴ is a C1-C4 alkyl group, R⁵ is a C1-C2 alkyl group;
an amide compound represented by formula (2) wherein R¹ is a chlorine atom, R² is a hydrogen atom, R³ is a hydrogen atom, R⁴ is a C1-C4 alkyl group, R³ is a C1-C4 alkyl group;
an amide compound represented by formula (2) wherein R¹ is a chlorine atom, R² is a hydrogen atom, R³ is a hydrogen atom, R⁴ is a C1-C4 alkyl group, R⁵ is a C3-C4 alkynyl group;
an amide compound represented by formula (2) wherein R¹ is a chlorine atom, R² is a hydrogen atom, R⁵ is a hydrogen atom, R⁴ is a C1-C4 alkyl group, R⁵ is a C1-C2 alkyl group;
an amide compound represented by formula (2) wherein R¹ is a chlorine atom, R² is a hydrogen atom, R³ is a hydrogen atom, R⁴ is a C1-C4 alkyl group, R⁵ is a 2-propynyl group;
an amide compound represented by formula (2) wherein R¹ is a methyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a methyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a methyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a methyl group, R² is a hydrogen atom, R³ is a hydrogen atom, R⁴ is a C1-C4 alkyl group, R⁵ is a C3-C4 alkynyl group;
an amide compound represented by formula (2) wherein R¹ is a methyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a methyl group, R² is a hydrogen atom, R³ is a hydrogen atom, R⁹ is a C1-C4 alkyl group, R⁵ is a 2-propynyl group;
an amide compound represented by formula (2) wherein R¹ is a trifluoromethyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a trifluoromethyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a trifluoromethyl group, R² is a hydrogen atom, R³ is a hydrogen atom, R⁴ is a C1-C4 alkyl group, R⁵ is a C1-C4 alkyl group;
an amide compound represented by formula (2) wherein R¹ is a trifluoromethyl group, R² is a hydrogen atom, R³ is a hydrogen atom, R⁴ is a C1-C4 alkyl group, R⁵ is a C3-C4 alkynyl group; an amide compound represented by formula (2) wherein R¹ is a trifluoromethyl group, R² is a hydrogen atom, R³ is a hydrogen atom, R⁴ is a C1-C4 alkyl group, R⁵ is a C1-C2 alkyl group;
an amide compound represented by formula (2) wherein R¹ is a trifluoromethyl group, R² is a hydrogen atom, R³ is a hydrogen atom, R⁴ is a C1-C4 alkyl group, R⁵ is a 2-propynyl group;
an amide compound represented by formula (2) wherein R² is a hydrogen atom, R³ is a hydrogen atom;
an amide compound represented by formula (2) wherein R² is a hydrogen atom, R⁴ is a C1-C4 alkyl group;
an amide compound represented by formula (2) wherein R² is a hydrogen atom, R⁹ is a C1-C2 alkyl group;
an amide compound represented by formula (2) wherein R² is a hydrogen atom, R⁵ is a C1-C4 alkyl group;
an amide compound represented by formula (2) wherein R² is a hydrogen atom, R⁵ is a C3-C4 alkynyl group;
an amide compound represented by formula (2) wherein R² is a hydrogen atom, R⁵ is a C1-C2 alkyl group;
an amide compound represented by formula (2) wherein R² is a hydrogen atom, R⁵ is a 2-propynyl group;
an amide compound represented by formula (2) wherein R³ is a hydrogen atom, R⁴ is a C1-C4 alkyl group;
an amide compound represented by formula (2) wherein R³ is a hydrogen atom, R⁹ is a C1-C2 alkyl group;
an amide compound represented by formula (2) wherein R³ is a hydrogen atom, R⁵ is a C1-C4 alkyl group;
an amide compound represented by formula (2) wherein R³ is a hydrogen atom, R⁵ is a C3-C4 alkynyl group;
an amide compound represented by formula (2) wherein R³ is a hydrogen atom, R⁵ is a C1-C2 alkyl group;
an amide compound represented by formula (2) wherein R³ is a hydrogen atom, R⁵ is a 2-propynyl group;
an amide compound represented by formula (2) wherein R⁴ is a C1-C4 alkyl group, R⁵ is a C1-C4 alkyl group;
an amide compound represented by formula (2) wherein R⁴ is a C1-C4 alkyl group, R⁵ is a C3-C4 alkynyl group;
an amide compound represented by formula (2) wherein R⁴ is a C1-C4 alkyl group, R⁵ is a C1-C2 alkyl group;
an amide compound represented by formula (2) wherein R⁴ is a C1-C4 alkyl group, R⁵ is a 2-propynyl group;
an amide compound represented by formula (2) wherein R⁴ is a C1-C2 alkyl group, R⁵ is a C1-C4 alkyl group;
an amide compound represented by formula (2) wherein R⁴ is a C1-C2 alkyl group, R⁵ is a C3-C4 alkynyl group;
an amide compound represented by formula (2) wherein R⁴ is a C1-C2 alkyl group, R⁵ is a C1-C2 alkyl group;
an amide compound represented by formula (2) wherein R⁴ is a C1-C2 alkyl group, R⁵ is a 2-propynyl group;
an amide compound represented by formula (2) wherein R² is a hydrogen atom, R⁴ is a C1-C2 alkyl group, R⁴ is a C1-C4 alkyl group;
an amide compound represented by formula (2) wherein R² is a hydrogen atom, R⁴ is a C1-C2 alkyl group, R⁴ is a C3-C4 alkynyl group;
an amide compound represented by formula (2) wherein R² is a hydrogen atom, R⁴ is a C1-C2 alkyl group, R⁴ is a C1-C2 alkyl group;
an amide compound represented by formula (2) wherein R² is a hydrogen atom, R⁴ is a C1-C2 alkyl group, R⁴ is a 2-propynyl group; an amide compound represented by formula (2) wherein R² is a hydrogen atom, R⁴ is a C1-C4 alkyl group, R⁴ is a C1-C4 alkyl group;
an amide compound represented by formula (2) wherein R² is a hydrogen atom, R⁴ is a C1-C4 alkyl group, R⁴ is a C3-C4 alkynyl group;
an amide compound represented by formula (2) wherein R² is a hydrogen atom, R⁴ is a C1-C4 alkyl group, R⁴ is a C1-C2 alkyl group;
an amide compound represented by formula (2) wherein R² is a hydrogen atom, R⁴ is a C1-C4 alkyl group, R⁴ is a 2-propynyl group; an amide compound represented by formula (2) wherein R¹ is a halogen atom, X is an oxygen atom;
an amide compound represented by formula (2) wherein R¹ is a C1-C4 alkyl group, X is an oxygen atom;
an amide compound represented by formula (2) wherein R¹ is a C1-C4 haloalkyl group, X is an oxygen atom;
an amide compound represented by formula (2) wherein R¹ is a di(C1-C4 alkyl)amino group, X is an oxygen atom;
an amide compound represented by formula (2) wherein R¹ is a chlorine atom, X is an oxygen atom;
an amide compound represented by formula (2) wherein R¹ is a methyl group, X is an oxygen atom;
an amide compound represented by formula (2) wherein R¹ is a trifluoromethyl group, X is an oxygen atom;
an amide compound represented by formula (2) wherein R² is a hydrogen atom, X is an oxygen atom;
an amide compound represented by formula (2) wherein R² is a halogen atom, X is an oxygen atom;
an amide compound represented by formula (2) wherein R² is a C1-C4 haloalkyl group, X is an oxygen atom;
an amide compound represented by formula (2) wherein R³ is a hydrogen atom, X is an oxygen atom;
an amide compound represented by formula (2) wherein R⁴ is a C1-C4 alkyl group, X is an oxygen atom;
an amide compound represented by formula (2) wherein R⁴ is a C1-C2 alkyl group, X is an oxygen atom;
an amide compound represented by formula (2) wherein R⁵ is a C1-C4 alkyl group, X is an oxygen atom;
an amide compound represented by formula (2) wherein R⁵ is a C3-C4 alkynyl group, X is an oxygen atom;
an amide compound represented by formula (2) wherein R⁵ is a C1-C2 alkyl group, X is an oxygen atom;
an amide compound represented by formula (2) wherein R⁵ is a 2-propynyl group, X is an oxygen atom;
an amide compound representedby formula (2) wherein R¹ is a halogen atom, R² is a hydrogen atom, X is an oxygen atom;
an amide compound representedby formula (2) wherein R¹ is a halogen atom, R² is a halogen group, X is an oxygen atom;
an amide compound represented by formula (2) wherein R¹ is a halogen atom, R² is a C1-C4 haloalkyl group, X is an oxygen atom;
an amide compound represented by formula (2) wherein R¹ is a halogen atom, R³ is a hydrogen atom, X is an oxygen atom;
an amide compound represented by formula (2) wherein R¹ is a halogen atom, R⁴ is a C1-C4 alkyl group, X is an oxygen atom;
an amide compound represented by formula (2) wherein R¹ is a halogen atom, R⁴ is a C1-C2 alkyl group, X is an oxygen atom;
an amide compound represented by formula (2) wherein R¹ is a halogen atom, R⁵ is a C1-C4 alkyl group, X is an oxygen atom;
an amide compound represented by formula (2) wherein R¹ is a halogen atom, R⁵ is a C3-C4 alkynyl group, X is an oxygen atom;
an amide compound represented by formula (2) wherein R¹ is a halogen atom, R⁵ is a C1-C2 alkyl group, X is an oxygen atom;
an amide compound represented by formula (2) wherein R¹ is a halogen atom, R⁵ is a 2-propynyl group, X is an oxygen atom;
an amide compound represented by formula (2) wherein R¹ is a C1-C4 alkyl group, R² is a hydrogen atom, X is an oxygen atom;
an amide compound represented by formula (2) wherein R¹ is a C1-C4 alkyl group, R² is a halogen group, X is an oxygen atom;
an amide compound represented by formula (2) wherein R¹ is a C1-C4 alkyl group, R² is a C1-C4 haloalkyl group, X is an oxygen atom; an amide compound represented by formula (2) wherein R¹ is a C1-C4 alkyl group, R³ is a hydrogen atom, X is an oxygen atom;
an amide compound represented by formula (2) wherein R¹ is a C1-C4 alkyl group, R⁴ is a C1-C4 alkyl group, X is an oxygen atom; an amide compound represented by formula (2) wherein R¹ is a C1-C4 alkyl group, R⁴ is a C1-C2 alkyl group, X is an oxygen atom; an amide compound represented by formula (2) wherein R¹ is a C1-C4 alkyl group, R⁵ is a C1-C4 alkyl group, X is an oxygen atom; an amide compound represented by formula (2) wherein R¹ is a C1-C4 alkyl group, R⁵ is a C3-C4 alkynyl group, X is an oxygen atom; an amide compound represented by formula (2) wherein R¹ is a C1-C4 alkyl group, R⁵ is a C1-C2 alkyl group, X is an oxygen atom; an amide compound represented by formula (2) wherein R¹ is a C1-C4 alkyl group, R⁵ is a 2-propynyl group, X is an oxygen atom;
an amide compound represented by formula (2) wherein R¹ is a C1-C4 haloalkyl group, R² is a hydrogen atom, X is an oxygen atom; an amide compound represented by formula (2) wherein R¹ is a C1-C4 haloalkyl group, R² is a halogen group, X is an oxygen atom; an amide compound represented by formula (2) wherein R¹ is a C1-C4 haloalkyl group, R² is a C1-C4 haloalkyl group, X is an oxygen atom;
an amide compound represented by formula (2) wherein R¹ is a C1-C4 haloalkyl group, R³ is a hydrogen atom, X is an oxygen atom; an amide compound represented by formula (2) wherein R¹ is a C1-C4 haloalkyl group, R⁴ is a C1-C4 alkyl group, X is an oxygen atom; an amide compound represented by formula (2) wherein R¹ is a C1-C4 haloalkyl group, R⁴ is a C1-C2 alkyl group, X is an oxygen atom; an amide compound represented by formula (2) wherein R¹ is a C1-C4 haloalkyl group, R⁵ is a C1-C4 alkyl group, X as an oxygen atom; an amide compound represented by formula (2) wherein R¹ is a C1-C4 haloalkyl group, R⁵ is a C3-C4 alkynyl group, X is an oxygen atom; an amide compound represented by formula (2) wherein R¹ is a C1-C4 haloalkyl group, R⁵ is a C1-C2 alkyl group, X is an oxygen atom; an amide compound represented by formula (2) wherein R¹ is a C1-C4 haloalkyl group, R⁵ is a 2-propynyl group, X is an oxygen atom; an amide compound represented by formula (2) wherein R¹ is a chlorine atom, R² is a hydrogen atom, X is an oxygen atom;
an amide compound represented by formula (2) wherein R¹ is a chlorine atom, R² is a halogen group, X is an oxygen atom;
an amide compound represented by formula (2) wherein R¹ is a chlorine atom, R² is a C1-C4 haloalkyl group, X is an oxygen atom; an amide compound represented by formula (2) wherein R¹ is a chlorine atom, R³ is a hydrogen atom, X is an oxygen atom;
an amide compound represented by formula (2) wherein R¹ is a chlorine atom, R⁴ is a C1-C4 alkyl group, X is an oxygen atom; an amide compound represented by formula (2) wherein R¹ is a chlorine atom, R⁴ is a C1-C2 alkyl group, X is an oxygen atom; an amide compound represented by formula (2) wherein R¹ is a chlorine atom, R⁵ is a C1-C4 alkyl group, X is an oxygen atom; an amide compound represented by formula (2) wherein R¹ is a chlorine atom, R⁵ is a C3-C4 alkynyl group, X is an oxygen atom; an amide compound represented by formula (2) wherein R¹ is a chlorine atom, R⁵ is a C1-C2 alkyl group, X is an oxygen atom; an amide compound represented by formula (2) wherein R¹ is a chlorine atom, R⁵ is a 2-propynyl group, X is an oxygen atom; an amide compound represented by formula (2) wherein R¹ is a methyl group, R² is a hydrogen atom, X is an oxygen atom;
an amide compound represented by formula (2) wherein R¹ is a methyl group, R² is a halogen group, X is an oxygen atom;
an amide compound represented by formula (2) wherein R¹ is a methyl group, R² is a C1-C4 haloalkyl group, X is an oxygen atom; an amide compound represented by formula (2) wherein R¹ is a methyl group, R³ is a hydrogen atom, X is an oxygen atom;
an amide compound represented by formula (2) wherein R¹ is a methyl group, R⁴ is a C1-C4 alkyl group, X is an oxygen atom; an amide compound represented by formula (2) wherein R¹ is a methyl group, R⁴ is a C1-C2 alkyl group, X is an oxygen atom; an amide compound represented by formula (2) wherein R¹ is a methyl group, R⁵ is a C1-C4 alkyl group, X is an oxygen atom; an amide compound represented by formula (2) wherein R¹ is a methyl group, R⁵ is a C3-C4 alkynyl group, X is an oxygen atom; an amide compound represented by formula (2) wherein R¹ is a methyl group, R⁵ is a C1-C2 alkyl group, X is an oxygen atom; an amide compound represented by formula (2) wherein R¹ is a methyl group, R⁵ is a 2-propynyl group, X is an oxygen atom;
an amide compound represented by formula (2) wherein R¹ is a trifluoromethyl group, R² is a hydrogen atom, X is an oxygen atom; an amide compound represented by formula (2) wherein R¹ is a trifluoromethyl group, R² is a halogen group, X is an oxygen atom; an amide compound represented by formula (2) wherein R¹ is a trifluoromethyl group, R² is a C1-C4 haloalkyl group, X is an oxygen atom; an amide compound represented by formula (2) wherein R¹ is a trifluoromethyl group, R³ is a hydrogen atom, X is an oxygen atom; an amide compound represented by formula (2) wherein R¹ is a trifluoromethyl group, R⁴ is a C1-C4 alkyl group, X is an oxygen atom;
an amide compound represented by formula (2) wherein R¹ is a trifluoromethyl group, R⁴ is a C1-C2 alkyl group, X is an oxygen atom;
an amide compound represented by formula (2) wherein R¹ is a trifluoromethyl group, R⁵ is a C1-C4 alkyl group, X is an oxygen atom;
an amide compound represented by formula (2) wherein R¹ is a trifluoromethyl group, R⁵ is a C3-C4 alkynyl group, X is an oxygen atom;
an amide compound represented by formula (2) wherein R¹ is a trifluoromethyl group, R⁵ is a C1-C2 alkyl group, X is an oxygen atom;
an amide compound represented by formula (2) wherein R¹ is a trifluoromethyl group, R⁵ is a 2-propynyl group, X is an oxygen atom;
an amide compound represented by formula (2) wherein R¹ is a di (C1-C4 alkyl) amino group, R² is a hydrogen atom, X is an oxygen atom;
an amide compound represented by formula (2) wherein R¹ is a di (C1-C4 alkyl) amino group, R² is a halogen group, X is an oxygen atom;
an amide compound represented by formula (2) wherein R¹ is a di(C1-C4 alkyl) amino group, R² is a C1-C4 haloalkyl group, X is an oxygen atom; an amide compound represented by formula (2) wherein R¹ is a di (C1-C4 alkyl) amino group, R³ is a hydrogen atom, X is an oxygen atom;
an amide compound represented by formula (2) wherein R¹ is a di(C1-C4 alkyl)amino group, R⁴ is a C1-C4 alkyl group, X is an oxygen atom;
an amide compound represented by formula (2) wherein R¹ is a di(C1-C4 alkyl)amino group, R⁴ is a C1-C2 alkyl group, X is an oxygen atom;
an amide compound represented by formula (2) wherein R¹ is a di(C1-C4 alkyl)amino group, R⁵ is a C1-C4 alkyl group, X is an oxygen atom;
an amide compound represented by formula (2) wherein R¹ is a di(C1-C4 alkyl)amino group, R⁵ is a C3-C4 alkynyl group, X is an oxygen atom;
an amide compound represented by formula (2) wherein R¹ is a di (C1-C4 alkyl) amino group, R⁵ is a C1-C2 alkyl group, X is an oxygen atom;
an amide compound represented by formula (2) wherein R¹ is a di(C1-C4 alkyl)amino group, R⁵ is a 2-propynyl group, X is an oxygen atom; an amide compound represented by formula (2) wherein R¹ is a halogen atom, R² is a hydrogen atom, R³ is a hydrogen atom, X is an oxygen atom;
an amide compound represented by formula (2) wherein R¹ is a halogen atom, R² is a hydrogen atom, R⁴ is a C1-C4 alkyl group, X is an oxygen atom;
an amide compound represented by formula (2) wherein R¹ is a halogen atom, R² is a hydrogen atom, R⁴ is a C1-C2 alkyl group, X is an oxygen atom;
an amide compound representedby formula (2) wherein R¹ is a halogen atom, R² is a hydrogen atom, R⁵ is a C1-C4 alkyl group, X is an oxygen atom;
an amide compound represented by formula (2) wherein R¹ is a halogen atom, R² is a hydrogen atom, R⁵ is a C3-C4 alkynyl group, X is an oxygen atom;
an amide compound represented by formula (2) wherein R¹ is a halogen atom, R² is a hydrogen atom, R⁵ is a C1-C2 alkyl group, X is an oxygen atom;
an amide compound representedby formula (2) wherein R¹ is a halogen atom, R² is a hydrogen atom, R⁵ is a 2-propynyl group, X is an oxygen atom;
an amide compound represented by formula (2) wherein R¹ is a C1-C4 alkyl group, R² is a hydrogen atom, R³ is a hydrogen atom, X is an oxygen atom;
an amide compound represented by formula (2) wherein R¹ is a C1-C4 alkyl group, R² is a hydrogen atom, R⁴ is a C1-C4 alkyl group, X is an oxygen atom;
an amide compound represented by formula (2) wherein R¹ is a C1-C4 alkyl group, R² is a hydrogen atom, R⁴ is a C1-C2 alkyl group, X is an oxygen atom;
an amide compound represented by formula (2) wherein R¹ is a C1-C4 alkyl group, R² is a hydrogen atom, R⁵ is a C1-C4 alkyl group, X is an oxygen atom;
an amide compound represented by formula (2) wherein R¹ is a C1 -C4 alkyl group, R² is a hydrogen atom, R⁵ is a C3-C4 alkynyl group, X is an oxygen atom;
an amide compound represented by formula (2) wherein R¹ is a C1-C4 alkyl group, R² is a hydrogen atom, R⁵ is a C1-C2 alkyl group, X is an oxygen atom;
an amide compound represented by formula (2) wherein R¹ is a C1-C4 alkyl group, R² is a hydrogen atom, R⁵ is a 2-propynyl group, X is an oxygen atom;
an amide compound represented by formula (2) wherein R¹ is a C1-C4 haloalkyl group, R² is a hydrogen atom, R³ is a hydrogen atom, X is an oxygen atom;
an amide compound represented by formula (2) wherein R¹ is a C1-C4 haloalkyl group, R² is a hydrogen atom, R⁴ is a C1-C4 alkyl group, X is an oxygen atom;
an amide compound represented by formula (2) wherein R¹ is a C1-C4 haloalkyl group, R² is a hydrogen atom, R⁴ is a C1-C2 alkyl group, X is an oxygen atom;
an amide compound represented by formula (2) wherein R¹ is a C1-C4 haloalkyl group, R² is a hydrogen atom, R⁵ is a C1-C4 alkyl group, X is an oxygen atom;
an amide compound represented by formula (2) wherein R¹ is a C1-C4 haloalkyl group, R² is a hydrogen atom, R⁵ is a C3-C4 alkynyl group, X is an oxygen atom;
an amide compound represented by formula (2) wherein R¹ is a C1-C4 haloalkyl group, R² is a hydrogen atom, R⁵ is a C1-C2 alkyl group, X is an oxygen atom;
an amide compound represented by formula (2) wherein R¹ is a C1-C4 haloalkyl group, R² is a hydrogen atom, R⁵ is a 2-propynyl group, X is an oxygen atom;
an amide compound represented by formula (2) wherein R¹ is a chlorine atom, R² is a hydrogen atom, R³ is a hydrogen atom, X is an oxygen atom;
an amide compound represented by formula (2) wherein R¹ is a chlorine atom, R² is a hydrogen atom, R⁴ is a C1-C4 alkyl group, X is an oxygen atom;
an amide compound represented by formula (2) wherein R¹ is a chlorine atom, R² is a hydrogen atom, R⁴ is a C1-C2 alkyl group, X is an oxygen atom;
an amide compound represented by formula (2) wherein R¹ is a chlorine atom, R² is a hydrogen atom, R⁵ is a C1-C4 alkyl group, X is an oxygen atom;
an amide compound represented by formula (2) wherein R¹ is a chlorine atom, R² is a hydrogen atom, R⁵ is a C3-C4 alkynyl group, X is an oxygen atom;
an amide compound represented by formula (2) wherein R¹ is a chlorine atom, R² is a hydrogen atom, R⁵ is a C1-C2 alkyl group, X is an oxygen atom;
an amide compound represented by formula (2) wherein R¹ is a chlorine atom, R² is a hydrogen atom, R⁵ is a 2-propynyl group, X is an oxygen atom; an amide compound represented by formula (2) wherein R¹ is a methyl group, R² is a hydrogen atom, R³ is a hydrogen atom, X is an oxygen atom;
an amide compound represented by formula (2) wherein R¹ is a methyl group, R² is a hydrogen atom, R⁴ is a C1-C4 alkyl group, X is an oxygen atom;
an amide compound represented by formula (2) wherein R¹ is a methyl group, R² is a hydrogen atom, R⁴ is a C1-C2 alkyl group, X is an oxygen atom;
an amide compound represented by formula (2) wherein R¹ is a methyl group, R² is a hydrogen atom, R⁵ is a C1-C4 alkyl group, X is an oxygen atom;
an amide compound represented by formula (2) wherein R¹ is a methyl group, R² is a hydrogen atom, R⁵ is a C3-C4 alkynyl group, X is an oxygen atom;
an amide compound represented by formula (2) wherein R¹ is a methyl group, R² is a hydrogen atom, R⁵ is a C1-C2 alkyl group, X is an oxygen atom;
an amide compound represented by formula (2) wherein R¹ is a methyl group, R² is a hydrogen atom, R⁵ is a 2-propynyl group, X is an oxygen atom;
an amide compound represented by formula (2) wherein R¹ is a trifluoromethyl group, R² is a hydrogen atom, R³ is a hydrogen atom, X is an oxygen atom;
an amide compound represented by formula (2) wherein R¹ is a trifluoromethyl group, R² is a hydrogen atom, R⁴ is a C1-C4 alkyl group, X is an oxygen atom;
an amide compound represented by formula (2) wherein R¹ is a trifluoromethyl group, R² is a hydrogen atom, R⁴ is a C1-C2 alkyl group, X is an oxygen atom;
an amide compound represented by formula (2) wherein R¹ is a trifluoromethyl group, R² is a hydrogen atom, R⁵ is a C1-C4 alkyl group, X is an oxygen atom;
an amide compound represented by formula (2) wherein R¹ is a trifluoromethyl group, R² is a hydrogen atom, R⁵ is a C3-C4 alkynyl group, X is an oxygen atom;
an amide compound represented by formula (2) wherein R¹ is a trifluoromethyl group, R² is a hydrogen atom, R⁵ is a C1-C2 alkyl group, X is an oxygen atom;
an amide compound represented by formula (2) wherein R¹ is a trifluoromethyl group, R² is a hydrogen atom, R⁵ is a 2-propynyl group, X is an oxygen atom;
an amide compound represented by formula (2) wherein R¹ is a halogen atom, R² is a hydrogen atom, R³ is a hydrogen atom, R⁴ is a C1-C4 alkyl group, X is an oxygen atom;
an amide compound represented by formula (2) wherein R¹ is a halogen atom, R² is a hydrogen atom, R³ is a hydrogen atom, R⁴ is a C1-C2 alkyl group, X is an oxygen atom;
an amide compound represented by formula (2) wherein R¹ is a halogen atom, R² is a hydrogen atom, R³ is a hydrogen atom, R⁵ is a C1-C4 alkyl group, X is an oxygen atom;
an amide compound represented by formula (2) wherein R¹ is a halogen atom, R² is a hydrogen atom, R³ is a hydrogen atom, R⁵ is a C3-C4 alkynyl group, X is an oxygen atom;
an amide compound represented by formula (2) wherein R¹ is a halogen atom, R² is a hydrogen atom, R³ is a hydrogen atom, R⁵ is a C1-C2 alkyl group, X is an oxygen atom;
an amide compound represented by formula (2) wherein R¹ is a halogen atom, R² is a hydrogen atom, R³ is a hydrogen atom, R⁵ is a 2-propynyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, X is an oxygen atom;
an amide compound represented by formula (2) wherein R¹ is a C1-C4 alkyl group, R² is a hydrogen atom, R³ is a hydrogen atom, R⁴ is a C1-C2 alkyl group, X is an oxygen atom;
an amide compound represented by formula (2) wherein R¹ is a C1-C4 alkyl group, R² is a hydrogen atom, R³ is a hydrogen atom, R⁵ is a C1-C4 alkyl group, X is an oxygen atom;
an amide compound represented by formula (2) wherein R¹ is a C1-C4 alkyl group, R² is a hydrogen atom, R³ is a hydrogen atom, R⁵ is a C3-C4 alkynyl group, X is an oxygen atom;
an amide compound represented by formula (2) wherein R¹ is a C1-C4 alkyl group, R² is a hydrogen atom, R³ is a hydrogen atom, R⁵ is a C1-C2 alkyl group, X is an oxygen atom;
an amide compound represented by formula (2) wherein R¹ is a C1-C4 alkyl group, R² is a hydrogen atom, R³ is a hydrogen atom, R⁵ is a 2-propynyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, X is an oxygen atom;
an amide compound represented by formula (2) wherein R¹ is a C1-C4 haloalkyl group, R² is a hydrogen atom, R³ is a hydrogen atom, R⁴ is a C1-C2 alkyl group, X is an oxygen atom;
an amide compound represented by formula (2) wherein R¹ is a C1-C4 haloalkyl group, R² is a hydrogen atom, R³ is a hydrogen atom, R⁵ is a C1-C4 alkyl group, X is an oxygen atom;
an amide compound represented by formula (2) wherein R¹ is a C1-C4 haloalkyl group, R² is a hydrogen atom, R³ is a hydrogen atom, R⁵ is a C3-C4 alkynyl group, X is an oxygen atom;
an amide compound represented by formula (2) wherein R¹ is a C1-C4 haloalkyl group, R² is a hydrogen atom, R³ is a hydrogen atom, R⁵ is a C1-C2 alkyl group, X is an oxygen atom;
an amide compound represented by formula (2) wherein R¹ is a C1-C4 haloalkyl group, R² is a hydrogen atom, R³ is a hydrogen atom, R⁵ is a 2-propynyl group, X is an oxygen atom;
an amide compound represented by formula (2) wherein R¹ is a chlorine atom, R² is a hydrogen atom, R³ is a hydrogen atom, R⁴ is a C1-C4 alkyl group, X is an oxygen atom;
an amide compound represented by formula (2) wherein R¹ is a chlorine atom, R² is a hydrogen atom, R³ is a hydrogen atom, R⁴ is a C1-C2 alkyl group, X is an oxygen atom;
an amide compound represented by formula (2) wherein R¹ is a chlorine atom, R² is a hydrogen atom, R³ is a hydrogen atom, R⁵ is a C1-C4 alkyl group, X is an oxygen atom;
an amide compound represented by formula (2) wherein R¹ is a chlorine atom, R² is a hydrogen atom, R³ is a hydrogen atom, R⁵ is a C3-C4 alkynyl group, X is an oxygen atom;
an amide compound represented by formula (2) wherein R¹ is a chlorine atom, R² is a hydrogen atom, R³ is a hydrogen atom, R⁵ is a C1-C2 alkyl group, X is an oxygen atom;
an amide compound represented by formula (2) wherein R¹ is a chlorine atom, R² is a hydrogen atom, R³ is a hydrogen atom, R⁵ is a 2-propynyl group, X is an oxygen atom;
an amide compound represented by formula (2) wherein R¹ is a methyl group, R² is a hydrogen atom, R³ is a hydrogen atom, R⁴ is a C1-C4 alkyl group, X is an oxygen atom;
an amide compound represented by formula (2) wherein R¹ is a methyl group, R² is a hydrogen atom, R³ is a hydrogen atom, R⁴ is a C1-C2 alkyl group, X is an oxygen atom;
an amide compound represented by formula (2) wherein R¹ is a methyl group, R² is a hydrogen atom, R³ is a hydrogen atom, R⁵ is a C1-C4 alkyl group, X is an oxygen atom;
an amide compound represented by formula (2) wherein R¹ is a methyl group, R² is a hydrogen atom, R³ is a hydrogen atom, R⁵ is a C3-C4 alkynyl group, X is an oxygen atom;
an amide compound represented by formula (2) wherein R¹ is a methyl group, R² is a hydrogen atom, R³ is a hydrogen atom, R⁵ is a C1-C2 alkyl group, X is an oxygen atom;
an amide compound represented by formula (2) wherein R¹ is a methyl group, R² is a hydrogen atom, R³ is a hydrogen atom, R⁵ is a 2-propynyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, X is an oxygen atom;
an amide compound represented by formula (2) wherein R¹ is a trifluoromethyl group, R² is a hydrogen atom, R³ is a hydrogen atom, R⁴ is a C1-C2 alkyl group, X is an oxygen atom;
an amide compound represented by formula (2) wherein R¹ is a trifluoromethyl group, R² is a hydrogen atom, R³ is a hydrogen atom, R⁵ is a C1-C4 alkyl group, X is an oxygen atom;
an amide compound represented by formula (2) wherein R¹ is a trifluoromethyl group, R² is a hydrogen atom, R³ is a hydrogen atom, R⁵ is a C3-C4 alkynyl group, X is an oxygen atom;
an amide compound represented by formula (2) wherein R¹ is a trifluoromethyl group, R² is a hydrogen atom, R³ is a hydrogen atom, R⁵ is a C1-C2 alkyl group, X is an oxygen atom;
an amide compound represented by formula (2) wherein R¹ is a trifluoromethyl group, R² is a hydrogen atom, R³ is a hydrogen atom, R⁵ is a 2-propynyl group, X is an oxygen atom;
an amide compound represented by formula (2) wherein R¹ is a halogen atom, R² is a hydrogen atom, R³ is a hydrogen atom, R⁴ is a C1-C4 alkyl group, R⁵ is a C1-C4 alkyl group, X is an oxygen atom; an amide compound represented by formula (2) wherein R¹ is a halogen atom, R² is a hydrogen atom, R³ is a hydrogen atom, R⁴ is a C1-C4 alkyl group, R⁵ is a C1-C2 alkyl group, X is an oxygen atom; an amide compound represented by formula (2) wherein R¹ is a halogen atom, R² is a hydrogen atom, R³ is a hydrogen atom, R⁴ is a C1-C4 alkyl group, R⁵ is a C1-C4 alkyl group, X is an oxygen atom; an amide compound represented by formula (2) wherein R¹ is a halogen atom, R² is a hydrogen atom, R³ is a hydrogen atom, R⁴ is a C1-C4 alkyl group, R⁵ is a C3-C4 alkynyl group, X is an oxygen atom; an amide compound represented by formula (2) wherein R¹ is a halogen atom, R² is a hydrogen atom, R³ is a hydrogen atom, R⁴ is a C1-C4 alkyl group, R⁵ is a C1-C2 alkyl group, X is an oxygen atom; an amide compound represented by formula (2) wherein R¹ is a halogen atom, R² is a hydrogen atom, R³ is a hydrogen atom, R⁴ is a C1-C4 alkyl group, R⁵ is a 2-propynyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R⁵ is a C3-C4 alkynyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R³ is a C1-C2 alkyl group, X is an oxygen atom;
an amide compound represented by formula (2) wherein R¹ is a C1-C4 alkyl group, R² is a hydrogen atom, R³ is a hydrogen atom, R⁴ is a C1-C4 alkyl group, R⁵ is a 2-propynyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C3-C4 alkynyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a C1-C4 haloalkyl group, R² is a hydrogen atom, R³ is a hydrogen atom, R⁴ is a C1-C4 alkyl group, R⁵ is a 2-propynyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a C3-C4 alkynyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a chlorine atom, R² is a hydrogen atom, R³ is a hydrogen atom, R⁴ is a C1-C4 alkyl group, R⁵ is a 2-propynyl group, X is an oxygen atom;
an amide compound represented by formula (2) wherein R¹ is a methyl group, R² is a hydrogen atom, R³ is a hydrogen atom, R⁴ is a C1-C4 alkyl group, R⁵ is a C1-C4 alkyl group, X is an oxygen atom; an amide compound represented by formula (2) wherein R¹ is a methyl group, R² is a hydrogen atom, R³ is a hydrogen atom, R⁴ is a C1-C4 alkyl group, R⁵ is a C1-C2 alkyl group, X is an oxygen atom; an amide compound represented by formula (2) wherein R¹ is a methyl group, R² is a hydrogen atom, R³ is a hydrogen atom, R⁴ is a C1-C4 alkyl group, R⁵ is a C1-C4 alkyl group, X is an oxygen atom; an amide compound represented by formula (2) wherein R¹ is a methyl group, R² is a hydrogen atom, R³ is a hydrogen atom, R⁴ is a C1-C4 alkyl group, R⁵ is a C3-C4 alkynyl group, X is an oxygen atom; an amide compound represented by formula (2) wherein R¹ is a methyl group, R² is a hydrogen atom, R³ is a hydrogen atom, R⁴ is a C1-C4 alkyl group, R⁵ is a C1-C2 alkyl group, X is an oxygen atom; an amide compound represented by formula (2) wherein R¹ is a methyl group, R² is a hydrogen atom, R³ is a hydrogen atom, R⁴ is a C1-C4 alkyl group, R⁵ is a 2-propynyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a C1-C4 alkyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a C3-C4 alkynyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a C1-C2 alkyl group, X is an oxygen atom;
an amide compound represented by formula (2) wherein R¹ is a trifluoromethyl group, R² is a hydrogen atom, R³ is a hydrogen atom, R⁴ is a C1-C4 alkyl group, R⁵ is a 2-propynyl group, X is an oxygen atom;
an amide compound represented by formula (2) wherein R² is a hydrogen atom, R³ is a hydrogen atom, X is an oxygen atom;
an amide compound represented by formula (2) wherein R² is a hydrogen atom, R⁴ is a C1-C4 alkyl group, X is an oxygen atom; an amide compound represented by formula (2) wherein R² is a hydrogen atom, R⁹ is a C1-C2 alkyl group, X is an oxygen atom; an amide compound represented by formula (2) wherein R² is a hydrogen atom, R⁵ is a C1-C4 alkyl group, X is an oxygen atom; an amide compound represented by formula (2) wherein R² is a hydrogen atom, R⁵ is a C3-C4 alkynyl group, X is an oxygen atom; an amide compound represented by formula (2) wherein R² is a hydrogen atom, R⁵ is a C1-C2 alkyl group, X is an oxygen atom; an amide compound represented by formula (2) wherein R² is a hydrogen atom, R⁵ is a 2-propynyl, X is an oxygen atom;
an amide compound represented by formula (2) wherein R³ is a hydrogen atom, R⁴ is a C1-C4 alkyl group, X is an oxygen atom; an amide compound represented by formula (2) wherein R³ is a hydrogen atom, R⁴ is a C1-C2 alkyl group, X is an oxygen atom; an amide compound represented by formula (2) wherein R³ is a hydrogen atom, R⁵ is a C1-C4 alkyl group, X is an oxygen atom; an amide compound represented by formula (2) wherein R³ is a hydrogen atom, R⁵ is a C3-C4 alkynyl group, X is an oxygen atom; an amide compound represented by formula (2) wherein R³ is a hydrogen atom, R⁵ is a C1-C2 alkyl group, X is an oxygen atom; an amide compound represented by formula (2) wherein R³ is a hydrogen atom, R⁵ is a 2-propynyl group, X is an oxygen atom; an amide compound represented by formula (2) wherein R⁴ is a C1-C4 alkyl group, R⁵ is a C1-C4 alkyl group, X is an oxygen atom; an amide compound represented by formula (2) wherein R⁴ is a C1-C4 alkyl group, R⁵ is a C3-C4 alkynyl group, X is an oxygen atom; an amide compound represented by formula (2) wherein R⁴ is a C1-C4 alkyl group, R⁵ is a C1-C2 alkyl group, X is an oxygen atom; an amide compound represented by formula (2) wherein R⁴ is a C1-C4 alkyl group, R⁵ is a 2-propynyl group, X is an oxygen atom;
an amide compound represented by formula (2) wherein R⁴ is a C1-C2 alkyl group, R⁵ is a C1-C4 alkyl group, X is an oxygen atom; an amide compound represented by formula (2) wherein R⁴ is a C1-C2 alkyl group, R⁵ is a C3-C4 alkynyl group, X is an oxygen atom; an amide compound represented by formula (2) wherein R⁴ is a C1-C2 alkyl group, R⁵ is a C1-C2 alkyl group, X is an oxygen atom; an amide compound represented by formula (2) wherein R⁴ is a C1-C2 alkyl group, R⁵ is a 2-propynyl group, X is an oxygen atom;
an amide compound represented by formula (2) wherein R² is a hydrogen atom, R⁴ is a C1-C2 alkyl group, R⁴ is a C1-C4 alkyl group, X is an oxygen atom;
an amide compound represented by formula (2) wherein R² is a hydrogen atom, R⁹ is a C1-C2 alkyl group, R⁴ is a C3-C4 alkynyl group, X is an oxygen atom;
an amide compound represented by formula (2) wherein R² is a hydrogen atom, R⁴ is a C1-C2 alkyl group, R⁴ is a C1-C4 alkyl group, X is an oxygen atom;
an amide compound represented by formula (2) wherein R² is a hydrogen atom, R⁴ is a C1-C2 alkyl group, R⁴ is a 2-propynyl group, X is an oxygen atom;
an amide compound represented by formula (2) wherein R² is a hydrogen atom, R⁴ is a C1-C4 alkyl group, R⁴ is a C1-C4 alkyl group, X is an oxygen atom;
an amide compound represented by formula (2) wherein R² is a hydrogen atom, R⁴ is a C1-C4 alkyl group, R⁴ is a C3-C4 alkynyl group, X is an oxygen atom;
an amide compound represented by formula (2) wherein R² is a hydrogen atom, R⁴ is a C1-C4 alkyl group, R⁴ is a C1-C4 alkyl group, X is an oxygen atom;
an amide compound represented by formula (2) wherein R² is a hydrogen atom, R⁴ is a C1-C4 alkyl group, R⁴ is a 2-propynyl group, X is an oxygen atom.

Next, production methods for the compounds of the present invention are described. The compounds of the present invention can be produced, for example, according to the following (Production Method A), (Production Method B), (Production Method C) or (Production Method D).

### (Production Method A)

The compound (1-1) among the compound of the present invention wherein R⁵⁷ is a hydrogen atom and X is an oxygen atom can be produced by making the compound (5) react with the compound (6). 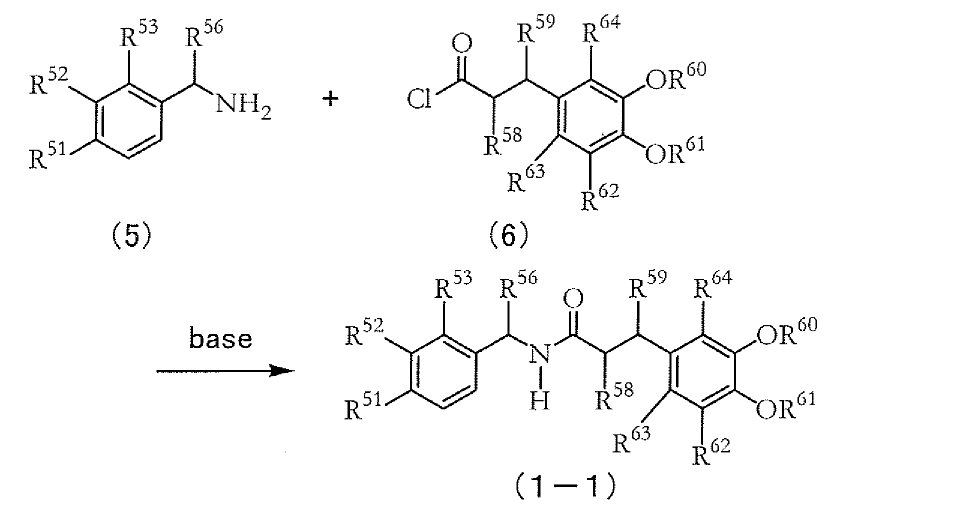 wherein, in the formula,
R⁵¹, R⁵², R⁵³, R⁵⁶, R⁵⁸, R⁵⁹, R⁶⁰, R⁶⁵ , R⁶², R⁶³ and R⁶⁴ represent same meaning as described above.

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 10 moles per 1 mole of the compound (6) and that to the compound (5) is usually from 1 to 5 moles.

The reaction temperature is usually in the range of from -20 to 100 °C, and the reaction time is usually in the range of from 0.1 to 24 hours.

After completion of the reaction, the compound (1-1) can be isolated by subjecting to post-treatment such as (i) the reaction mixture is poured into water, extracted with organic solvent; the organic layer is washed with acidic water (diluted hydrochloric acid and the like), basic water (aqueous solution of sodium bicarbonate and the like), if necessary; then the organic layer is dried and concentrated, or (ii) to the reaction mixture small amount of water is added , and concentrated under reduced pressure, then the obtained solid is collected by filtration. The isolated compound (1-1) can be purified by a technique such as chromatography, recrystallization and the like.

### (Production Method B)

The compound (1-1) among the compound of the present invention wherein R⁵⁷ is a hydrogen atom and X is an oxygen atom can be also produced by making the compound (7) react with the compound (8). 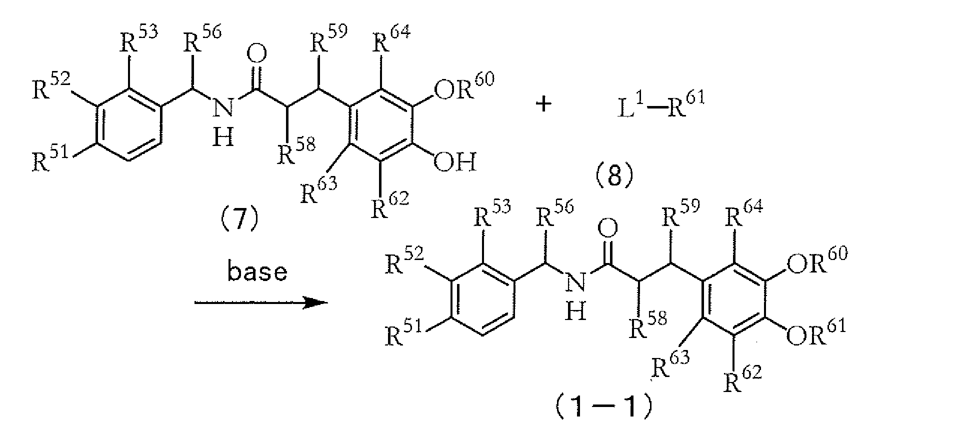 wherein, in the formula,
L¹ represents a halogen atom, a methansulfonyloxy group, a trifluoromethanesulfonyloxy group or a p-toluenesulfonyloxy group, R⁵¹, R⁵² R⁵³, R⁵⁶, R⁵⁸ R⁵⁹, R⁶⁰, R⁶¹, R⁶², R⁶³ and R⁶⁴ represent
same meaning as described above.

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, alkali metal hydrides such as sodium hydride, potassium hydride and the like, tertiary amines such as triethylamine, diisopropylethylamine, 1,8-diazabicyclo [5.4.0] undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 10 moles per 1 mole of the compound (7) and that to the compound (8) is usually from 1 to 5 moles.

The reaction temperature is usually in the range of from -20 to 100 °C, and the reaction time is usually in the range of from 0.1 to 24 hours.

After completion of the reaction, the compound (1-1) can be isolated by subjecting to post-treatment such as the reaction mixture is poured into water, extracted with organic solvent; the organic layer is washed with acidic water (diluted hydrochloric acid and the like), basic water (aqueous solution of sodium bicarbonate and the like), if necessary; then the organic layer is dried and concentrated. The isolated compound (1-1) can be purified by a technique such as chromatography, recrystallization and the like.

Further, the compound (1-1) can be also produced by making the compound (7') react with the compound (11). 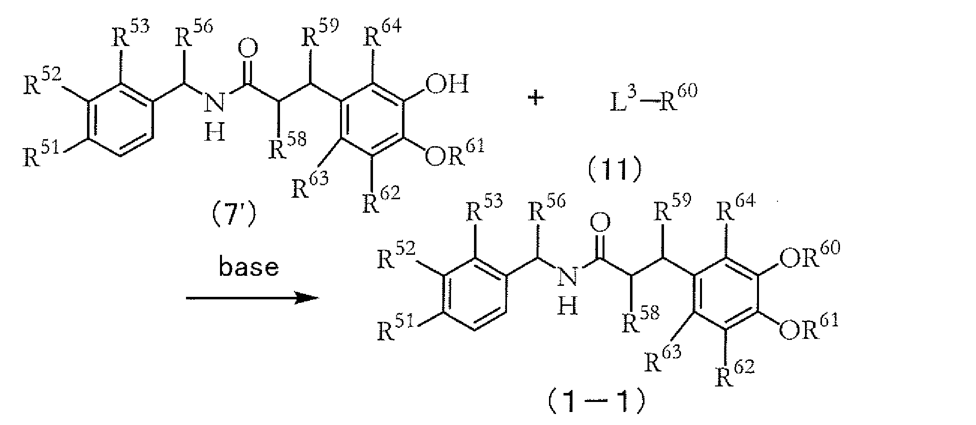 wherein, in the formula,
L³ represents a halogen atom, a methansulfonyloxy group, a trifluoromethanesulfonyloxy group or a p-toluenesulfonyloxy group, R⁵¹, R⁵², R⁵³ R⁵⁶, R⁵⁸, R⁵⁹, R⁶⁰, R⁶¹, R⁶², R⁶³ and R⁶⁴ represent same meaning as described above.

The reaction is carried out in a similar condition as the above mentioned reaction.

### (Production Method C)

The compound (1-2) among the compound of the present invention wherein R⁵⁷ is a C1-C4 alkyl group, a C3-C4 alkenyl group or a C3-C4 alkynyl group and X is an oxygen atom can be produced by making the compound (1-1) react with the compound (9) . 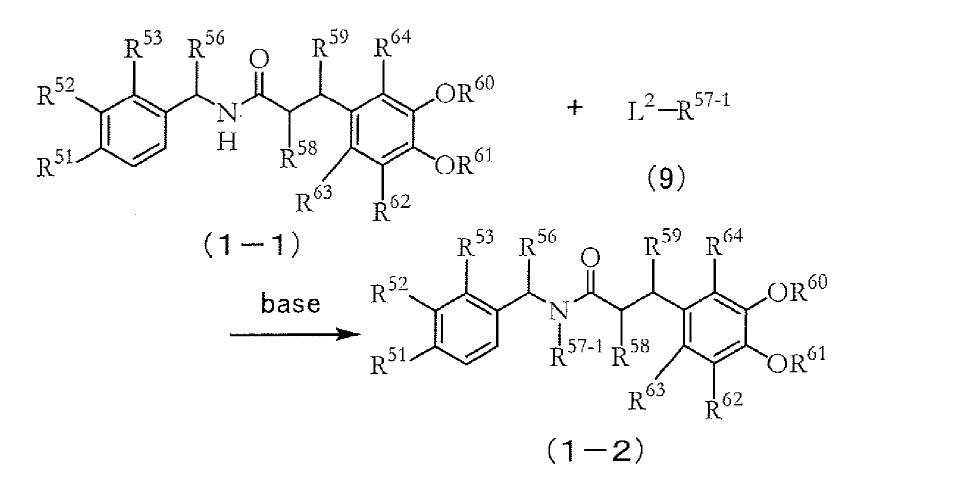 wherein, in the formula,
L² represents a halogen atom, a methansulfonyloxy group, a trifluoromethanesulfonyloxy group or a p-toluenesulfonyloxy group, R⁵⁷⁻¹ represents a C1-C4 alkyl group, a C3-C4 alkenyl group or a C3-C4 alkynyl group, R⁵¹, R⁵², R⁵³, R⁵⁶, R⁵⁸, R⁵⁹, R⁶⁰, R⁶¹, R⁶², R⁶³ and R⁶⁴ represent same meaning as described above.

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, alkali metal hydrides such as sodium hydride potassium hydride and the like, tertiary amines such as triethylamine, diisopropylethylamine, 1,8-diazabicyclo[5.4.0]undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 10 moles per 1 mole of the compound (1-1) and that to the compound (9) is usually from 1 to 5 moles.

The reaction temperature is usually in the range of from 0 to 100°C, and the reaction time is usually in the range of from 0.1 to 24 hours.

After completion of the reaction, the compound (1-2) can be isolated by subjecting to post-treatment such as the reaction mixture is poured into water, extracted with organic solvent, then the organic layer is dried and concentrated. The isolated compound (1-2) can be purified by a technique such as chromatography, recrystallization and the like.

### (Production Method D)

The compound (1-4) among the compound of the present invention wherein X is a sulfur atom can be produced by making the compound (1-3), which X is an oxygen atom among the compound of the present invention, react with 2,4-bis(4-methoxyphenyl)-1,3-dithia-2,4-diphosphetane-2,4-d isulfide (which is generally known as "Lawesson's Reagent"). 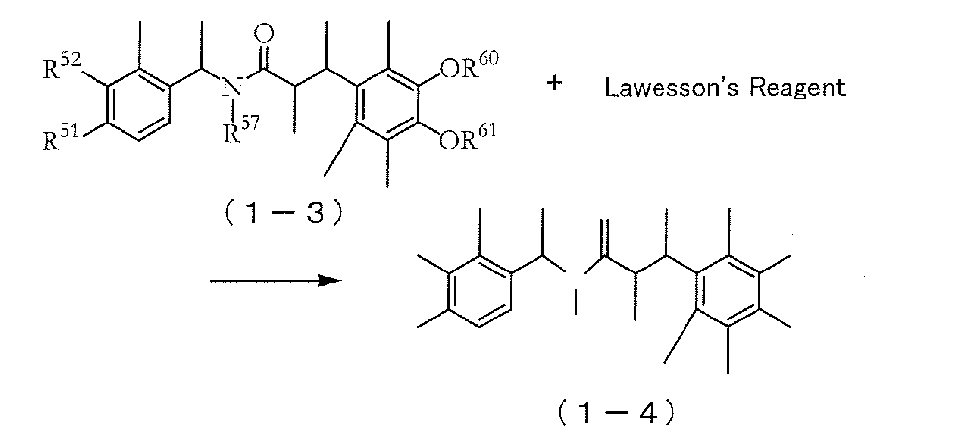 ran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, nitriles such as acetonitrile, butyronitrile and the like, sulfoxides such as dimethyl sulfoxide and the mixture thereof.

The molar ratio applied to the Lawesson's Reagent is usually from 1 to 10 moles per 1 mole of the compound (1-3).

The reaction temperature is usually in the range of from 50 to 150 °C, and the reaction time is usually in the range of from 0.5 to 24 hours.

After completion of the reaction, the compound (1-4) can be isolated by subjecting to post-treatment such as the reaction mixture is poured into water, extracted with organic solvent, then the organic layer is dried and concentrated. The isolated compound (1-4) can be purified by a technique such as chromatography, recrystallization and the like.

Next, production methods for the intermediate compounds for the present invention are described.

The compound (6) can be produced, for example, according to following scheme. 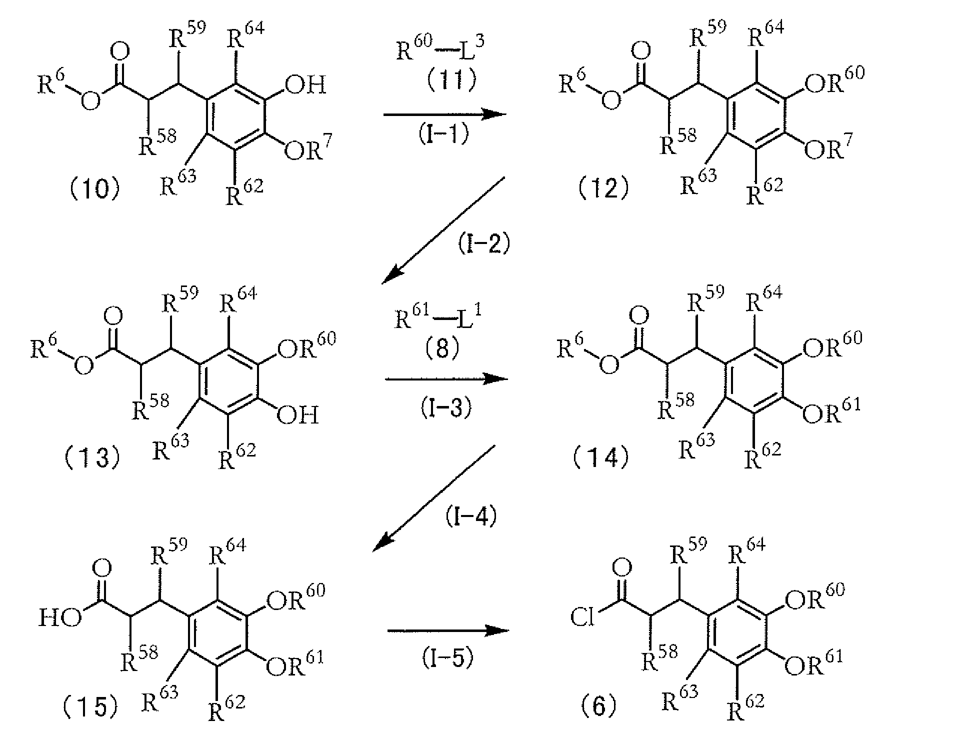 wherein, in the formula,
R⁶ represents a C1-C6 alkyl group, R⁷ represents a benzyl group or a methoxymethyl group, R⁵⁸, R⁵⁹, R⁶⁰, R⁶¹, R⁶², R⁶³, R⁶⁴, L¹ and L³ represent same meaning as described above.

### Step(I-1)

The compound (12) can be produced by making the compound (10) react with the compound (11).

The reaction is carried out in the presence of a solvent or absence of a solvent, and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, alkali metal hydrides such as sodium hydride potassium hydride and the like, tertiary amines such as triethylamine, diisopropylethylamine, 1,8-diazabicyclo[5.4.0]undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 50 moles per 1 mole of the compound (10) and that to the compound (11) is usually from 1 to 5 moles.

The reaction temperature is usually in the range of from 0 to 100 °C, and the reaction time is usually in the range of from 0.1 to 24 hours.

After completion of the reaction, the compound (12) can be isolated by subjecting to post-treatment such as the reaction mixture is poured into water, extracted with organic solvent, then the organic layer is dried and concentrated. The isolated compound (12) can be purified by a technique such as chromatography, recrystallization and the like.

The compound (10) is, for example, the compound described in Tetrahedron Letters, vol.36, No.51, pp.9369-9372, 1995 or can be produced according to similar methods described in that literature.

### Step(I-2)

### (A) In case of R⁷ is a benzyl group

The compound (13) can be produced by making the compound (12) react with hydrogen in the presence of a hydrogenation catalyst and an acid.

The reaction is carried out usually under hydrogen atmosphere and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like and the mixture thereof.

The hydrogenation catalyst used for the reaction includes, for example, transition metal compounds such as palladium charcoal, palladium hydroxide, Raney nickel, platinum oxide and the like. The acid used for the reaction includes, for example, hydrochloric acid.

The molar ratio applied to hydrogen is 1 mole per 1 mole of the compound (13) and that to the hydrogenation catalyst is usually from 0.001 to 0.5 moles.

The reaction is usually carried out under hydrogen atmosphere of from 1 to 100 atoms.

The reaction temperature is usually in the range of from -20 to 100 °C, and the reaction time is usually in the range of from 0.1 to 24 hours.

After completion of the reaction, the compound (13) can be isolated by subjecting to post-treatment such as the reaction mixture is filtered, and the filtrate is extracted with organic solvent, then the obtained organic layer is dried and concentrated. The isolated compound (13) can be purified by a technique such as chromatography, recrystallization and the like.

### (B) In case of R⁷ is a methoxymethyl group

The compound (11) can be produced by subjecting the compound (12) to hydrolysis reaction in the presence of an acid.

The reaction is usually carried out in the presence of water and an organic solvent.

The organic solvent used for the reaction includes, for example, ethers such as 1, 4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide, alcohols such as methanol, ethanol, propanol and the like and the mixture thereof.

The acid used for the reaction includes, for example, inorganic acids such as hydrochloric acid, sulfuric acid and the like, organic acids such as p-toluenesulfonic acid, methansulfonic acid and the like.

The molar ratio applied to the acid is usually from 1 to 10 moles per 1 mole of the compound (12).

The reaction temperature is usually in the range of from 0 to 100 °C, and the reaction time is usually in the range of from 0.1 to 24 hours.

After completion of the reaction, the compound (13) can be isolated by subjecting to post-treatment such as the reaction mixture is extracted with organic solvent, then the organic layer is dried and concentrated. The isolated compound (13) can be purified by a technique such as chromatography, recrystallization and the like.

### Step(I-3)

The compound (14) can be produced by making the compound (13) react with the compound (8).

The reaction is carried out in the presence of a solvent or absence of a solvent, and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 50 moles per 1 mole of the compound (13) and that to the compound (8) is usually from 1 to 5 moles.

The reaction temperature is usually in the range of from 0 to 100°C, and the reaction time is usually in the range of from 0.1 to 24 hours.

After completion of the reaction, the compound (14) can be isolated by subjecting to post-treatment such as following: after adding an organic solvent to the reaction mixture if necessary, the reaction mixture is filterd and the filtrate is concentrated. The isolated compound (14) can be purified by a technique such as distillation, chromatography, recrystallization and the like.

### Step(I-4)

The compound (15) can be produced by subjecting the compound (14) to hydrolysis reaction in the presence of a base.

The reaction is usually carried out in the presence of water and an organic solvent.

The organic solvent used for the reaction includes, for example, ethers such as 1,4-dioxane, tetrahydrofuran, ethylene glycol dimethyl ether, tert-butyl methyl ether and the like, aromatic hydrocarbons such as toluene, xylene and the like, halogenated hydrocarbons such as chlorobenzene, nitriles such as acetonitrile, butyronitrile and the like, alcohols such as methanol, ethanol, propanol and the like and the mixture thereof.

The base used for the reaction includes, for example, alkali metal hydroxides such as lithium hydroxide, sodium hydroxide, potassium hydroxide and the like.

The molar ratio applied to the base is usually from 1 to 10 moles per 1 mole of the compound (14).

The reaction temperature is usually in the range of from 0 to 150 °C, and the reaction time is usually in the range of from 0.1 to 24 hours.

After completion of the reaction, the compound (15) can be isolated by subjecting to post-treatment such as acidic water (hydrochloric acid and the like) is added to the reaction mixture, and the mixture is extracted with organic solvent, then the organic layer is dried and concentrated. The isolated compound (15) can be purified by a technique such as distillation, chromatography, recrystallization and the like.

### Step(I-5)

The compound (6) can be produced by making the compound (15) react with a chlorinating agent.

The reaction is carried out in the presence of a solvent or absence of a solvent.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane and the like, aromatic hydrocarbons such as toluene, xylene and the like, halogenated hydrocarbons such as chlorobenzene and the mixture thereof.

The chlorinating agent used for the reaction includes, for example, thionyl chloride, oxalyl chloride, phosphorus oxychloride.

The molar ratio applied to the chlorinating agent is usually from 1 to 100 moles per 1 mole of the compound (15).

The reaction temperature is usually in the range of from 30 to 150 °C, and the reaction time is usually in the range of from 0.1 to 24 hours.

After completion of the reaction, the compound (6) can be isolated by subjecting to post-treatment such as the reaction mixture is concentrated. The isolated compound (6) usually can be used to the reaction of next step without further purification.

The compound in the compound (14) wherein R⁶⁰ is a C1-C4 alkyl group, R⁶¹ is a C3-C6 alkynyl group, R⁶ is a methyl group, an ethyl group, a propyl group, an isopropyl group, an isobutyl group, a tert-butyl group; the compound in the compound (15) wherein R⁶⁰ is a methyl group or an ethyl group, R⁶¹ is a C3-C6 alkynyl group; and the compound which is described the formula (6) wherein R⁶⁰ is a methyl group or an ethyl group, R⁶¹ is a C3-C6 alkynyl group, R⁵⁸, R⁵⁹, R⁶², R⁶³ and R⁶⁴ are hydrogen atoms, are the intermediate 1 of the present invention.

The compound (13) can also be produced by according to following scheme. 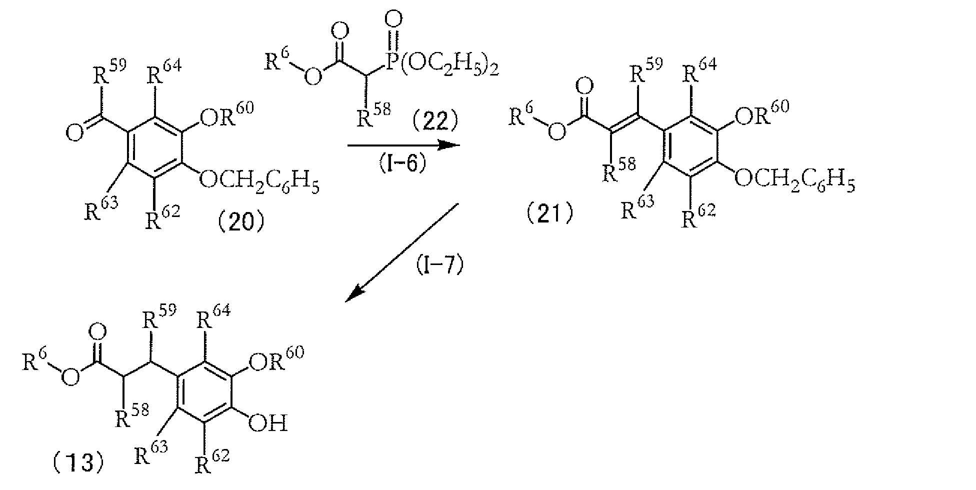 wherein, in the formula,
R⁶ represents a C1-C6 alkyl group, R⁷ represents a benzyl group or a methoxymethyl group, R⁵⁸, R⁵⁹, R⁶⁰, R⁶², R⁶³, R⁶⁴, L¹ and L³ represent same meaning as described above.

### Step(I-6)

The compound (21) can be produced by making the compound (20) react with the compound (22) described below and a base.

The reaction is carried out in the presence of a solvent .

The solvent used for the reaction includes, for example, tetrahydrofuran. The base used to the reaction includes carbonates such as potassium carbonate and the like, alkali metal hydrides such as sodium hydride and the like.

The molar ratio applied to the compound (22) is usually from 1 to 2 moles per 1 mole of the compound (20) and that to the base is usually from 1 to 2 moles. The reaction temperature is usually in the range of from 0 to 80 °C, and the reaction time is usually in the range of from 0.1 to 24 hours.

After completion of the reaction, the compound (20) can be isolated by subjecting to post-treatment such as the reaction mixture is poured into water, and extracted with organic solvent, then the organic layer is dried and concentrated. The isolated compound (20) can be purified by a technique such as chromatography, recrystallization and the like.

The compound (20) is commercially available or can be produced in the similar manner as well known methods.

### Step(I-7)

The compound (13) can be produced by making the compound (20) react with hydrogen in the presence of a hydrogenation catalyst and an acid.

The solvent used for the reaction includes, for example, alcohols such as methanol, ethanol, propanol and the like, esters such as ethyl acetate, butyl acetate and the like, ethers such as tetrahydrofuran, 1,4-dioxane and the like and the mixture thereof.

The hydrogenation catalyst used for the reaction includes, for example, transition metal compounds such as palladium charcoal, palladium hydroxide, Raney nickel, platinum oxide and the like. The acid used for the reaction includes, for example, hydrochloric acid.

The molar ratio applied to hydrogen is usually 2 moles per 1 mole of the compound (13) and that to the hydrogenation catalyst is usually from 0.01 to 0.5 moles.

The reaction is usually carried out under hydrogen atmosphere of from 1 to 100 atoms.

The reaction temperature is usually in the range of from -20 to 100 °C, and the reaction time is usually in the range of from 0.1 to 24 hours.

After completion of the reaction, the compound (13) can be isolated by subjecting to post-treatment such as the reaction mixture is filtered, and the filtrate is extracted with organic solvent, then the obtained organic layer is dried and concentrated. The isolated compound (13) can be purified by a technique such as chromatography, recrystallization and the like.

The compound (14) can also be produced by according to following scheme. 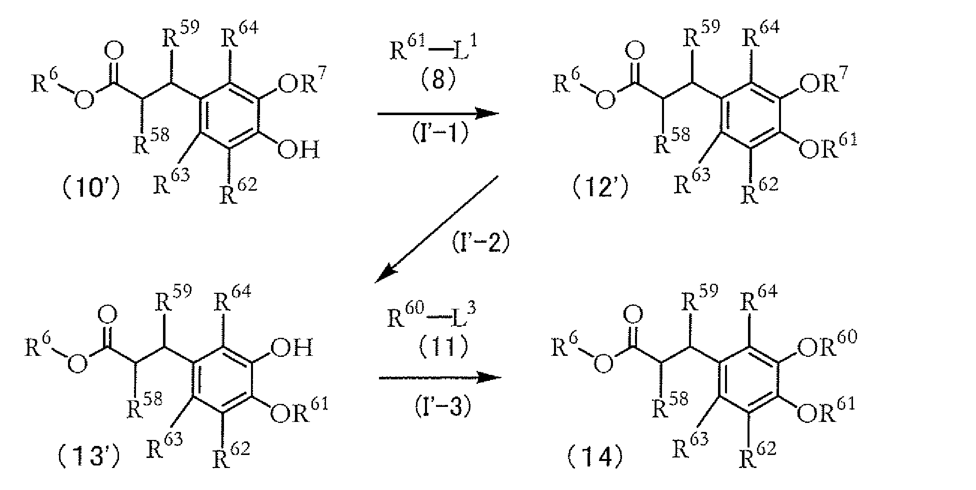 wherein, in the formula,
R⁶ represents a C1-C6 alkyl group, R⁷ represents a benzyl group or a methoxymethyl group, R⁵⁸, R⁵⁹, R⁶⁰, R⁶¹, R⁶², R⁶³, R⁶⁴, L¹ and L³ represent same meaning as described above.

Step(I'-1) can be carried out in a similar manner as Step(I-3) described above. Step(I'-2) can be carried out in a similar manner as Step(I-2) described above. Step(I'-3) is similar condition as Step(I-1).

The compound (10') is, for example, the compound described in Tetrahedron Letters, vol.36, No.51, pp.9369-9372, 1995 or can be produced according to similar methods described in that literature.

The compound (13') can also be produced by according to following scheme. 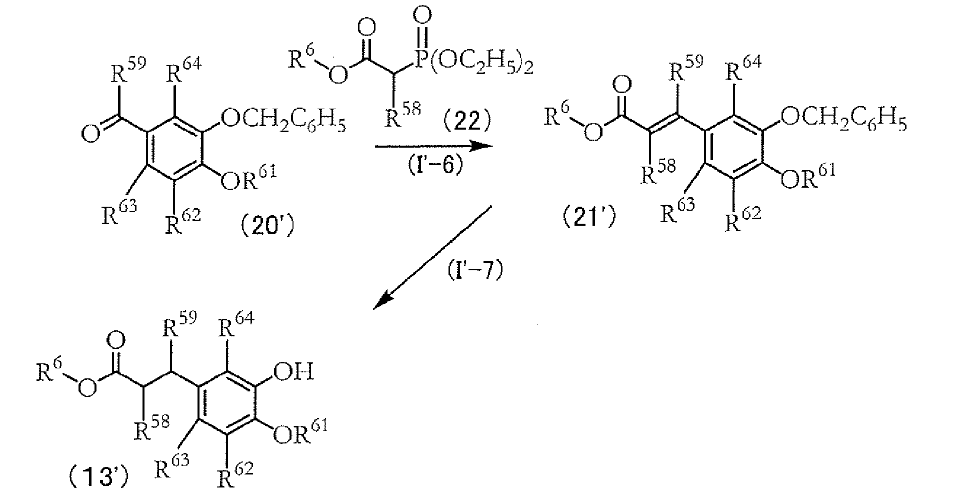 wherein, in the formula,
R^{&} represents a C1-C6 alkyl group, R⁷ represents a benzyl group or a methoxymethyl group, R⁵⁸, R⁵⁹, R⁶¹, R⁶², R⁶³, R⁶⁴, L¹ and L³ represent same meaning as described above.

Step (I'-6) can be carried out in a similar manner as Step (I-6) describedabove. Step (I' -7) can be carried in a similar manner as Step (I-7) described above.

The compound (20') is commercially available or can be produced by in a similar manner as well known methods.

The compound (7) can be produced, for example, by according to following scheme. 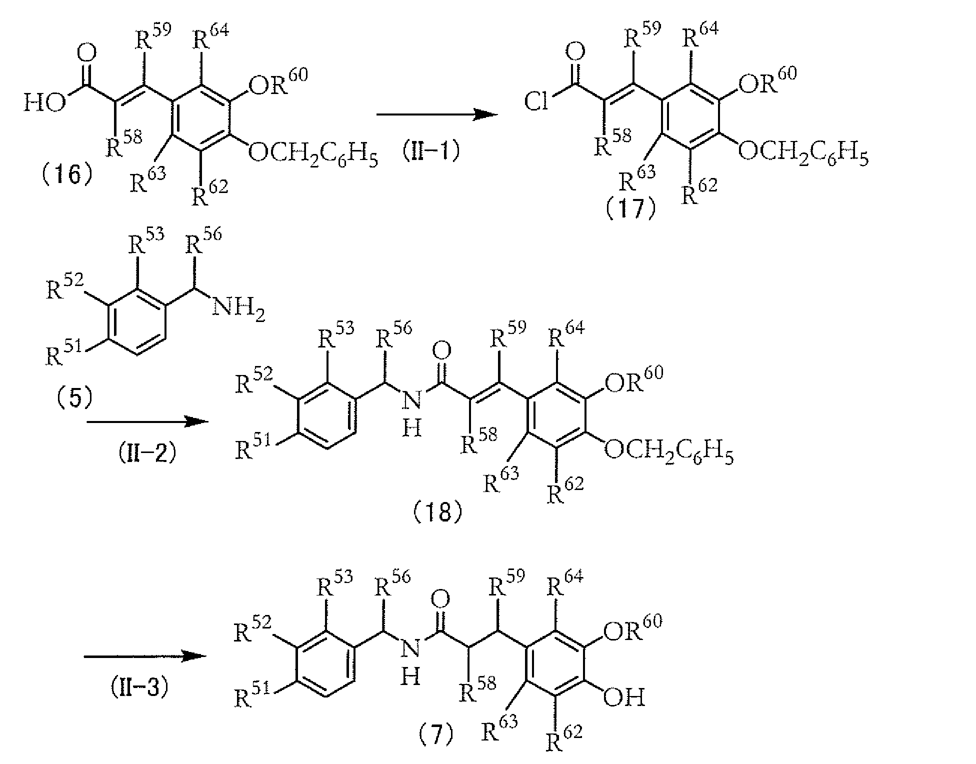 wherein, in the formula,
R⁵¹, R⁵², R⁵³, R⁵⁶, R⁵⁸, R⁵⁹, R⁶⁰, R⁶¹, R⁶², R⁶³, R⁶⁴ represent same meaning as described above.

### Step(II-1)

The compound (17) can be produced by makeing the compound (16) react with a chlorinating agent.

The reaction is carried out in the presence of a solvent or absence of a solvent.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane and the like, aromatic hydrocarbons such as toluene, xylene and the like, halogenated hydrocarbons such as chlorobenzene and the mixture thereof.

The chlorinating agent used for the reaction includes, for example, thionyl chloride, oxalyl chloride and phosphorus oxychloride.

The molar ratio applied to the chlorinating agent is usually from 1 to 100 moles per 1 mole of the compound (16).

The reaction temperature is usually in the range of from 30 to 150 °C, and the reaction time is usually in the range of from 0.1 to 24 hours.

After completion of the reaction, the compound (17) can be isolated by subjecting to post-treatment such as the reaction mixture is concentrated. The isolated compound (17) usually can be used to the reaction of next step without further purification.

The compound (16) is, for example, the compound described in Synthetic Communications, 29(4), pp. 573-581 (1999) or can be produced according to similar methods described in that literature.

### Step(II-2)

The compound (18) can be produced by making the compound (17) react with the compound (5).

The reaction is carried out usually in the presence of a solvent and usually in the presence of a base.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide and the mixture thereof.

The base used for the reaction includes, for example, carbonates such as sodium carbonate, potassium carbonate and the like, tertiary amines such as triethylamine, diisopropylethylamine, 1,8-diazabicyclo[5.4.0]undec-7-ene, 1,5-diazabicyclo[4.3.0]non-5-ene and the like, nitrogen-contained aromatic compounds such as pyridine, 4-dimethylaminopyridine.

The molar ratio applied to the base is usually from 1 to 10 moles per 1 mole of the compound (17) and that to the compound (5) is usually from 1 to 5 moles.

The reaction temperature is usually in the range of from -20 to 100°C, and the reaction time is usually in the range of from 0.1 to 24 hours.

After completion of the reaction, the compound (18) can be isolated by subjecting to post-treatment such as (i) the reaction mixture is poured into water, extracted with organic solvent; the organic layer is washed with acidic water (diluted hydrochloric acid and the like), basic water (aqueous solution of sodium bicarbonate and the like), if necessary; then the organic layer is dried and concentrated, or (ii) to the reaction mixture small amount of water is added , and concentrated under reduced pressure, then the obtained solid is collected by filtration. The isolated compound (18) can be purified by a technique such as chromatography, recrystallization and the like.

### Step(II-3)

The compound (7) can be produced by making the compound (18) react with hydrogen in the presence of a hydrogenation catalyst and an acid.

The reaction is carried out usually under hydrogen atmosphere and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like and the mixture thereof.
The hydrogenation catalyst used for the reaction includes, for example, transition metal compounds such as palladium charcoal, palladium hydroxide, Raney nickel, platinum oxide and the like. The acid used for the reaction includes, for example, such as hydrochloric acid.

The molar ratio applied to hydrogen is usually 2 moles per 1 mole of the compound (18) and that to the hydrogenation catalyst is usually from 0.001 to 0.5 moles.

The reaction is usually carried out under hydrogen atmosphere of from 1 to 100 atoms.

The reaction temperature is usually in the range of from -20 to 100 °C, and the reaction time is usually in the range of from 0.1 to 24 hours.

After completion of the reaction, the compound (7) can be isolated by subjecting to post-treatment such as the reaction mixture is filtered, and the filtrate is extracted with organic solvent, then the obtained organic layer is dried and concentrated. The isolated compound (7) can be purified by a technique such as chromatography, recrystallization and the like.

The compound in the compound (7) wherein R⁵¹ is a halogen atom, a C1-C4 alkyl group, a C1-C4 haloalkyl group, a C1-C4 alkoxy group, a C1-C4 haloalkoxy group, a di(C1-C4 alkyl) amino group or a cyano group, R⁵² is a hydrogen atom, a halogen atom, a C1-C4 alkyl group or a C1-C4 haloalkyl group; or both of R⁵¹ and R⁵² are combined together to be a C3-C5 alkylene group or a group of -CH=CH-CH=CH-, R⁵³ is a hydrogen atom, R⁵⁶ is a hydrogen atom, R⁶⁰ is a C1-C4 alkyl group, is the intermediate 2 of the present invention.

The compound (7') can be produced, for example, by according to following scheme. 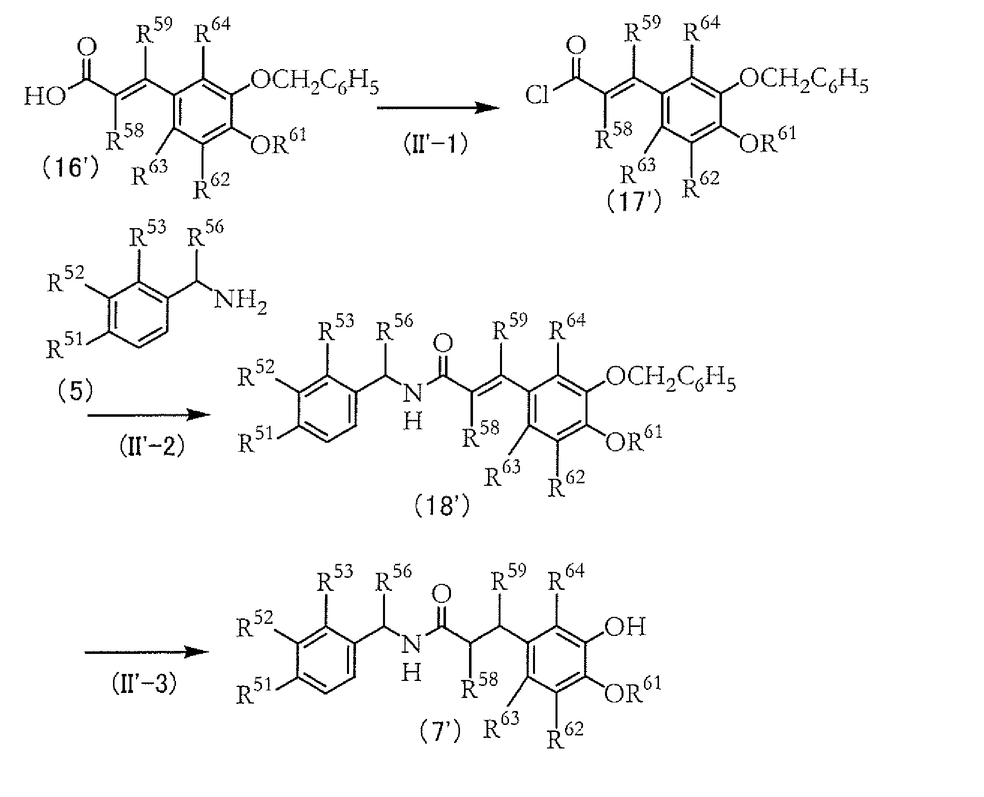 wherein, in the formula,
R⁵¹, R⁵², R⁵³, R⁵⁶, R⁵⁸, R⁵⁹, R⁶⁰, R⁶¹, R⁶², R⁶³, R⁶⁴ represent same meaning as described above.

Step(II'-1) can be carried out in a similar manner as Step (II-1) described above. Step(II'-2) can be carried out in a similar manner as Step (II-2) described above. Step (II'-3) can be carried out in a similar manner as Step (II-3) described above.

The compound (16') is, for example, the compound described in Synthetic Communications, 29(4), pp.573-581 (1999) or can be produced according to similar methods described in that literature.

The compound (5-1) among the compound (5) wherein R⁵⁶ is a hydrogen atom can be produced, for example, by subjecting the compound (19) to reduction reaction. 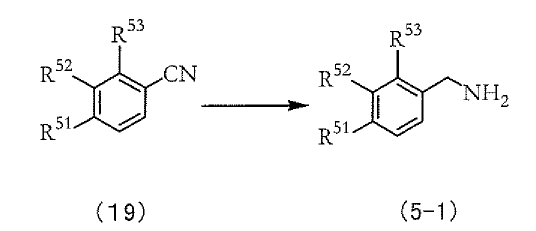 wherein, in the formula,
R⁵¹, R⁵² and R⁵³ represent same meaning as described above.

The reaction is usually carried out in the presence of a solvent.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like and the mixture thereof.

The reducing agent used for the reaction includes, for example, metal hydrides such as lithium aluminium hydride, diisobutyl aluminium hydride.

The molar ratio applied to the reducing agent is usually from 0.5 to 5 moles per 1 mole of the compound (19), but it is changeable depend on the kind of reducing agent .

After completion of the reaction, the compound (5) can be isolated by subjecting to post-treatment such as the reaction mixture is poured into water, extracted with organic solvent; the organic layer washed with basic water (aqueous solution of sodium hydroxide and the like) if necessary, then dried and concentrated. The isolated compound (5) can be purified by a technique such as distillation, chromatography and the like.

The compound (5) can be produced by according to following scheme. 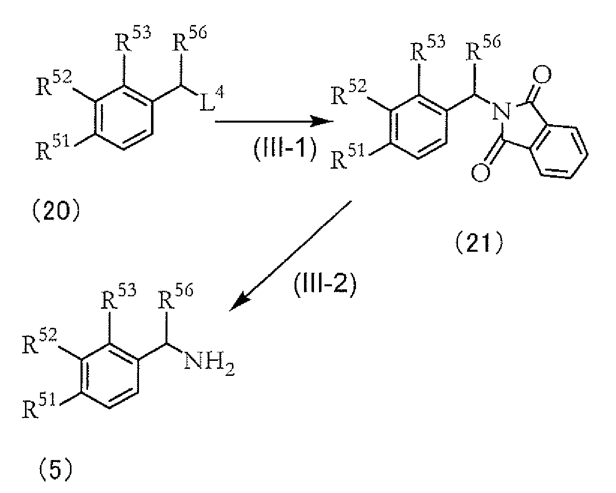 wherein, in the formula,
R⁵¹, R⁵² , R⁵³ and R⁵⁶ represent same meaning as described above, L⁴ represents a chlorine atom or a bromine atom.

### Step(III-1)

The compound (21) can be produced by making the compound (20) react with potassium phthalimide. The reaction is usually carried out in the presence of a solvent.

The solvent used for the reaction includes ethers such as 1, 4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide, water and the mixture thereof.

The molarration applied to potassium phthalimide is usually from 1 to 3 moles per 1 mole of the compound (20).

The reaction temperature is usually in the range of from -20 to 100 °C, and the reaction time is usually in the range of from 0.1 to 24 hours.

After completion of the reaction, the compound (21) can be isolated by subjecting to post-treatment such as the reaction mixture is poured into water and extracted with organic solvent; the organic layer is dried and concentrated. The isolated compound (21) can be purified by a technique such as chromatography, recrystallization and the like.

### Step(III-2)

The compound (5) can be produced by making the compound (21) react with hydrazine.

The reaction is usually carried out in the presence of a solvent.

The solvent used for the reaction includes alcohols such as methanol, ethanol, propanol and the like, water and the mixture thereof.

Hydrazine used for the reaction may be its hydrate. The molar ratio applied to hydrazine is usually from 1 to 10 moles per 1 mole of the compound (21).

The reaction temperature is usually in the range of from 0 to 150 °C, and the reaction time is usually in the range of from 0.1 to 24 hours.

After completion of the reaction, the compound (5) can be isolated by subjecting to post-treatment such as the reaction mixture is filtered, water is added to the filtrate, and extracted with organic solvent; then the organic layer is dried and concentrated. The isolated compound (5) can be purified by a technique such as distillation, chromatography and the like.

The compound (5) can be produced, for example, by subjecting the compound (22) to Leuckart Reaction. 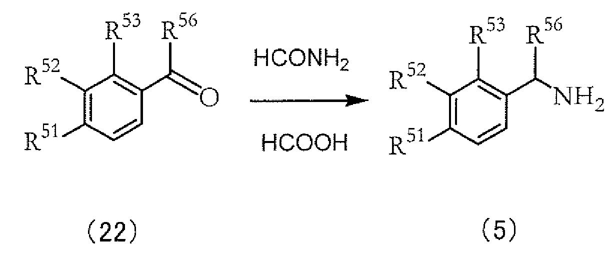 wherein, in the formula,
R⁵¹, R⁵², R⁵³, and R⁵⁶ represent same meaning as described above.

The reaction is carried out in the presence of a solvent or absence of solvent, in the presence of formamide and formic acid.

The solvent used for the reaction includes, for example, ethers such as 1,4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like and the mixture thereof.

The molar ratio applied to formamide is usually from 1 to 15 moles per 1 mole of the compound (22), and that to formic acid is usually from 0.1 to 2 moles.

After completion of the reaction, the compound (5) can be isolated by subjecting to post-treatment such as the reaction mixture is poured into water, extracted with organic solvent; if necessary, the organic layer washed with basic water (aqueous solution of sodium hydroxide and the like); then dried and concentrated. The isolated compound (5) can be purified by a technique such as distillation, chromatography and the like.

The compound (5) can be produced by according to following scheme. 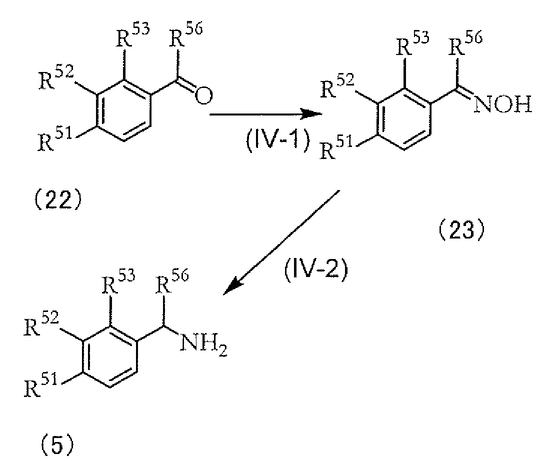 wherein, in the formula,
R⁵¹, R⁵², R⁵³, and R⁵⁶ represent same meaning as described above.

### Step(VI-1)

The compound (23) can be produced by making the compound (22) react with hydroxyl amine.

The reaction is usually carried out in the presence of a solvent.

The solvent used for the reaction includes ethers such as 1, 4-dioxane, tetrahydrofuran, ethylene glycol dimethyl ether, tert-butyl methyl ether and the like, aliphatic hydrocarbons such as hexane, heptane, octane and the like, aromatic hydrocarbons such as toluene, xylene and the like, halogenated hydrocarbons such as chlorobenzene, esters such as ethyl acetate, butyl acetate and the like, nitriles such as acetonitrile, butyronitrile and the like, acid amides such as N,N-dimethylformamide, sulfoxides such as dimethyl sulfoxide, alcohols such as methanol, ethanol, propanol, isopropanol and the like, water and the mixture thereof.

The molar ratio applied to hydroxyl amine is usually from 1 to 5 moles per 1 mole of the compound (22).

The reaction temperature is usually in the range of from 0 to 150 °C, and the reaction time is usually in the range of from 0.1 to 24 hours.

After completion of the reaction, the compound (23) can be isolated by subjecting to post-treatment such as the reaction mixture is poured into water, and extracted with organic solvent, then the organic layer is dried and concentrated. The isolated compound (23) can be purified by a technique such as chromatography, recrystallization and the like.

### Step(VI-2)

The compound (5) can be produced by making the compound (23) react with hydrogen in the presence of a hydrogenation catalyst.

The reaction is usually carried out under hydrogen atmosphere, and usually in the presence of a solvent.

The solvent used for the reaction includes, for example, alcohols such as methanol, ethanol, propanol and the like, esters such as ethyl acetate, butyl acetate and the like, ethers such as tetrahydrofuran, 1,4-dioxane and the like and the mixture thereof.

The hydrogenation catalyst used for the reaction includes, for example, transition metal compounds such as palladium charcoal, palladium hydroxide, Raney nickel, platinum oxide and the like.

The molar ratio applied to hydrogen is usually 2 moles per 1 mole of the compound (23) and that to the hydrogenation catalyst is usually from 0.001 to 0.5 moles.

The reaction is usually carried out under hydrogen atmosphere of from 1 to 100 atoms. The reaction can be carried out by added an acid (hydrochloric acid and the like), ifnecessary.

The reaction temperature is usually in the range of from -20 to 100°C, and the reaction time is usually in the range of from 0.1 to 24 hours.

After completion of the reaction, the compound (5) can be isolated by subjecting to post-treatment such as the reaction mixture is filtered, and the filtrate is extracted with organic solvent, then the obtained organic layer is dried and concentrated. The isolated compound (5) can be purified by a technique such as chromatography, recrystallization and the like.

Plant diseases against which the compound of the present invention has controlling activity include, for example, a disease due to Phycomycetes, specifically following diseases are illustrated:
*Peronospora brassicae* of vegetables and radishes; *Peronospora spinaciae* of spinach; *Peronospora tabacina* of tobacco; *Pseudoperonospora cubensis* of gourds; *Plasmopara* viticola of grapes; Phytophthora *cactorum* of apple, strawberry and ginseng; *Phytophora capsici* of tomato and cucumber; *Phytophthora cinnamomi* of pineapple; *Phytophthora infestans* of potato and tomato; *Phytophthora nicotianae var. nicotianae* of tobacco, broad bean and leek; *Pythium sp.* of spinach; *Pythium aphanidermatum* of cucumber; *Pythium sp.* of wheat; *Pythium debaryanum* of tobaco; and *Pythium rot* of soybean (*Pythium aphanidermatum, P. debaryanum, P. irregulare,* P. *myriotylum*, *P. ultimum*).

The compound of the present invention can control plant diseases by applying for plants or soils growing the plant as it is. But usually it is used a composition which contains the compound of the present invention and a carrier, namely it is a form of plant diseases controlling composition which the compound of the present invention is held by proper carrier. The plant diseases controlling composition of the present invention is a formulation such as emulsifiable concentrate, wettable powder, dry flowable, flowable, dust, granule and the like; obtained by mixing the compound of the present invention, and solid carrier, a liquid carrier, surfactant and other adjuvant for formulation. These formulations usually contain 0.1 to 90 % by weight of the compound of the present invention.

Solid carriers used for formulation include, for example, fine powders or granules of minerals such as kaolin clay, attapulgite clay, bentonite, montmorillonite, terra alba, pyrophyllite, talc, diatomaceous earth, calcite and the like; natural organic substancss such as corncob powder, walnut shell powder and the like; synthetic organic substances such as urea and the like; salts such as calcium carbonate, ammonium sulfate and the like; synthetic inorganic substances such as synthetic hydrous silicon oxide and the like. Liquid carriers include, for example, aromatic hydrocarbons such as xylene, alkylbenzene, methylnaphthalene and the like; alcohols such as 2-propanol, ethylene glycol, propylene glycol, cellosolve and the like; ketones such as acetone, cyclohexanone, isophorone and the like; vegetable oils such as soybean oil, cottonseed oil and the like; aliphatic hydrocarbons, esters, dimethylsulfoxide, acetonitrile and water.

Surfactants include, for example, anionic surfactants such as alkylsulfuric acid ester salt, alkylarylsulfonic acid salt, dialkylsulfosuccinic acid salt, polyoxyethylenealkylaryletherphosphoric acid ester salt, lignin sulfonic acid salt, polycondensed naphthalenesulfonateformaldehyde and the like; and nonionic surfactants such as polyoxyethylenealkylarylether, polyoxyethylenealkylpolyoxypropylene block copolymer, sorbitan fatty acid ester and the like.

Another adjuvant for formulation includes, for example, water-soluble polymers such as polyvinylalcohol, polyvinylpyrrolidone and the like; Arabian gum; alginic acid and its salt thereof; polysaccharides such as CMC (carboxymethylcellulose), xanthan gum and the like; inorganic substances such as alminum magnesium silicate, alumina sol and the like; and preservatives, colorants, PAP(isopropyl acidic phosphate), stabilizing agents such as BHT and the like.

By applying the plant diseases controlling composition of the present invention to treatment for foliage of plants, said plants can be protected from plant diseases; and by applying the plant diseases controlling composition of the present invention to treatment for soils, the plants grown on said soils can be protected from plant diseases.

When the plant diseases controlling composition of the present invention is applied to foliage treatment for plants or soil treatment, the application amount thereof, which may be varied with a kind of control-object plants, a kind of control-object disases, an infestation level of control-object diseases, formulation types, application timings, weather conditions and the like, is usually 1 to 5000 g, preferably 5 to 1000 g, of the compound of the present invention per 10,000 m².

Emulsifiable concentrate, wettable powder, flowable and the like are usually sprayed after diluted with water. In this case, the concentration of the compound of the present invention is usually in the range of from 0.0001 to 3 % by weight, preferably from 0.0005 to 1 % by weight. Dust, granule and the like are usually directly applied without dilution.

The plant diseases controlling composition of the present invention can be also applied in treatment methods such as seed disinfection and the like. The methods include, for example, a method to soak seeds of a plant in the plant diseases controlling composition of the present invention which prepared in 1 to 1,000 ppm in terms of concentration of the compound of the present invention, a method to spray or coat seeds of a plant with the liquid plant diseases controlling composition of the present invention which prepared in 1 to 1,000 ppm in terms of concentration of the compound of the present invention, and a method to coat seeds of a plant with the plant diseases controlling composition of the present invention which is formulated to dust.

The method for controlling plant diseases of the present invention is usually performed by applying effective amount of the composition of the present invention to a plant or a soil growing the plant in which infection is predictable and/or to a plant or a soil growing the plant in which infection is confirmed.

The plant diseases controlling composition of the present invention is usually used as an agent controlling plant diseases for agriculture or gardening, that is, as an agent controlling plant diseases to control plant diseases on plowed fields, paddy fields, orchards, tea fields, pastures, lawn and the like.

The plant diseases controlling composition of the present invention may be used together with other plant disease controlling compositions, pesticides, acaricides, nematicides, herbicides, plant growth regulators and/or fertilizers.

Examples of the active ingredient of such other plant diseases controlling composition include:
chlorothalonil; fluazinam; dichlofluanid; fosetyl-Al; cyclic imide derivatives such as captan, captafol, folpet and the like; dithiocarbamate derivatives such as maneb, mancozeb, thiuram, ziram, zineb, propineb and the like; inorganic or organic copper derivatives such as basic copper sulfate, basic copper chloride, copper hydroxide, copper-oxinate and the like; acylalanine derivatives such as metalaxyl, furalaxyl, ofurace, cyprofuram, benalaxyl, oxadixyl and the like; strobilurine like compound such as kresoxim-methyl, azoxystrobin, trifloxystrobin, picoxystrobin, pyraclostrobin, dimoxystrobin and the like; anilinopyrimidine derivatives such as cyprodinil, pyrimethanil, mepanipyrim and the like; phenyl pyrrole derivatives such as fenpiclonil, fludioxonil and the like; imide derivatives such as procymidone, iprodione, vinclozolin and the like; benzimidazole derivatives such as carbendazim, benomyl, thiabendazole, thiophanate methyl and the like; aminederivatives such as fenpropimorph, tridemorph, fenpropidin, spiroxamine and the like; azole derivatives such as propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol and the like; cymoxanil; dimethomorph; famoxyadone, fenamidone; iprovalicarb; benthiavalicarb; cyazofamid, zoxamide, ethaboxam; nicobifen; fenhexamid; quinoxyfen; diethof encarb and acibenzolar S-methyl.

Specific examples of the compound of the present invention are described below:
amide compounds shown by formula(i) to (xxxxxv)

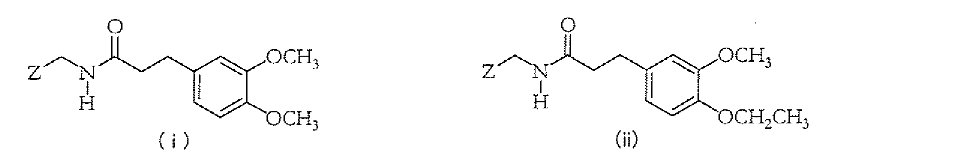

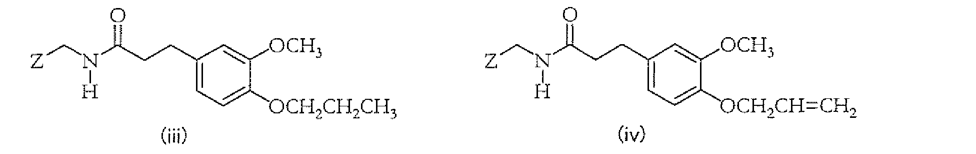

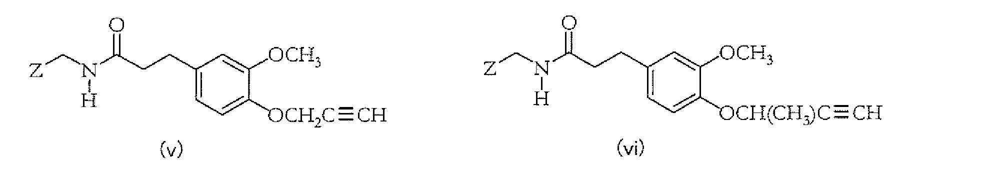

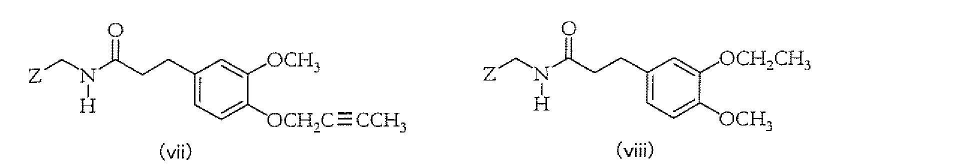

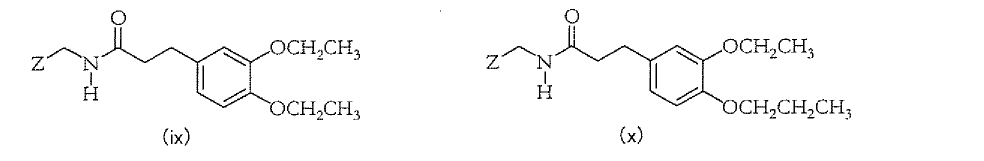

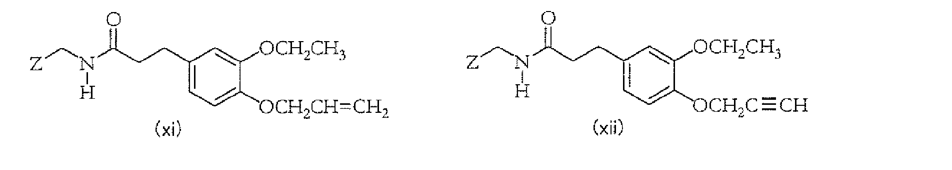

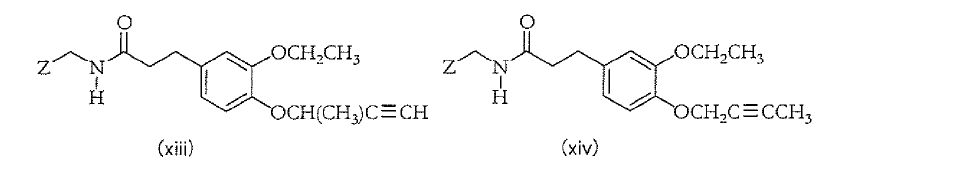

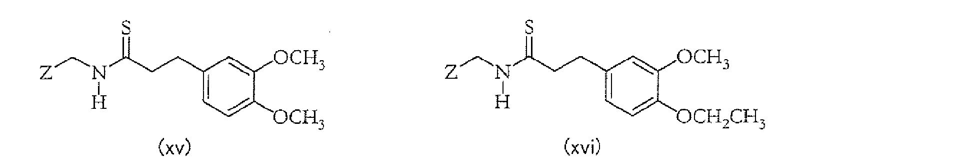

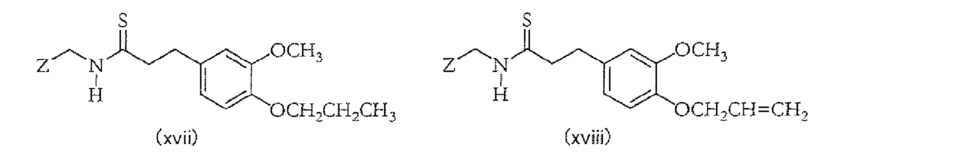

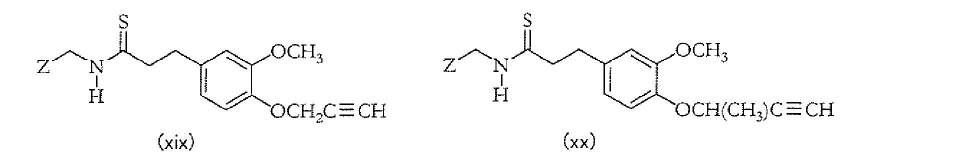

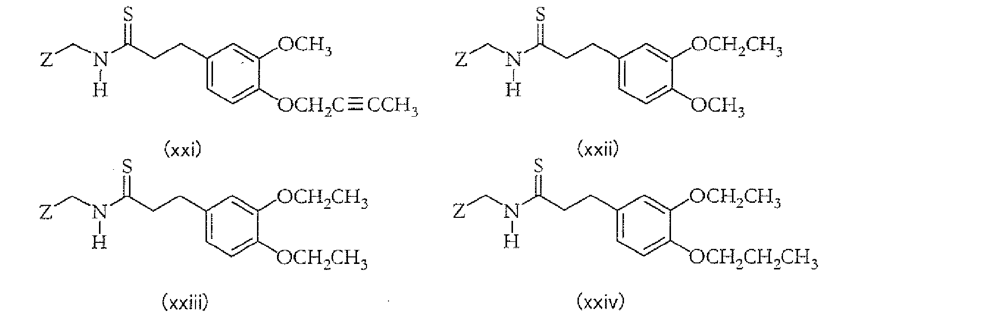

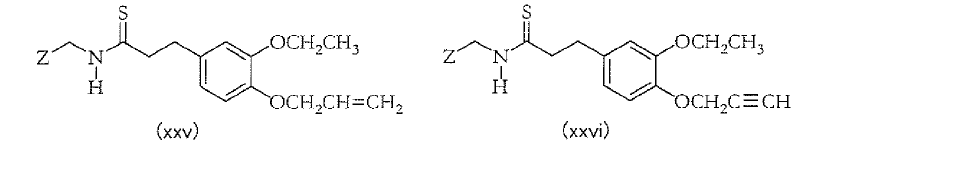

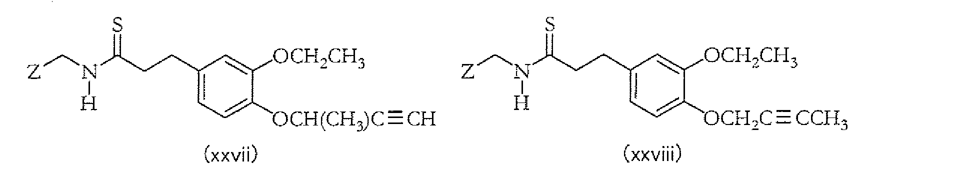

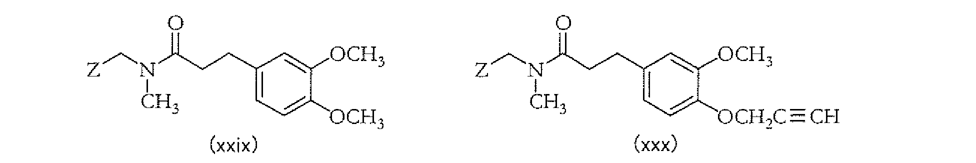

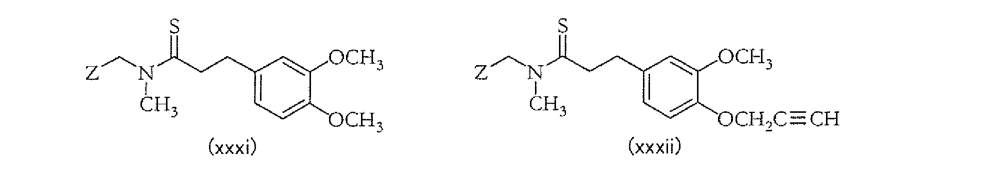

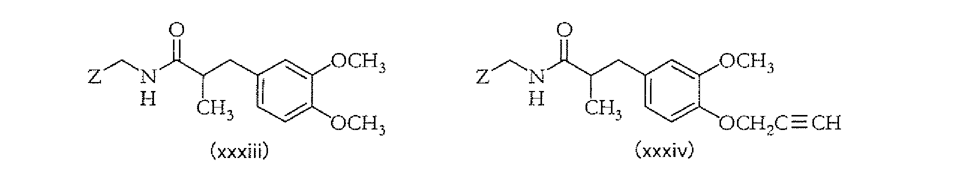

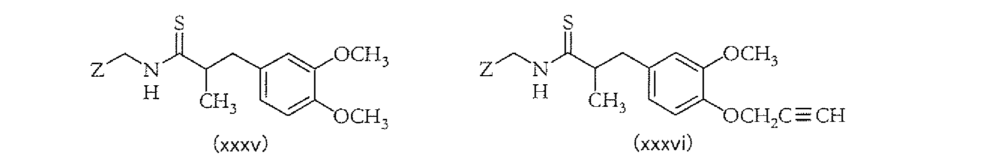

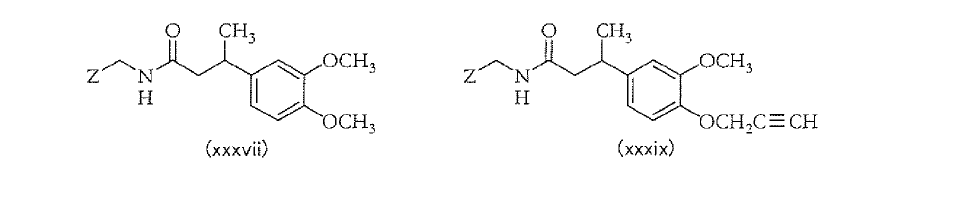

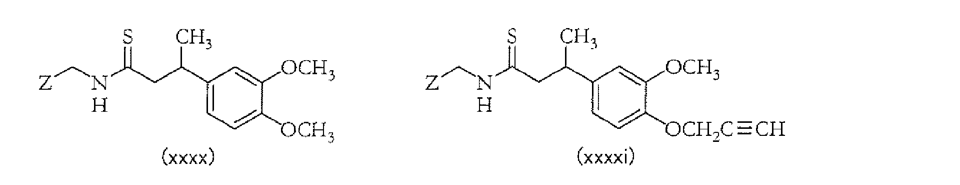

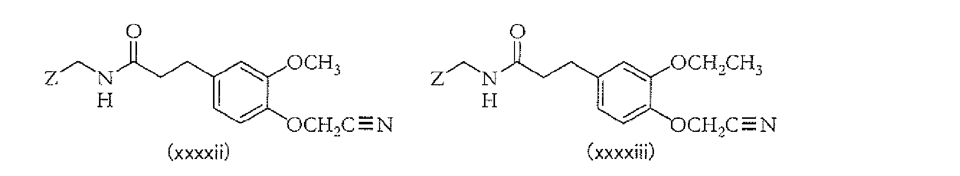

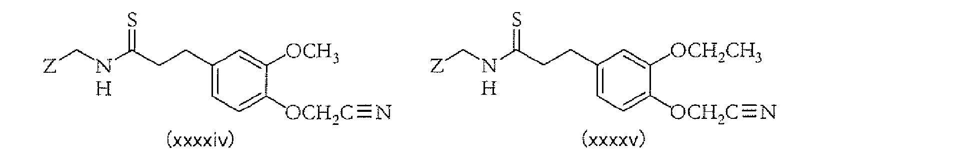

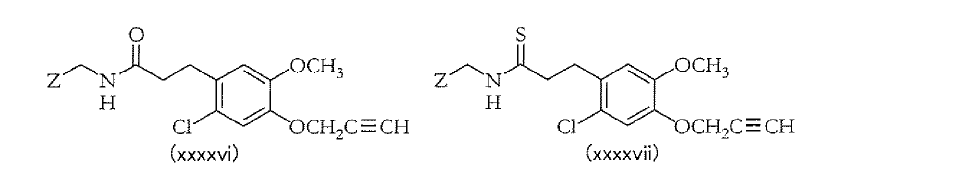

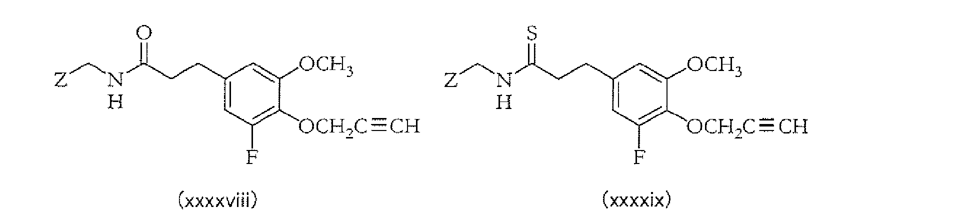

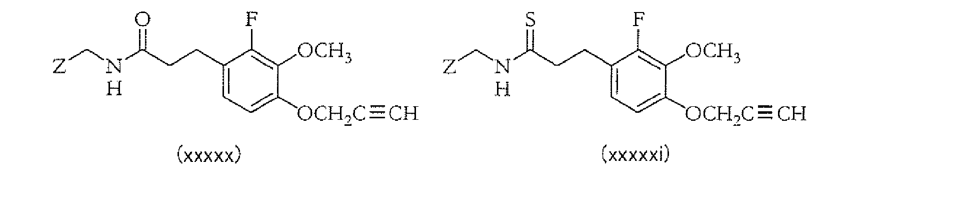

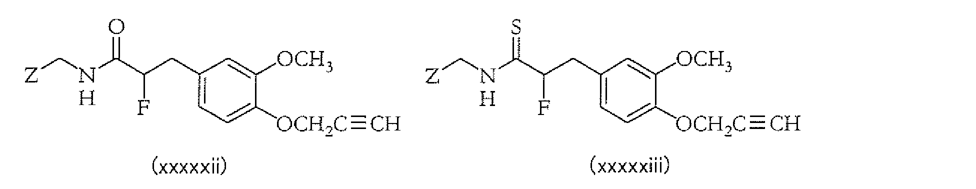

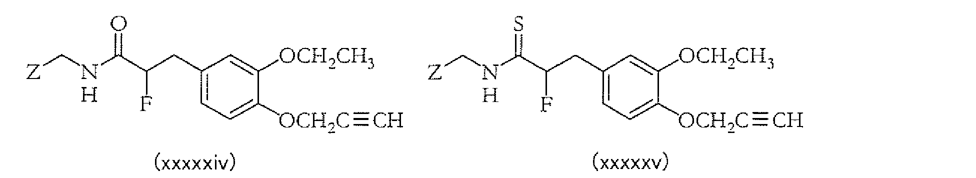

In formula (i) to (xxxxxv), Z represents any one of group below:
4-fluorophenylgroup,4-chlorophenyl group, 4-bromophenyl group, 4-iodophenyl group, 4-methylphenyl group, 4-ethylphenyl group, 4-propylphenyl group, 4-isopropylphenyl group, 4-butylphenyl group, 4-(sec-butyl)phenyl group, 4-isobutylphenyl group, 4-(tert-butyl)phenyl group, 4-vinylphenyl group, 4-(1-methylvinyl)phenyl group, 4-(1-propenyl)phenyl group, 4-(2-methyl-1-propenyl) phenyl group, 4-(1-butenyl) phenyl group, 4-ethynylphenyl group, 4-(1-propynyl)phenyl group, 4-(1-butynyl)phenyl group, 4-(1-pentanyl)phenyl group, 4-(3-methyl-1-butynyl)phenyl group, 4- (3, 3-dimethyl-1-butynyl)phenyl group, 4- (fluoromethyl) phenyl group, 4-(difluoromethyl) phenyl group, 4-(trifluoromethyl)phenyl group, 4-methoxyphenyl group, 4-ethoxyphenyl group, 4-(fluoromethoxy)phenyl group, 4-(difluoromethoxy)phenyl group, 4-(trifluoromethoxy)phenyl group, 4-cyanophenyl group, 4-(N,N-dimethylamino)phenyl group, 4- (N, N-diethylamino)phenyl group, 4-(N,N-dipropylamino)phenyl group, 3,4-difluorophenylgroup, 4-chloro-3-fluorophenyl group, 4-bromo-3-fluorophenyl group, 3-fluoro-4-methylphenyl group, 4-ethyl-3-fluorophenyl group, 3-fluoro-4-(trifluoromethyl)phenyl group, 3-fluoro-4-methoxyphenyl group, 4-cyano-3-fluorophenyl group, 4-fluoro-3-chlorophenyl group, 3,4-dichlorophenyl group, 4-bromo-3-chlorophenyl group, 3-chloro-4-methylphenyl group, 3-chloro-4-ethylphenyl group, 3-chloro-4-(trifluoromethyl)phenyl group, 3-chloro-4-methoxyphenyl group, 4-cyano-3-chlorophenyl group, 3-bromo-4-fluorophenyl group, 3-bromo-4-chlorophenyl group, 3,4-dibromophenyl group, 3-bromo-4-methylphenyl group, 3-bromo-4-ethylphenyl group, 3-bromo-4-(trifluoromethyl)phenyl group, 3-bromo-4-methoxyphenyl group, 3-bromo-4-cyanophenyl group, 4-fluoro-3-methylphenyl group, 4-chloro-3-methylphenyl group, 4-chloro-3-methylphenyl group, 3,4-dimethylphenyl group, 4-ethyl-3-methylphenyl group, 3-methyl-4-(trifluoromethyl)phenyl group, 4-methoxy-3-methylphenyl group, 4-cyano-3-methylphenyl group, 3-ethyl-4-fluoromethyl group, 4-chloro-3-ethylphenyl group, 4-bromo-3-ethylphenyl group, 3-ethyl-4-methylphenyl group, 3,4-diethylphenyl group, 3-ethyl-4-(trifluoromethyl)phenyl group, 3-ethyl-4-methoxyphenyl group, 4-cyano-3-ethylphenyl group, 4-fluoro-3-(trifluoromethyl)phenyl group, 4-chloro-3-(trifluoromethyl)phenyl group, 4-bromo-3-(trifluoromethyl)phenyl group, 4-methyl-3-(trifluoromethyl)phenyl group, 4-ethyl-3-(trifluoromethyl)phenyl group, 3,4-di-(trifluoromethyl)phenyl group, 4-methoxy-3-(trifluoromethyl)phenyl group, 4-cyano-3-(trifluoromethyl)phenyl group, 4-methoxycarbonylphenyl group, 4-ethoxycarbonylphenyl group, 4-phenylphenyl group, 3-fluoro-4-phenylphenyl group, 3-chloro-4-phenylphenyl group, 3-bromo-4-phenylphenyl group, 3-methyl-4-phenylphenyl group, 3-ethyl-4-phenylphenyl group, 4-phenoxyphenyl group, 3-fluoro-4-phenoxyphenyl group, 3-chloro-4-phenoxyphenyl group, 3-bromo-4-phenoxyphenyl group, 3-methyl-4-phenoxyphenyl group, 3-ethyl-4-phenoxyphenyl group, 4-nitrophenyl group, 3-fluoro-4-nitrophenyl group, 3-chloro-4-nitrophenyl group, 3-bromo-4-nitrophenyl group, 3-methyl-4-nitrophenyl group, 3-ethyl-4-nitrophenyl group, 4-methylthiophenyl group, 3-fluoro-4-methylthiophenyl group, 3-chloro-4-methylthiophenylgroup, 3-bromo-4-methylthiophenyl group, 3-methyl-4-methylthiophenyl group, 3-ethyl-4-methylthiophenyl group,
Indan-5-yl group, 5,6,7,8-tetrahydronaphthalene-2-yl group, 6,7,8,9-tetrahydro-5H-benzocycloheptene-2-yl group, 5,6,7,8,9,10-hexahydro-benzocyclooctene-2-yl group, 2-naphthyl group, 4-fluoronaphthalene-2-yl group, 5-fluoronaphthalene-2-yl group, 6-fluoronaphthalene-2-yl group, 7-fluoronaphthalene-2-yl group,4-chloronaphthalene-2-yl group, 5-chloronaphthalene-2-yl group, 6-chloronaphthalene-2-yl group, 7-chloronaphthalene-2-yl group, 4-bromonaphthalene-2-yl group, 5-bromonaphthalene-2-yl group, 6-bromonaphthalene-2-yl group, 7-bromonaphthalene-2-yl group, 4-methylnaphthalene-2-yl group, 5-methylnaphthalene-2-yl group, 6-methylnaphthalene-2-yl group, 7-methylnaphthalene-2-yl group, 4-methoxynaphthalene-2-yl group, 5-methoxynaphthalene-2-yl group, 6-methoxynaphthalene-2-yl group, 7-methoxynaphthalene-2-yl group, 4-trifluoromethylnaphthalene-2-yl group, 5-trifluoromethylnaphthalene-2-yl group, 6-trifluoromethylnaphthalene-2-yl group, 7-trifluoromethylnaphthalene-2-yl group, 5,6-difluoronaphthalene-2-yl group, 5,6-dichloronaphthalene-2-yl group, 5,6-dimethylnaphthalene-2-yl group, 5-fluoro-6-methylnaphthalene-2-yl group, 6-fluoro-5-methylnaphthalene-2-yl group, 5-chloro-6-methylnaphthalene-2-yl group, 6-chloro-5-methylnaphthalene-2-yl group, 6-chloro-5-fluoronaphthalene-2-yl group, 5-chloro-6-fluoronaphthalene-2-yl group, 4-(2-fluorovinyl)phenyl group, 4- (2-chlorovinyl) phenyl group, 4-(2-bromovinyl)phenyl group, 4-(2,2-difluorovinyl)phenyl group, 4-(2,2-dichlorovinyl)phenyl group, 4-(2,2-dibromovinyl)phenyl group, 4-(1-methyl-2,2-dichlorovinyl)phenyl group, 4-(1-methyl-2,2-dibromovinyl)phenyl group, 4-chloroethynylphenyl group, 4-bromoethynylphenyl group, 4-iodoethynylphenyl group, 4-methoxymethylphenyl group, 4-ethoxymethylphenyl group, 4-propyloxymethylphenyl group, 4-(1-methoxyethyl)phenyl group, 4-(2-methoxyethyl) phenyl group, 4-(2-methoxypropyl) phenyl group, 4-(2-methoxyisopropyl) phenyl group, 4-phenoxymethylphenyl group, 4-(1-phenoxyethyl)phenyl group, 4-(2-phenoxyethyl)phenyl group, 4-(1-phenoxypropyl)phenyl group, 4-(3-phenoxypropyl)phenyl group, 4- (4-phenoxybutyl) phenylgroup, 4- (hydroxymethyl) phenyl group, 4-(1-hydroxyethyl)phenyl group, 4-(2-hydroxyethyl)phenyl group, 4-(1-hydroxypropyl)phenyl group, 4-(1-hydroxybutyl)phenyl group, 4-methylsulfonyloxymethyl)phenyl group, 4-ethylsultonyloxymethyl)phenyl group, 4-(1-methylsulfonyloxyethyl)phenyl group, 4-(2-sulfonyloxyethyl)phenyl group, 4-(1-methylsulfonyloxypropyl)phenyl group, 4-carbonylphenyl group, 4-methylcarbonylphenyl group, 4-ethylcarbonylphenyl group, 4-propylcarbonylphenylgroup, 4-isopropylcarbonylphenyl group, 4-methoxyiminomethylphenyl group, 4-(1-methoxyiminoethyl)phenyl group, 4-(1-methoxyiminopropyl)phenyl group, 4-(1-methoxyiminobutyl)phenyl group, 4-ethoxyiminomethylphenylgroup, 4-(1-ethoxyiminoethyl)phenyl group, 4-propoxyiminomethylphenyl group, 4-(1-isopropoxyiminoethyl)phenyl group, 4-(butoxyiminomethyl)phenyl group, 4-pentyloxyiminomethylphenyl group, 4-(1-hexyloxyiminoethyl)phenyl group, 4-benzyloxyiminomethylphenyl group, 4-(1-benzyloxyiminoethyl)phenyl group, 4-(1-benzyloxyiminopropyl)phenyl group, 4-(1-benzyloxyiminobutyl)phenyl group, 4-dimethylaminoiminomethylphenyl group, 4-(1-dimethylaminoiminoethyl)phenyl group, 4-diethylaminoiminomethylphenyl group, 4-(1-diethylaminoiminoethyl)phenyl group, 4-trimethylsilylphenyl group, 4-triethylsilylphenyl group, 4-tert-butyldimethylsilylphenyl group.

The present invention is further illustrated in detail with production examples, formulation examples, test examples and other manner, but should not be limited as follows.

At first, the production examples of the compound of the present invention are illustrated as follows.

### Production Example 1

200 mg of 3-{3-methoxy-4-(2-propynyloxy)phenyl}propionyl chloride, 112 mg of 4-chlorobenzylamine, 0.17 ml of triethylamine and 5 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 212 mg of N-(4-chlorobenzyl)-3-{3-methoxy-4-(2-propynyloxy)phenyl}pro panamide (referred as the compound of the present invention 1 hereinafter).
the compound of the present invention 1 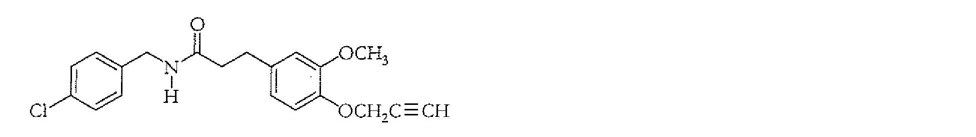 ¹H-NMR (CDCl₃, TMS) δ (ppm) : 7.25-7.27 (2H, m) , 7.05 (2H, d, J=8.2 Hz), 6.94 (1H, d, J=8.0 Hz), 6.71-6.74 (2H, m), 5.59 (1H, br.s), 4.73 (2H, d, J=2.5 Hz), 4.36 (2H, d, J=5.9 Hz), 3.82 (3H, s), 2.94 (2H, t, J=7.5 Hz), 2.45-2.52 (3H, m)

### Production Example 2

By using 200 mg of 3-{3-methoxy-4-{2-propynyloxy}phenyl}propionyl chloride and 99 mg of 4-methylbenzylamine according to the Production Example 1 was obtained 204 mg of N-(4-methylbenzyl)-3-{3-methoxy-4-(2-propynyloxy)phenyl}pro panamide (referred as the compound of the present invention 2 hereinafter).
the compound of the present invention 2 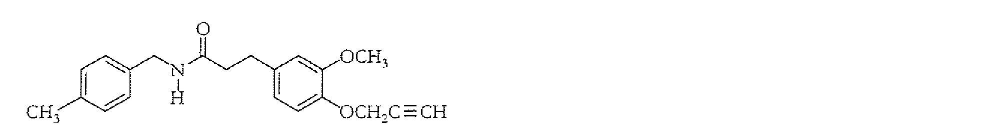 ¹H-NMR (CDCl₃, TMS) 5 (ppm): 7.11 (2H, d, J=8.0 Hz), 7.05 (2H, d, J=8.0 Hz), 6.94 (1H, d, J=8.0 Hz), 6.71-6.75 (2H, m), 5.53 (1H, br.s), 4.73 (2H, d, J=2. 4 Hz), 4.36 (2H, d, J=5. 5 Hz), 3.82 (3H, s), 2.94 (2H, t, J=7.5 Hz), 2.46-2.50 (3H, m), 2.32 (3H, s)

### Production Example 3

By using 200 mg of 3-(3-methoxy-4-(2-propynyloxy) phenyl)propionyl chloride and 99 mg of 4-fluorobenzylamine according to the Production Example 1 was obtained 0.13 g of N-(4-fluorobenzyl)-3-{3-methoxy-4-(2-propynyloxy)phenyl}pro panamide (referred as the compound of the present invention 3 hereinafter).
the compound of the present invention 3 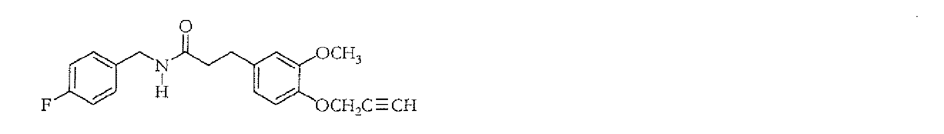 ¹H-NMR (CDCl₃, TMS) δ (ppm): 7.09-7.23 (2H, m), 6.93-7.00 (3H, m), 6.71-6.75 (2H, m), 5.57 (1H, br.s), 4.73 (2H, d, J=2. 4 Hz), 4.36 (2H, d, J=5.8 Hz), 3.82 (3H, s), 2.94 (2H, t, J=7.5 Hz), 2.48-2.52 (3H, m)

### Production Example 4

200 mg of 3-{3-methoxy-4-(2-propynyloxy)phenyl}propionyl chloride, 176 mg of 4 -bromobenzylamine hydrochloride, 0.29 ml of triethylamine and 5 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 117 mg of N-(4-bromobenzyl)-3-{3-methoxy-4-(2-propynyloxy)phenyl}prop anamide (referred as the compound of the present invention 4 hereinafter).
the compound of the present invention 4 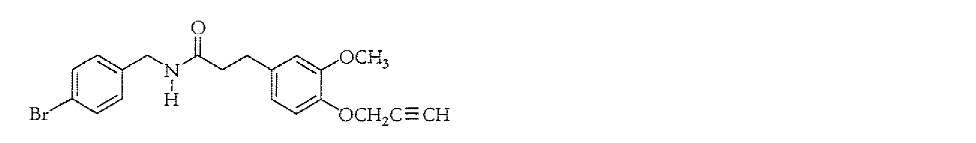 ¹H-NMR (CDCl₃, TMS) δ (ppm): 7.41 (2H, d, J=8 Hz), 7.00 (2H, d, J=8 Hz), 6.94 (1H, d, J=8.0 Hz), 6.71-6.74 (2H, m), 5.59 (1H, br.s), 4.74 (2H, d, J=2.4 Hz) , 4.34 (2H, d, J=5.8Hz) , 3.82 (3H, s), 2.94 (2H, t, J=7.5 Hz), 2.49-2.52 (3H, m)

### Production Example 5

300 mg of 3-{3-methoxy-4-(2-propynyloxy)phenyl}-propionyl chloride, 163 mg of 4-methoxybenzylamine, 0.25 ml of triethylamine and 10 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 280 mg of N-(4-methoxybenzyl)-3-{3-methoxy-4-(2-propynyloxy)phenyl}pr opanamide (referred as the compound of the present invention 5 hereinafter).
the compound of the present invention 5 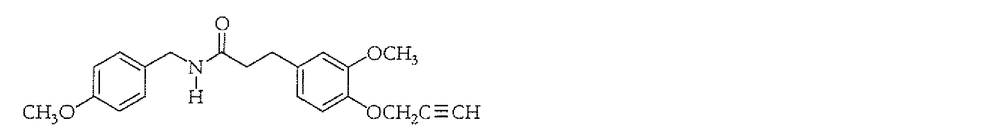 ¹H-NMR (CDCl₃, TMS) δ (ppm): 7.08 (2H, d, J=8.5 Hz), 6.93 (1H, d, J=8.0 Hz), 6.83 (2H, d, J=8.5 Hz), 6.71-6.74 (2H, m), 5.54 (1H, br.s), 4.74 (2H, d, J=2.5 Hz), 4.33 (2H, d, J=5. 6 Hz), 3.82 (3H, s), 3.79 (3H, s), 2.94 (2H, t, J=7.8 Hz), 2.46-2.49 (3H, m)

### Production Example 6

By using 300 mg of 3-{3-methoxy-4-(2-propynyloxy)phenyl}propionyl chloride and 208 mg of 4-(trifluoromethyl)benzylamine according to the Production Example 5 was obtained 208 mg of N-{(4-trifluoromethyl)benzyl}-3-{3-methoxy-4-(2-propynyloxy )phenyl}propanamide (referred as the compound of the present invention 6 hereinafter).
the compound of the present invention 6 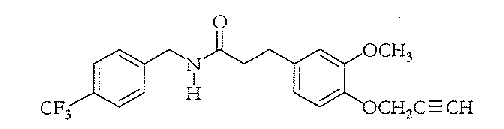 ¹H-NMR (CDCl₃, TMS) δ (ppm) : 7.54 (2H, d, J=8.0 Hz), 7.23 (1H, d, J=8.2 Hz), 6.94 (2H, d, J=8.0 Hz), 6.71-6.75 (2H, m), 5.68 (1H, br. s), 4.73 (2H, d, J=2. 4 Hz), 4.51 (2H, d, J=6.1 Hz), 3.81 (3H, s), 2.36 (2H, t, J=7 Hz), 2.53 (2H, t, J=7 Hz), 2.49 (2H, t, J=2.4 Hz)

### Production Example 7

By using 300 mg of 3-{3-methoxy-4-(2-propynyloxy)phenyl}propionyl chloride and 209 mg of 3,4-dichlorobenzylamine according to the Production Example 5 was obtained 430 mg of N-(3,4-difluoxobenzyl)-3-{3-methoxy-4-(2-propynyloxy)phenyl }propanamide (referred as the compound of the present invention 7 hereinafter).
the compound of the present invention 7 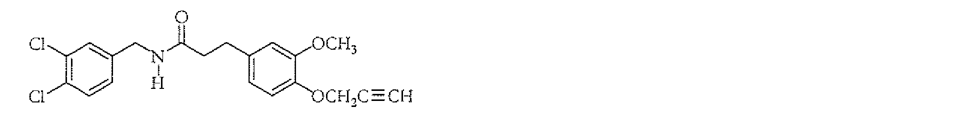 ¹H-NMR (CDCl₃, TMS) 5 (ppm): 7.35 (1H, d, J=8.2 Hz), 7.28 (1H, d, J=1.9 Hz), 6.93-6.99 (2H, m), 6.71-6.74 (2H, m), 5.64 (1H, br.s), 4.73 (2H, d, J=2.4 Hz), 4.34 (2H, d, J=6.1 Hz), 3.83 (3H, s), 2.95 (2H, t, J=7.5 Hz), 2.48-2.54 (3H, m)

### Production Example 8

By using 300 mg of 3-{3-methoxy-4-(2-propynyloxy)phenyl}propionyl chloride and 194 mg of 4-tert-butylbenzylamine according to the Production Example 5 was obtained 511 mg of N-(4-tert-butylbenzyl)-3-{3-methoxy-4-(2-propynyloxy)phenyl }propanamide (referred as the compound of the present invention 8 hereinafter).
the compound of the present invention 8 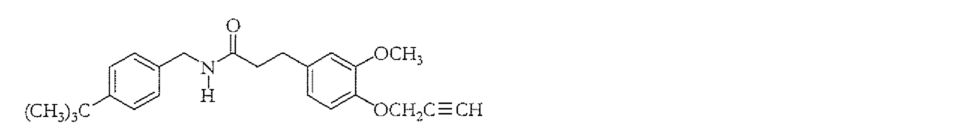 ¹H-NMR (CDCl₃, TMS) δ (ppm): 7.33 (2H, d, J=8.5 Hz), 7.10 (2H, d, J=8.5 Hz), 6.94 (1H, d, J=8.0 Hz), 6.71-6.75 (2H, m), 5.67 (1H, br.s), 4.73 (2H, d, J=2. 4 Hz), 4.37 (2H, d, J=5. 8 Hz), 3. 82 (3H, s), 2.95 (2H, t, J=7.5 Hz), 2.46-2.50 (3H, m), 1.30 (9H, s)

### Production Example 9

By using 300 mg of 3-{3-methoxy-4-(2-propynyloxy)phenyl}propionyl chloride and 227 mg of 4-(trifluoromethoxy)benzylamine according to the Production Example 5 was obtained 209 mg of N-{4-(trifluoromethoxy)benzyl}-3-{3-methoxy-4-(2-propynylox y)phenyl}propanamide (referred as the compound of the present invention 9 hereinafter).
the compound of the present invention 9 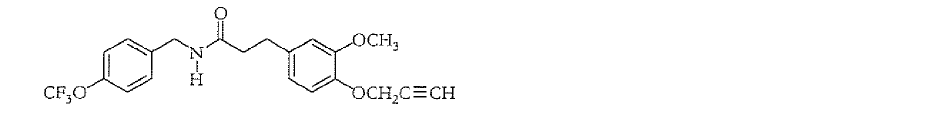 ¹H-NMR (CDCl₃, TMS) δ (ppm) : 7.12-7.17 (4H, m), 6.95 (1H, d, J=8.0 Hz), 6.71-6.76 (2H, m), 5. 63 (1H, br.s), 4.74 (2H, d, J=2.4 Hz), 4.40 (2H, d, J=5.9 Hz), 3.83 (3H, s), 2.95 (2H, t, J=7.6 Hz), 2.48-2.53 (3H, m)

### Production Example 10

By using 0.20 g of 3-{3-methoxy-4-(2-propynyloxy)phenyl}propionyl chloride, 0.27 g of 4-dimethylaminobenzylamine hydrochloride and 0.28 ml of triethylamine according to the Production Example 5 was obtained 0.40 g of N-(4-dimethylaminobenzyl)-3-{3-methoxy-4-(2-propynyloxy)phe nyl}propanamide (referred as the compound of the present invention 10 hereinafter).
the compound of the present invention 10 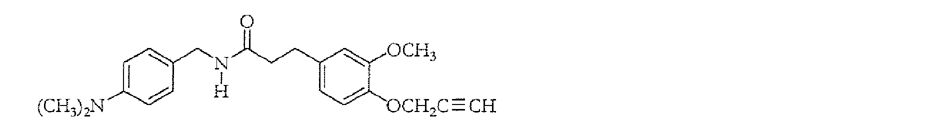 ¹H-NMR (CDCl₃, TMS) δ (ppm) : 7.03-7.06 (2H, m), 6. 93 (1H, d, J=8.0 Hz), 6.65-6.76 (4H, m), 5.46 (1H, br.s), 4.72 (2H, d, J=2.4 Hz), 4.29 (2H, d, J=5.5 Hz), 3.83 (3H, s) , 2.89-2.95 (8H, m), 2.43-2.49 (3H, m)

### Production Example 11

0.81 g of 3-{3-ethoxy-4-(2-propynyloxy)phenyl}propionyl chloride, 0.46 g of 4-chlorobenzylamine, 0.64 ml of triethylamine and 10 ml of tetrahydrofuran were mixed, and stirred at room temperature for 20 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.79 g of N-(4-chlorobenzyl)-3-{3-ethoxy-4-(2-propynyloxy)phenyl}prop anamide (referred as the compound of the present invention 11 hereinafter).
the compound of the present invention 11 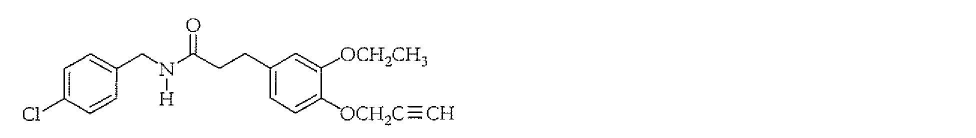 ¹H-NMR (CDCl₃, TMS) δ (ppm): 7.24-7.26 (2H, m), 7.04-7.06 (2H, m), 6.95 (1H, d, J=8.0 Hz), 6.70-6.74 (2H, m), 5.60 (1H, br.s), 4.74 (2H, d, J=2.4 Hz), 4.35 (2H, d, J=5.8 Hz), 4.02 (2H, q, J=7 Hz), 2.92 (2H, t, J=7.5 Hz), 2.27-2.51 (3H, m), 1.42 (3H, t, J=7 Hz)

### Production Example 12

By using 0.30 g of 3-{3-methoxy-4-(2-propynyloxy)phenyl}propionylchloride 0.19 g of (naphthalene-2-yl)methylamine and 0.5ml of triethylamine according to the Production Example 11 was obtained 0.25 g of N-{ (naphthalene-2-yl)methyl}-3-{3-methoxy-4-(2-propynyloxy) phenyl}propanamide (referred as the compound of the present invention 12 hereinafter).
the compound of the present invention 12 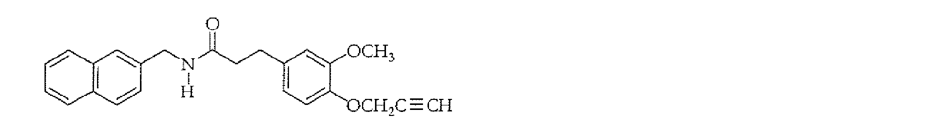 ¹H-NMR (CDCl₃, TMS) δ (ppm) : 7.76-7.82 (3H, m), 7.60 (1H, s), 7.44-7.49 (2H, m), 7.29-7.29 (1H, m), 6.89-6.90 (1H, m), 6.71-6.95 (2H, m), 5.74 (1H, br.s), 4.69 (2H, d, J=2.2 Hz), 4.55 (2H, d, J=5.9 Hz), 3.78 (3H, s), 2.96 (2H, t, J=7.6 Hz), 2.46-2.54 (3H, m)

### Production Example 13

By using 0.30 g of 3-(3-methoxy-4-(2-propynyloxy) phenyl)propionyl chloride, 0.23 g of (5,6,7,8-tetrahydronaphthalene-2-yl)methylamine hydrochloride and 0.42ml of triethylamine according to the Production Example 11 was obtained 0.41 g of N-{(5,6,7,8tetrahydronaphthalene-2-yl)methyl}-3-{3-methoxy-4-(2-propynyloxy)phenyl}propanamide (referred as the compound of the present invention 13 hereinafter).
the compound of the present invention 13 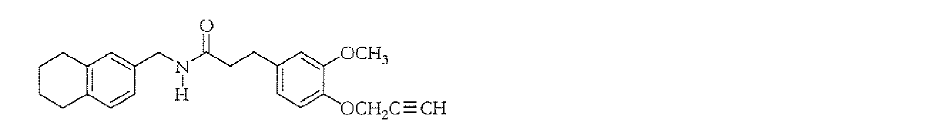 ¹H-NMR (CDCl₃, TMS) δ (ppm) : 6.89-7.02 (4H, m), 6.71-6.76 (2H, m), 5.52 (1H, br.s), 4.73 (2H, d, J=2.5 Hz), 4.33 (2H, d, J=5.4 Hz), 3.83 (3H, s), 2.92 (2H, t, J=7.8 Hz), 2.49-2.72 (4H, m), 2.45-2.49 (3H, m), 1.76-1.79 (4H, m)

### Production Example 14

By using 0.30 g of 3-{3-methoxy-4-(2-propynyloxy)phenyl}propionyl chloride and 0.16 g of 4-ethylbenzylamine according to the Production Example 5 was obtained 0.40 g of N-(4-ethylbenzyl)-3-{3-methoxy-4-(2-propynyloxy)phenyl}prop anamide (referred as the compound of the present invention 14 hereinafter).
the compound of the present invention 14 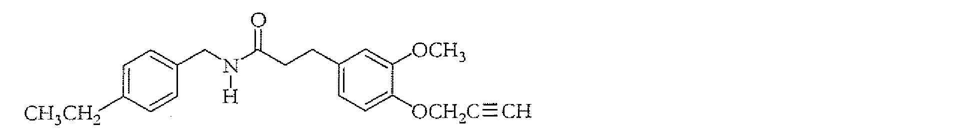 ¹H-NMR (CDCl₃, TMS) δ (ppm): 7.08-7.15 (4H, m) , 6.94 (1H, d, J=8.1 Hz), 6. 72-6. 75 (2H, m), 5.55 (1H, br.s), 4.73 (2H, d, J=2.2 Hz), 4.37 (2H, d, J=5.6 Hz), 3.82 (3H, s), 2.95 (2H, t, J=7.3 Hz), 2.63 (2H, q, J=7 Hz), 2.47-2.50 (3H, m), 1.22 (3H, t, J=7 Hz)

### Production Example 15

1.1 g of 3-{3-methoxy-4-(2-propynyloxy)phenyl}propionyl chloride, 1.0 g of 4-iodobenzylamine, 0.98 ml of triethylamine and 20 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magneium sulfate, and concentrated under reduced pressure. The residue was washed by hexane to obtain 1.5 g of N-(4-iodobenzyl)-3-{3-methoxy-4-(2-propynyloxy)phenyl}propa neamide (referred as the compound of the present invention 15 hereinafter).
the compound of the present invention 15 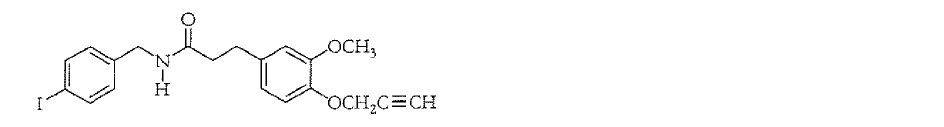 ¹H-NMR (CDCl₃, TMS) δ (ppm) : 7.60-7.62 (2H, m), 6. 93 (1H, d, J=8 .0 Hz), 6.86-6.89 (2H, m), 6.70-6.74 (2H, m), 5. 61 (1H, br.s), 4.77 (2H, d, J=2.5 Hz), 4.32 (2H, d, J=6.1 Hz), 3.82 (3H, s), 2.94 (2H, t, J=7.2 Hz), 2.48-2.52 (3H, m)

### Production Example 16

0.50 g of 3-{3-methoxy-4-(2-propynyloxy)phenyl}propionylchloride 0.32 g of 4-butylbenzylamine, 0.41 ml of triethylamine and 15 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 0.60 g of N- (4-butylbenzyl) -3-{3-methoxy-4- (2-propynyloxy) phenyl}prop anamide (referred as the compound of the present invention 16 hereinafter).
the compound of the present invention 16 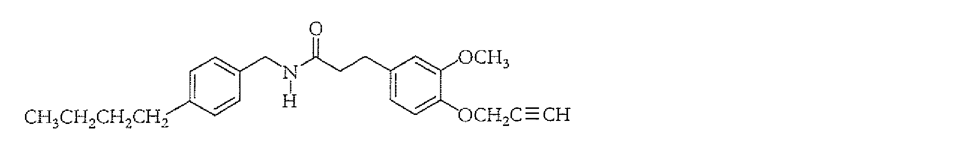 ¹H-NMR (CDCl₃, TMS) δ (ppm) : 7.06-7.12 (4H, m), 6.94 (1H, d, J=8.2 Hz), 6.71-6.75 (2H, m), 5.59 (1H, br.s), 4. 73 (2H, d, J=2.4 Hz), 4.36 (2H, d, J=5.5 Hz), 3.82 (3H, s), 2.95 (2H, t, J=7.5 Hz), 2.58 (2H, t, J=7.5 Hz), 2.46-2.50 (3H, m), 2.46-2.50 (3H, m), 1.53-1.61 (2H, m), 1.30-1.39 (2H, m), 0.92 (3H, t, J=7.3 Hz)

### Production Example 17

By using 0.30 g of 3-{3-methoxy-4-(2-propynyloxy)phenyl}propionyl chloride, 0.17 g of 3-fluoro-4-methylbenzylamine and 0.25ml of triethylamine according to the Production Example 11 was obtained 0.34 g of N-(3-fluoro-4-methylbenzyl)-3-{3-methoxy-4-(2-propynyloxy)p henyl}propanamide (referred as the compound of the present invention 17 hereinafter).
the compound of the present invention 17 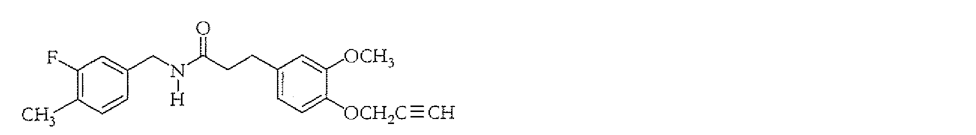 ¹H-NMR (CDCl₃, TMS) δ (ppm): 7.09 (1H, t, J=7.8 Hz), 6.94 (1H, d, J=8.0 Hz), 6.70-6.83 (4H, m), 5.58 (1H, br.s), 4.73 (2H, d, J=2.2 Hz), 4.35 (2H, d, J=5.8 Hz), 3.83 (3H, s), 2.95 (2H, t, J=7.2 Hz), 2.48-2.53 (3H, m), 2.24 (3H, s)

### Production Example 18

0.50 g of 3-{3-methoxy-4-(2-propynyloxy)phenyl}proplonylchloride, 0.50 g of 3-fluoro-4-(trifluoromethyl)benzylamine, 0.41 ml of triethylamine and 10 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 0.73 g of N-{3-fluoro-4-(trifluoromethyl)benzyl}-3-{3-methoxy-4-(2-pr opynyloxy)phenyl}propanamide (referred as the compound of the present invention 18 hereinafter).
the compound of the present invention 18 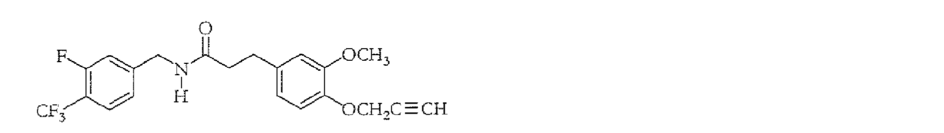 ¹H-NMR (CDCl₃, TMS) δ (ppm): 7.51 (1H, t, J=7.7 Hz), 6.94-7.01 (3H, m), 6.72-6.74 (2H, m), 5.71 (1H, br.s), 4.73 (2H, d, J=2.4 Hz), 4.43 (2H, d, J=6.3 Hz), 3.83 (3H, s), 2.96 (2H, t, J=7.5 Hz), 2.55 (2H, t, J=7.5 Hz), 2.48 (1H, t, J=2.4 Hz)

### Production Example 19

By using 0.5 g of 3-{3-methoxy-4-(2-propynyloxy)phenyl}propionyl chloride, 0.42 g of 4-chloro-3-(trifluoromethyl)benzylamine and 0.42 ml of triethylamine according to the Production Example 18 was obtained 0.76 g of N-{4-chloro-3-(trifluoromethyl)benzyl}}-3-{3-methoxy-4-(2-p ropynyloxy)phenyl}propanamide (referred as the compound of the present invention 19 hereinafter).
the compound of the present invention 19 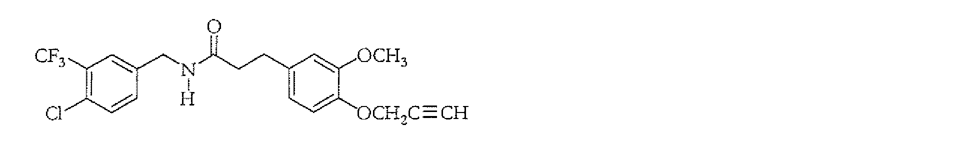 ¹H-NMR (CDCl₃, TMS) 5 (ppm): 7.53 (1H, d, J=2.2 Hz), 7.42 (1H, d, J=8.2 Hz), 7.20-7.22 (1H, m), 6.94 (1H, d, J=7.8Hz), 6.71-6.74 (2H, m), 5.69 (1H, br.s), 4.73 (2H, d, J=2.4 Hz), 4.41 (2H, d, J=5.8 Hz), 3.82 (3H, s), 2.95 (2H, t, J=7.5 Hz), 2.53 (2H, t, J=7.6 Hz), 2.49 (2H, t, J=2.4 Hz)

### Production Example 20

0.50 g of 3-{3-methoxy-4-(2-propynyloxy)phenyl}propionyl chloride, 0.36 g of (indane-5-yl) methylamine hydrochloride, 0.8 ml of triethylamine and 20 ml of tetrahydrofuranweremixed, and stirred at room temperature for 20 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 0.34 g of N-{ (indan-5-yl)methyl}-3-{3-methoxy-4-(2-propynyloxy)phenyl }propanamide (referred as the compound of the present invention 20 hereinafter).
the compound of the present invention 20 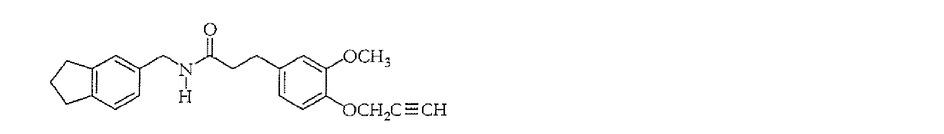 ¹H-NMR (CDCl₃, TMS) δ (ppm): 6.71-7.16 (6H, m), 5.54 (1H, br. s), 4.72 (2H, m), 4.36 (2H, d, J=4.6 Hz), 3.82 (3H, s), 2.65-2.94 (6H, m), 2.48 (3H, m), 2.04-2.08 (2H, m)

### Production Example 21

By using 0.30 g of 3-(3,4-dimethoxyphenyl)propionyl chloride and 0.19 g of 4-chlorobenzylamine according to the Production Example 5 was obtained 0.32 g of N-(4-chlorobenzyl)-3-(3,4-dimethoxyphenyl)propanamide (referred as the compound of the present invention 21 hereinafter).
the compound of the present invention 21 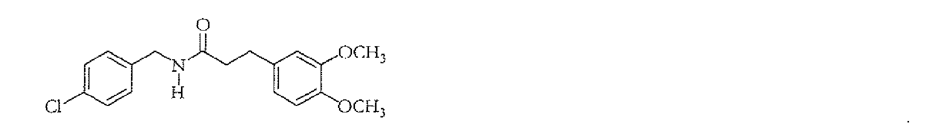 ¹H-NMR (CDCl₃, TMS) δ (ppm): 7.24-7.27 (2H, m), 7.05-7.07 (2H, m), 6.70-6.77 (3H, m), 5.61 (1H, br.s), 4.36 (2H, d, J=5.9 Hz), 3.86 (3H, s), 3.84 (3H, s), 2.94 (2H, t, J=7.6 Hz), 2.50 (2H, t, J=7.6 Hz)

### Production Example 22

0.30 g of 3-(3,4-dimethoxyphenyl)propionyl chloride, 0.25 g of C-(5,5,7,8-tetrahydronaphthalene-2-yl)methylamine hydrochloride, 0.40 ml of triethylamine and 10 ml of tetrahydrofuran were mixed, and stirred at room temperature for 20 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 0.35 g of N-{ (5,6,7,8tetrahydronaphthalene-2-yl)methyl}-3-(3,4-dimeth oxyphenyl)propanamide (referred as the compound of the present invention 22 hereinafter).
the compound of the present invention 22 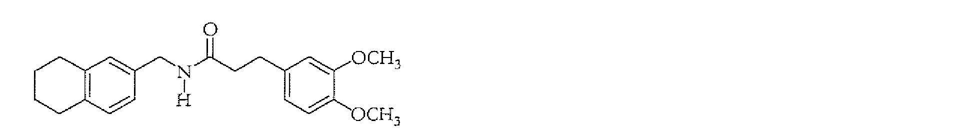 ¹H-NMP, (CDCl₃, TMS) δ (ppm): 7.00 (1H, d, J=8.3 Hz), 6.89 (2H, m), 6.72-6.79 (3H, m), 5.51 (1H, br.s), 4.33 (2H, d, J=5.4 Hz), 3.85 (3H, s), 3.84 (3H, s), 2.94 (2H, t, J=7.6 Hz), 2.73 (4H, m), 2.47 (2H, t, J=7.6 Hz), 1.78 (4H, m)

### Production Example 23

300 mg of N-(4-methylbenzyl)-3-(4-hydroxy-3-methoxyphenyl)propanamide, 0.075ml g of methyl iodide, 0.21 g of potassium carbonate and 5 ml of N,N-dimethylformamide were mixed, and stirred at room temperature for 6 hours. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 226 mg of N-(4-methylbenzyl)-3-(3,4-dimethoxyphenyl)propanamide (referred as the compound of the present invention 23 hereinafter).
the compound of the present invention 23 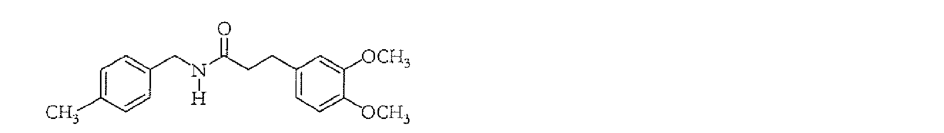 ¹H-NMR (CDCl₃, TMS) δ (ppm) : 7.10 (2H, d, J=8.0 Hz), 7.05 (2H, d, J=8.0 Hz), 6.70-6.78 (3H, m), 5.53 (1H, br.s), 4.35 (2H, d, J=5.6 Hz), 3.85 (3H, s), 3.83 (3H, s), 2.94 (2H, t, J=7.5 Hz), 2.48 (2H, t, J=7.7 Hz), 2.32 (3H, s)

### Production Example 24

By using 300 mg of N-(4-methylbenzyl)-3-(4-hydroxy-3-methoxyphenyl)propanamide and 0.1 ml of ethyl iodide according to the Production Example 23 was obtained 225 mg of N-(4-methylbenzyl)-3-(4-ethoxy-3-methoxyphenyl)propanamide (referred as the compound of the present invention 24 hereinafter).
the compound of the present invention 24 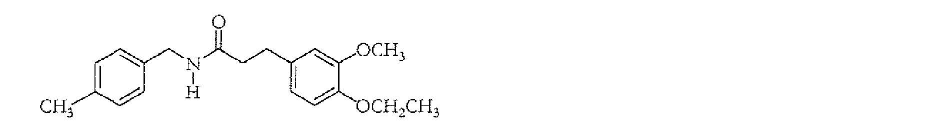 ¹H-NMR (CDCl₃, TMS) δ (ppm): 7.10 (2H, d, J=8.0 Hz), 7.05 (2H, d, J=8.0 Hz), 6.68-6.78 (3H, m), 5.53 (1H, br.s), 4.35 (2H, d, J=5.5 Hz)4.07 (2H, q, J=7.0 Hz), 3.80 (3H, s), 2.93 (2H, t, J=7.5 Hz), 2.48 (2H, t, J=7.7 Hz), 2.32 (3H, s), 1.45 (2H, t, J=7.0 Hz)

### Production Example 25

By using 300 mg of N-(4-methylbenzyl)-3-(4-hydroxy-3-methoxyphenyl)propanamide and 0.12 ml of propyl iodide according to the Production Example 23 was obtained 262 mg of N-(4-methylbenzyl)-3-(3-methoxy-4-prppoxyphenyl)propanamide (referred as the compound of the present invention 25 hereinafter).
the compound of the present invention 25 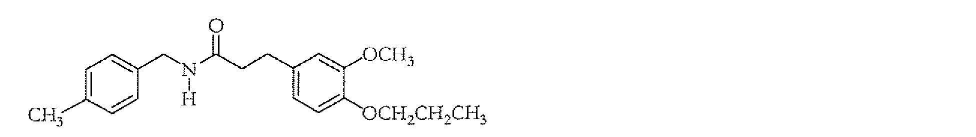 ¹H-NMR (CDCl₃, TMS) δ (ppm) : 7.11 (2H, d, J=8.1 Hz), 7.05 (2H, d, J=8.0 Hz), 6.69-6.78 (3H, m), 5.54 (1H, br.s), 4.35 (2H, d, J=5.6 Hz), 3.95 (2H, t, J=6.8 Hz), 3.83 (3H, s), 2.93 (2H, t, J=7.6 Hz), 2.48 (2H, t, J=7.6 Hz), 2.32 (3H, s), 1.83-1.89 (2H, m) 1.03 (2H, t, J=7.6 Hz)

### Production Example 26

300 mg of N-(4-methylbenzyl)-3-(4-hydroxy-3-methoxyphenyl)propanamide, 0.104 ml g of allyl bromide, 0.21 g of potassium carbonate and 5 ml of N,N-dimethylformamide were mixed, and stirred at 60 °C for 6 hours. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was washed by hexane to obtain 366 mg of N-(4-methylbenzyl)-3-{3-methoxy-4-(2-propenyloxy)pheny}pro panamide (referred as the compound of the present invention 26 hereinafter).
the compound of the present invention 26 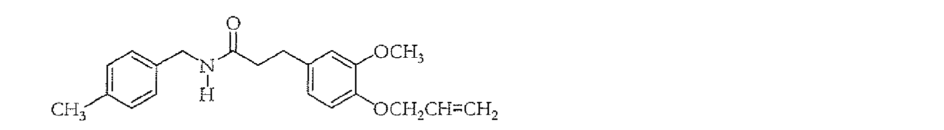 ¹H-NMR (CDCl₃, TMS) δ (ppm): 7.10 (2H, d, J=8.0 Hz), 7.05 (2H, d, J=8.0 Hz), 6.68-6.83 (3H, m), 6.02-6.12 (1H, m), 5.54 (1H, br.s), 5.39 (2H, d, J=17.2 Hz), 5.27 (2H, d, J=10.5 Hz), 4.57 (2H, d, J=5.3 Hz), 4.35 (2H, d, J=5.5 Hz), 3.82 (3H, s), 2.93 (2H, t, J=7.8 Hz), 2.47 (2H, t, J=7.8 Hz), 2.32 (3H, s)

### Production Example 27

By using 0.30 g of H-(4-methylbenzyl)-3-(4-hydroxy-3-methoxyphenyl)propanamide and 0.11 ml of 1-bromo-2-butyne according to the Production Example 23 was obtained 0.31 g of N-(4-methylbenzyl)-3-{3-methoxy-4-(2-butynyloxy)phenyl}prop anamide (referred as the compound of the present invention 27 hereinafter).
the compound of the present invention 27 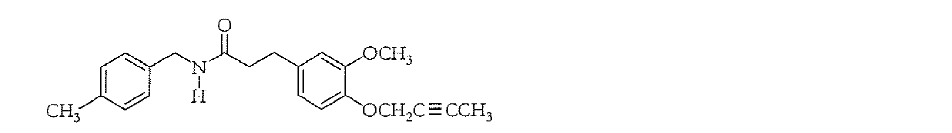 ¹H-NMR (CDCl₃, TMS) δ (ppm) : 7.04-7.12 (4H, m), 5. 92 (1H, d, J=7.8 Hz), 6.71-6.73 (2H, m), 5.53 (1H, br.s), 4.68 (2H, d, J-2.2 Hz), 4.36 (2H, d, J=5.6 Hz), 3.82 (3H, s), 2.94 (2H, t, J=7.8 Hz), 2.48 (2H, t, J=7.8 Hz), 2.33 (3H, s), 1.84 (3H, t, J=2.2 Hz)

### Production Example 28

0.11 g of 1-methyl-2-propynol, 0.11 ml of methanesulfonyl chloride, 0.28 ml of triethylamine and 3 ml of tetrahydrofuran were mixed, and stirred at room temperature for 30 minutes. Then ethyl acetate was added to the reaction mixture, and filtered. The filtrate was concentrated under reduced pressure. The obtained residue, 0.30 g of N-(4-methylbenzyl)-3-(4-hydroxy-3-methoxyphenyl)propanamide 0.21 g of potassium carbonate and 5 ml of N, N-dimethylformamide were mixed, and stirred at 60 °C for 6 hours. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.16 g of N-(4-methylbenzyl)-3-{3-methoxy-4-(1-methyl-2-propynyloxy)p henyl}propanamide (referred as the compound of the present invention 28 hereinafter).
the compound of the present invention 28 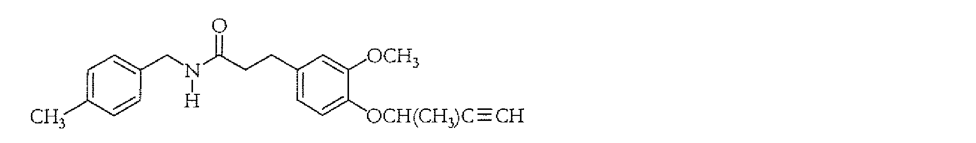 ¹H-NMR (CDCl₃, TMS) δ (ppm): 6.98-7.12 (4H, m), 6.82 (1H, d, J=7.8 Hz), 6.66-6.74 (2H, m), 5.54 (1H, br. s), 4.84-4.87 (1H, m), 4.35 (2H, d, J=5.5 Hz), 3.83 (3H, s), 2.92 (2H, t, J=7.2 Hz), 2.43-2.50 (2H, m), 2.32 (3H, s), 2.17 (3H, s), 1.69 (1H, d, J=6.6 Hz)

### Production Example 29

1.4 g of N-(4-trifluoromethylbenzyl)-3-{3-methoxy-4-(2-propynyloxy)p henyl}propanamide, 1.6 g of Lawesson's Reagent and 20 ml of tetrahydrofuran were mixed, and stirred at 65 °C for 3 hours. Then the reaction mixture was cooled, water was added to the reaction mixture, and extracted with ethyl acetate. The organic layer was washed successively with 3 % of aqueous solution of sodium hydroxide, 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 1.3 g of N-(4-trifluoromethylbenzyl)-3-{3-methoxy-4-(2-pxopynyloxy)p henyl}propanthioamide (referred as the compound of the present invention 29 hereinafter).
the compound of the present invention 29 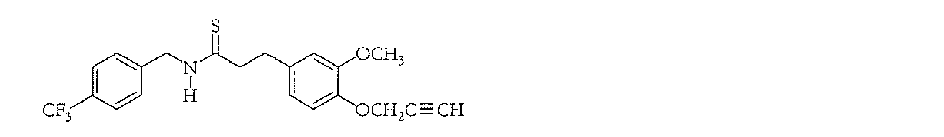 ¹H-NMR (CDCl₃, TMS) δ (ppm): 7.56 (2H, d, J=8 Hz), 7.19 (2H, d, J=8 Hz), 7.12 (1H, br.s), 6.93 (1H, d, J=8.2 Hz), 6.72-6.76 (2H, m), 4.81 (2H, d, J=5.3 Hz), 4.73 (2H, d, J=2.4 Hz), 3.81 (3H, s), 3.11 (2H, t, J=6.8 Hz), 2.97 (2H, t, J=6.8 Hz), 2.47 (1H t, J=2.4 Hz)

### Production Example 30

0.59 g of N-(4-chlorolbenzyl)-3-{3-methoxy-4-(2-propynyloxy)phenyl}pr opanamide, 0.67 g of Lawesson's Reagent and 10 ml of tetrahydrofuran were mixed, and stirred at 65 °C for 3 hours. Then the reaction mixture was cooled, and concentrated under reduced pressure. Water was added to the residue and extracted with ethyl acetate. The organic layer was washed successively with 3 % of aqueous solution of sodium hydroxide, 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.59 g of N-(4-chlorobenzyl)-3-{3-methoxy-4-(2-propynyloxy)phenyl}pro panthioamide (referred as the compound of the present invention 30 hereinafter).
the compound of the present invention 30 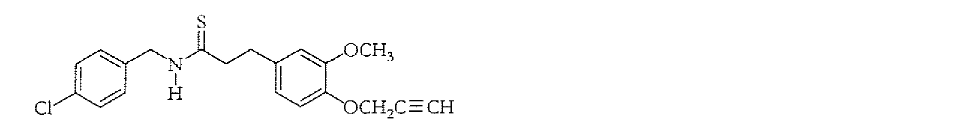 ¹H-NMR (CDCl₃, TMS) δ (ppm): 7.26-7.29 (2H, m), 6.99-7.06 (3H, m), 6.92 (1H, d, J=8.0 Hz), 6.70-6.75 (2H, m), 4.73 (2H, d, J=2 Hz), 4.70 (2H, d, J=5.3 Hz), 3.82 (3H, s), 3.09 (2H, t, J=7.2 Hz), 2.97 (2H, t, J=7.2 Hz), 2.49 (1H, t, J=2 Hz)

### Production Example 31

0.40 g of N-(3,4-dichlorolbenzyl)-3-{3-methoxy-4-(2-propynyloxy)pheny 1}propanamide, 0.45 g of Lawesson's Reagent and 15 ml of tetrahydrofuran were mixed, and stirred at 65 °C for 3 hours. Then the reaction mixture was cooled. Water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.42 g of N-(3,4-dichlorobenzyl)-3-{3-methoxy-4-(2-propynyloxy)phenyl }propanthioamide (referred as the compound of the present invention 31 hereinafter).
the compound of the present invention 31 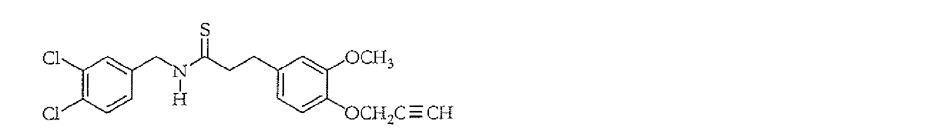 ¹H-NMR (CDCl₃, TMS) δ (ppm): 7.34 (1H, d, J=8.2 Hz), 7.28 (1H, d, J=2.2 Hz), 7.12 (1H, br.s), 6.89-6.99 (2H, m), 6.71-6.75 (2H, m)4.70-4.73 (4H, m), 3.82 (3H, s), 3.09 (2H, t, J=7.5 Hz), 2.96 (2H, t, J=7.5 Hz), 2.49 (1H, t, J=2.1 Hz)

### Production Example 32

0.40 g of N-(4-methylbenzyl)-3-{3-methoxy-4-(2-propynyloxy)phenyl}pro panamide, 0.53 g of Lawesson's Reagent and 10 ml of tetrahydrofuran were mixed, and stirred at 65 °C for 3 hours. Then the reaction mixture was cooled, and concentrated under reduced pressure. Water was added to the residue and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.38 g of N-(4-methylbenzyl)-3-{3-methoxy-4-(2-propynyloxy)phenyl}pro panthioamide (referred as the compound of the present invention 32 hereinafter).
the compound of the present invention 32 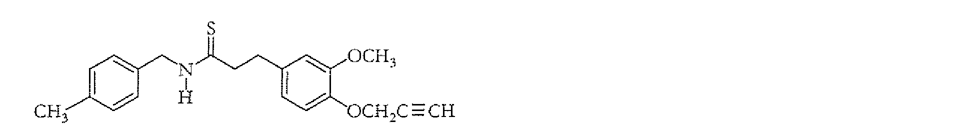 ¹H-NMR (CDCl₃, TMS) δ (ppm) : 7.12 (2H, d, J=7.8 Hz), 6.91-7.02 (3H, m), 6.92 (1H, d, J=8.2 Hz), 6.75 (1H, d, J=1.9 Hz), 6.72 (1H, dd, J=8.2 Hz, 1.9 Hz), 4.72 (2H, d, J=2 Hz), 4.66 (2H, d, J=4.8 Hz), 3.82 (3H, s), 3.08 (2H, t, J=7.2 Hz), 2.97 (2H, t, J=7.2 Hz), 2.48 (1H, t, J=2 Hz), 2.33 (3H, s)

### Production Example 33

By using 0.61 g of N-{(naphthalene-2-yl)methyl}-3-{3-methoxy-4-(2-propynyloxy) phenyl}propanamide and 745 mg of Lawesson's Reagent according to the Production Example 32 was obtained 0.38 g of N-{(naphthalene-2-yl)methyl}-3-{3-methoxy-4-(2-propynyloxy) phenyl}propanthioamide (referred as the compound of the present invention 33 hereinafter).
the compound of the present invention 33 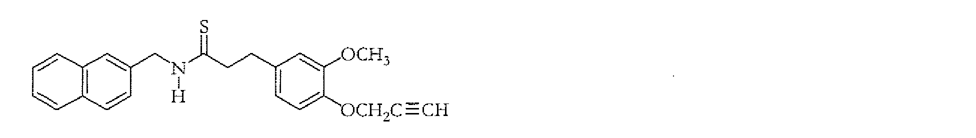 ¹H-MMR (CDCl₃, TMS) δ (ppm) : 8.7-8.9 (3H, m), 7.60 (1H, s), 7.4-7.6 (2H, m), 7.20 (1H, dd, J=8.5 Hz, 1.7 Hz), 7.15 (1H, br), 6.88 (1H, d, J=8.0 Hz), 6.77 (1H, d, J=1.9 Hz), 6.72 (1H, dd, J=8.2 Hz, 1.8 Hz), 4.88 (2H, d, J=5.1 Hz), 4.67 (2H, d, J=2.4 Hz), 3.80 (3H, s), 3.10 (2H, t, J=7.2 Hz), 2.96 (2H, t, J=7.2 Hz), 2.44 (1H, t, J=2.4 Hz)

### Production Example 34

By using 686 mg of 3-(3,4-dimethoxyphenyl)propionyl chloride, 563 mg of 4-methoxy-3-methylbenzylamine hydrochloride and 758 mg of triethylamine according to the Production Example 4 was obtained 900 mg of N-(4-methoxy-3-methylbenzyl)-3-(3,4-dimethoxyphenyl)propana mide (referred as the compound of the present invention 34 hereinafter).
the compound of the present invention 34 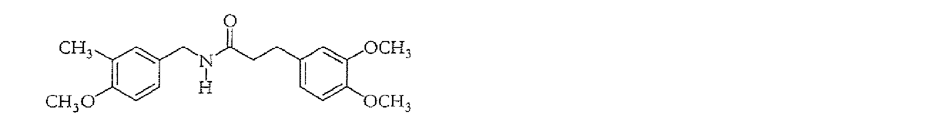 ¹H-NMR (CDCl₃, TMS) δ (ppm): 6.9-7.0 (2H, m), 6.7-6.8 (4H, m), 5.49 (1H, br), 4.30 (2H, d, J=5.6 Hz), 3.84 (3H, s), 3.83 (3H, s), 3.80 (3H, s), 2.93 (2H, t, J=7.5 Hz), 2.46 (2H, t, J=7.6 Hz), 2.18 (3H, s)

### Production Example 35

By using 0.30 g of N-(4-methylbenzyl)-3-(4-hydroxy-3-methoxyphenyl)propanamide and 0.08 ml of chloroacetonitrile according to the Production Example 23 was obtained 0.19 g of N-(4-methylbenzyl)-3-{3-methoxy-4-cyanomethoxyphenyl}propan amide (referred as the compound of the present invention 35 hereinafter).
the compound of the present invention 35 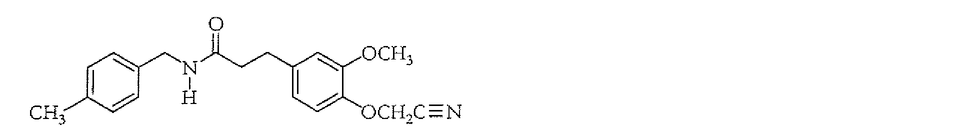 ¹H-NMR (CDCl₃, TMS) δ (ppm) : 7.09-7.12 (4H, m), 6.96 (1H, d, J=8.2 Hz), 6.73-6.80 (2H, m), 5.59 (1H, br.s), 4.78 (2H, s), 4.47 (2H, d, J=5.5 Hz), 3.83 (3H, s), 2.96 (2H, t, J=7.3 Hz), 2.48 (2H, t)

### Production Example 36

By using 1.0 g of 3-{3-methoxy-4-(2-propynyloxy)phenyl}propionyl chloride and 0.90 g of 4-methoxycarbonylbenzylamine hydrochloride according to the Production Example 18 was obtained 0.82 g of N-(4-methoxycarbonylbenzyl)-3-{3-methoxy-4-(2-propynyloxy)p henyl}propanamide (referred as the compound of the present invention 36 hereinafter).
the compound of the present invention 36 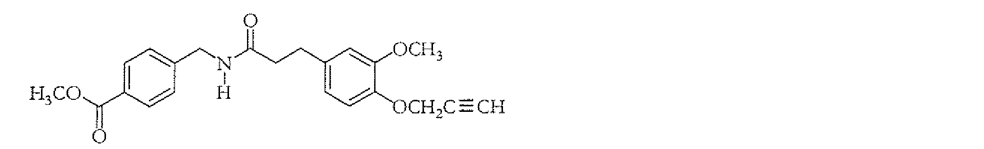 ¹H-NMR (CDCl₃, TMS) δ (ppm): 7.94-7.97 (2H, m), 7.12-7.21 (2H, m), 6.94 (1H, d, J=8.0 Hz), 6.71-6.75 (2H, m), 5.48 (1H, br.s), 4.73 (2H, d, J=2.4 Hz), 4.46 (2H, d, J=6.0 Hz), 3.91 (3H, s), 3.82 (3H, s), 2.96 (2H, t, J=7.2 Hz), 2.47-2.55 (3H, m)

### Production Example 37

884 mg of 3-{3-methoxy-4-(2-propynyloxy)phenyl}propionyl chloride, 641 mg of 4-phenylbenzylamine, 530 mg of triethylamine and 10 ml tetrahydrofuran were mixed, and stirred at room temperature for 1 hour. Then water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was washed by hexane to obtain 1.00 g of N-(4-phenylbenzyl)-3-{3-methoxy-4-(2-propynyloxy)phenyl}pro panamide (referred as the compound of the present invention 37 hereinafter).
the compound of the present invention 37 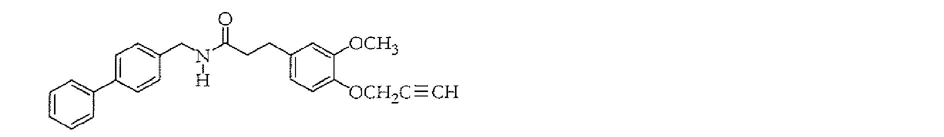 ¹H-NMR (CDCl₃, TMS) δ (ppm): 7.50-7.60 (4H, m), 7.40-7.47 (2H, m), 7.31-7.38 (1H, m), 7.22 (2H, d, J=8.3 Hz), 6.94 (1H, d, J=7.8 Hz), 6.71-6.77 (2H, m), 5. 63 (1H, br.s), 4.72 (2H, d, J=2.2 Hz), 4.44 (2H, d, J=5.8 Hz), 3.81 (3H, s), 2.96 (2H, t, J=7.5 Hz), 2.52 (2H, t, J=7.5 Hz), 2.45 (1H, t, J=2.4 Hz)

### Production Example 38

In the same way as in the Production Example 37, 100 mg of N-(3-chloro-4-methylbenzyl)-3-{3-methoxy-4-(2-propynyloxy)p henyl}propanamide (referred as the compound of the present invention 38 hereinafter) was obtained from 884 mg of 3-{3-methoxy-4-(2-propynyloxy)phenyl}propionyl chloride and 554 mg of 3-chloro-4-methyl-benzylamine.
the compound of the present invention 38 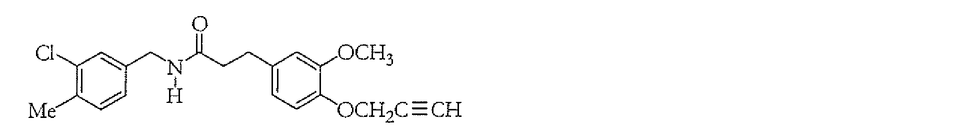 ¹H-NMR (CDCl₃, TMS) δ (ppm): 7.18 (1H, d, J=1.7 Hz), 7.14 (1H, d, J=7.7 Hz), 6.91-6.96 (2H, m), 6.70-6.75 (2H, m), 4.73 (2H, d, J=2.4 Hz)5.57 (1H, br.s), 4.34 (2H, d, J=5.8 Hz), 3.82 (3H, s), 2.94 (2H, t, J=7.5 Hz), 2.45-2.53 (3H, m), 2.34 (3H, s)

### Production Example 39

In the same way as in the Production Example 37, 1.03 g of N-(3-chloro-4-fluorobenzyl)-3-{3-methoxy-4-(2-propynyloxy)p henyl}propanamide (referred as the compound of the present invention 39 hereinafter) was obtained from 884 mg of 3-{3-methoxy-4-(2-propynyloxy)phenyl}propionyl chloride and 559 mg of 3-chloro-4-fluorobenzylamine.
the compound of the present invention 39 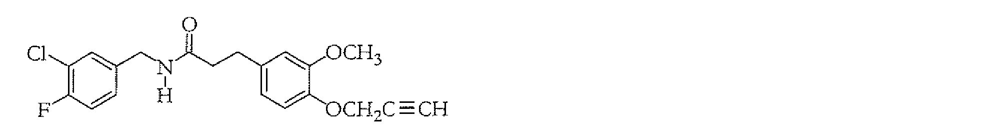 ¹H-NMR (CDCl₃, TMS) δ (ppm) : 7.21-7.26 (2H, m), 6.92-7.10 (3H, m), 6.70-6.76 (2H, m), 5.63 (1H, br.s), 4.73 (2H, d, J=2.4 Hz), 4.34 (2H, d, J=5.8 Hz), 3.83 (3H, s), 2.95 (2H, t, J=7.3 Hz), 2.51 (2H, t, J=7.3 Hz), 2.48 (1H, t, J=2.2 Hz)

### Production Example 40

637 mg of N-(5,6,,7,8-tetrahydronaphtalene-2-ylmethyl)-3-{3-methoxy-4 -(2-propynyloxy)phenyl}propanamide, 769 mg of Lawesson's Reagent and 10 ml of tetrahydrofuran were mixed, and stirred at 65 °C for 3 hours. Then the reaction mixture was cooled, and concentrated under reduced pressure. Water was added to the residue and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 394 mg of N- (5,6,7,8-tetrahydronaphtalene-2-ylmethyl)-3-{3-methoxy-4-(2-propynyloxy)phenyl}propanthioamide (referred as the compound of the present invention 40 hereinafter).
the compound of the present invention 40 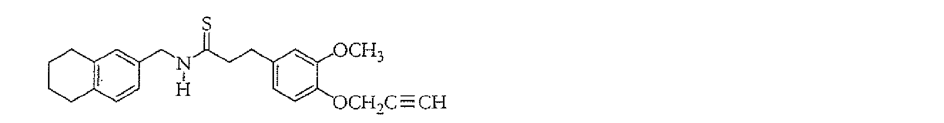 ¹H-NMR (CDCl₃, TMS) δ (ppm) : 6.91-7.03 (3H, m), 6.85 (2H, d, J=7.5 Hz), 6.76 (1H, d, J=2.0 Hz), 6.72 (1H, dd, J=8.1 Hz, 2.0 Hz), 4.72 (2H, d, J=2.2 Hz), 4.62 (2H, d, J=4.6 Hz), 3.82 (3H, s), 3.08 (2H, t, J=7.3 Hz), 2.91 (2H, t, J=7.3 Hz), 2.69-2.77 (4H, m), 2.48 (1H, t, J=2.4 Hz), 1.75-1.81 (4H, m)

### Production Example 41

In the same way as in the Production Example 37, 700 mg of N-(4-difluoromethoxybenzyl)-3-{3-methoxy-4-(2-propynyloxy)p henyl}propanamide (referred as the compound of the present invention 41 hereinafter) was obtained from 884 mg of 3-{3-methoxy-4-(2-propynyloxy)phenyl}propionyl chloride and 606 mg of 4-difluoromethoxybenzylamine.
the compound of the present invention 41 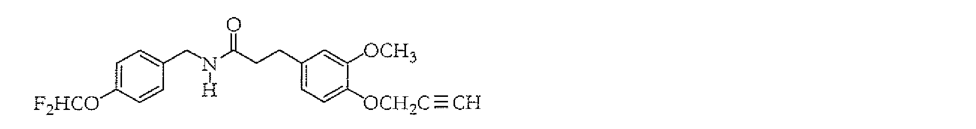 ¹H-NMR (CDCl₃, TMS) δ (ppm): 7.12 (2H, d, J=8.4 Hz) , 7.03 (2H, d, J=8.4 Hz), 6.93 (1H, d, J=7.7 Hz), 6.70-6.75 (2H, m), 6.49 (1H, t, J=73 Hz), 5.59 (1H, br.s), 4.73 (2H, d, J=2.2 Hz), 4.37 (2H, d, J-5.8 Hz), 3.81 (3H, s), 2.94 (2H, t, J=7.5 Hz), 2.46-2. 54 (3H, m)

### Production Example 42

In the same way as in the Production Example 37, 1.02 g of N-(4-methylbenzyl)-3-{3-ethoxy-4-(2-propynyloxy)phenyl}prop anamide (referred as the compound of the present invention 42 hereinafter) was obtained from 0.93 g of 3-{3-ethoxy-4-(2-propynyloxy)phenyl}propionyl chloride and 0.42 g of 4-methylbenzylamine.
the compound of the present invention 42 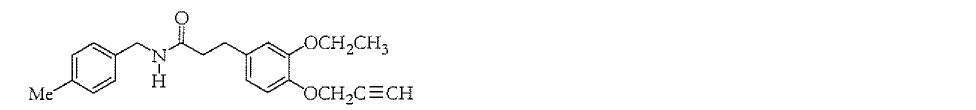 ¹H-NMR (CDCl₃, TMS) 5 (ppm): 7.03-7.12 (4H, m), 6.94 (1H, d, J=8.2 Hz), 6.74 (1H, d, J=1.9 Hz), 6.70 (1H, dd, J=2.1 Hz, 8.2 Hz), 5.53 (1H, br.s), 4.72 (2H, d, J-2.4 Hz), 4.35 (2H, d, J=5.5 Hz), 4.03 (2H, q, J=7.0 Hz), 2.92 (2H, t, J=7.7 Hz), 2.45-2.50 (3H, m), 2.32 (3H, s), 1.42 (3H, t, J=7.0 Hz)

### Production Example 43

In the same way as in the Production Example 37, 1.34 g of N-(3,4-dichlorobenzyl)-3-{3-ethoxy-4-(2-propynyloxy)phenyl} propanamide (referred as the compound of the present invention 43 hereinafter) was obtained from 0.93 g of 3-{3-ethoxy-4-(2-propynyloxy)phenyl}propionyl chloride and 0.62 g of 3,4-dichlorobenzylamine.
the compound of the present invention 43 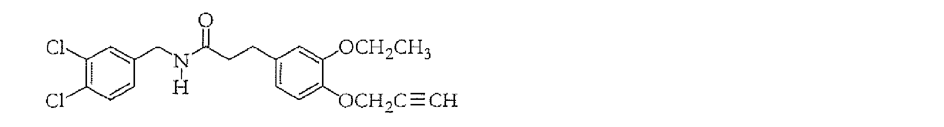 ¹H-NMR (CDCl₃, TMS) δ (ppm): 7.27-7.37 (2H, m), 6.92-6.98 (2H, m), 6. 68-0.75 (2H, m), 5.62 (1H, br.s), 4.73 (2H, d, J=2.4 Hz), 4.34 (2H, d, J=6.0 Hz), 4.03 (2H, q, J=7.0 Hz), 2.93 (2H, t, J=7.2 Hz), 2.45-2.55 (3H, m), 1.42 (3H, t, J=7.0 Hz)

### Production Example 44

In the same way as in the Production Example 40, 350 mg of N-(indan-5-yl methyl)-3-{3-methoxy-4-(2-propynyloxy)phenyl}propanthioamid e (referred as the compound of the present invention 44 hereinafter) was obtained from 500 mg of N-(indan-5-yl methyl)-3-{3-methoxy-4-(2-propynyloxy)phenyl}propanamide and 627 mg of Lawesson's Reagent.
the compound of the present invention 44 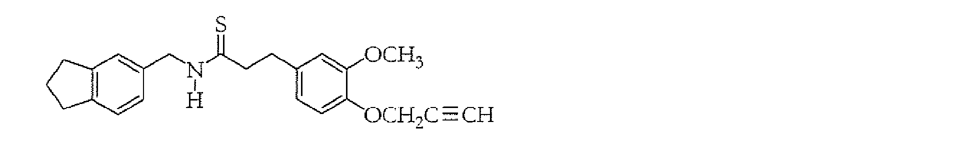 ¹H-NMR (CDCl₃, TMS) δ (ppm) : 7.16 (1H, d, J=7.7 Hz), 6.86-7.10 (4H, m), 6.75 (1H, d, J=1.9 Hz), 6.72 (1H, dd, J=8.2 Hz, 1.9 Hz), 4.72 (2H, d, J=2.4 Hz), 4.65 (2H, d, J=4.8 Hz), 3.81 (3H, s), 3.08 (2H, t, J=7.4 Hz), 2.84-2.95 (6H, m), 2.47 (1H, t, J=2.4 Hz), 2.01-2.11 (2H, m)

### Production Example 45

In the same way as in the Production Example 40, 314 mg of N-(4-phenylbenzyl)-3-{3-methoxy-4-(2-propynyloxy)phenyl}pro panthioamide (referred as the compound of the present invention 45 hereinafter) was obtained from 500 mg of N-(4-phenylbenzyl)-3-{3-methoxy-4-(2-propynyloxy)phenyl}pro panamide and 571 mg of Lawesson's Reagent.
the compound of the present invention 45 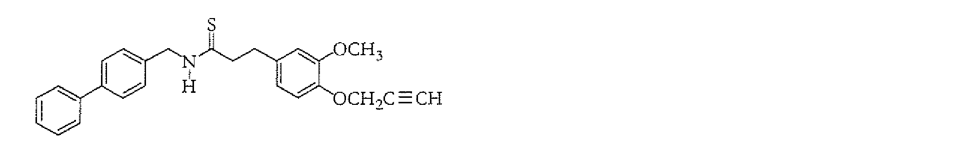 ¹H-NMR (CDCl₃, TMS) δ (ppm): 7.52-7.38 (4H, m), 7.40-7.46 (2H, m), 7.33-7.37 (1H, m), 7.15-7.23 (3H, m), 6.92 (1H, d, J=8.1 Hz), 6.71-6.77 (2H, m), 4.76 (2H, d, J=5.1 Hz), 4.70 (2H, d, J=2.4 Hz), 3.79 (3H, s), 3.09 (2H, t, J=7.2 Hz), 2.94 (2H, t, J=7.2 Hz), 2.44 (1H, t, J=2.4 Hz)

### Production Example 46

0.50 g of 3-{3-methoxy-4-(propynyloxy)phenyl}propionyl chloride, 0.39 g of 4-nitrobenzylamine, 0.69 ml triethylamine and 10 ml of tetrahydrofuran were mixed, and stirred at room temperature for 1 hour. Then water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.32 g of N-(4-nitrobenzyl)-3-{3-methoxy-4-(2-propynyloxy)phenyl}prop anamide (referred as the compound of the present invention 46 hereinafter).
the compound of the present invention 46 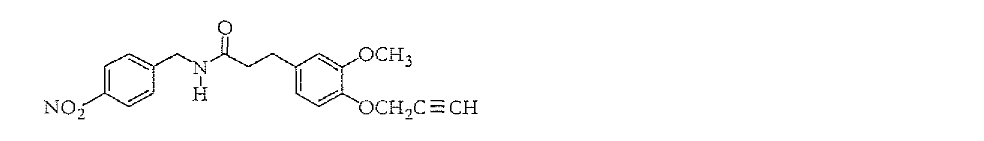 ¹H-NMR (CDCl₃, TMS) δ (ppm): 8.12-8.15 (2H, m), 7.25-7.28 (2H, m), 6.96 (1H, d, J=7.1 Hz), 6.72-6.75 (2H, m), 5.72 (1H, br.s), 4.75 (2H, d, J=2. Hz), 4.49 (2H, d, J=6.3 Hz), 3.83 (3H, s), 2.96 (2H, t, J=7.5 Hz), 2.56 (2H, t, J=7.5 Hz), 2.50 (1H, t, J=2.4 Hz)

### Production Example 47

0.50 g of 3-(3,4-dimethoxyphenyl)propionyl chloride, 0.43 g of 4-nitrobenzylamine, 0.71 ml triethylamine and 10 ml of tetrahydrofuran were mixed, and stirred at room temperature for 1 hour. Then water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.30 g of N-(4-nitrobenzyl)-3-(3,4-dimethoxyphenyl)propanamide (referred as the compound of the present invention 47 hereinafter).
the compound of the present invention 47 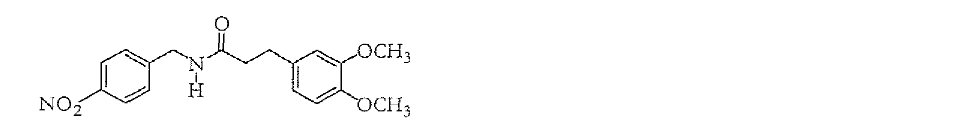 ¹H-NMR (CDCl₃, TMS) δ (ppm): 8.10-8.12 (2H, m), 7.24-7.26 (2H, m), 6.73-6.78 (3H, m), 5.78 (1H, br.s) 4.49 (2H, d, J=5.8 Hz), 3.86 (3H, s), 3.84 (3H, s), 2.96 (2H, t, J=7.2 Hz), 2.55 (2H, t, J=7.2 Hz)

### Production Example 48

In the same way as in the Production Example 40, 338 mg of N-(3-chloro-4-methylbenzyl)-3-{3-methoxy-4-(2-propynyloxy)p henyl}propanthioamide (referred as the compound of the present invention 48 hereinafter) was obtained from 560 mg of N-(3-chloro-4-methylbenzyl)-3-{3-methoxy-4-(2-propynyloxy)p henyl}propanamide and 683 mg of Lawesson's Reagent.
the compound of the present invention 48 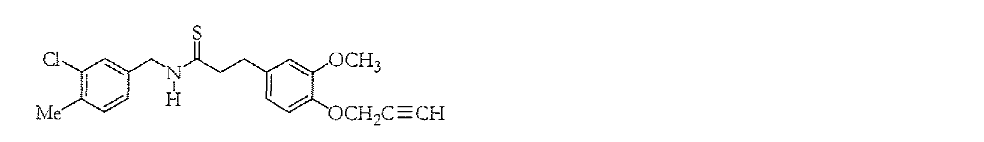 ¹H-NMR (CDCl₃, TMS) δ (ppm): 7.13-7.18 (2H, m), 7.05 (1H, br.s), 6.86-6.95 (2H, m), 6.70-6.76 (2H, m), 4.72 (2H, d, J=2.4 Hz), 4.67 (2H, d, J=5.3 Hz), 3.82 (3H, s), 3.08 (2H, t, J=7.0 Hz), 2. 93 (2H, t, J=7.0 Hz), 2.47 (1H, t, J=2.1 Hz), 2.34 (3H, s)

### Production Example 49

In the same way as in the Production Example 40, 345 mg of N-(3-chloro-4-fluorobenzyl)-3-{3-methoxy-4-(2-propynyloxy)p henyl}propanthioamide (referred as the compound of the present invention 49 hereinafter) was obtained from 500 mg of N- (3-chloro-4-fluorobenzyl) -3-(3-methoxy-4-(2-propynyloxy)p henyl}propanamide and 384 mg of Lawesson's Reagent.
the compound of the present invention 49 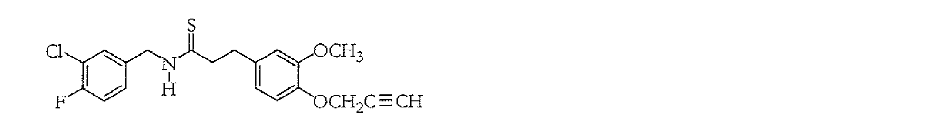 ¹H-NMR (CDCl₃, TMS) δ (ppm): 7.23 (1H, dd, J=7.0 Hz, 2.0 Hz), 7.19 (1H, br. s), 7.07 (1H, t, J=8.6Hz), 6.90-6.97 (2H, m), 6.75 (1H, d, J=1.7 Hz), 6.72 (1H, dd, J=8.0 Hz, 1.9 Hz), 4.72 (2H, d, J=2.4 Hz), 4.70 (2H, d, J=5.3 Hz), 3.81 (3H, s), 3.08 (2H, t, J=7.2 Hz), 2.94 (2H, t, J=7.2 Hz), 2.48 (1H, t, J=2.4 Hz)

### Production Example 50

In the same way as in the Production Example 40, 483 mg of N-(4-difluoromethoxybenzyl)-3-{3-methoxy-4-(2-propynyloxy)p henyl}propanthioamide (referred as the compound of the present invention 50 hereinafter) was obtained from 550 mg of N-(4-difluoromethoxybenzyl)-3-{3-methoxy-4-(2-propynyloxy)p henyl}propanamide and 408 mg of Lawesson's Reagent.
the compound of the present invention 50 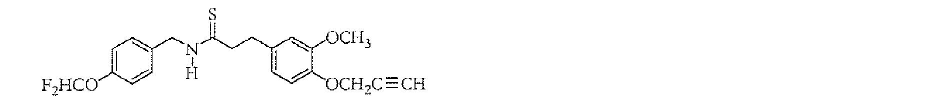 ¹H-NMR (CDCl₃, TMS) δ (ppm): 7.24 (1H, br.s), 7.01-7.12 (4H, m), 6.91 (1H, d, J=8.0 Hz), 6.71-6.74 (2H, m), 6.52 (1H, t, J=74 Hz), 4.67-4.73 (4H, m), 3.78 (3H, s), 3.07 (2H, t, J=7.0 Hz), 2.93 (2H, t, J=7.0 Hz), 2.48 (1H, t, J=2.4 Hz)

### Production Example 51

In the same way as in the Production Example 40, 360 mg of N-(4-chlorobenzyl)-3-{3-ethoxy-4-(2-propynyloxy)phenyl}prop anthioamide (referred as the compound of the present invention 51 hereinafter) was obtained from 425 mg of N-(4-chlorobenzyl)-3-{3-ethoxy-4-(2-propynyloxy)phenyl}prop anamide and 330 mg of Lawesson's Reagent.
the compound of the present invention 51 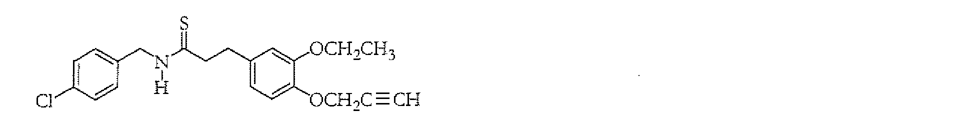 ¹H-NMR (CDCl₃, TMS) δ (ppm): 7.41 (1H, br.s), 7.25 (2H, d, J=8.4 Hz), 7.01 (2H, d, J=8.2 Hz), 6.91 (1H, d, J=8.2 Hz), 6.73 (1H, d, J=1.9 Hz), 6.69 (1H, dd, J=8.0 Hz, 1.9 Hz), 4.67-4.71 (4H, m), 3.99 (2H, q, J=7.0 Hz), 3.05 (2H, t, J=7.1 Hz), 2.91 (2H, t, J=7.1 Hz), 2.48 (1H, t, J=2.4 Hz), 1.40 (3H, t, J=6.9 Hz)

### Production Example 52

In the same way as in the Production Example 40, 423 mg of N-(4-methylbenzyl)-3-{3-ethoxy-4-(2-propynyloxy)phenyl}prop anthioamide (referred as the compound of the present invention 52 hereinafter) was obtained from 0.53 g of N-(4-methylbenzyl)-3-(3-ethoxy-4-(2-propynyloxy)phenyl)prop anamide and 0.44 g of Lawesson's Reagent.
the compound of the present invention 52 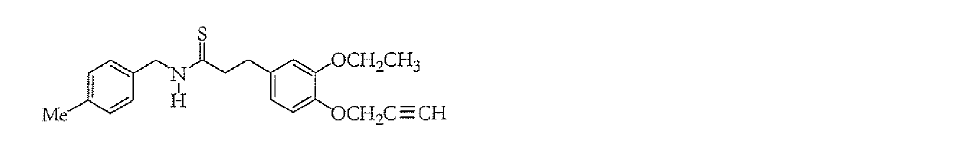 ¹H-NMR (CDCl₃, TMS) δ (ppm) : 7.21 (1H, br.s), 7.10 (2H, d, J=7.7 Hz), 7.00 (2H, d, J=7.9 Hz), 6.91 (1H, d, J=7.9 Hz), 6.74 (1H, d, J=1.9 Hz), 6. 69 (1H, dd, J=8.2 Hz, 1.9 Hz), 4. 69 (2H, d, J=2.1 Hz), 4.65 (2H, d, J=5.0H), 4.00 (2H, q, J=7.0 Hz), 3.04 (2H, t, J=7.2 Hz), 2.89 (2H, t, J=7.2 Hz), 2.46 (1H, t, J=2.1 Hz), 2.32 (3H, s), 1.40 (3H, t, J=6.7 Hz)

### Production Example 53

In the same way as in the Production Example 40, 463 mg of N-(4-bromobenzyl)-3-{3-methoxy-4-(2-propynyloxy)phenyl}prop anthioamide (referred as the compound of the present invention 53 hereinafter) was obtained from 660 mg of N-(4-bromobenzyl)-3-{3-methoxy-4-(2-propynyloxy)phenyl}prop anamide and 435 mg of Lawesson's Reagent.
the compound of the present invention 53 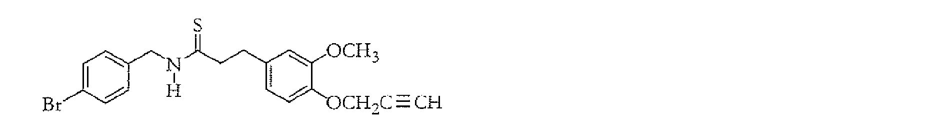 ¹H-NMR (CDCl₃, TMS) δ (ppm) : 7.39-7.43 (2H, m), 7.28 (1H, br.s), 6.95 (2H, d, J=8.4 Hz), 6.90 (1H, d, J=8.2 Hz), 6.74 (1H, d, J=1.7 Hz), 6.70 (1H, dd, J=7.9 Hz, 2.0 Hz), 4.71. (2H, d, J=2.4 Hz), 4.68 (2H, d, J=5.3 Hz), 3.79 (3H, s), 3.07 (2H, t, J=7.1 Hz), 2.93 (2H, t, J=7.1 Hz), 2.49 (1H, t, J=2.4 Hz)

### Production Example 54

In the same way as in the Production Example 40, 360 mg of N-(4-iodobenzyl)-3-{3-methoxy-4-(2-propynyloxy)phenyl}propa nthioamide (referred as the compound of the present invention 54 hereinafter) was obtained from 541 mg of N-(4-iodobenzyl)-3-{3-methoxy-4-(2-propynyloxy)phenyl}propa namide and 348 mg of Lawesson's Reagent.
the compound of the present invention 54 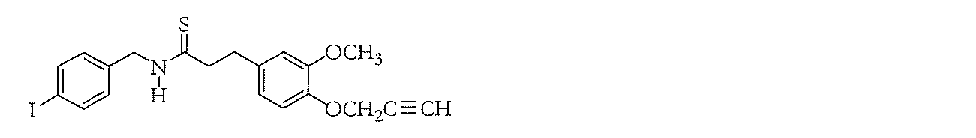 ¹H-NMR (CDCl₃, TMS) δ (ppm): 7.63 (2H, d, J=7. 9 Hz), 7.03 (1H, br.s), 6.92 (1H, d, J=7.9 Hz), 6.83 (2H, d, J=7.7 Hz), 6.75 (1H, d, J=1.9 Hz), 6.71 (1H, dd, J=7.9 Hz, 1.7 Hz), 4.74 (2H, d, J=2.4 Hz), 4.67 (2H, d, J=5.0 Hz), 3.81 (3H, s), 3.08 (2H, t, J=7.1 Hz), 2.94 (2H, t, J=7.1 Hz), 2.49 (1H, t, J=2.4 Hz)

### Production Example 55

In the same way as in the Production Example 37, 830 mg of N-(4-methylthiobenzyl)-3-{3-methoxy-4-(2-propynyloxy)phenyl }propanamide (referred as the compound of the present invention 55 hereinafter) was obtained from 632 mg of 3-{3-methoxy-4-(2-propynyloxy)phenyl}propionyl chloride and 383 mg of 4-methylthiobenzylamine.
the compound of the present invention 55 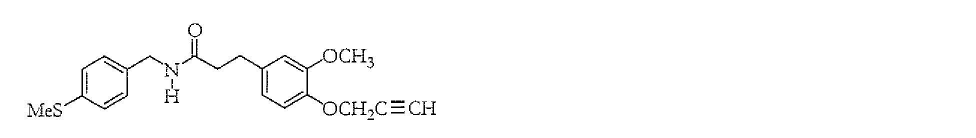 ¹H-NMR (CDCl₃, TMS) δ (ppm): 7.18 (2H, d, J=8.0 Hz), 7.06 (2H, d, J=8.2 Hz), 6.93 (1H, d, J=8.0 Hz), 6.70-6.75 (2H, m), 5.56 (1H, br.s), 4.73 (2H, d, J=2.4Hz), 4.35 (2H, d, J=5.8 Hz), 3.82 (3H, s), 2.94 (2H, t, J=7.1 Hz), 2.45-2.53 (6H, m)

### Production Example 56

In the same way as in the Production Example 37, 830 mg of N-(3,4-dimethylbenzyl)-3-{3-methoxy-4-(2-propynyloxy)phenyl }propanamide (referred as the compound of the present invention 56 hereinafter) was obtained from 632 mg of 3-{3-methoxy-4-(2-propynyloxy)phenyl}propionyl chloride and 338 mg of 3,4-dimethylbenzylamine.
the compound of the present invention 56 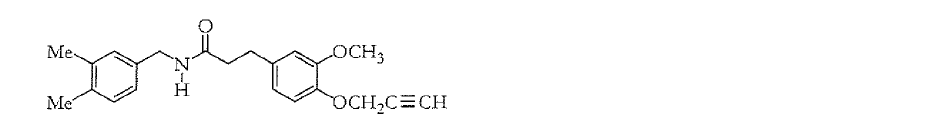 ¹H-NMR (CDCl₃, TMS) δ (ppm): 7.06 (1H, d, J=7.5 Hz), 6.88-7.00 (3H, m), 6. 75 (1H, d, J=1.9 Hz), 6.73 (1H, dd, J=8.0 Hz, 1. 9 Hz), 5.51 (1H, br.s), 4.73 (2H, d, J=2.4 Hz), 4.34 (2H, d, J=5.3 Hz), 3. 82 (3H, s), 2. 94 (2H, t, J=7.3 Hz), 2.44-2.51 (3H, m), 2.23 (6H, s)

### Production Example 57

In the same way as in the Production Example 37, 1.44 g of N-(4-cyano-benzyl)-3-{3-methoxy-4-(2-propynyloxy)phenyl}pro panamide (referred as the compound of the present invention 57 hereinafter) was obtained from 1.61 g of 3-{3-methoxy-4-(2-propynyloxy)phenyl}propionyl chloride and 0.84 g of 4-cyanobenzylamine.
the compound of the present invention 57 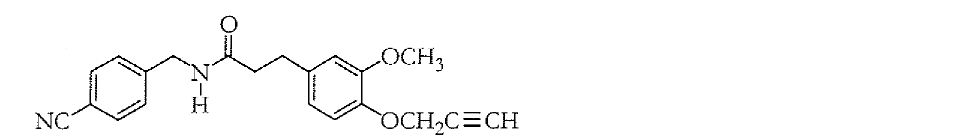 ¹H-NMR (CDCl₃, TMS) δ (ppm): 7.56 (2H, d, J=7.9 Hz), 7.18 (2H, d, J=7.9 Hz), 6.95 (1H, d, J=7.7 Hz), 6.71-6.75 (2H, m), 5.71 (1H, br.s), 4.74 (2H, d, J=2.4 Hz), 4.44 (2H, d, J=6.0 Hz), 3.82 (3H, s), 2.95 (2H, t, J=7.4 Hz), 2.54 (2H, t, J=7.2 Hz), 2.50 (1H, t, J=2.4 Hz)

### Production Example 58

In the same way as in the Production Example 40, 390 mg of N-(4-methylthiobenzyl)-3-{3-methoxy-4-(2-propynyloxy)phenyl }propanthioamide (referred as the compound of the present invention 58 hereinafter) was obtained from 0.52 g of N-(4-methylthiobenzyl)-3-{3-methoxy-4-(2-propynyloxy)phenyl }propanamide and 0.41 g of Lawesson's Reagent.
the compound of the present invention 58 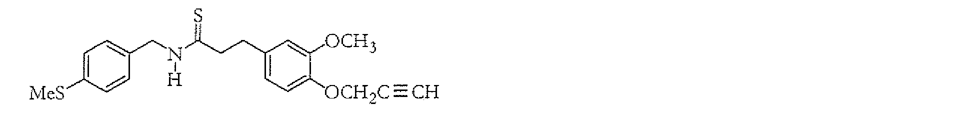 ¹H-NMR (CDCl₃, TMS) δ (ppm) : 7.18 (1H, d, J=8.2Hz), 6.99-7.06 (3H, m), 6.91 (1H, d, J=8.2Hz), 6.75 (1H d, J=1.9Hz), 6.71 (1H, dd, J=7.9Hz, 1.9Hz), 4.72 (2H, d, J=2.1Hz), 4.66 (2H, d, J=5.0Hz), 3.81(3H, s), 3.08 (2H, t, J=7 .2Hz), 2.93 (2H, t, J=7.2Hz), 2.49 (1H, t, J=2.4Hz), 2.47 (3H, s)

### Production Example 59

In the same way as in the Production Example 40, 345 mg of N-(3,4-dimethylbenzyl)-3-{3-methoxy-4-(2-propynyloxy)phenyl }propanthioamide) (referred as the compound of the present invention 59 hereinafter) was obtained from 0.43 g of N-(3,4-dimethylbenzyl)-3-{3-methoxy-4-(2-propynyloxy)phenyl }propanamide and 0.36 g of Lawesson's Reagent.
the compound of the present invention 59 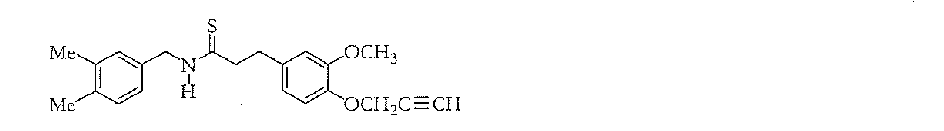 ¹H-NMR (CDCl₃, TMS) δ (ppm): 7.08 (1H, d, J=7.8 Hz), 7.01 (1H, br.s), 6.84-6.96 (3H, m), 6.76 (1H, d, J=1.9Hz), 6.72 (1H, dd, J=8.1 Hz, 1.9 Hz), 4.72 (2H, d, J=2.2 Hz), 4.63 (2H, d, J=4.8 Hz), 3.82 (3H, s), 3.08 (2H, t, J=7.3 Hz), 2.91 (2H, t, J=7.3 Hz), 2.47 (1H, t, J=2.4 Hz), 2.43 (3H, s), 2.23 (3H, s)

### Production Example 60

In the same way as in the Production Example 37, 830 mg of N-(4-methoxy-3-methylbenzyl)-3-{3-methoxy-4-(2-propynyloxy) phenyl}propanamide (referred as the compound of the present invention 60 hereinafter) was obtained from 0.76 g of 3-{3-methoxy-4-(2-propynyloxy)phenyl}propionyl chloride and 0.56 g of 4-methoxy-3-methylbenzylamine hydrochloride.
the compound of the present invention 60 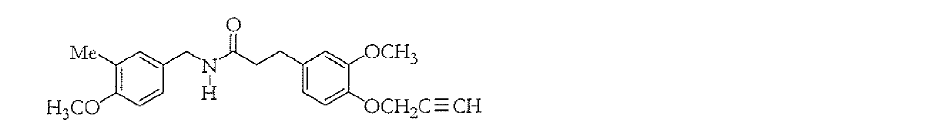 ¹H-NMR (CDCl₃, TMS) δ (ppm): 6.91-6.98 (3H, m), 6.70-6.76 (3H, m), 5.49 (1H, br.s), 4.72 (2H, d, J=2.4 Hz), 4.30 (2H, d, J=5.5 Hz), 3.82 (3H, s), 3.81 (3H, s), 2.94 (2H, t, J=7.5 Hz), 2.47-2.49 (3H, m), 2.18 (3H, s)

### Production Example 61

In the same manner way as in the Production Example 40, 449 mg of N-(4-cyanobenzyl)-3-{3-methoxy-4-(2-propynyloxy)phenyl}prop anthioamide (referred as the compound of the present invention 61 hereinafter) was obtained from 532 mg of N-(4-cyanobenzyl)-3-{3-methoxy-4-(2-propynyloxy)phenyl}prop anamide and 435 mg of Lawesson's Reagent.
the compound of the present invention 61 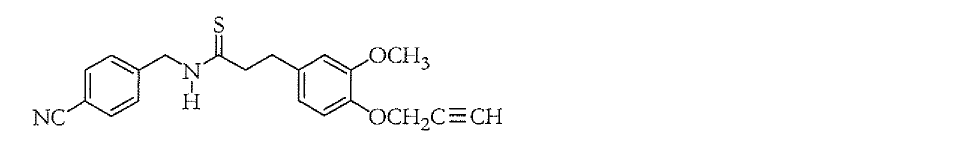 ¹H-NMR (CDCl₃, TMS) δ (ppm): 7.55 (2H, d, J=7.9 Hz), 7.33 (1H, br.s), 7.12 (2H, d, J=7.9 Hz), 6.93 (1H, d, J=7.9 Hz), 6.72-6.76 (2H, m), 4.83 (2H, d, J=5.5 Hz), 4.74 (2H, d, J=2.4 Hz), 3.80 (3H, s), 3.10 (2H, t, J=7.0 Hz), 2.98 (2H, t, J=7.0 Hz), 2.50 (1H, t, J=2.4 Hz)

### Production Example 62

In the same way as in the Production Example 40, 383 mg of N-(4-methoxy-3-methylbenzyl)-3-{3-methoxy-4-(2-propynyloxy) phenyl}propanthioamide (referred as the compound of the present invention 62 hereinafter) was obtained from 0.55 g of N-(4-methoxy-3-methylbenzyl)-3-{3-methoxy-4-(2-propynyloxy) phenyl}propanamide and 0.44 g of Lawesson's Reagent.
the compound of the present invention 62 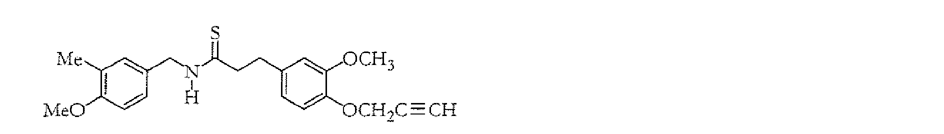 ¹H-NMR (CDCl₃, TMS) δ (ppm): 6.89 (1H, br.s) . 6.91-6.96 (3H, m), 6.68-6.78 (3H, m), 4.72 (2H, d, J=2.4 Hz), 4.59 (2H, d, J=4.6 Hz), 3.82 (3H, s), 3.81 (3H, s), 3.08 (2H, t, J=7.3 Hz), 2.91 (2H, t, J=7.3 Hz), 2.47 (1H, t, J=2.4 Hz), 2.18 (3H, s)

### Production Example 63

The mixed solution of 500 mg of N-(4-chlorobenzyl)-3-{3-methoxy-4-(2-propynyloxy)phenyl}pro panamide and 5 ml of N, N-dimethylformamide was stirred at 0 to 5 °C, added 61 mg of sodium hydride, and stirred for 30 minutes . Then, 199 mg of methyl iodide was added to the solution, and stirred at 0 to 5 °C for 30 minutes and at room temperature for 2 hours. After that, water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 312 mg of N-(4-chlorobenzyl)-N-methyl-3-{3-methoxy-4-(2-propynyloxy)p henyl}propanamide (referred as the compound of the present invention 63 hereinafter).
the compound of the present invention 63 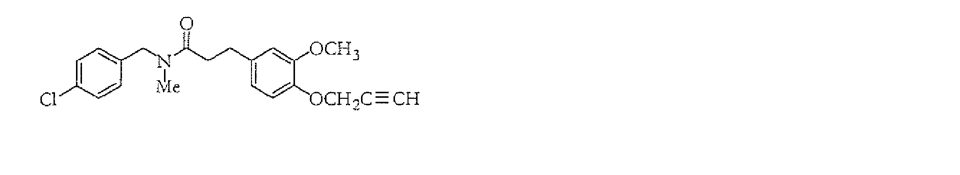 ¹H-NMR (CDCl₃, TMS) δ (ppm) : 7.26-7.32 (2.2H, m), 7.10-7.15 (1.3H, m), 6.90-7.01 (1.5H, m), 6.66-6.82 (2H, m), 4.70-4.75 (2H, m), 4.54 (2H, s), 4.41 (1H, s), 3.85 (2H, s), 3.82 (1H, s), 2.94-3.01 (2H, m), 2.93 (1H, s), 2.85 (2H, s), 2.59-2.69 (2H, m), 2.47-2.50 (1H, m)

### Production Example 64

In the same way as in the Production Example 37, 720 mg of N-(4-chlorobenzyl)-2-methyl-3-{3-methoxy-4-(2-propynyloxy)p henyl}propanamide (referred as the compound of the present invention 64 hereinafter) was obtained from 934 mg of 2-methyl-3-{3-methoxy-4-(2-propynyloxy)phenyl}propionyl chloride and 496 mg of 4-chlorolbenzylamine.
the compound of the present invention 64 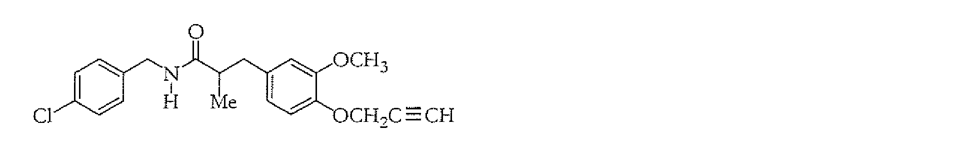 ¹H-NMR (CDCl₃, TMS) δ (ppm): 7.21-7.26 (2H, m), 6.90-6.96 (3H, m), 6.66-6.71 (2H, m), 5.45 (1H, br.s), 4.74 (2H, d, J=2.4 Hz), 4.38 (1H, dd, J=15 Hz, 6.3 Hz), 4.21 (1H, dd, J=15 Hz, 5.5 Hz), 3.80 (3H, s), 2.87-2.94 (1H, m), 2.64-2.70 (1H, m), 2.49 (1H, t, J=2.4 Hz), 2.42-2.46 (1H, m), 1.23 (3H, d, J=6.8 Hz)

### Production Example 65

In the same way as in the of Production Example 37, 720 mg of N-(4-methylbenzyl)-2-methyl-3-{3-methoxy-4-(2-propynyloxy)p henyl}propanamide (referred as the compound of the present invention 65 hereinafter) was obtained from 934 mg of 2-methyl-3-{3-methoxy-4-(2-propynyloxy)phenyl}propionyl chloride and 424 mg of 4-methylbenzylamine.
the compound of the present invention 65 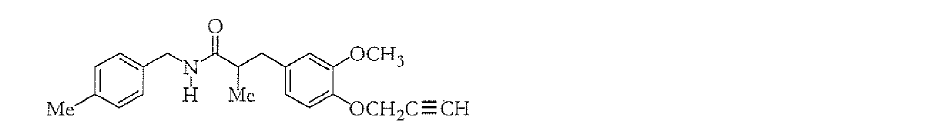 ¹H-NMR (CDCl₃, TMS) δ (ppm): 7.07 (2H, d, J=7.7 Hz), 6.94 (2H, d, J=7.9 Hz), 6.91 (1H, d, J=7.9 Hz), 6.66-6.71 (2H, m), 5.40 (1H, br.s), 4.73 (2H, d, J=2.4 Hz), 4.20-4.38 (2H, m), 3.80 (3H, s), 2.88-2.97 (1H, m), 2.60-2.68 (1H, m), 2.48 (1H, t, J=2.4 Hz), 2.36-2.45 (1H, m), 2.31 (3H, s), 1.21 (3H, d, J=7.0 Hz)

### Production Example 66

In the same way as in the Production Example 37, 1.05 g of N-(3,4-dichlorobenzyl)-2-methyl-3-{3-methoxy-4-(2-propynylo xy)phenyl}propanamide (referred as the compound of the present invention 66 hereinafter) was obtained from 1.07 g of 2-methyl-3-{3-methoxy-4-(2-propynyloxy)phenyl}propionyl chloride and 704 mg of 3,4-dichlorobenzylamine.
the compound of the present invention 66 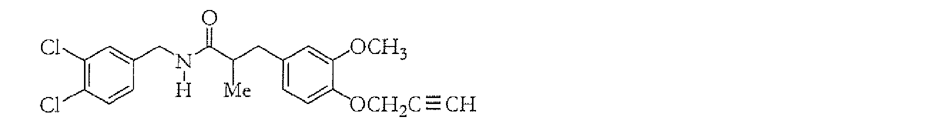 ¹H-NMR (CDCl₃, TMS) δ (ppm) : 7.32 (1H, d, J=8.2 Hz), 7.21 (1H, d, J-1.9 Hz), 6.91 (1H, d, J=7.7 Hz), 6.81 (1H, dd, J=8.2 Hz, 2.1 Hz), 6.65-6.72 (2H, m), 5.49 (1H, br. s), 4.73 (2H, d, J=2.4 Hz), 4.17-4.38 (2H, m), 3.81 (3H, s), 2. 86-2. 95 (1H, m), 2.62-2.70 (1H, m), 2.40-2.50 (2H, m), 1.23 (3H, d, J=6.7 Hz)

### Production Example 67

In the same way as in the Production Example 37, 480 mg of N-{(naphthalene-2-yl)methyl}-2-methyl-3-{3-methoxy-4-(2-pro pynyloxy)phenyl}propanamide (referred as the compound of the present invention 67 hereinafter) was obtained from 1.07 g of 2-methyl-3-{3-methoxy-4-(2-propynyloxy)phenyl}propionyl chloride and 628 mg of (naphthalene-2-yl)methylamine.
the compound of the present invention 67 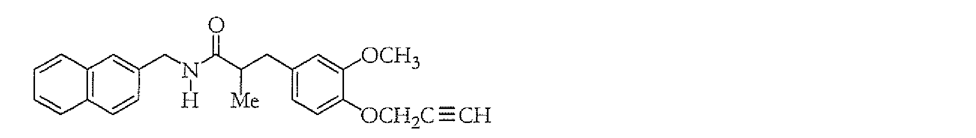 ¹H-NMR (CDCl₃, TMS) δ (ppm): 7.71-7.83 (3H, m), 7.53 (1H, s), 7.41-7.50 (2H, m), 7.15 (1H, dd, J=8.4 Hz, 1.7 Hz), 6.86 (1H, d, J=7.9 Hz), 6.72 (1H, d, J=1.7 Hz), 6.69 (1H, dd, J=7.9 Hz, 1.9 Hz), 5.52 (1H, br.s), 4.68 (2H, d, J=2.4 Hz), 4.40-4.60 (2H, m), 3.78 (3H, s), 2.91-2.99 (1H, m), 2.64-2.71 (1H, m), 2.41-2.53 (1H, m), 2.45 (1H, t, 2.4 Hz), 1.25 (3H, d, J=6.7 Hz)

### Production Example 68

In the same way as in the Production Example 40, 242 mg of N-(4-chloro-benzyl)-N-methyl-3-{3-methoxy-4-(2-propynyloxy) phenyl}propanthioamide (referred as the compound of the present invention 68 hereinafter) was obtained from 500 mg of N-(4-chlorobenzyl)-N-methyl-3-{3-methoxy-4-(2-propynyloxy)p henyl}propanamide and 390 mg of Lawesson's Reagent.
the compound of the present invention 68 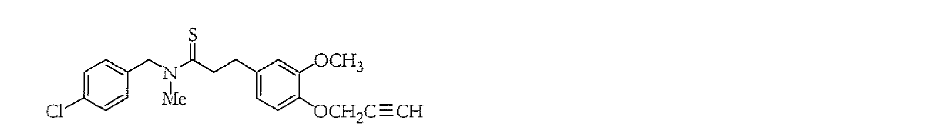 ¹H-NMR (CDCl₃, TMS) δ (ppm): 7.27 (2H, m), 7.18-7.24 (1.3H, m), 6.90-7.00 (1.7H, m), 6.66-6.83 (2H, m), 5.29 (1.3H, s), 4.73-4.74 (2H, m), 4.61 (0.7H, s), 3.85 (2H, s), 3.82 (1H, s), 3.42 (1H, s), 3.07-3.12 (4H, m), 3.04 (2H, s), 2.48-2.50 (1H, m)

### Production Example 69

In the same way as in the Production Example 40, 451 mg of N-(4-chlorobenzyl)-2-methyl-3-{3-methoxy-4-(2-propynyloxy)p henyl}propanthioamide (referred as the compound of the present invention 69 hereinafter) was obtained from 420 mg of N-(4-chlorobenzyl)-2-methyl-3-{3-methoxy-4-(2-propynyloxy)p henyl}propanamide and 328 mg of Lawesson's Reagent.
the compound of the present invention 69 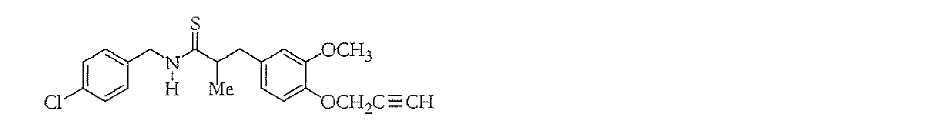 ¹H-NMR, (CDCl₃, TMS) δ (ppm): 7.23-7.26 (2H, m), 6.96 (1H, br.s), 6.87-6.93 (3H, m), 6.74 (1H, d, J=1.7 Hz), 6.69 (1H, dd, J=8.1 Hz, 1.7 Hz), 4.72-4.78 (3H, m), 4.54 (1H, dd, J=15 Hz, 4.8 Hz), 3.79 (3H, s), 2.97-3.04 (1H, m), 2.75-2.84 (2H, m), 2.49 (1H, t, J=2.4 Hz), 1.35 (3H, d, J=6.3 Hz)

### Production Example 70

In the same way as in the Production Example 37, 1.17 g of N-(4-chlorobenzyl)-3-{3-methoxy-4-(2-propynyloxy)phenyl}but aneamide (referred as the compound of the present invention 70 hereinafter) was obtained from 934 mg of 3-{3-methoxy-4-(2-propynyloxy)phenyl}butyryl chloride and 496 mg of 4-chlorobenzylamine.
the compound of the present invention 70 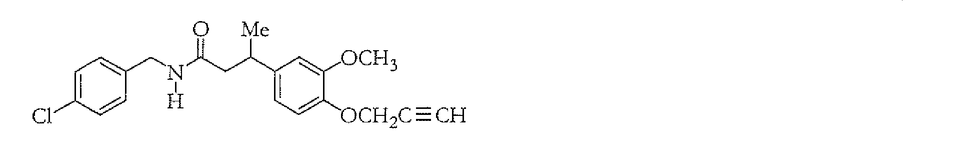 ¹H-NMR (CDCl₃, TMS) δ (ppm): 7.20-7.26 (2H, m), 6.93-6.97 (3H, m), 6.72-6.77 (2H, m), 5.50 (1H, br.s), 4.74 (2H, d, J=2.4 Hz), 4.36 (1H, dd, J=15 Hz, 6.0 Hz), 4.22 (1H, dd, J=15 Hz, 5.5 Hz), 3.82 (3H, s), 3.20-3.30 (1H, m), 2.42-2.50 (3H, m), 1.31 (3H, d, J=7.0 Hz)

### Production Example 71

In the same way as in the Production Example 37, 1.13 g of N-(4-methylbenzyl)-3-{3-methoxy-4-(2-propynyloxy)phenyl}but aneamide (referred as the compound of the present invention 71 hereinafter) was obtained from 934 mg of 3-{3-methoxy-4- (2-propynyloxy)phenyl}butyryl chloride and 424 mg of 4-methylbenzylamine.
the compound of the present invention 71 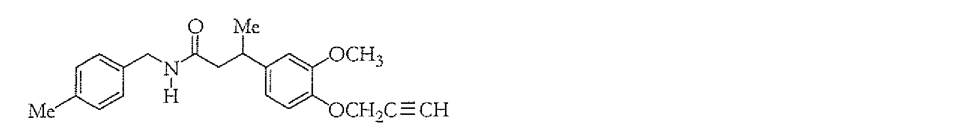 ¹H-NMR (CDCl₃, TMS) δ (ppm): 7.08 (2H, d, J=7.9 Hz), 6.92-6.98 (3H, m), 6.73-6.77 (2H, m), 5.43 (1H, br. s), 4.73 (2H, d, J=2.4 Hz), 4.21-4.37 (2H, m), 3.82 (3H, s), 3.22-3.34 (1H, m), 2.48 (1H, t, J=2.4 Hz), 2.38-2.44 (2H, m), 2.31 (3H, s), 1.30 (3H, d, J=7.0 Hz)

### Production Example 72

In the same way as in the Production Example 37, 1.28 g of N-(3,4-dichlorobenzyl)-3-{3-methoxy-4-(2-propynyloxy)phenyl }butaneamide (referred as the compound of the present invention 72 hereinafter) was obtained from 934 mg of 3-{3-methoxy-4- (2-propynyloxy)phenyl}butyryl chloride and 616 mg of 3,4-dichlorobenzylamine.
the compound of the present invention 72 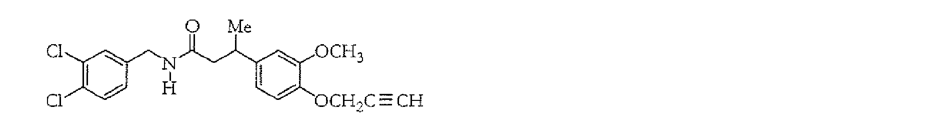 ¹H-NMR (CDCl₃, TMS) δ (ppm): 7.32 (1H, d, J=8.3 Hz), 7.21 (1H, d, J=1.7 Hz), 6.96 (1H, d, J=8.5 Hz), 6.81 (1H, dd, J=8.3 Hz, 1.6 Hz), 6.72-6.78 (2H, m), 5.53 (1H, br.s), 4.73 (2H, d, J=2.4 Hz), 4.18-4.34 (2H, m), 3.83 (3H, s), 3.20-3.33 (1H, m), 2.40-2.52 (3H, m), 1.31 (3H, d, J=7.1 Hz)

### Production Example 73

In the same way as in the Production Example 37, 1.21 g of N-{(naphthalene-2-yl)-methyl}-3-{3-methoxy-4-(2-propynyloxy )phenyl}butaneamide (referred as the compound of the present invention 73 hereinafter) was obtained from 934 mg of 3-{3-methoxy-4-(2-propynyloxy)phenyl}butyryl chloride and 550 mg of (naphthalene-2-yl)methylamine.
the compound of the present invention 73 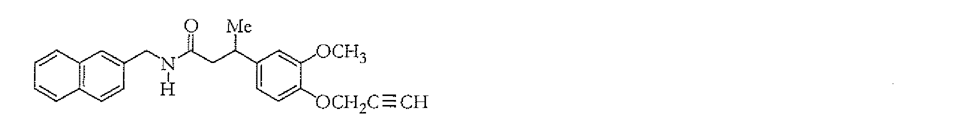 ¹H-NMR (CDCl₃, TMS) δ (ppm): 7.72-7.84 (3H, m), 7.54 (1H, s), 7.42-7.50 (2H, m), 7.15) 1H, dd, J=8.5 Hz, 1.7 Hz), 6.91 (1H, d, J=8.1 Hz), 6.70-6.78 (2H, m), 5.56 (1H, br.s), 4.68 (2H, m), 4.42-4.59 (2H, m), 3.80 (3H, s), 3.25-3.36 (1H, m), 2.42-2.49 (3H, m), 1.32 (3H, d, J=6.8 Hz)

### Production Example 74

In the same way as in the Production Example 40, 423 mg of N-(4-methylbenzyl)-2-methyl-3-{3-methoxy-4-(2-propynyloxy)p henyl}propanthioamide (referred as the compound of the present invention 74 hereinafter) was obtained from 420 mg of N-(4-methylbenzyl)-2-methyl-3-{3-methoxy-4-(2-propynyloxy)p henyl}propanamide and 348 mg of Lawesson's Reagent.
the compound of the present invention 74 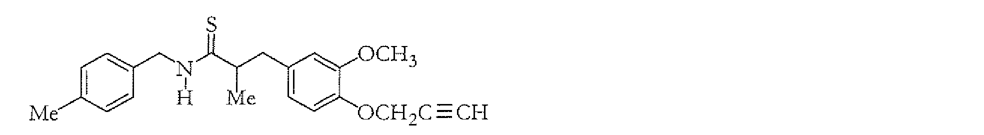 ¹H-NMR (CDCl₃, TMS) δ (ppm) : 7.09 (2H, d, J=7.7 Hz), 6.87-6.95 (4H, m), 6.75 (1H, d, J=1.9 Hz), 6.70 (1H, dd, J=8.1 Hz, 1.9 Hz), 4.73 (2H, d, J=2.4 Hz), 4.53-4.69 (2H, m), 3.81 (3H, s), 2.97-3.06 (1H, m), 2.71-2.83 (2H, m), 2.48 (1H, t, J=2.4 Hz) , 2.32 (3H, s), 1.34 (3H, d, J=6.5 Hz)

### Production Example 75

In the same way as in the Production Example 40, 451 mg of N-(3,4-dichloro-benzyl)-2-methyl-3-{3-methoxy-4-(2-propynyl oxy)phenyl}propanthioamide (referred as the compound of the present invention 75 hereinafter) was obtained from 500 mg of N-(3,4-dichloro-benzyl)-2-methyl-3-{3-methoxy-4-(2-propynyl oxy)phenyl}propanamide and 357 mg of Lawesson's Reagent.
the compound of the present invention 75 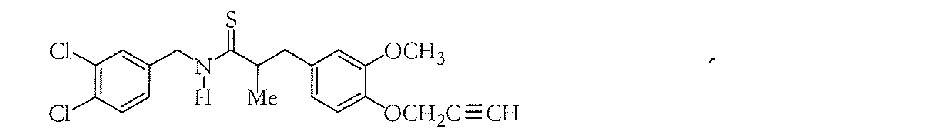 ¹H-NMR (CDCl₃, TMS) δ (ppm): 7.34 (1H, d, J=8.1 Hz), 7.20 (1H, d, J=1.9 Hz), 6.99 (1H, br.s), 6. 91 (1H, d, J=8.0 Hz), 6.68-6.76 (3H, m), 4.74 (2H, d, J=2.4 Hz), 4.55-4.77 (2H, m), 3.81 (3H, s), 2.96-3.05 (1H, m), 2.75-2.85 (2H, m), 2.48 (1H, t, J=2.4 Hz), 1.35 (3H, d, J=6. Hz)

### Production Example 76

In the same way as in the Production Example 40, 1.17 g of N-{(naphthalene-2-yl)methyl}-2-methyl-3-{3-methoxy-4-(2-pro pynyloxy)phenyl}propanthioamide (referred as the compound of the present invention 76 hereinafter) was obtained from 470 mg of N-{(naphthalene-2-yl)methyl}-2-methyl-3-{3-methoxy-4-(2-pro pynyloxy)phenyl}propanamide and 352 mg of Lawesson's Reagent. the compound of the present invention 76 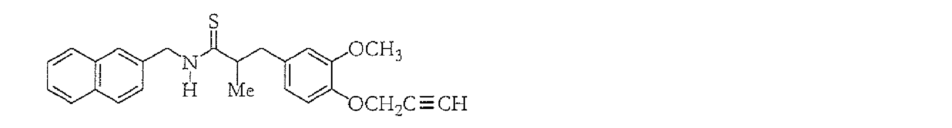 ¹H-NMR (CDCl₃, TMS) δ (ppm): 7.74-7.83 (3H, m), 7.45-7.54 (3H, m), 7.07 (1H, dd, J=8.4 Hz, 1.9 Hz), 6.89 (1H, br.s), 6.88 (1H, d, J=7.9 Hz), 6.77 (1H, d, J=1.9 Hz), 6.71 (1H, dd, J=8.2 Hz, 1.9 Hz), 4.73-4.92 (2H, m), 4.69 (2H, d, J=2.4 Hz), 3.80 (3H, s), 2.99-3.09 (1H, m), 2.74-2.85 (2H, m), 2.44 (1H, t, J=2.4 Hz), 1.37 (3H, d, J=6.3 Hz)

### Production Example 77

In the same way as in the Production Example 40, 395 mg of N-(4-methyl-benzyl)-3-{3-methoxy-4-(2-propynyloxy)phenyl}bu tanethioamide (referred as the compound of the present invention 77 hereinafter) was obtained from 500 mg of N-(4-methylbenzyl)-3-{3-methoxy-4-(2-propynyloxy)phenyl}but ylamide and 412 mg of Lawesson's Reagent.
the compound of the present invention 77 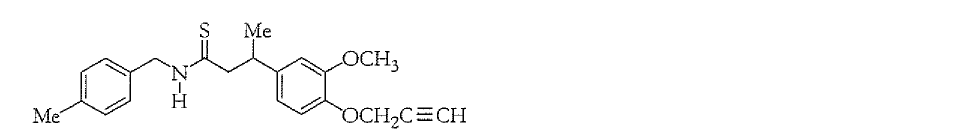 ¹H-NMR (CDCl₃, TMS) δ (ppm) : 7.09 (2H, d, J=7.7 Hz), 6.93 (1H, d, J=7.9 Hz), 6.89 (2H, d, J=7.9H), 6.84 (1H, br.s), 6.72-6.77 (2H, m), 4.72 (2H, d, J=2.4 Hz), 4.50-4.63 (2H, m), 3.82 (3H, s), 3.36-3.47 (1H, m), 2.93-3.01 (1H, m), 2.72-2.81 (1H, m), 2.48 (1H, t, J=2.4 Hz), 2.32 (3H, s), 1.32 (3H, d, J=7.0 Hz)

### Production Example 78

In the same way as in the Production Example 40, 313 mg of N-(3,4-dichlorobenzyl)-3-{3-methoxy-4-(2-propynyloxy)phenyl }butanethioamide (referred as the compound of the present invention 78 hereinafter) was obtained from 500 mg of N-(3,4-dichlorobenzyl)-3-(3-znethoxy-4-(2-propynyloxy)phenyl }butaneamide and 343 mg of Lawesson's Reagent.
the compound of the present invention 78 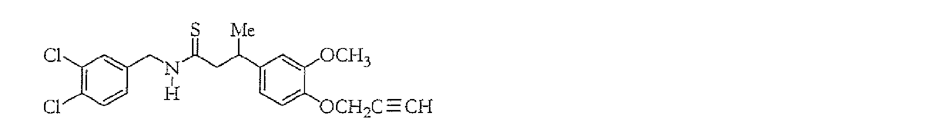 ¹H-NMR (CDCl₃, TMS) δ (ppm): 7.34 (1H, d, J=8.3 Hz), 7.20 (1H, d, J=1.9 Hz), 7.01 (1H, br.s), 6.91-6.96 (1H, m), 6.70-6.77 (3H, m), 4.73 (2H, d, J=2.4 Hz), 4.51-4.72 (2H, m), 3.82 (3H, s), 3.34-3.47 (1H, m), 2.97-3.05 (1H, m), 2.76-2.83 (1H, m), 2.49 (1H, t, J=2.4 Hz), 1.33 (3H, d, J=7.1 Hz)

### Production Example 79

In the same way as in the Production Example 40, 436 mg of N-{(naphthalene-2-yl)methyl}-3-{3-methoxy-4-(2-propynyloxy) phenyl}butanethioamide (referred as the compound of the present invention 79 hereinafter) was obtained from 500 mg of N-{(naphthalene-2-yl)methyl}-3-{3-methoxy-4-(2-propynyloxy) phenyl}butaneamide and 388 mg of Lawesson's Reagent.
the compound of the present invention 79 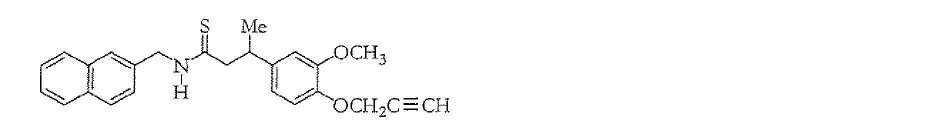 ¹H-NMR (CDCl₃, TMS) δ (ppm): 7.75-7.81 (3H, m), 7.46-7.52 (3H, m), 7.06 (1H, dd, J=8.3 Hz, 1.8 Hz), 6.93 (1H, br.s), 6.87 (1H, d, J=7.9 Hz), 6.77 (1H, d, J-1.9 Hz), 6.74 (1H, dd, J=8.1 Hz, 1.9 Hz), 4.71-4.85 (2H, m), 4.64 (2H, d, J=2.4 Hz), 3.79 (3H, s), 3.36-3.49 (1H, m), 2.98-3.06 (1H, m), 2.76-2.84 (1H, m), 2.43 (1H, t, J=2.4 Hz), 1.34 (3H, d, J=7.0 Hz)

### Production Example 80

566 mg of 4-chlorobenzylamine, 937 mg of 3-(4-methoxy-3-(2-propynyloxy) phenyl)propionic acid, 805 mg of 1-ethyl-3-(3-diethylaminopropyl)carbodiimide hydrochloride (referred as WSC hereinafter) and 10 ml of N,N-dimethylformamide were mixed at room temperature for 4 hours. Then water was added to the residue and extracted with ethyl acetate. The organic layer was washed successively with 5 % hydrochloric acid, water and saturated aqueous solution of sodium chloride, dried by magnesium sulfate, and concentrated under reduced pressure. The residue was purified by silica gel column to obtain 847 mg of N-(4-chlorobenzyl)-3-{4-methoxy-3-(2-propynyloxy)phenyl}pro panamide (referred as the compound of the present invention 80 hereinafter).
the compound of the present invention 80 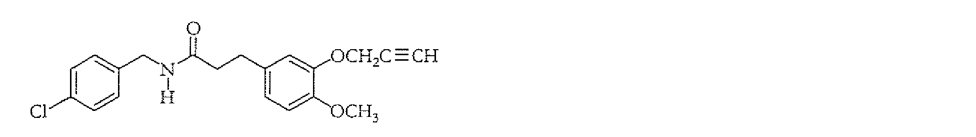 ¹H-NMR (CDCl₃, TMS) δ (ppm) : 7.22-7.27 (2H, m), 7.05 (2H, d, J=7. 5 Hz), 6.76-6.89 (3H, m), 5.55 (1H, br.s), 4.72 (2H, d, J=2.4 Hz), 4.34 (2H, d, J=5.7 Hz), 3.84 (3H, s), 2.93 (2H, t, J=7.4 Hz), 2.44-2.53 (3H, m)

### Production Example 81

517 mg of N-(4-chlorobenzyl)-3-{4-methoxy-3-(2-propynyloxy)phenyl}pro panamide, 419 mg of Lawesson's Reagent and 10 ml of tetrahydrofuran were mixed, and stirred at 65 °C for 5 hours. Then the reaction mixture was cooled, and concentrated under reduced pressure. The residue was purified by silica gel column to obtain 160 mg of N-(4-chlorobenzyl)-3-{4-methoxy-3-(2-propynyloxy)phenyl}pro panthioamide (referred as the compound of the present invention 81 hereinafter).
the compound of the present invention 81 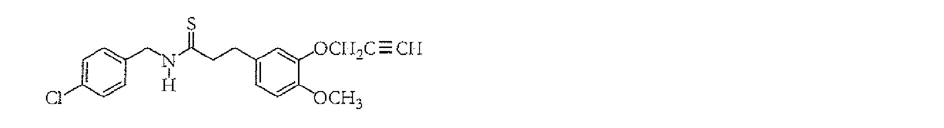 ¹H-NMR (CDCl₃, TMS) 5 (ppm): 7.24-7.26 (3H, m), 7.01 (2H, d, - J=8.2 Hz), 6.88 (1H, d, J=1.4 Hz), C.73-6.77 (2H, m), 4.72 (2H, d, J=2.4 Hz), 4.67 (2H, d, J=5.3 Hz), 3.84 (3H, s), 3.07 (2H, t, J=7.0 Hz), 2. 35 (2H, t, J=7.0 Hz), 2.44 (1H, t, J=2.4 Hz)

### Production Example 82

538 mg of N-(4-chlorobenzyl)-3-{3-methoxy-4-(2-propynyloxy)phenyl}pro panamide and 5 ml of N,N-dimethylformamide were mixed and cooled to 0 °C. 72 mg of sodium hydride was added to the mixture, and it was stirred at 0 °C for about 15 minutes. Then, 196 mg of propargyl bromide was added in it, the mixture was stirred at 0 °C for 30 minutes and at room temperature for 2 hours. After that, water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 105 mg of N-(4-chlorobenzyl)-N-propynyl-3-{4-methoxy-3-(2-propynyloxy )phenyl}propanamide (referred as the compound of the present invention 82 hereinafter).
the compound of the present invention 82 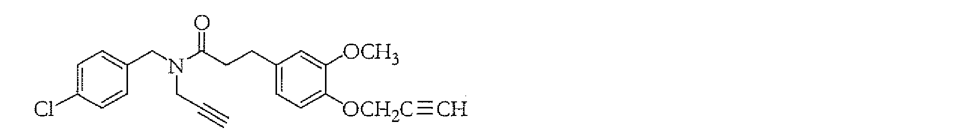 ¹H-NMR (CDCl₃, TMS) δ (ppm) : 7.26-7.30 (2H, m), 7.16 (1H, d, - J=8.2 Hz), 7.02 (1H, d, J=8.2 Hz), 6.91-6.97 (1H, m), 6.67-6.82 (2H, m), 4.73 (2H, d, J=2.1 Hz), 4.64 (1H, s), 4.54 (1H, s), 4.20 (1H, s), 3.84 (3H, s), 3.82 (1H, s), 2.90-3.03 (2H, m), 2.75 (1H, t, J=7.6 Hz), 2.62 (1H, t, J=7.5 Hz), 2.49 (1H, t, J=2.1 Hz), 2.26 (0.5H, s), 2.19 (0.5H, s)

### Production Example 83

943 mg of 4-phenoxybenzylamine hydrochloride, 1.01 g of 3-{3-methoxy-4-(2-propynyloxy) phenyl}propionic acid, 844 mg of WSC and 10 ml of pyridine were mixed at room temperature for 4 hours . Then water was added to the reacton mixture and extracted with ethyl acetate. The organic layer was washed successively with 5 % hydrochloric acid, water and saturated aqueous solution of sodium chloride, dried by magnesium sulfate, and concentrated under reduced pressure. The residue was purified by silica gel column to obtain 1.31 g of N-(4-phenoxybenzyl)-3-{3-methoxy-4-(2-propynyloxy)phenyl}pr opanamide (referred as the compound of the present invention 83 hereinafter).
the compound of the present invention 83 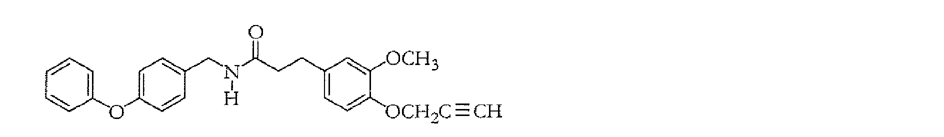 ¹H-NMR (CDCl₃, TMS) δ (ppm): 7.30-7.36 (2H, m), 7.08-7.15 (3H, m), 6.92-7.01 (5H, m), 6.75 (1H, d, J=1.9 Hz), 6.73 (1H, dd, J=8.2 Hz, 2.2 Hz), 5.61 (1H, br.s), 4.71 (2H, d, J=2.4 Hz) , 4.38 (2H, d, J=5.5 Hz), 3.83 (3H, s), 2.95 (2H, t, J=7.5 Hz), 2.50 (2H, t, J=7.5 Hz), 2.46 (1H, t, J=2.4 Hz)

### Production Example 84

In the same way as in the Production Example 40, 250 mg of N-(4-phenoxybenzyl)-3-{3-methoxy-4-(2-propynyloxy)phenyl}pr opanthioamide (referred as the compound of the present invention 84 hereinafter) was obtained from 1.0 g of N-(4-phenoxybenzyl)-3-{3-methoxy-4-(2-propynyloxy)phenyl}pr opameamide and 681 mg of Lawesson's Reagent.
the compound of the present invention 84 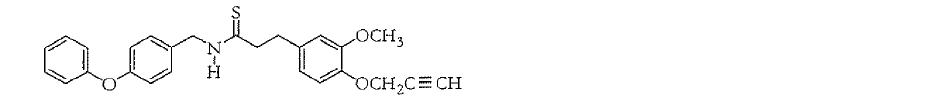 ¹H-NMR (CDCl₃, TMS) δ (ppm): 7.31-7.37 (2H, m), 7.03-7.15 (4H, m), 6.90-7.02 (5H, m), 6.70-6.78 (2H, m), 4.60-4.70 (4H, m), 3.82 (3H, s), 3.09 (2H, t, J=7.2 Hz), 2.93 (2H, t, J=7.2 Hz), 2.44 (1H, t, J=2.4 Hz)

### Production Example 85

By using 300 mg of 3-{3-methoxy-4-(2-propynyloxy)phenyl}propionyl chloride and 209 mg of 2,4-dichlorobenzylamine according to the Production Example 5 was obtained 450 mg of N-(2,4-dichlorobenzyl)-3-{3-methoxy-4-(2-propynyloxy)phenyl }propanamide (referred as the compound of the present invention 85 hereinafter).
the compound of the present invention 85 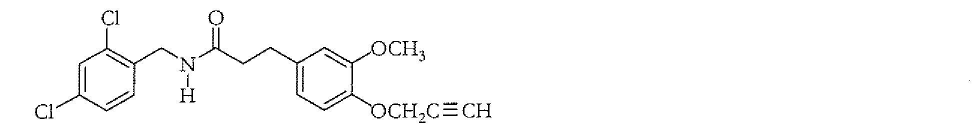 ¹H-NMR (CDCl₃, TMS) δ (ppm): 7.34 (1H, d, J=1.9 Hz), 7.11-7.19 (2H, m), 6.92 (1H, d, J=8.0 Hz), 6.68-6.71 (2H, m), 5.78 (1H, br.s), 4.73 (2H, d, J=2.5 Hz), 4.43 (2H, d, J=6.1 Hz), 3.81 (3H, s), 2.92 (2H, t, J=7.5 Hz), 2.48-2.52 (3H, m)

### Production Example 86

1.19 g of N-(4-hydroxybenzyl)-3-{3-methoxy-4-(2-propynyloxy)phenyl}pr opanamide, 418 mg of propargyl bromide, 531 mg of potassium carbonate and 5 ml of acetonitrile were mixed, and stirred at 80 °C for 3 hours. Then the reaction mixture was cooled to room temperature, and water was added to the reaction mixture and extracted with ethyl acetate. The organic layer was washed successively with 5 % hydrochloric acid, water and saturated aqueous solution of sodium chloride, dried by magnesium sulfate, and concentrated under reduced pressure. The residue was washed by hexane to obtain 1.12 g of N-{4-(2-propynyloxy)benzyl}-3-{3-methoxy-4-(2-propynyloxy)p henyl}propanamide (referred as the compound of the present invention 86 hereinafter).
the compound of the present invention 86 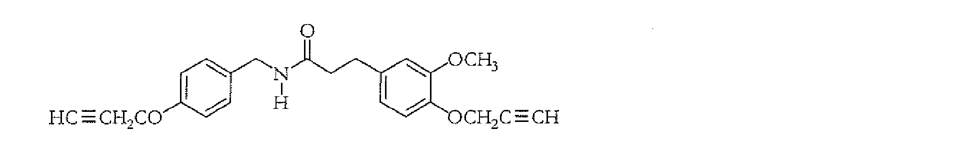 ¹H-NMR (CDCl₃, TMS) δ (ppm): 7.09 (2H, d, J=8.5 Hz), 6.88-6.95 (3H, m), 6.70-6.75 (2H, m), 5.61 (1H, br.s), 4.73 (2H, d, J=2.4 Hz), 4.68 (2H, d, J=2.4 Hz), 4.33 (2H, d, J=5.6 Hz), 3.81 (3H, s), 2.93 (2H, t, J=7.4 Hz), 2.46-2.53 (4H, m)

### Production Example 87

In the same way as in the Production Example 40, 780 mg of N-{4-(2-propynyloxy)benzyl}-3-{3-methoxy-4-(2-propynyloxy)p henyl}propanthioamide (referred as the compound of the present invention 87 hereinafter) was obtained from 793 mg of N-{4-(2-propynyloxy)benzyl}-3-{3-methoxy-4-(2-propynyloxy)p henyl}propanamide and 594 mg of Lawesson's Reagent.
the compound of the present invention 87 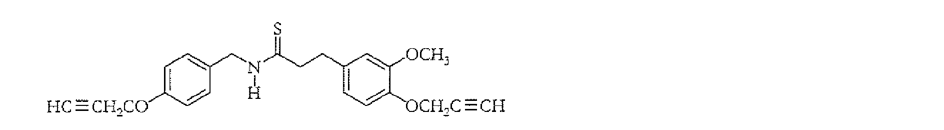 ¹H-NMR (CDCl₃, TMS) δ (ppm) : 7.04-7.09 (2H, m), 7.03 (1H, br.s), 6.90-6.94 (3H, m), 6.75 (1H, d, J=1.9 Hz), 6.72 (1H, dd, J=8.0 Hz, 1.9 Hz), 4.73 (2H, d, J=2.4 Hz), 4.69 (2H, d, J=2.4 Hz), 4.64 (2H, d, J=5.1 Hz), 3.81 (3H, s), 3.08 (2H, t, J=7.1 Hz), 2.92 (2H, t, J=7.2), 2.52 (1H, t, J=2.4 Hz), 2.49 (1H, t, J=2.4)

### Production Example 88

1.9 g of N-(4-chlorobenzyl)-3-{2-chloro-5-methoxy-4-hydroxyphenyl}pr opanamide, 0.57 ml of propargyl bromide, 1.0 g of potassium carbonate and 40 ml of acetonitrile were mixed, and stirred at 80 °C for 1 hour. Then the reaction mixture was cooled to room temperature, and ethyl acetate was added to the reaction mixture and filtered. The filtrate was concentrated under reduced pressure. The obtained residue was washed by hexane to obtain 1.9 g of N-(4-cholorobenzyl)-3-{2-chloro-5-methoxy-4-(2-propynyloxy) phenyl}propanamide (referred as the compound of the present invention 88 hereinafter).
the compound of the present invention 88 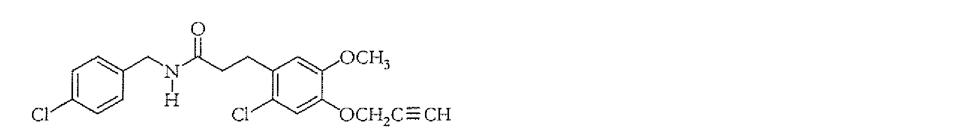 ¹H-NMR (CDCl₃, TMS) δ (ppm): 7.26 (2H, d, J=8.3 Hz), 7.04 (2H, d, J=8.3 Hz), 7.04 (1H, s), 6.78 (1H, s), 5.71 (1H, br.s), 4.73 (2H, d, J=2.2 Hz), 4.36 (2H, d, J=5.9 Hz), 3.79 (3H, s), 3.04 (2H, t, J=7.4 Hz), 2.50-2.54 (3H, m)

### Production Example 89

By using 586 mg of 3-{3-methoxy-4 - (2-propynyloxy) phenyl}propionic acid and 519 mg of (6-methylnaphthalene-2-yl)methylamine hydrochloride according to the Production Example 83 was obtained 850 mg of N-(6-methylnaphthalene-2-yl)-3-{3-methoxy-4-(2-propynyloxy) phenyl}propanamide (referred as the compound of the present invention 89 hereinafter).
the compound of the present invention 89 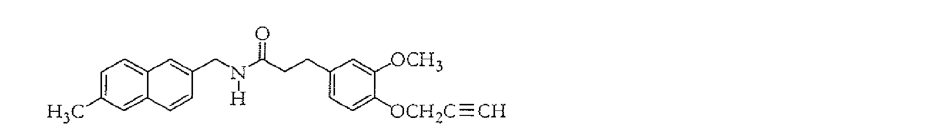 ¹H-NMR (CDCl₃, TMS) 5 (ppm): 7.68 (2H, t, J=9.1 Hz) , 7.57 (2H, d, J=5.1 Hz), 7.31 (1H, d, J=8.3 Hz), 7.23 (1H, d, J=8.3 Hz), 6.91 (1H, d, J=8.3 Hz), 6.70-6.77 (2H, m), 5.63 (1H, br.s), 4.70 (2H, d, J=2.4 Hz), 4.55 (2H, d, J=5.5 Hz), 3.80 (3H, s), 2.97 (2H, t, J=7.5 Hz), 2.49-2.56 (5H, m), 2.46 (1H, t, J=2.4 Hz)

### Production Example 90

By using 810 mg of 3-{3-methoxy-4 - (2-propynyloxy) phenyl}propionic acid and 720 mg of (7-methylnaphthalene-2-yl)methylamine hydrochloride according to the Production Example 83 was obtained 730 mg of N-(7-methylnaphthalene-2-yl)-3-{3-methoxy-4-(2-propynyloxy) phenyl}propanamide (referred as the compound of the present invention 90 hereinafter).
the compound of the present invention 90 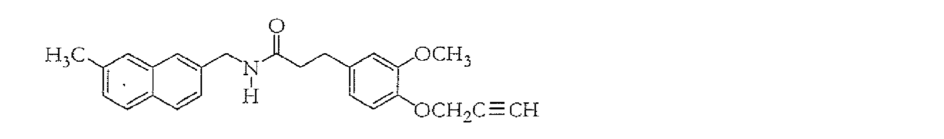 ¹H-NMR (CDCl₃, TMS) δ (ppm) : 7.68-7.75 (2H, m), 7.53 (1H, s), 7.52 (1H, s), 7.29 (1H, dd, J=8.4 Hz, 1.7 Hz), 7.19 (1H, dd, J=8.2 Hz, 1.7 Hz), 6.91 (1H, d, J=8.2 Hz), 6.76 (1H, d, J=1.9 Hz), 6.73 (1H, dd, J=8.0 Hz, 1.9 Hz), 5.64 (1H, br.s), 4.70 (2H, d, J=2.4 Hz), 4.54 (2H, d, J=5.5 Hz), 3.80 (3H, s), 2.96 (2H, t, J=7.5 Hz), 2.49-4.56 (5H, m), 2.46 (1H, t, J=2.4 Hz)

### Production Example 91

In the same way as in the Production Example 40, 292 mg of N-(7-methylnaphthalene-2-yl)-3-{3-methoxy-4-(2-propynyloxy) phenyl}propanthioamide (referred as the compound of the present invention 91 hereinafter) was obtained from 420 mg of N-(7-methylnaphthalene-2-yl)-3-{3-methoxy-4-(2-propynyloxy) phenyl}propanamide and 310 mg of Lawesson's Reagent.
the compound of the present invention 91 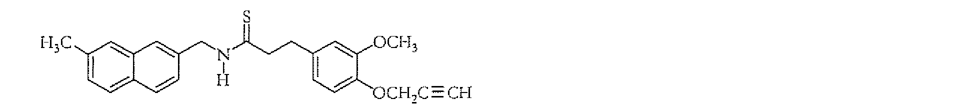 ¹H-NMR (CDCl₃, TMS) δ (ppm): 7.74 (1H, d, J=8.2 Hz), 7.72 (1H, d, J=8.4 Hz), 7.55 (1H, s), 7.51 (1H, s), 7.32 (1H, dd, J=8.3 Hz, 1.5 Hz), 7.13 (1H, dd, J=8.2 Hz, 1.7 Hz), 7.10 (1H, br.s), 6.88 (1H, d, J=8.2 Hz), 6.77 (1H, d, J=1.9 Hz), 6.72 (1H, dd, J=8.3 Hz, 1.8 Hz), 4.85 (2H, d, J=5.1 Hz), 4.67 (2H, d, J=2.4 Hz), 3.80 (3H, s), 2.96 (2H, t, J=7.2 Hz), 3.10 (2H, t, J=7.2 Hz), 2.51 (3H, s), 2.44 (1H, t, J=2.4 Hz)

### Production Example 92

0.2 g of 3-{3-methoxy-4-(2-propynyloxy)phenyl}propionyl chloride, 0.12 g of 1-(4-chlorophenyl)ethylamine, 0.2 ml of triethylamine and 5 ml of tetrahydrofuran were mixed, and stirred at room temperature for 30 minutes. Then water was added to the reaction mixture, and extracted with ethyl acetate. The organic layer was washed successively with 5 % of hydrochloric acid, saturated aqueous solution of sodium bicarbonate and saturated aqueous solution of sodium chloride, dried by magneium sulfate, and concentrated under reduced pressure. The residue was washed by hexane to obtain 0.21 g of N-{1-(4-chlorophenyl)ethyl}-3-{3-methoxy-4-(2-propynyloxy)p henyl}propanamide (referred as the compound of the present invention 92 hereinafter).
the compound of the present invention 92 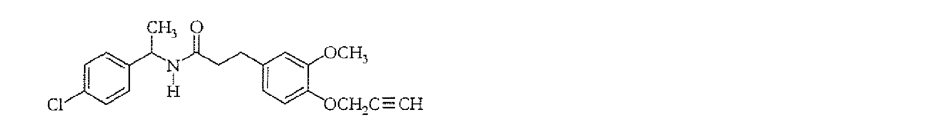 ¹H-NMR (CDCl₃, TMS) δ (ppm) : 7.25-7 .27 (2H, m), 7.09 (2H, d, J=8.0 Hz), 6.93 (1H, d, J=8.0Hz), 6.69-6.71 (2H, m), 5.67 (1H, br.d), 5.03-5.06 (1H, m), 4.73-4.74 (2H, m), 3.80 (3H, s), 2.91 (2H, t, J=6.8 Hz), 2.41-2.47 (3H, m), 1.38 (3H, d, J=7.0 Hz)

### Production Example 93

By using 0.84 g of 3-{3-methoxy-4(2-propynyloxy)phenyl}propionic acid and 0.85 g of 1-(4-chlorophenyl)-3-butunylamine according to the Production Example 92 was obtained 0.40 g of N-{1-(4-chlorophenyl)-3-butynyl}-3-{3-methoxy-4-(2-propynyl oxy)phenyl}propanamide (referred as the compound of the present invention 93 hereinafter).
the present invention 93 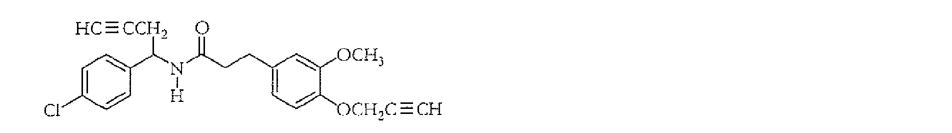 ¹H-NMR (CDCl₃, TMS) δ (ppm): 7.28 (2H, d, J=8.5 Hz), 7.12 (2H, d, J=8.5 Hz), 6.95 (1H, d, J=8.8 Hz), 6.72-6.74 (2H, m), 5.85 (1H, br.d, J=8.1 Hz), 5.14 (1H, dt, J=8.0 Hz, 5.6 Hz), 4.75 (2H, d, J=2.2 Hz), 3.80 (3H, s), 2.93 (2H, t, J=7.1 Hz), 2.63-2.67 (2H, m), 2.47-2.57 (3H, m), 1.99 (1H, t, J=2.7 Hz)

### Production Example 94

0.30 g of 3-{3-methoxy-4- (2-propynyloxy)phenyl}propionic acid, 0.24 g of 1-(3,4-dichlorophenyl)ethylamine, 0.26 g of WSC and 10 ml of dimethylformamide were mixed, and stirred at room temperature for 3 hours. Then water was added to the reaction mixture and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was washed by hexane to obtain 0.25 g of N-{1-(3,4-dichlorophenyl)ethyl}-3-{3-methoxy-4-(2-propynylo xy) phenyl}propanamide (referred as the compound of the present invention 94 hereinafter).
the compound of the present invention 94 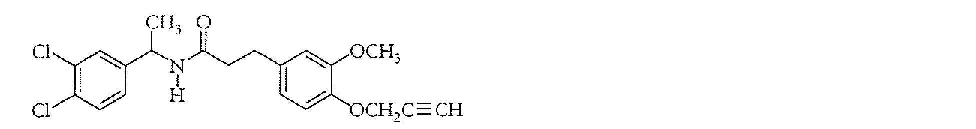 ¹H-NMR (CDCl₃, TMS) δ (ppm): 7.34 (1H, d, J=8.5 Hz), 7.30 (1H, d, J=2.2 Hz) 6.98 (1H, dd, J=8.5 Hz, 2.2 Hz), 6.94 (1H, d, J=8.2 Hz), 6.70-6.72 (2H, m), 5.67 (1H, br.d, J=7.5 Hz), 5.00-5.04 (1H, m), 4.74 (2H, d, J=2.5 Hz), 3.81 (3H, s), 2.91 (2H, t, J=7.2 Hz), 2.47-2.50 (3H, m), 1.37 (3H, d, J=6.8 Hz)

### Production Example 95

By using 0.30 g of 3-{3-methoxy-4-(2-propynyloxy)phenyl}propionic acid and 0.29 g of 1-(naphthalene-2-yl) ethylamine according to the Production Example 94 was obtained 0.23 g of N-{1-(naphthalene-2-yl)ethyl}3-{3-methoxy-4-(2-propynyloxy) phenyl}propanamide (referred as the compound of the present invention 95 hereinafter).
the present invention 95 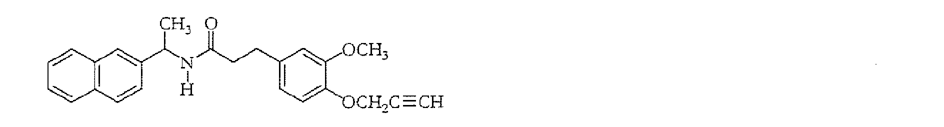 ¹H-NMR (CDCl₃, TMS) δ (ppm): 7.78-7.90 (3H, m), 7.65 (1H, s), 7.47-7.48 (2H, m), 7.28-7.30 (1H, m), 6.90 (1H, d, J=8.0 Hz), 6.70-6.72 (2H, m), 5.60 (1H, br.d, J=7.0 Hz), 5.31-5.24 (1H, m), 4.70 (2H, m), 3.76 (3H, s), 2.93 (2H, t, J=7.2 Hz), 2.46-2.50 (3H, m), 1.51 (3H, d, J=6.8 Hz)

### Production Example 96

By using 0.30 g of 3-{3-methoxy-4-(2-propynyloxy)phenyl}propionic acid and 0.22 g of 1-(5, 6, 7,8 tetrahydronaphthalene-2-yl) ethylamine according to the Production Example 94 was obtained 0.22 g of N-{1-(5,6,7,8tetrahydronaphthalene-2-yl)ethyl}3-{3-methoxy-4-(2-propynyloxy)phenyl}propanamide (referred as the compound of the present invention 97 hereinafter).
the present invention 96 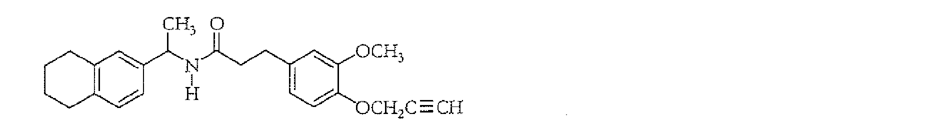 ¹H-NMR (CDCl₃, TMS) δ (ppm): 7.01 (1H, d, J=8.5 Hz), 6.92-5.95 (3H, m), 6.71-6.73 (2H, m), 5.47 (1H, br.d, J=7. 2 Hz), 5.00-5.07 (1H, m), 4.73 (2H, d, J-2.4 Hz), 3.81 (3H, s), 2. 92 (2H, t, J=7.5 Hz), 2.70-2.75 (4H, m), 2.48 (1H, t, J=2.5 Hz), 2.44 (2H, t, J=7.5 Hz), 1.76-1.86 (4H, m), 1.40 (3H, d, J=7.0 Hz)

### Production Example 97

By using 0.30 g of 3-{3-fluoro-5-methoxy-4-(2-propynyloxy)phenyl}propionic acid and 0.22 g of 4-chlorobenzylamine according to the Production Example 94 was obtained 0.23 g of N-(4-chlorobenzyl)-3-{3-fluoro-5-methoxy-(2-propynyloxy)phe nyl}propanamide (referred as the compound of the present invention 97 hereinafter).
the compound of the present invention 97 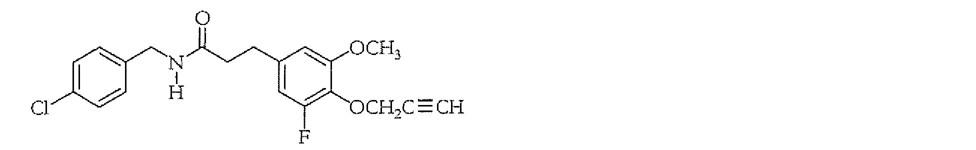 ¹H-NMR (CDCl₃, TMS) δ (ppm): 7.28 (2H, d, J=8.3 Hz), 7.11 (2H, d, J=8.3 Hz), 6.54-6.58 (2H, m), 5.65 (1H, br.s), 4.73 (2H, d, J=2.4 Hz), 4.38 (2H, d, J=5.9 Hz), 3.81 (3H, s), 2.93 (2H, t, J=7.5 Hz), 2.49 (2H, t, J=7.5 Hz), 2.45 (1H, t, J=2.4 Hz)

### Production Example 98

By using 196 mg of 3-{3-methoxy-4-(2-propynyloxy)phenyl}propionic acid and 234 mg of 4-chloro-3-fluorobenzylamine hydrochloride according to the Production Example 83 was obtained 310 mg of N-(4-chloro-3-fluorobenzyl)-3-{3-methoxy-4-(2-propynyloxy)p henyl}propanamide (referred as the compound of the present invention 98 hereinafter).
the compound of the present invention 98 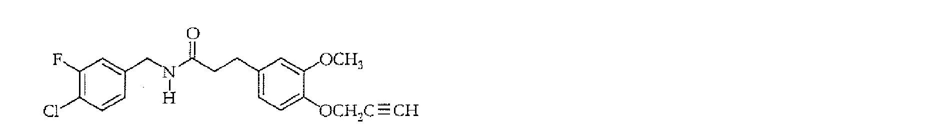 ¹H-NMR (CDCl₃, TMS) δ (ppm): 7.30 (1H, t, J=7.8 Hz), 6.92-6.98 (2H, m), 6.82-6.86 (1H, m), 6.71-6.76 (2H, m), 5.66 (1H, br.s), 4.74 (2H, d, J=2.4 Hz), 4.36 (2H, d, J=6.1 Hz), 3.83 (3H, s), 2.95 (2H, t, J=7.4 Hz), 2.52 (2H, t, J=7.4 Hz), 2.49 (1H, t, J=2.4 Hz)

### Production Example 99

By using 196 mg of 3-{3-methoxy-4-(2-propynyloxy)phenyl}propionic acid and 234 mg of 4-chloro-2-fluorobenzylaminne hydrochloride according to the Production Example 83 was obtained 330 mg of N-(4-chloro-2-fluorobenzyl)-3-{3-methoxy-4-(2-propynyloxy)p henyl}propanamide (referred as the compound of the present invention 99 hereinafter).
the compound of the present invention 99 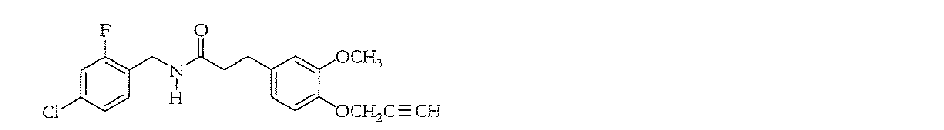 ¹H-NMR (CDCl₃, TMS) δ (ppm) : 7.03-7.15 (3H, m), 6.92 (1H, d, J=8.0 Hz), 6.67-6.72 (2H, m), 5.69 (1H, br.s), 4.73 (2H, d, J=2.4 Hz), 4.39 (2H, d, J=6.1 Hz), 3.81 (3H, s), 2.92 (2H, t, J=7.5 Hz), 2.46-2.51 (3H, m)

### Production Example 100

By using 562 mg of 3-{3-methoxy-4-(2-propynyloxy)phenyl}propionic acid and 508 mg of (6-fluoronaphthalene-2-yl) methylamine hydrochloride according to the Production Example 83 was obtained 540 mg of N-{(6-fluoronaphthalene-2-yl)methyl}-3-{3-methoxy-4-(2-prop ynyloxy)phenyl}propanamide (referred as the compound of the present invention 100 hereinafter).
the compound of the present invention 100 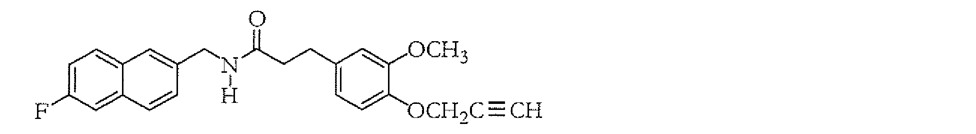 ¹H-NMR (CDCl₃, TMS) δ (ppm): 7.70-7.78 (2H, m), 7.59 (1H, s), 7.42 (1H, dd, J=9.8 Hz, 2.2 Hz), 7.22-7.30 (2H, m), 6.91 (1H, d, J=8.0 Hz), 6.72-6.77 (2H, m), 5.68 (1H, br.s), 4.71 (2H, d, J=2.4 Hz), 4.55 (2H, d, J=5.6 Hz), 3.79 (3H, s), 2.97 (2H, t, J=7.1 Hz), 2.53 (2H, t, J=7.1 Hz), 2.46 (1H, t, J=2.4 Hz)

### Production Example 101

10.7 g of 4-(2-methyl-[1,3]dioxolane-2-yl)benzylamine, 11.8g of 3-{3-methoxy-4-(2-propynyloxy)phenyl}propionic acid, 10. 6 g of WSC and 150 ml of dimethylformamide were mixed at room temperature for 2 hours. Then water was added to the reaction mixture, and extracted with ethyl acetate. The organic layer was washed successively with 5 % hydrochloric acid, water and saturated aqueous solution of sodium chloride, dried by magnesium sulfate, and concentrated under reduced pressure. About 100 ml of acetone and about 0.1 g of p-toluenesulfonic acid were added to the residue, then the mixture was stirred at 50 °C for 3 hours. The mixture was cooled, added saturated aqueous solution of sodium bicarbonate was added to the mixture, and concentrated. Ethyl acetate was added to the residue, washed with water, dried by dried by magnesium sulfate, and concentrated under reduced pressure. The obtained solid was washed by hexane to obtain 12.5 g of N-(4-methylcarbonylbenzyl)-3-{3-methoxy-4-(2-propynyloxy)ph enyl}propanamide (referred as the compound of the present invention 101 hereinafter).
the compound of the present invention 101 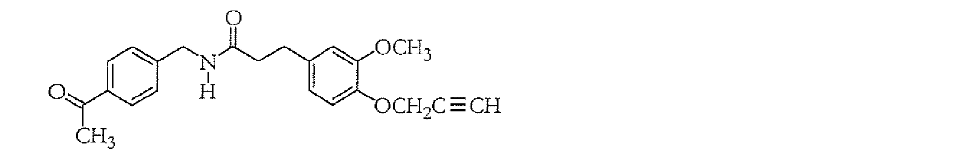 ¹H-NMR (CDCl₃, TMS) 5 (ppm) : 7.87 (2H, d, J=8.3 Hz), 7.20 (2H, d, J=8.3 Hz), 6.94 (1H, d, J=7.5 Hz), 6.72-6.74 (2H, m), 5.73 (1H, br.s), 4.74 (2H, d, J=2. 4 Hz), 4.50 (2H, d, J=6.3 Hz), 3.81 (3H, s), 2.59 (3H, s), 2.53 (2H, t, J=7.5Hz), 2.96 (2H, t, J=7.5 Hz), 2.50 (1H, J=2.4 Hz)

### Production Example 102

0.52 g of N-(4-methylcarbonylbenzyl)-3-{3-methoxy-4-(2-propynyloxy)ph enyl}propanamide, 0.18 g of O-methoxyhydroxylamine hydrochloride, 0.16 ml of pyridine and 10 ml of ethanol were mixed, and stirred at 50 °C for 2 hours. Then water was added to the reaction mixture, and extracted with ethyl acetate. The organic layer was washed successively with 5 % hydrochloric acid twice, saturated aqueous solution of sodium bicarbonate and saturated aqueous solution of sodium chloride, dried by magnesium sulfate, and concentrated under reduced pressure. The obtained solid was washed by hexane to obtain 0.49 g of N-(4-(1-methoxyiminoethyl)benzyl)-3-{3-methoxy-4-(2-propyny loxy)phenyl}propanamide (referred as the compound of the present invention 102 hereinafter). 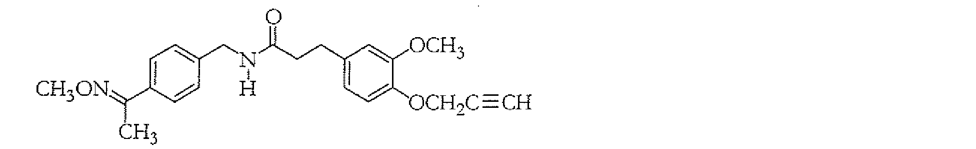 ¹H-NMR (CDCl₃, TMS) δ (ppm): 7.58 (2H, d, J=8.0 Hz), 7.40 (2H, d, J=8.0 Hz), 6.94 (1H, d, J=8.0 Hz), 6.71-6.75 (2H, m), 5.59 (1H, br.s), 4.73 (2H, d, J=2.4 Hz), 4.41 (2H, d, J=6.0 Hz), 3. 98 (3H, s), 3.82 (3H, s), 2.95 (2H, t, J=7.6 Hz), 2.49-2.52 (3H, m), 2.20 (3H, s)

### Production Example 103

0.50 g of N-(4-methylcarbonylbenzyl)-3-{3-methoxy-4-(2-propynyloxy)ph enyl}propanamide, 0.26 g of O-benzyloxyhydroxylamine hydrochloride, 0.12 ml of pyridine and 10 ml of ethanol were mixed, and stirred at 50 °C for 2 hours. Then water was added to the reaction mixture, and extracted with ethyl acetate. The organic layer was washed successively with 5 % hydrochloric acid twice, saturated aqueous solution of sodium bicarbonate and saturated aqueous solution of sodium chloride, dried by magnesium sulfate, and concentrated under reduced pressure. The obtained solid was washed by hexane to obtain 0.54 g of N-(4-(1-benzyloxyiminoethyl)benzyl)-3-{3-methoxy-4-(2-propy nyloxy)phenyl}propanamide (referred as the compound of the present invention 103 hereinafter). 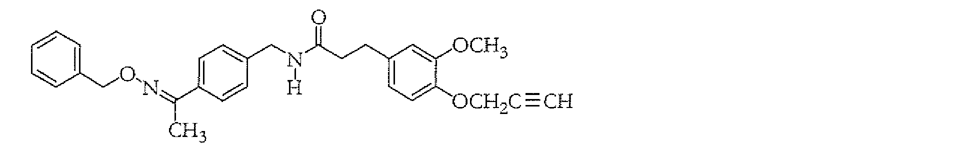 ¹H-NMR (CDCl₃, TMS) δ (ppm): 7.57 (2H, d, J=7.9 Hz), 7.20-7.46 (5H, m), 7.13 (2H, d, J=7. 9 Hz), 6.93 (1H, d, J=7.5 Hz), 6.71-6.74 (2H, m), 5.58 (1H, br.s), 5.53 (2H, s), 4.71-4.72 (2H, m), 4.39 (2H, d, J=5.5 Hz), 3.81 (3H, s), 2.94 (2H, t, J=7.5Hz), 2.48-2.51 (3H, m), 2.24 (3H, s)

### Production Example 104

0.50 g of N-(4-methylcarbonylbenzyl)-3-{3-methoxy-4-(2-propynyloxy)ph enyl}propanamide, 0.21ml of 1,1-dimethylhydrazine, 1ml of acetic acid and 10 ml of methanol were mixed, and stirred under the condition of reflux for 1 hour. Then water was added to the reaction mixture, and extracted with ethyl acetate. The organic layer was washed successively with 5 % hydrochloric acid twice, saturated aqueous solution of sodium bicarbonate and saturated aqueous solution of sodium chloride, dried by magnesium sulfate, and concentrated under reduced pressure. The obtained solid was washed by hexane to obtain 0.46 g of N-(4-(1-dimethylaminoimino)ethyl)benzyl)-3-(3-methoxy-4-(2-propynyloxy)phenyl}propanamide (referred as the compound of the present invention 104 hereinafter).
the compound of the present invention 104 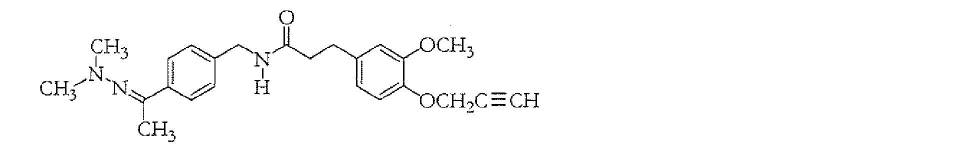 ¹H-NMR (CDCl₃, TMS) δ (ppm): 7.66 (2H, d, J=8.0 Hz), 7.14 (2H, d, J=8.0 Hz), 6.94 (1H, d, J=8.0 Hz), 6.72-6.76 (2H, m), 5.58 (1H, br.s), 4.73 (2H, d, J=2.4 Hz), 4.41 (2H, d, J=5.6 Hz), 3.82 (3H, s), 2.95 (2H, t, J=7.2 Hz), 2.59 (6H, s), 2.48-2.52 (3H, m), 2.33 (3H, s)

### Production Example 105

By using 13.0 g of 4-([1,3]dioxolane-2-yl)benzylamine and 15.5 g of 3-{3-methoxy-4-(2-propynyloxy)phenyl}propionic acid according to the Production Example 101 was obtained 14.7 g of N-(4-formylbenzyl)-3-{3-methoxy-4-(2-propynyloxy)phenyl}pro panamide (referred as the compound of the present invention 105 hereinafter).
the compound of the present invention 105 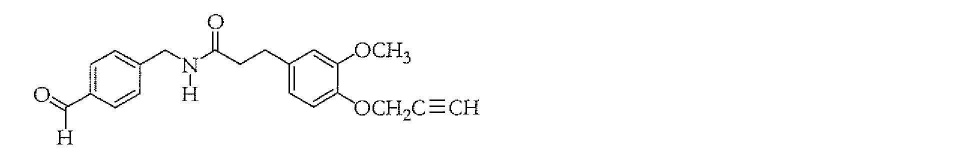 ¹H-NMR (CDCl₃, TMS) 5 (ppm): = 9.98 (1H, s), 7.79-7.81 (2H, m), 7.26-7.28 (2H, m), 6.35 (1H, d, J=7.9 Hz), 6.71-6.75 (2H, m), 5.74 (1H, br.s), 4.74 (2H, d, J=2.4 Hz), 4.48 (2H, d, J=5.5 Hz), 3.82 (3H, s), 2.96 (2H, t, J=7.5 Hz), 2.55 (2H, t, J=7.5 Hz), 2.50 (2H, t, J=2.4 Hz)

### Production Example 106

By using 0.54 g of N-(4-formylbenzyl)-3-{3-methoxy-4-(2-propynyloxy)phenyl}pro panamide, 0.15 g of O-methoxyhydroxylamine hydrochloride according to the Production Example 102 was obtained 0.54 g of N-(4-methoxyiminomethylbenzyl)-3-{3-methoxy-4-(2-propynylox y)phenyl}-propanamide (referred as the compound of the present invention 106 hereinafter).
the compound of the present invention 106 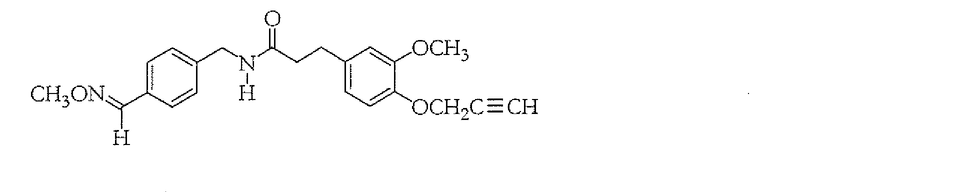 ¹H-NMR (CDCl₃, TMS) δ (ppm): 8.03 (1H, s), 7.51 (2H, d, J=8.0 Hz), 7.14 (2H, d, J=8.0 Hz), 6.94 (1H, d, J=8.0 Hz), 6.72-6.76 (2H, m), 5.61 (1H, br.s), 4.74 (2H, d, J=2.4 Hz), 4.42 (2H, d, J=4.8 Hz), 3.97 (3H, s), 3.82 (3H, s), 2.95 (2H, t, J=7.6 Hz), 2.48-2.53 (3H, m)

### Production Example 107

By using 0.56 g of N-(4-formylbenzyl)-3-{3-methoxy-4-(2-propynyloxy)phenyl}pro panamide, 0.31 g of O-benzyloxyhydroxylamine hydrochloride according to the Production Example 103 was obtained 0.64 g of N-(4-benzyloxyiminomethylbenzyl)-3-{3-methoxy-4-(2-propynyl oxy)phenyl}propanamide (referred as the compound of the present invention 107 hereinafter).
the compound of the present invention 107 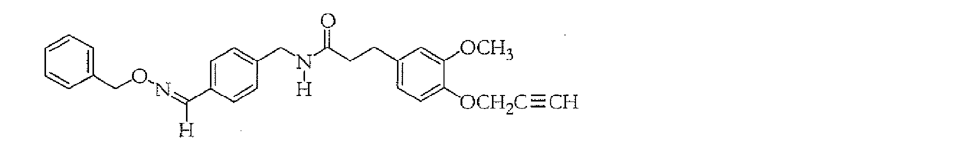 ¹H-NMR (CDCl₃, TMS) δ (ppm): 8.10 (1H, s), 7.50 (2H, d, J=8.1 Hz), 7.29-7.42 (5H, m), 7.13 (2H, d, J=8.1 Hz), 6.93 (1H, d, J=7.9 Hz), 6.71-6.74 (2H, m), 5.58 (1H, br.s), 5.20 (2H, s), 4.72 (2H, d, J=2.4 Hz), 4.40 (2H, d, J=5.5 Hz), 3.82 (3H, s), 2.95 (2H, t, J=7.5 Hz), 2.48-2.53 (3H, m)

### Production Example 108

3.48 g of N-(4-formylbenzyl)-3-{3-methoxy-4-(2-propynyloxy)phenyl}pro panamide and 50 ml of ethanol were mixed, and 0.10 g of sodium borohydride was mixed with it at 0°C and followed by stirring at room temperature for 3 hours, then 0.10 g of sodium borohydride was added again followed by stirring at room temperature for 1 hour. After that, saturated aqueous solution of ammonium chloride and water were added to the reaction mixture, then concentrated under reduced pressure. The residue was extracted with ethyl acetate. The organic layer was washed successively with saturated aqueous solution of sodium chloride, dried by magnesium sulfate, and concentrated under reduced pressure. The obtained solid was washed by hexane to obtain 3.7 g of N-(4-hydroxymethylbenzyl)-3-{3-methoxy-4-(2-propynyloxy)phe nyl}propanamide (referred as the compound of the present invention 108 hereinafter).
the compound of the present invention 108 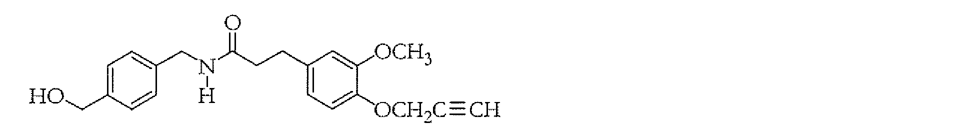 ¹H-NMR (CDCl₃, TMS) δ (ppm): 7.29 (2H, d, J=7.9 Hz), 7.13 (2H, d, J=7.9 Hz), 6.93 (1H, d, J=8.7 Hz), 6.70-6.73 (2H, m), 5.58 (1H, br.s), 4.73 (2H, d, J=2. 4 Hz), 4.67 (2H, d, J=5.5 Hz), 4.38 (2H, d, J=5.9 Hz), 3.81 (3H, s), 2.94 (2H, t, J=7.3 Hz), 2.48-2.51 (3H, m), 1.75 (1H, br.t)

### Production Example 109

1.5 g of N-(4-hydroxymethylbenzyl)-3-{3-methoxy-4-(2-propynyloxy)phe nyl}propanamide, 0.71 ml of triethylamine and 50 ml of tetrahydrofuran were mixed, 0.35 ml of methansulfonyl chloride was mixed with it at 0°C and followed by stirring at room temperature for 30 minutes. Then water was added to the residue and concentrated under reduced pressure. The residue was extracted with ethyl acetate. The organic layer was washed successively with 5 % hydrochloric acid, water and saturated aqueous solution of sodium chloride, dried by magnesium sulfate, and concentrated under reduced pressure. The obtained residue was washed by hexane to obtain 1.7 g of 4-{(3-{3-methoxy-4-(2-propynyloxy)phenyl}propionyl)aminomet hyl}benzyl methansulfonate (referred as the compound of the present invention 109 hereinafter).
the compound of the present invention 109 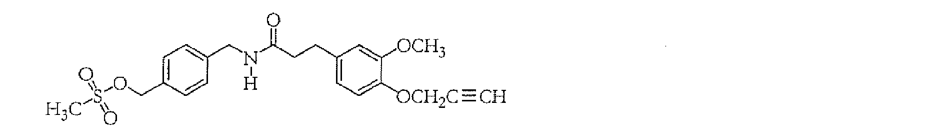 ¹H-NMR (CDCl₃, TMS) 5 (ppm): 7.34 (2H, d, J=8.1 Hz), 7.16 (2H, d, J=8.1 Hz), 6.95 (1H, d, J=7.9 Hz), 6.72-6.75 (2H, m), 5.61 (1H, br.s), 5.21 (2H, s), 4.74 (2H, d, J=2.8 Hz), 4.42 (2H, d, J=5.5Hz), 3.82 (3H, s), 2.94-2.97 (5H, m), 3.82 (3H, s), 2.49-2.54 (3H, m)

### Production Example 110

1.2 g of N-(4-formylbenzyl)-3-{3-methoxy-4-(2-propynyloxy)phenyl}pro panamide and 30 ml of methylene chloride were mixed, 5.3 g of triphenylphosphine and 3.6 g of carbontetrabromide were mixed with it under ice cooling followed by stirring under ice cooling for 2 hours. Then ethyl acetate was added to the reaction mixture, and it was washed successively with saturated aqueous solution of sodium bicarbonate and saturated aqueous solution of sodium chloride, dried by magnesium sulfate, and concentrated under reduced pressure. The residue was purified by silica gel column to obtain 0.2 g of N-(4-(2,2-dibromovinyl)benzyl)-3-{3-methoxy-4-(2-propynylox y)phenyl}propanamide (referred as the compound of the present invention 110 hereinafter).
the compound of the present invention 110 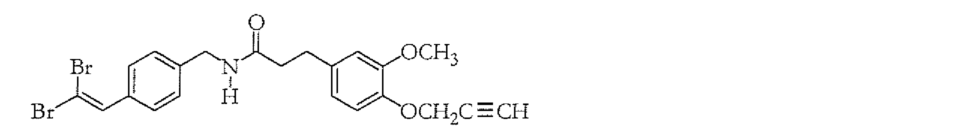 ¹H-NMR (CDCl₃, TMS) δ (ppm) : 7.75-7.47 (3H, m), 7. 13 (2H, d, J=7. 9 Hz), 6.94 (1H, d, J=7.9 Hz), 6.72-6.75 (2H, m), 5.60 (1H, br.s), 4.73 (2H, d, J=2.4 Hz), 4.39 (2H, d, J=5.9 Hz), 3.82 (3H, s), 2.95 (2H, t, J=7.1 Hz), 2.48-2.53 (3H, m)

### Production Example 111

0.37 g of 4-{(3-{3-methoxy-4-(2-propynyloxy)phenyl}propionyl)aminomet hyl}benzyl methansulfonate, 2.0 g of lithium bromide and 20 ml of acetone were mixed, and stirred under the condition of reflux for 3 hours. Then ethyl acetate was added to the reaction mixture. It was washed with water, dried by magnesium sulfate, and concentrated under reduced pressure. The obtained solid was washed by hexane to obtain 0.34 g of N-(4-bromomethylbenzyl)-3-{3-methoxy-4-(2-propynyloxy)pheny 1 }propanamide (referred as the compound of the present invention 111 hereinafter).
the compound of the present invention 111 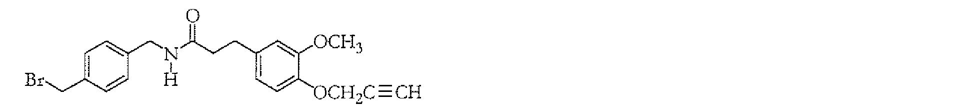 ¹H-NMR (CDCl₃, TMS) δ (ppm) : 7.32 (2H, d, J=8.0 Hz), 7.11 (2H, d, J=8.0 Hz), 6.95 (1H, d, J=8.2 Hz), 6.71-6.75 (2H, m), 5.59 (1H, br.s), 4.74 (2H, d, J=2.4 Hz), 4.72 (2H, s), 4.67 (2H, d, J=5.1 Hz), 3.82 (3H, s), 2.95 (2H, t, J=7.5 Hz), 2.48-2.52 (3H, m)

### Production Example 112

0.23 g of 4-{(3-{3-methoxy-4-(2-propynyloxy)phenyl}propionyl)aminomet hyl}benzyl methansulfonate, 0.11 g of sodium methoxied (28 % methanol solution) and 5 ml of tetrahydrofuran were mixed and stirred at room temperature for 2 hours. Then ethyl acetate was added to the reaction mixture. It was washed with water and saturated aqueous solution of sodium bicarbonate, dried by magnesium sulfate, and concentrated under reduced pressure. The obtained solid was washed by hexane to obtain 0.15 g of N-(4-methoxymethylbenzyl)-3-{3-methoxy-4-(2-propynyloxy)phe nyl}propanamide (referred as the compound of the present invention 112 hereinafter).
the compound of the present invention 112 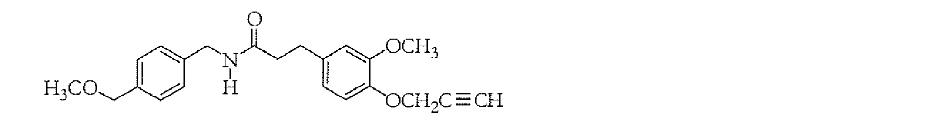 ¹H-NMR (CDCl₃, TMS) δ (ppm) : 7.27 (2H, d, J=7.9 Hz), 7.14 (2H, d, J=7.9 Hz), 6.93 (1H, d, J=8.3 Hz), 6.71-6.75 (2H, m), 5.58 (1H, br.s), 4.73 (2H, d, J=2.4 Hz), 4.43 (2H, s), 4.40 (2H, d, J=5.5 Hz), 3.82 (3H, s), 3.37 (3H, s), 2.95 (2H, t, J=7.5 Hz), 2.48-2.53 (3H, m)

### Production Example 113

52 mg of phenol, 5 ml of dimethylformamide and 24 mg of 55 % sodium hydride were mixed and stirred at room temperature for 15 minutes. 0.20 g of 4-{(3-{3-methoxy-4-(2-propynyloxy)phenyl}propionyl)aminomet hyl}benzyl methansulfonate was mixed with the reaction mixture and stirred at room temperature for 3 hours. Then ethyl acetate was added to the reaction mixture, and washed with 5 % hydrochloric acid, water, saturated aqueous solution of sodium bicarbonate and saturated aqueous solution of sodium chloride. After that, it was dried by magnesium salfate, and concentrated under reduced pressure. The obtained solid was purified by silica gel column to obtain 97 mg of N- (4-phenoxymethylbenzyl) -3-{3-methoxy-4- (2-propynyloxy)phe nyl}propanamide (referred as the compound of the present invention 113 hereinafter).
the compound of the present invention 113 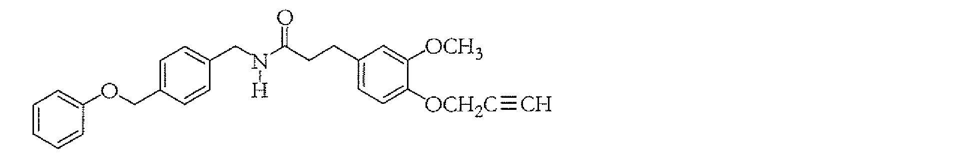 ¹H-NMR (CDCl₃, TMS) δ (ppm): 7.37 (2H, d, J=8.1 Hz), 7.26-7.30 (2H, m), 7.17 (2H, d, J=8.1 Hz), 6.93-6.99 (4H, m), 6.71-6.75 (2H, m), 5.57 (1H, br.s), 5.04 (2H, s), 4.73 (2H, d, J=2.4 Hz), 4.41 (2H, d, J=5.6 Hz), 3.82 (3H, s), 3.37 (3H, s), 2.95 (2H, t, J=7.5 Hz), 2.47-2.52 (3H, m)

### Production Example 114

By using 1.0 g of N-(4-methylcarbinylbenzyl)-3-{3-methoxy-4-(2-propynyloxy)ph enyl}propanamide, 0.06 g of sodium borohydride according to the Production Example 108 was obtained 1.0 g of N-{4-(1-hydroxyethyl)benzyl}-3-{3-methoxy-4-(2-propynyloxy) phenyl}propanamide (referred as the compound of the present invention 114 hereinafter).
the compound of the present invention 114 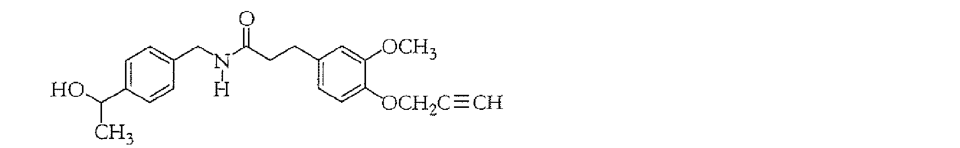 ¹H-NMR (CDCl₃, TMS) δ (ppm): 7.29 (2H, d, J=8. 1 Hz), 7.12 (2H, d, J=8.1 Hz), 6.93 (1H, d, J=8.5 Hz), 6.69-6.71 (2H, m), 5.63 (1H, br. s), 4.87-4.88 (1H, m), 4.72 (2H, d, J=2.5 Hz), 4.37 (2H, d, J=5.8 Hz), 3.80 (3H, s), 2.94 (2H, t, J=7.5 Hz), 2.47-2.53 (3H, m), 1.63 (1H, br.s), 1.47 (3H, d, J=6.5 Hz)

### Production Example 115

By using 0.30 g of C-(6,7,8,9-tetrahydro-5H-benzocycloheptene-2-yl)methylamine and 0.30 g of 3-{3-methoxy-4-(2-propynyloxy)phenyl}propionic acid according to the Production Example 94 was obtained 0.34 g of N-{(6,7,8,9-tetrahydro-5H-benzocycloheptene-2-yl)methyl}-3-{3-methoxy-4-(2-propynyloxy)phenyl}propanamide (referred as the compound of the present invention 115 hereinafter).
the compound of the present invention 115 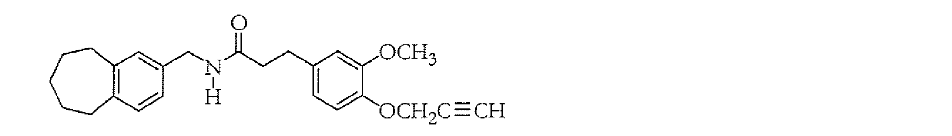 ¹H-NMR (CDCl₃, TMS) δ (ppm) : 7.03 (1H, d, J=7.6 Hz), 6.93-6.95 (2H, m), 6.88 (1H dd, J=7.6 Hz, 1.7 Hz), 6.72-6.76 (2H, m), 5.56 (1H, br.s), 4.73 (2H, d, J=2.4 Hz), 4.34 (2H, d, J=5.6 Hz), 3. 83 (3H, s), 2. 95 (2H, t, J=7.5 Hz), 2.74-2.77 (4H, m), 2.46-2.50 (3H, m), 1.80-1.85 (2H, m), 1.59-1.65 (4H, m)

### Production Example 116

0.3 g of 2-fluoro-3-{3-methoxy-4-(2-propynyloxy)phenyl}-propionic acid, 0.15 ml of thionyl chloride and 5 ml of toluene were mixed, and stirred at 80 °C for 1 hour. Aftercoolingtoroomtemperature, it was concentrated under reduced pressure. The obtained residue was mixed to the mixture of 0.22 g of 4-chlorobenzylamine, 0.5 ml of triethylamine and tetrahydrofuran at 0 °C, and stirred at room temperature for 1 hour. Water and ethyl acetate were added to the reaction mixture, then organic layer was separated. The organic layer was washed with 5 % hydrochloric acid, saturated aqueous solution of sodium bicarbonate and saturated aqueous solution of sodium chloride, dried by magnesium sulfate, concentrated under reduced pressure. The obtained solid was washed by hexane to obtain 0.20 g of N-(4-chlorolbenzyl)-2-fluoro-3-{3-methoxy-4-(2-propynyloxy) phenyl}propanamide (referred as the compound of the present invention 116 hereinafter).
the compound of the present invention 116 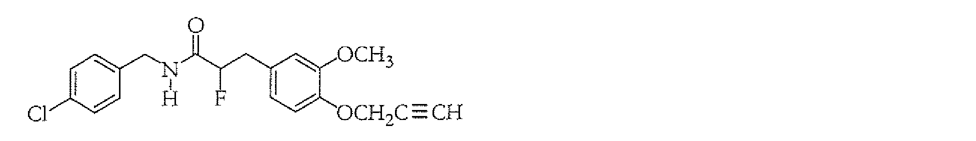 ¹H-NMR (CDCl₃, TMS) δ (ppm): 7.25 (2H, d, J=8.2 Hz), 6.93-6.98 (3H, m), 6.77-6.78 (2H, m), 6.44 (1H, br.s), 5.13 (1H, ddd, J=49 Hz, 6.1 Hz, 3.9 Hz), 4.76 (2H, d, J=2.4 Hz), 4.48 (1H, dd, J=15 Hz, 6.5 Hz), 4.26 (1H dd, J=15 Hz, 5.3 Hz), 3.82 (3H, s), 3.28 (1H, ddd, J=26 Hz, 15 Hz, 3.9 Hz), 3.14 (1H, ddd, J=33 Hz, 15 Hz, 6.0 Hz), 2.50 (1H, t, J=2.4 Hz)

### Production Example 117

By using 0.65 g of 4-trimethylsilylbenzylamine, 0.78 g of 3-{3-methoxy-4-(2-propynyloxy)phenyl}-propionic acid according to the Production Example 94 was obtained 0.68 g of N-(4-trimethylsilylbenzyl)-3-{3-methoxy-4-(2-propynyloxy)ph enyl}propanamide (referred as the compound of the present invention 117 hereinafter).
the compound of the present invention 117 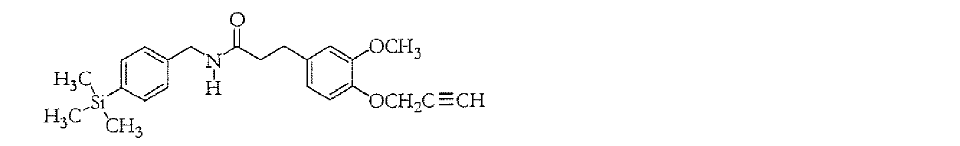 ¹H-NMR (CDCl₃, TMS) δ (ppm): 7.46 (2H, d, J=8.0 Hz), 7.16 (2H, d, J=8.0 Hz), 6.94 (1H, d, J=8.0 Hz), 6.71-6.76 (2H, m), 5.32 (1H, br.s), 4.73 (2H, d, J=2.4 Hz), 4.40 (2H, d, J=5.8 Hz), 3.82 (3H, s), 2.95 (2H, t, J=7.5 Hz), 2.47-2.51 (3H, m), 0.26 (9H, s)

### Production Example 118

By using 0.30 g of 4-{(3-{3-methoxy-4-(2-propynyloxy)phenyl}propionyl)aminomet hyl}-benzylmethansulfonate, 0.30g of lithium chloride according to the Production Example 109 was obtained 0.25 g of N-(4-chloromethyllbenzyl)-3-{3-methoxy-4-(2-propynyloxy)phe nyl}propanamide (referred as the compound of the present invention 118 hereinafter).
the compound of the present invention 118 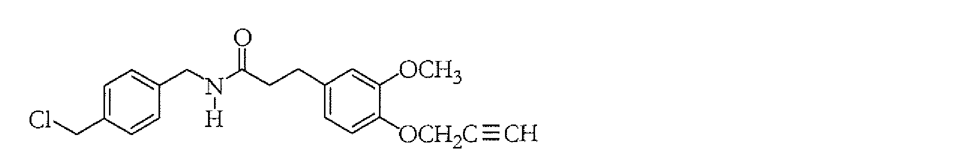 ¹H-NMR (CDCl₃, TMS) 5 (ppm): 7.32 (2H, d, J=7.9 Hz), 7.13 (2H, d, J-7.9 Hz), 6.94 (1H, d, J=7.9 Hz), 6.71-6.75 (2H, m), 5.59 (1H, br.s), 4.74 (2H, d, J=2.4 Hz), 4.56 (2H, s), 4.40 (2H, d, J=5.9 Hz), 3.82 (3H, s), 2.95 (2H, t, J=7.3 Hz), 2.48-2.52 (3H, m)

### Production Example 119

0.30 g of 3-(3,4-dimethoxyphenyl)propionyl chloride, 0.21 g of (naphthalene-2-yl) methylamine, 0.50 ml of triethylamine and 10 ml of tetrahydrofuran were mixed, and stirred at room temperature for 20 minutes. Then water was added to the reaction mixture, extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was washed by hexane to obtain 0.25 g of N-{(naphtalene-2-yl)methyl}-3-(3,4-dimethoxyphenyl)propanam ide (referred as the compound of the present invention 119 hereinafter).
the compound of the present invention 119 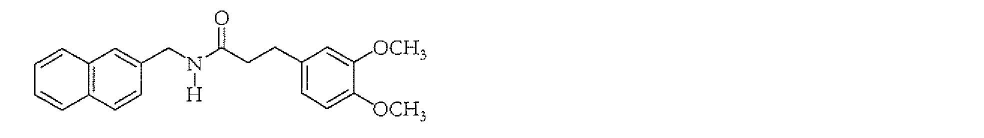 ¹H-NMR (CDCl₃, TMS) δ (ppm): 7.75-7.82 (3H, m), 7.60 (1H, s), 7.44-7.48 (2H, m), 7.25-7.28 (1H, m), 6.73-6.74 (3H, m), 5.66 (1H, br.s), 4.56 (2H, d, J=5.8 Hz), 3.82 (3H, s), 3.82 (3H, s), 2.96 (2H, t, J=7.5 Hz), 2.53 (2H, t, J=7.5 Hz)

### Production Example 120

0.65 g of N-(4-formylbenzyl)-3-{3-methoxy-4-(2-propyonyl)phenyl}propa namide and 10 ml of methylenechloride were mixed, 1.5 g of triphenylphosphine and 2 ml of carbontetrachloride were mixed with it under ice cooling, followed by stirring under ice cooling for 2 hours and at room temperature for 15 hours. Then the reaction mixture was concentrated under reduced pressure. The obtained residue was purified by silica gel column twice to obtain 0.15 g of N-(4-(2,2-dichlorovinyl)benzyl)-3-{3-methoxy-4-(2-propynylo xy)phenyl}propanamide (referred as the compound of the present invention 120 hereinafter).
the compound of the present invention 120 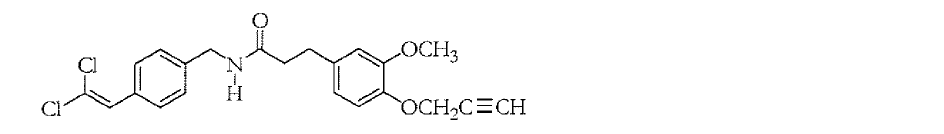 ¹H-NMR (CDCl₃, TMS) δ (ppm) : 7.49 (0.8H, d, J=8.5 Hz), 7.46 (1.2H, d, J=8.2 Hz), 7.12-7.17 (2H, m), 6.93-6.95 (1H, m), 6.82 (0.6H, s), 6.71-6.75 (2H, m), 6.68 (0.4H, s), 5.62 (1H, hr.s), 4.73-4.74 (2H, m), 4.39-4.42 (2H, m), 3.82 (3H, s), 2.95 (2H, t, J=7.5 Hz), 2.48-2.53 (3H, m)

### Production Example 121

0.52 g of 4-(trimethylsilylethynyl)benzylamine acetic acid salt, 0.42 g of 3-{3-methoxy-4-(2-propynyloxy)phenyl}propionic acid, 0.38 g of WSC and 10 ml of dimethylformamide were mixed at room temperature for 4 hours. Then water was added to the reaction mixture, and extracted with ethyl acetate. The organic layer was washed successively with 5%hydrochloric acid, water, saturated aqueous solution of sodium bicarbonate and saturated aqueous solution of sodium chloride, dried by magnesium sulfate, and concentrated under reduced pressure. Obtained residue , 10 ml of methanol and 0.5 g of potassium carbonate were stirred at room temperature for 2 hours. The reaction mixture was concentrated under reduced pressure. It was added water, and extracted with ethyl acetate. The organic layer was washed successively with water, saturated aqueous solution of sodium bicarbonate and saturated aqueous solution of sodium chloride, dried by magnesium sulfate, and concentrated under reduced pressure. The obtained residue was purified by silica gel column to obtain 0.21 g of N-(4-ethynylbenzyl)-3-{3-methoxy-4-(2-propynyloxy)phenyl}pr opanamide (referred as the compound of the present invention 121 hereinafter).
the compound of the present invention 121 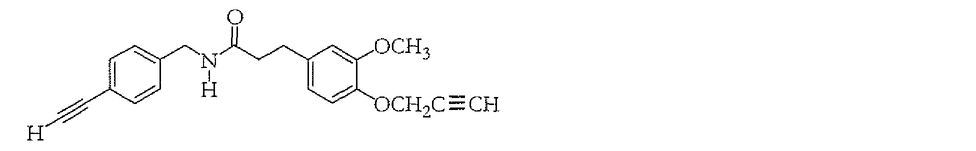 ¹H-NMR (CDCl₃, TMS) δ (ppm) : 7.40-7.42 (2H, m), 7.09 (2H, d, J=8.3 Hz), 6.93 (1H, d, J=8.3 Hz), 6.70-6.75 (2H, m), 5.60 (1H, br.s), 4.74 (2H, d, J=2.4 Hz), 4.39 (2H, d, J=5.9 Hz), 3.82 (3H, s), 3.06 (1H, s), 2.95 (1H, t, J=7.5 Hz), 2.47-2.59 (3H, m)

### Production Example 122

0.51 g of 4-cyclopropylbenzylamine, 0.74 g of 3-{3-methoxy-4-(2-propynyloxy)phenyl}propionic acid, 0.67 g of WSC and 10 ml of dimethylformamide were mixed at room temperature for 2 hours. Then water was added to the reaction mixture and extracted with ethyl acetate. The organic layer was washed successively with 5 % hydrochloric acid, water, saturated aqueous solution of sodium bicarbonate and saturated aqueous solution of sodium chloride, dried by magnesium sulfate, and concentrated under reduced pressure. The obtained residue was subjected to silica gel column to obtain 0.47 g of N-(4-cyclopropylbenzyl)-3-{3-methoxy-4-(2-propynyloxy)pheny 1}propanamide (referred as the compound of the present invention 122 hereinafter).
the compound of the present invention 122 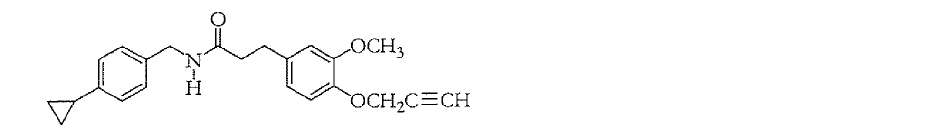 ¹H-NMR (CDCl₃, TMS) δ (ppm) : 7.05 (2H, d, J=8.2 Hz), 7.00 (2H, d, J=8.2 Hz), 6.93 (1H, d, J=8.1 Hz), 6.70-6.75 (2H, m), 5.59 (1H, br.s), 4.72 (2H, d, J=2.4 Hz), 4.35 (2H, d, J=5.6 Hz), 3.82 (3H, s), 2.94 (2H, t, J=7.3 Hz), 2.46-2.49 (3H, m)

### Production Example 123

0.2 g of 2-fluoro-3-{3-methoxy-4-(2-propynyloxy)phenyl}propionic acid, 0.15 g of C-naphtalene-2-ylmethylamione hydrochloride, 0.19 g of WSC, 1 ml of pyridine and 5 ml of dimethylformamide were mixed at room temperature for 2 hours. Then water was added to the reaction mixture and extracted with ethyl acetate. The organic layer was washed successively with 5 % hydrochloric acid, water, saturated aqueous solution of sodium bicarbonate and saturated aqueous solution of sodium chloride, dried by magnesium sulfate, and concentrated under reduced pressure. The obtained residue was subjected to silica gel column to obtain 0.13 g of N-{(naphthalene-2-yl)methyl}-2-fluoro-3-{3-methoxy-4-(2-pro pynyloxy)phenyl}propanamide (referred as the compound of the present invention 123 hereinafter).
the compound of the present invention 123 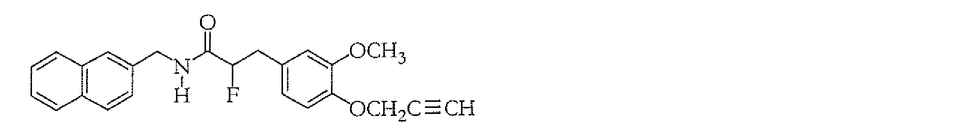 ¹H-NMR (CDCl₃, TMS) δ (ppm) : 7.76-7.82 (3H, m), 7.57 (1H, br.s), 7.46-7.56 (2H, m), 7.17 (1H, d d, J=8.2 Hz, 1.7 Hz), 6.91 (1H, d, 8.7 Hz), 6.78-6.80 (2H, m), 6.50 (1H, br.s), 5.18 (1H, ddd, J=49 Hz, 6.1 Hz, 3.6 Hz), 6.63-6.70 (3H, m), 4.49 (1H, dd, J=15 Hz, 5.3 Hz), 3.80 (3H, s), 3.14-3.32 (2H, m), 2.47 (1H, t, J=2.4 Hz)

### Production Example 124

0.5 g of crude product of 2-fluoro-3-{3-methoxy-4-(2-propynyloxy)phenyl}propionyl chloride, 0.49 g of 4-bromobenzylamine hydrochloride, 0.64 ml of triethylamine, about 10 mg of dimethylaminopyridine and 10 ml of tetrahydrofuran were mixed at room temperature for 20 minutes. Then water was added to the reaction mixture and extracted with ethyl acetate. The organic layer was washed successively with 5 % hydrochloric acid, water, saturated aqueous solution of sodium bicarbonate and saturated aqueous solution of sodium chloride, dried by magnesium sulfate, and concentrated under reduced pressure. The residue was subjected to silica gel column to obtain 0.23 g of N-(4-bromobenzyl)-2-fluoro-2-{3-methoxy-4-(2-propynyloxy)ph enyl}propanamide (referred as the compound of the present invention 124 hereinafter).
the compound of the present invention 124 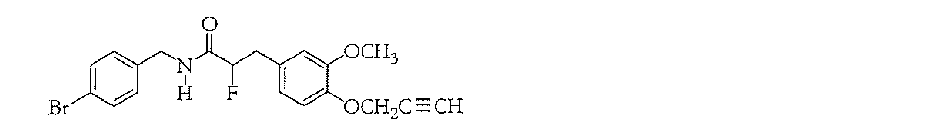 ¹H-NMR (CDCl₃, TMS) δ (ppm): 7.40 (2H, d, J=8.6 Hz), 6.94 (2H, d, J=8.6 Hz), 6.91 (1H, d, J=8.2 Hz), 6.77-6.78 (2H, m), 6.44 (1H, br.s), 5.16 (1H, ddd, J=49 Hz, 5.8 Hz, 3.6 Hz), 4.76 (2H, d, J=2. 4 Hz), 4.47 (1H, dd, J=15 Hz, 6.8 Hz), 4.24 (1H, dd, J=15 Hz, 5.3 Hz), 3.82 (3H, s), 3.24 (1H, ddd, J=25 Hz, 15 Hz, 3.6 Hz), 3.14 (1H, ddd, J=34 Hz, 15 Hz, 5.8 Hz), 2. 50 (1H, t, J=2.4 Hz)

### Production Example 125

By using 0.5 g of crude product of 2-fluoro-3-{3-methoxy-4-(2-propynyloxy)phenyl}propionyl chloride and 0.39 g of 3,4-dichlorobenzylamine according to the Production Example 124 was obtained 0.31 g of N-(3,4-dichlorobenzyl)-2-fluoro-3-{3-methoxy-4-(2-propynylo xy)phenyl}propanamide (referred as the compound of the present invention 125 hereinafter).
the compound of the present invention 125 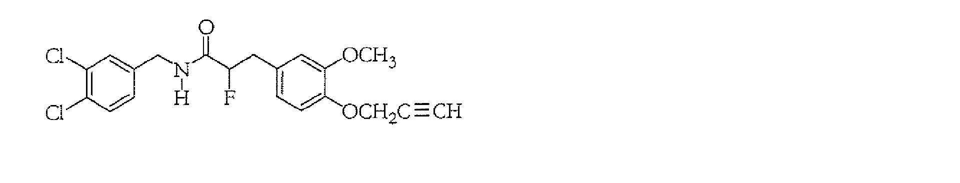 ¹H-NMR (CDCl₃, TMS) δ (ppm) : 7.35 (1H, d, J=8.2 Hz), 7.25-2.26 (1H, m), 6.94 (1H, d, J=8.7 Hz), 6.83 (1H, dd, J=8.2 Hz, 2.2 Hz), 6.76-6.78 (2H, m), 6.49 (1H, br.s), 5.16 (1H, ddd, J=49 Hz, 6.1 Hz, 3.9 Hz), 4.75 (2H, d, J=2.4 Hz), 4.44 (1H, dd, J=15 Hz, 6.8 Hz), 4.27 (1H, dd, J=15 Hz, 5.5 Hz), 3.82 (3H, s), 3.28 (1H, ddd, J=26 Hz, 15 Hz, 3.9 Hz), 3.14 (1H ddd, J=33 Hz, 15 Hz, 6.1 Hz), 2.49 (1H, t, J=2.4 Hz)

### Production Example 126

By using 0.5 g of crude product of 2-fluoro-3-(3-methoxy-4- (2-propynyloxy) phenyl)propionyl chloride and 0.27 g of 4-methylbenzylamine according to the Production Example 124 was obtained 0.30 g of N- (4-methylbenzyl) -2-fluoro-3-{3-methoxy-4- (2-propynyloxy)p henyl}propanamide (referred as the compound of the present invention 126 hereinafter).
the compound of the present invention 126 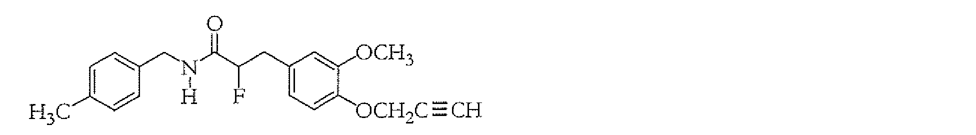 ¹H-NMR (CDCl₃, TMS) δ (ppm): 7.10 (2H, d, J=7.8 Hz), 6.94-6.98 (3H, m), 6.78-6.79 (2H, m), 6.39 (1H, br.s), 5. 13 (1H, ddd, J=49 Hz, 6.4 Hz, 3.4 Hz), 4.75 (2H, d, J=2.4 Hz), 4.44 (1H, dd, J=15 Hz, 6.5 Hz), 4.24 (1H, dd, J=15 Hz, 5.3 Hz), 3.82 (3H, s), 3.28 (1H, ddd, J=27 Hz, 15 Hz, 3.4 Hz), 3.13 (1H, ddd, J=32 Hz, 15 Hz, 6.4 Hz), 2.49 (1H, t, J=2.4 Hz)

### Production Example 127

By using 0.5 g of crude product of 2-fluoro-3-{3-methoxy-4-(2-propynyloxy)phenyl}propionyl chloride and 0.39 g of 4-trifluoromethylbenzylamine according to the Production Example 124 was obtained 0.29 g of N-(4-trifluoromethylbenzyl)-2-fluoro-3-{3-methoxy-4-(2-prop ynyloxy)phenyl}propanamide (referred as the compound of the present invention 127 hereinafter).
the compound of the present invention 127 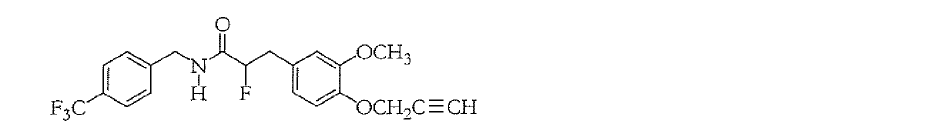 ¹H-NMR (CDCl₃, TMS) δ (ppm): 7.54 (2H, d, J=8.2 Hz), 7.13 (2H, d, J=8.2 Hz), 6.95 (1H, d, J=8.7 Hz), 6.78-6.79 (2H, m), 6.52 (1H, br.s), 5.18 (1H, ddd, J=49 Hz, 5.8 Hz, 3.8 Hz), 4.75 (2H, d, J=2.4 Hz), 4.58 (1H, dd, J=15 Hz, 6.8 Hz), 4.34 (1H, dd, J=15 Hz, 5.3 Hz), 3.81 (3H, s), 3.09-3.35 (2H, m), 2.48 (1H, t, J=2.4 Hz)

### Production Example 128

By using 0.5 g of crude product of 2-fluoro-3-{3-methoxy-4-(2-propynyloxy)phenyl}propionylchloride and 0.44 g of (5,6,7,8-tetrahydronaphthalene-2-yl)methylamine hydrochloride according to the Production Example 124 was obtained 0.33 g of N-{(5,6,7,8tetrahydronaphthalene-2-yl)methyl}-2-fluoro-3-{3 -methoxy-4-(2-propynyloxy)phenyl}propanamide (referred as the compound of the present invention 128 hereinafter).
the compound of the present invention 128 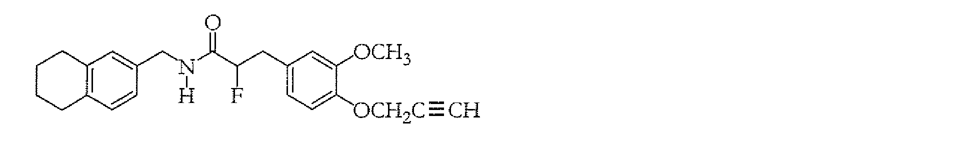 ¹H-NMR (CDCl₃, TMS) δ (ppm): 7.00 (1H, d, J=7.8 Hz), 6.95 (1H, d, J=8.7 Hz), 6.78-6.85 (4H, m), 6.39 (1H, br.s), 5.12 (1H, ddd, J=49 Hz, 6.5 Hz, 3.4 Hz), 4.74 (2H, d, J=2.4 Hz), 4.28-4.39 (2H, m), 3.83 (3H, s), 3.28 (1H, ddd, J=28 Hz, 15 Hz, 3.4 Hz), 3.12 (1H, ddd, J=31 Hz, 15 Hz, 6.5 Hz), 2.75-2.80 (4H, m), 2.49 (1H, t, J=2.4 Hz), 1.74-1.82 (4H, m)

### Production Example 129

1.1 g of N-(4-chlorobenzyl)-2-fluoro-3-{3-methoxy-4-(2-propynyloxy)p henyl}propanamide, 0.86 g of Lawesson's Reagent and 30 ml of tetrahydrofuran were mixed, and stirred at 65 °C for 3 hours. Then the reaction mixture was cooled, and concentrated under reduced pressure. Water was added to the residue, and extracted with ethyl acetate. The organic layer was washed successively with 5 % hydrochloric acid, saturated aqueous solution of sodium bicarbonate and saturated aqueous solution of sodium chloride, dried by magnesium sulfate, and concentrated under reduced pressure. The residue was purified by silica gel column to obtain 0.15 g of N-(4-chlorobenzyl)-2-fluoro-3-{3-methoxy-4-(2-propynyloxy)p henyl}propanthioamide (referred as the compound of the present invention 129 hereinafter).
the compound of the present invention 129 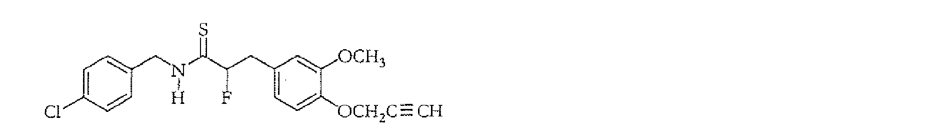 ¹H-NMR (CDCl₃, TMS) δ (ppm): 7.79 (1H, br.s), 7.24-7.29 (2H, m), 6.92-6.94 (3H, m), 6.78-6.80 (2H, m), 5.52 (1H, dt, J=50 Hz, 4.5 Hz), 4.83 (1H, dd, J=15 Hz, 6.1 Hz), 4.76 (2H, d, 2.4 Hz), 4.57 (1H, dd, J=15 Hz, 4.8 Hz), 3.81 (3H, s), 3.27-3.51 (2H, m), 2.50 (1H, t, J=2.4 Hz)

Next, the production of the intermediate of the present invention is described as Reference Production Example.

### Reference Production Example 1

23.8 g of ethyl 3-(4-hydroxy-3-methoxyphenyl)propionate, 11.4 ml of propargyl bromide, 20.5 g of potassium carbonate and 250 ml of acetonitrile were mixed, and stirred at 80 °C for 2 hours. Then the reaction mixture was cooled to room temperature. Ethyl acetate was added to it, and filtered. The filtrate was concentrated under reduced pressure to obtain 28.9 g of ethyl 3-{3-methoxy-4-(2-propynyloxy)phenyl}propionate. 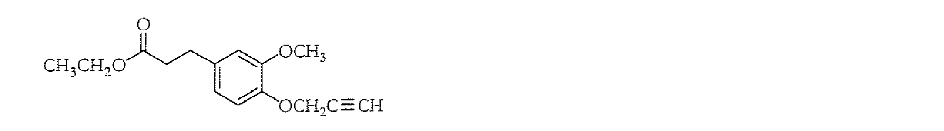 ¹H-NMR (CDCl₃, TMS) δ (ppm): 6.95 (1H, d, J=7.7 Hz), 6.72-6.75 (2H, m), 4.73 (2H, d, J=2.4 Hz), 4.13 (2H, q, J=7.2 Hz), 3.86 (3H, s), 2.90 (2H, t, J=7.5 Hz), 2.60 (2H, t, J=7.5 Hz), 2.49 (1H, t, J=2.4 Hz), 1.24 (3H, t, J=7.0 Hz)

### Reference Production Example 2

28.9 g of ethyl 3-{3-methoxy-4-(2-propynyloxy)phenyl}propionate, 4.0 g of lithium hydroxide, 300 ml of tetrahydrofuran and 100 ml of water were mixed, and stirred at 65 °C for 3 hours. Then water was added to the reaction mixture, and concentrated under reduced pressure. 5 % hydrochloric acid was added to the residue and extracted with chloroform by three times. The organic layer was dried by magnesium sulfate, and concentrated under reduced pressure. The residue was washed by hexane to obtain 22.7 g of 3-{3-methoxy-4-(2-propynyloxy)phenyl}propionic acid. 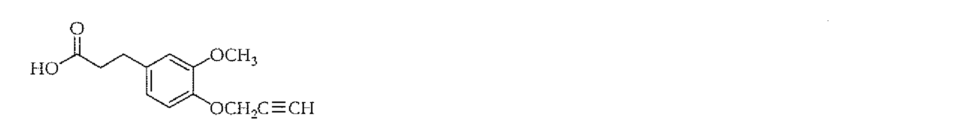 ¹H-NMR (CDCl₃, TMS) δ (ppm): 6.96 (1H, d, J=8.2 Hz), 6.73-6.75 (2H, m), 4.73 (2H, d, J=2.4 Hz), 3.85 (3H, s), 2.91 (2H, t, J=8 Hz), 2.67 (2H, t, J=8 Hz), 2.49 (1H, t, J=2.4 Hz)

### Reference Production Example 3

12.7 g of 3-{3-methoxy-4-(2-propynyloxy) phenyl}propionic acid, 4.3 ml of thionyl chloride, 100 ml of toluene and about 0.05 g of N,N-dimethylformamide were mixed, and stirred at 80°C for 30 minutes. Then the reaction mixture was cooled to room temperature, and concentrated under reduced pressure to obtain 14.6 g of 3-{3-methoxy-4-(2-propynyloxy)phenyl}proionyl chloride. 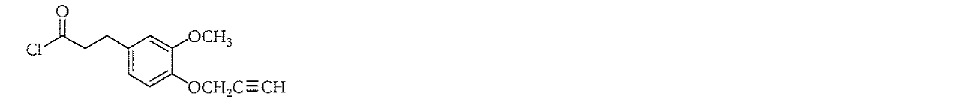 ¹H-NMR (CDCl₃, TMS) δ (ppm): 6.97 (1H, d, J=8.8 Hz), 6.72-6.74 (2H, m), 4.73 (2H, d, J=2.4 Hz), 3.87 (3H, s), 3.19 (2H, t, J=7.2 Hz), 2.99 (2H, t, J=7.2 Hz), 2.49 (1H, t, J=2.4 Hz)

### Reference Production Example 4

6.2 g of N-(4-methylbenzyl)-3-{3-methoxy-4-(benzyloxy)phenyl}acrylam ide 0.6 g of 5 % palladium charcoal, 0.3 g of palladium hydroxide, 100 ml of ethanol, 100 ml of ethyl acetate, 100 ml of tetrahydrofuran and 1 ml of 36 % hydrochloric acid were stirred under hydrogen atmosphere. After stopping the absorption of hydrogen, the reaction mixture was filtered, and the filtrate was concentrated under reduced pressure. Ethyl acetate and water was added to the residue, and separated into two layer. The organic layer was dried by magnesium sulfate, and concentrated under reduced pressure. The residue was washed by hexane to obtain 4.5 g of N-(4-methylbenzyl)-3-(4-hydroxy-3-methoxyphenyl)propanamide 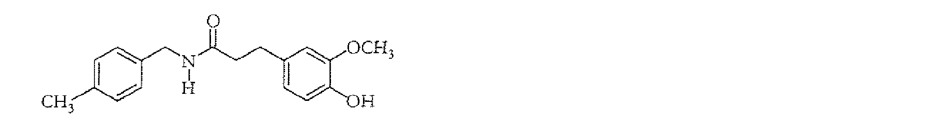 ¹H-NMR (CDCl₃, TMS) δ (ppm): 7.10 (2H, d, J=8 Hz), 7.03 (2H, d, J=8 Hz), 6.81 (1H, d, J=8.0 Hz), 6.66-6.70 (2H, m), 5.78 (1H, br.s), 4.35 (2H, d, J=5.1 Hz), 3.82 (3H, s), 2.92 (2H, d, J=7 Hz), 2.47 (2H, d, J=7 Hz), 2.32 (3H, s)

### Reference Production Example 5

50 g of 3- (4-hydroxy-3-methoxyphenyl)propionic acid, 50 ml of propargyl bromide, 88 g of potassium carbonate and 500 ml of acetonitrile were mixed, and stirred at 80 °C for 3 hours. Then the reaction mixture was cooled to room temperature. Ethyl acetate was added to it, and filtered. The filtrate was concentrated under reduced pressure to obtain 67 g of (2-propynyl) 3-{3-methoxy-4-(2-propynyloxy)phenyl}propionate. 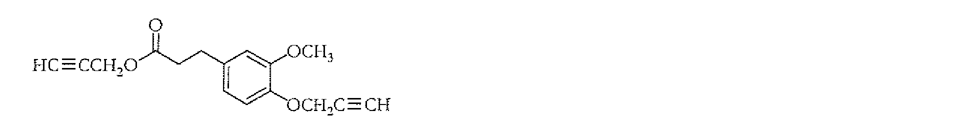 ¹H-NMR (CDCl₃, TMS) δ (ppm): 6.96 (1H, d, J=7.8 Hz), 6.68-6.75 (2H, m), 4.73 (2H, d, J=2.2 Hz), 4.68 (2H, d, J=2.2 Hz), 3.87 (3H, s), 2.93 (2H, t, J=7.3 Hz), 2.67 (2H, t, J=7.3Hz), 2.47-2.50 (2H, m)

### Reference Production Example 6

67 g of (2-propynyl) 3-{3-methoxy-4-(2-propynyloxy)phenyl}propionate, 8.08 g of lithium hydroxide, 400 ml of tetrahydrofuran and 200 ml of water were mixed, and stirred at 65 °C for 3 hours. Then water was added to the reaction mixture, and concentrated under reduced pressure. 5 % hydrochloric acid was added to the residue and extracted with chloroform by three times. The organic layer was dried by magnesium sulfate, and concentrated under reduced pressure. The residue was washed by hexane to obtain 51 g of 3-{3-methoxy-4-(2-propynyloxy)phenyl}propionic acid. 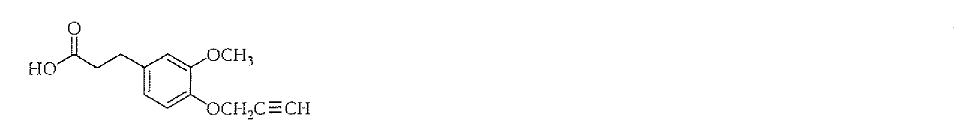 ¹H-NMR (CDCl₃, TMS) 5 (ppm): 6.96 (1H, d, J=8.2 Hz), 6.73-6.75 (2H, m), 4.73 (2H, d, J=2.4 Hz) , 3.85 (3H, s), 2.91 (2H, t, J=8 Hz), 2.67 (2H, t, J=8 Hz), 2.49 (1H, t, J=2.4 Hz)

### Reference Production Example 7

20.0 g of 3-(4-hydroxy-3-methoxyphenyl)propionic acid, 15 g of 4-chlorobenzylamine, 0.51 g of 2-nitrophenylboronic acid and 300 ml of toluene were mixed, and stirred under the condition of reflux and dehydration with Dean-Stark trap for 4 hours. Then the reaction mixture was cooled to room temperature, and added about 1000 ml of ethyl acetate. It was washed successively with 5 % hydrochloric acid, saturated aqueous solution of sodium bicarbonate and saturated aqueous solution of sodium chloride, dried by magneium sulfate, and concentrated under reduced pressure. The residue was recrystallized form toluene to obtain 28 g of N-(4-chlorobenzyl)-3-{4-hydroxy-3-methoxyphenyl}propanamide 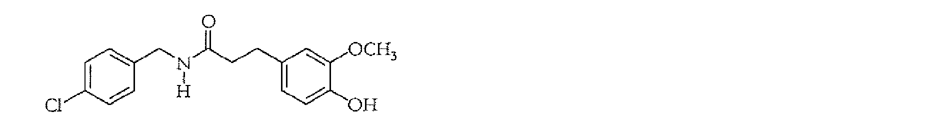 ¹H-NMR (CDCl₃, TMS) δ (ppm): 7.25 (2H, d, J=8.5 Hz), 7.03 (2H, d, J=8.5 Hz), 6.83 (1H, d, J=8.0 Hz), 6.66-6.69 (2H, m), 5.62 (1H, br.s), 5.53 (1H, s), 4.35 (2H, d, J=5.8 Hz), 3.82 (3H, s), 2.92 (2H, d, J=7.5 Hz), 2.49 (2H, d, J=7.5 Hz)

### Reference Production Example 8

12.5 g of 3-(4-hydroxy-3-methoxyphenyl)propionic acid, 10.0 g of (C-naphthalene-2-yl)methylamine, 13.4 g of WSC and 100 ml of N,N-dimethylformamide were mixed, and stirred at room temperature for 2 hours. Then water was added to the reaction mixture and extracted with chloroform. The organic layer was washed successively with 5 % hydrochloric acid and saturated aqueous solution of sodium chloride, dried by magnesium sulfate, and concentrated under reduced pressure. The residue was washed by hexane to obtain 15.1 g of N-(naphthalene-2-ylmethyl)-3-(4-hydroxy-3-methoxyphenyl)pro panamide. 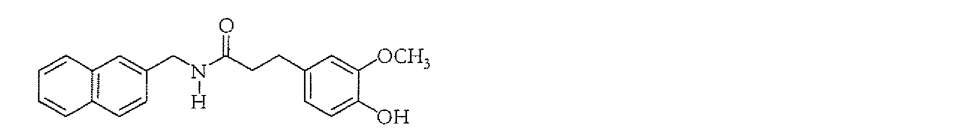 ¹H-NMR (CDCl₃, TMS) δ (ppm): 7.73-7.84 (3H, m), 7.58 (1H, s), 7.43-7.51 (2H, m), 7.23-7.27 (1H, m), 6.82 (1H, d, J=7.6 Hz), 6.67-6.72 (2H, m), 5.67 (1H, br.s), 5.49 (1H, s), 4.56 (2H, d, J=5.9 Hz), 3.77 (3H, s), 2.94 (2H, d, J=7.5 Hz), 2.51 (2H, d, J=7.5 Hz)

### Reference Production Example 9

16.2 g of ethyl 3- (4-hydroxy-3-ethoxyphenyl)propionate, 7.3 ml of propargyl bromide, 13.2 g of potassium carbonate and 160 ml of acetonitrile were mixed, and stirred at 80 °C for 2 hours. Then the reaction mixture was cooled to room temperature. Ethyl acetate was added to it, and filtered. The filtrate was concentrated under reduced pressure to obtain 18.8 g of ethyl 3-{3-ethoxy-4-(2-propynyloxy)phenyl}propionate. 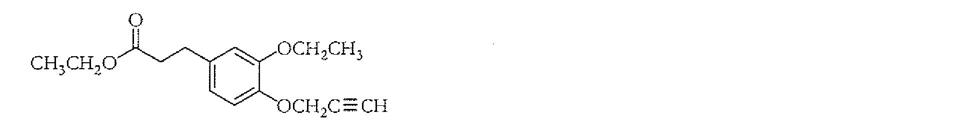 ¹H-NMR (CDCl₃, TMS) δ (ppm) : 6.96 (1H, d, J=8.0 Hz), 6.74 (1H, d, J=1.9Hz), 6.72 (1H, dd, J=8.0 Hz, 1.9 Hz), 4.73 (2 H, d, J=2. 4 Hz), 4.12 (2H, q, J=7.0 Hz), 4.07 (2H, q, J=7.0 Hz), 2.89 (2H, t, J=7.5 Hz), 2.59 (2H, t, J=7.5 Hz), 2.48 (1H, t, J=2.4 Hz), 1.44 (3H, t, J=7.0 Hz), 1.23 (3H, t, J=7.0 Hz)

### Reference Production Example 10

28.7 g of ethyl 3-{3-ethoxy-4-(2-propynyloxy)phenyl}propionate, 2.3 g of lithium hydroxide, 180 ml of tetrahydrofuran and 60 ml of water were mixed, and stirred at 65 °C for 3 hours. Then water was added to the reaction mixture, and concentrated under reduced pressure. The mixture was washed with methyl=tert-butyl ether and aqueous layer was separated. 5 % hydrochloric acid was added to the aqueous layer, and extracted with ethyl acetate. The organic layer was washed with saturated aqueous solution of sodium chloride, dried by magnesium sulfate, and concentrated under reduced pressure. The residue was washed by hexane to obtain 15.5 g of 3-(3-ethoxy-4-(2-propynyloxy)phenyl)propionic acid. 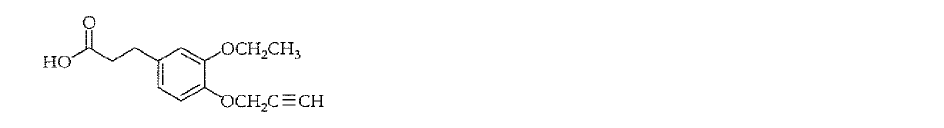 ¹H-NMR (CDCl₃, TMS) δ (ppm): 6.97 (1H, d, J=8.2 Hz), 6.74 (1H, d, J=1.9 Hz), 6.73 (1H, dd, J=8.2 Hz, 1.9 Hz), 4.73 (2H, d, J=2.4 Hz), 4.08 (2H, q, J=7.0 Hz), 2.90 (2H, t, J=7.7 Hz), 2.66 (2H, t, J=7.7 Hz), 2. 48 (1H, t, J=2.4 Hz), 1.44 (3H, t, J=7.0 Hz)

### Reference Production Example 11

6.7 g of 3-{3-ethoxy-4-(2-propynyloxy)phenyl}propionic acid, 3.2 ml of thionyl chloride, 100 ml of toluene and about 0.03 g of N,N-dimethylformamide were mixed, and stirred at 80 °C for 30 minutes followed by at 100 °C for 30 minutes. Then the reaction mixture was cooled to room temperature, and concentrated under reduced pressure to obtain 17.5 g of 3-{3-ethoxy-4-(2-propynyloxy)phenyl}propionyl chloride. 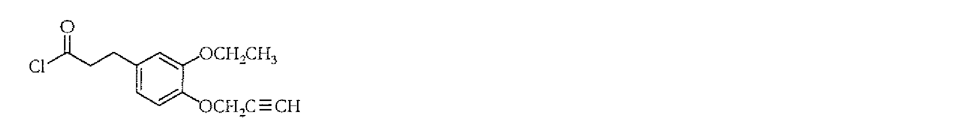 ¹H-NMR (CDCl₃, TMS) δ (ppm): 6.98 (1H, d, J=8.3 Hz), 6.71-6.73 (2H, m), 4.74 (2H, d, J=2.4 Hz), 4.08 (2H, q, J=7.1 Hz), 3.18 (2H, t, J=7.6 Hz), 2.95 (2H, t, J=7.6 Hz), 2.49 (1H, t, J=2.4 Hz), 1.45 (3H, t, J=7.1 Hz)

### Reference Production Example 12

7.14 g of ethyl 3-(4-hydroxy-3-mehoxyphenyl)butyrate, 3.93 g of 3-bromopropyn, 4.98 g of potassium carbonate and 50 ml of acetonitrile were mixed, and stirred at 80 °C for 4 hours. Then the reaction mixture was cooled to room temperature, water was added to it, and extracted with ethyl acetate. The organic layer was washed successively with water and aqueous solution of sodium chloride, dried by magnesium sulfate, and concentrated under reduced pressure to obtain 8.3 g of ethyl 3-{3-methoxy-4-(2-propynyloxy)phenyl}butyrate. 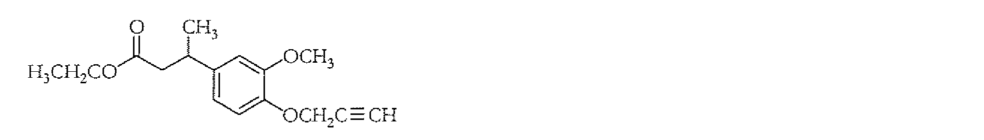 ¹H-NMR (CDCl₃, TMS) δ (ppm): 6.96 (1H, d, J=8.9 Hz), 6.73-6.78 (2H, m), 4.72 (2H, d, J=2.4 Hz), 4.08 (2H, q, J=7.0 Hz), 3.86 (3H, s), 3.18-3.29 (1H, m), 2.47-2.63 (3H, m), 1.28 (3H, d, J=7.0 Hz), 1.19 (3H, t, J=7.1 Hz)

### Reference Production Example 13

8.0 g of ethyl 3-{3-methoxy-4-(2-propynyloxy)phenyl}butyrate 1.04 g of lithium hydroxide, 40 ml of tetrahydrofuran and 15 ml of water were mixed, and stirred at 65 °C for 4 hours. Then water was added to the reaction mixture and concentrated under reduced pressure. 5 % hydrochloric acid was added to the residue, and extracted with chloroform by three times. The organic layer was washed with successively water and saturated aqueous solution of sodium chloride. The organic layer was dried by magnesium sulfate, and concentrated under reduced pressure. The residue was washed by hexane to obtain 7.0 g of 3-{3-methoxy-4-(2-propynyloxy)phenyl}butyric acid. 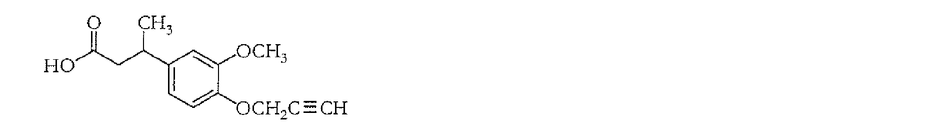 ¹H-NMR (CDCl₃, TMS) δ (ppm): 6.97 (1H, d, J=8.8 Hz), 6. 74-6. 78 (2H, m), 4.73 (2H, d, J=2.4 Hz), 3.86 (3H, s), 3.17-3.30 (1H, m), 2.52-2.70 (2H, m), 2.49 (1H, t, J=2.4 Hz), 1.31 (3H, d, J=7.1 Hz)

### Reference Production Example 14

7.0 g of 3-{3-methoxy-4-(2-propynyloxy)phenyl}butyric acid, 5.0 g of thionyl chloride and 100 ml of toluene were mixed, and stirred at 50°C for 30 minutes followed by at 80°C for 3 hours. Then the reaction mixture was cooled to room temperature, and concentrated under reduced pressure. The residue was washed by hexane to obtain 6.3 g of 3-{3-methoxy-4-(2-propynyloxy)phenyl}butyryl chloride. 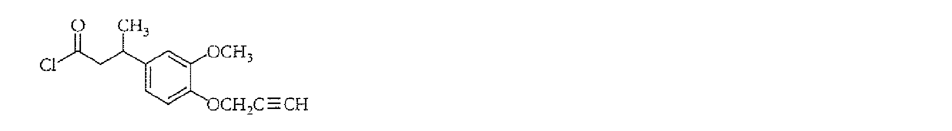 ¹H-NMR (CDCl₃, TMS) δ (ppm): 6.97 (1H, d, J=7.7 Hz), 6.72-6.78 (2H, m), 4.73 (2H, d, J=2.4 Hz), 3.87 (3H, s), 3.25-3.35 (1H, m), 3.04-3.21 (2H, m), 2.49 (1H, t, J=2.4 Hz), 1.34 (3H, d, J=7.0 Hz)

### Reference Production Example 15

By using 5.93 g of ethyl 3-(3-methoxy-4-hydroxyphenyl)-2-methylpropionate according to Reference Production Example 12 was obtained 6.84 g of ethyl 3-{3-methoxy-4-(2-propynyloxy)phenyl}-2-methylpropionate. 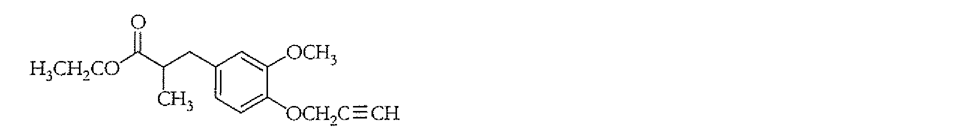 ¹H-NMR (CDCl₃, TMS) δ (ppm): 6.94 (1H, d, J=8 .8 Hz), 6.68-6.74 (2H, m), 4.73 (2H, d, J=2.4 Hz), 4.09 (2H, q, J=7.0 Hz), 3.85 (3H, s), 2.92-3.01 (1H, m), 2.58-2.74 (2H, m), 2.48 (1H, t, J=2.4 Hz), 1.19 (3H, t, J=7.2 Hz), 1.15 (3H, d, J=6.8 Hz)

### Reference Production Example 16

By using 6.50 g of ethyl 3-{3-methoxy-4-(2-propynyloxy)phenyl}-2-methylpropionate according to Reference Production Example 13 was obtained 5.33 g of 3-{3-methoxy-4-(2-propynyloxy)phenyl}-2-methylpropionic acid. 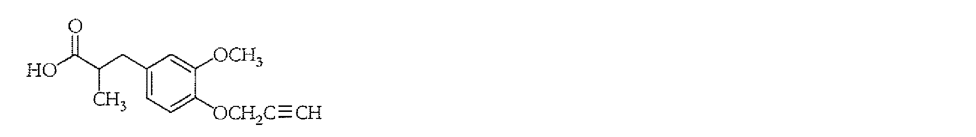 ¹H-NMR (CDCl₃, TMS) δ (ppm) : 6.95 (1H d, J=8 . Hz), 6.70-6.75 (2H, m), 4.73 (2H, d, J=2.2 Hz), 3.84 (3H, s), 2.97-3.06 (1H, m), 2.58-2.80 (2H, m), 2.49 (1H, t, J=2.4 Hz), 1.19 (3H, d, J=6.7 Hz)

### Reference Production Example 17

By using 5.03 g of 3-{3-methoxy-4-(2-propynyloxy)phenyl}-2-methylpropionic acid according to Reference Production Example 14 was obtained 5.09 g of 3-{3-methoxy-4-(2-propynyloxy)phenyl}-2-methylpropionyl chloride. 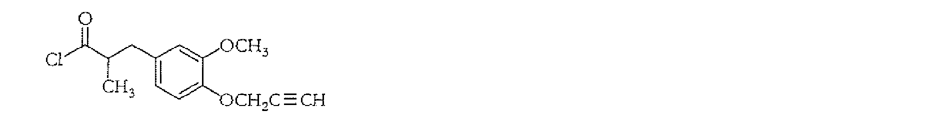 ¹H-NMR (CDCl₃, TMS) δ (ppm): 6.96 (1H, d, J=8.2 Hz), 6.72-6.75 (2H, m), 4.73 (2H, d, J=2.4 Hz), 3.85 (3H, s), 3.07-3.18 (2H, m), 2.67-2.77 (1H, m), 2.49 (1H, t, J=2.4 Hz), 1.28 (3H, d, J=6.8 Hz)

### Reference Production Example 18

By using 2.0 g of crude product of ethyl 3-(3-methoxy-4-hydroxyphenyl)-2-fluoropropionate and 0.86 ml of 3-bromopropyne according to Reference Production Example 12 was obtained 2.3 g of crude product of ethyl 3-{3-methoxy-4-(2-propynyloxy)phenyl}-2-fluoropropionate. 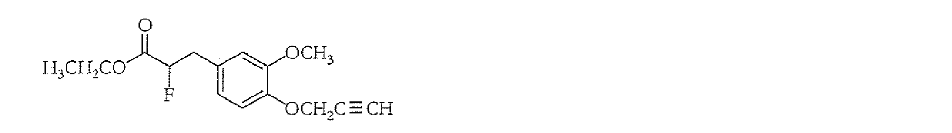 ¹H-NMR (CDCl₃, TMS) δ (ppm): 6.97 (1H, d, J=8.9 Hz), 6.72-6.79 (2H, m), 5.06 (1H, ddd, J=49 Hz, 7.5 Hz, 4.1 Hz), 4.74 (1H, d, J=2.4 Hz), 4.23 (2H, q, J=7.0 Hz), 3.86 (3H, s), 3.08-3.20 (2H, m), 2.49-2.50 (1H, m), 1.27 (3H, t, J=7.0 Hz)

### Reference Production Example 19

By using 2.3 g of crude product of ethyl 3-{3-methoxy-4-(2-propynyloxy)phenyl}-2-fluoropropionate according to Reference Production Example 13 was obtained 1.8 g of 3-{3-methoxy-4-(2-propynyloxy)phenyl}-2-fluoropropionic acid. 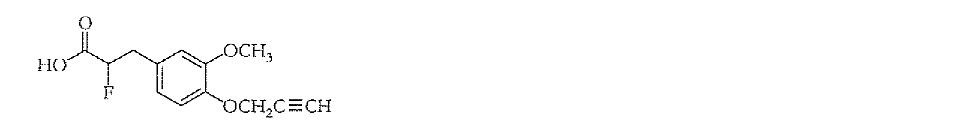 ¹H-NMR (CDCl₃, TMS) δ (ppm): 6.98 (1H, d, J=8.7 Hz), 6.74-6.82 (2H, m), 5.21 (1H, ddd, J=49 Hz, 7.5 Hz, 3.6 Hz), 4.75 (1H, d, J=2.4 Hz), 3.86 (3H, s), 3.08-3.31 (2H, m), 2.49 (1H, t, J=2.4 Hz)

### Reference Production Example 20

By using about 4.7 g of 3-{3-methoxy-4-(2-propynyloxy)phenyl}-2-fluoropropionic acid according to Reference Production Example 14 was obtained 5.0 g crude product of 3-{3-methoxy-4-(2-propynyloxy)phenyl}-2-fluoropropionyl chloride. 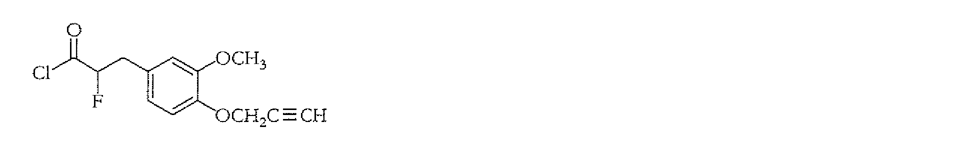 ¹H-NMR (CDCl₃, TMS) δ (ppm): 6.99 (1H, d, J=8.2 Hz), 6.79-6. 82 (2H, m), 5.20 (1H, ddd, J=49 Hz, 7.3 Hz, 3.9 Hz), 4.75 (2H, d, J=2.4 Hz), 3.87 (3H, s), 3.17-3.37 (2H, m), 2.50 (1H, t, J=2.4 Hz)

Next, the production of the intermediate is described as Reference Example.

### Reference Example 1

100 g of 4-benzyloxy-3-methoxybenzaldehyde, 120 g of ethyl diethylphosphonoacetate, 570 g of potassium carbonate and 570 ml of water were mixed, and stirred under condition of reflux for 20 hours. Then the reaction mixture was cooled to room temperature, water was added to it and extracted ethyl acetate twice. The separated organic layer was washed successively with water and saturated aqueous solution of sodium chloride, dried by magnesium sulfate, and concentrated under reduced pressure. The obtained residue was recrystallized from ethanol to obtain 58.6 g of ethyl 3-{3-methoxy-4-(benzyloxy)phenyl}acrylate. 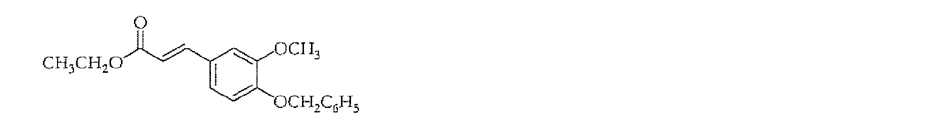 ¹H-NMR (CDCl₃, TMS) δ (ppm): 7.60 (1H, d, J=15 Hz), 7.29-7.44 (5H, m), 7.06 (1H, d, J=1.9 Hz), 7.02 (1H, dd, J=8.2 Hz, 1.9 Hz), 6.86 (1H, d, J=8.2 Hz), 6.29 (1H, d, J=15 Hz), 5.18 (2H, s), 4.25 (2H, q, J=7.3 Hz), 3.91 (3H, s), 1.33 (3H, t, J=7.3 Hz)

### Reference Example 2

33 g of ethyl 3-{3-methoxy-4-(benzyloxy)phenyl}acrylate, 0.3 g of 5 % palladium charcoal, about 0.05 g of 36 % hydrochloric acid and 200 ml of ethanol were stirred under hydrogen atmosphere. After stopping absorption of hydrogen gas, it was filtered, and the filtrate was concentrated under reduced pressure. Ethyl acetate and water were added to the residue and separated to two layer. The organic layer was dried by magnesium sulfate, added about 5 g of active carbon and about 5 g of caustic clay, filtered, and concentrated under reduced pressure. The residue was washed by hexane to obtain 23 g of ethyl 3-(4-hydroxy-3-methoxyphenyl)propionate. 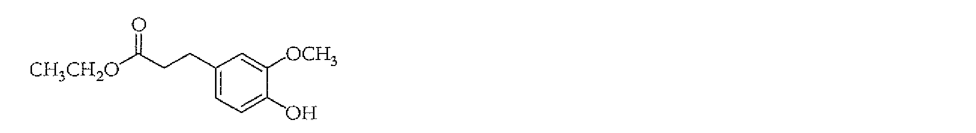 ¹H-NMR (CDCl₃, TMS) δ (ppm): 6.82 (1H, d, J=7.7 Hz), 6.67-6.70 (2H, m), 5.47 (1H, s), 4.19 (2H, q, J=7.2 Hz), 3.87 (3H, s), 2.88 (2H, t, J=7.5 Hz), 2.58 (2H, t, J=7.5 Hz), 1.24 (3H, t, J=7.2 Hz)

### Reference Example 3

To 10 g of ethyl 3-{3-methoxy-4-(benzyloxy)phenyl} acrylate and 10 g of 20 % aqueous solution of sodium hydroxide, ethanol was mixed until it was homogenized. The obtained mixture was stirred under reflux for 2 hours. The reaction mixture was cooled, ethanol was distilled off under reduced pressure. Water was added to it, then adjusted to PH=2 with 5 % hydrochloric acid, and extracted with ethyl acetate. The organic layer was washed with saturated aqueous solution of sodium chloride twice, dried by magnesium sulfate, and concentrated under reduced pressure. The residue was washed by hexane to obtain 9 g of 3-{3-methoxy-4-(benzyloxy)phenyl}acrylic acid. 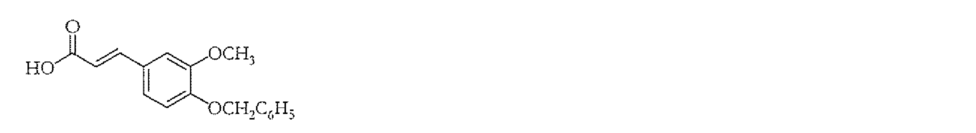 ¹H-NMR (CD₃SOCD₃, TMS) δ (ppm): 12.18 (1H, s), 7.51 (1H, d, J=16 Hz), 7.33-7.44 (6H, m), 7.05 (1H, d, J=8.2 Hz), 6.44 (1H, d, J=16 Hz), 6.66-6.70 (2H, m), 5. 13 (2H, s), 3.82 (3H, s)

### Reference Example 4

5.0 g of 3-{3-methoxy-4-(benzyloxy)phenyl} acrylic acid, 2 ml of thionyl chloride, 100 ml of toluene and about 0.05 g of N, N-dimethylformamide were mixed and stirred at 80 °C for 30 minutes. Then the reaction mixture was cooled to room temperature, and concentrated under reduced pressure to obtain 3-{3-methoxy-4-(benzyloxy)phenyl}acrylic chloride. The 3-{3-methoxy-4-(benzyloxy)phenyl}-acrylic chloride, 2.1 g of 4-methylbenzylamine, 3.7 ml of triethylamine and 100 ml of tetrahydrofuran were mixed, and stirred at room temperature for 1 hour. Then water was added to the reaction mixture, solvent was distilled off under reduced pressure. The obtained solid was collected by filtration. The solid was washed by hexane, and dried to obtain 6.5 g of N-(4-methylbenzyl)-3-{3-methoxy-4-(benzyloxy)phenyl}acrylam ide. 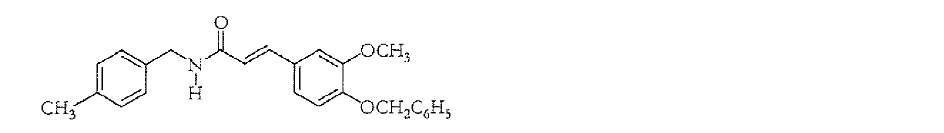 ¹H-NMR (CDCl₃, TMS) δ (ppm): 7.58 (1H, d, J=15 Hz), 7.14-7.43 (9H, m), 6.98-6.99 (2H, m), 6.85 (1H, d, J=8.2 Hz), 6.25 (1H, d, J=15 Hz), 5.75 (1H, br.s), 5.75 (2H, s), 4.52 (2H, d, J=5.6 Hz), 3. 90 (3H, s), 2.34 (3H, s)

### Reference Example 5

50 g of 3-(4-hydroxy-3-methoxyphenyl)acrylic acid, 0.5 g of 5 % of palladium charcoal, about 0.05 g of 36 % hydrochloric acid, 250 ml of ethanol and 100 ml of tetrahydrofuran were stirred under hydrogen atmosphere. After stopping absorption of hydrogen gas, it was filtered, and the filtrate was concentrated under reduced pressure to obtain 52 g of 3-(4-hydroxy-3-methoxyphenyl)propionic acid. 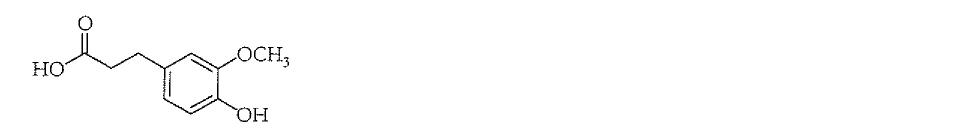 ¹H-NMR (CDCl₃, TMS) δ (ppm): 6.83 (1H, dd, J=7.3 Hz, 0.8 Hz), 6.81-6.70 (2H, m), 3.86 (3H, s), 2.88 (2H, t, J=7.6 Hz), 2.65 (2H, t, J=7.6 Hz)

### Reference Example 6

25 g of 3-ethoxy-4-hydroxybenzaldehyde, 27 g of benzyl bromide, 25 g of potassium carbonate and 250 ml of acetonitrile were mixed, and stirred under condition of reflux for 3 hours. Then the reaction mixture was cooled to room temperature. Ethyl acetate was added to it, and solid was filtered off. The obtained organic layer was concentrated under reduced pressure, the residue was washed bymethyl=tert-butylether and hexane to obtain 36 g of 4-benzyloxy-3-ethoxybenzaldehyde.
¹H-NMR (CDCl₃, TMS) δ (ppm): 9.82 (1H, s), 7.28-7.44 (7H, m), 6.98 (1H, d, J=8.2 Hz), 5.24 (2H, s), 4.18 (2H, q, J=7.0 Hz), 1.49 (3H, t, J=7.0 Hz)

### Reference Example 7

19 g of ethyl diethylphosphonoacetate and 400 ml of tetrahydrofuranweremixed, added 3.3 g of sodium hydride (content 60 %) under ice cooling, and stirred for 10 minutes. Then the mixture of 20 g of 4-benzyloxy-3-ethoxybenzaldehyde and 50 ml of tetrahydrofuran was added gradually to it under ice cooling, and stirred at room temperature for 1 hour. Water was added to the obtained mixture. Organic solvent was distilled off under reduced pressure, and then extracted with ethyl acetate. The oil layer was separated. It was washed with water, buffer solution of PH 6.8 and saturated aqueous solution of sodium chloride, dried by magnesium sulfate, concentrated under reduced pressure. The obtained residue was washed by hexane to obtain 25 g of ethyl 3-{3-ethoxy-4-(benzyloxy)phenyl}acrylate. 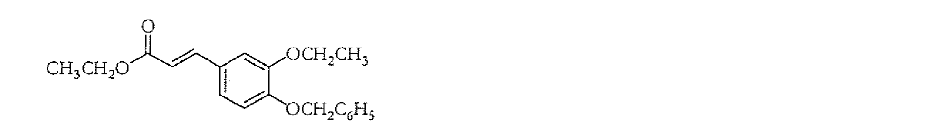 ¹H-NMR (CDCl₃, TMS) δ (ppm): 7.59 (1H, d, J=16 Hz), 7.28-7.44 (5H, m), 7.07 (1H, d, J=2.0 Hz), 7.02 (1H, dd, J=8.3 Hz, 2.0 Hz), 6.86 (1H, d, J=8.3 Hz), 6.28 (1H, d, J=16 Hz), 5.18 (2H, s), 4.28 (2H, q, J=7.1 Hz), 4.31 (2H, q, J=6.8 Hz), 1.47 (3H, t, J=6.8 Hz), 1.33 (3H, t, J=7.1 Hz)

### Reference Example 8

23 g of ethyl 3-{3-ethoxy-4-(benzyloxy)phenyl}acrylate, 0.2 g of 5 % palladium charcoal, about 0.04 g of 36 % hydrochloric acid, 120 ml of ethanol and 100 ml of tetrahydrofuran were stirred under hydrogen atmosphere. After stopping absorption of hydrogen gas, it was filtered, and the filtrate was concentrated under reduced pressure. The residue was washed by hexane to obtain 17 g of ethyl 3-(4-hydroxy-3-ethoxyphenyl)propionate. 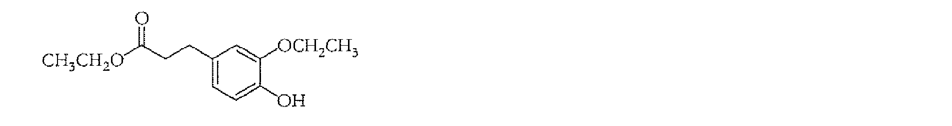 ¹H-NMR (CDCl₃, TMS) δ (ppm): 6.83 (1H, d, J=8.0 Hz), 6.66-6.70 (2H, m), 5.55 (1H, s), 4.01-4.15 (4H, m), 2.86 (2H, t, J=7.8 Hz), 2.57 (2H, t, J=7.8 Hz), 1.43 (3H, t, J=6.9 Hz), 1.23 (3H, t, J=7.2 Hz)

### Reference Example 9

30 g of 4-acetyl-2-methoxyphenol, 32 g of benzyl bromide, 28 g of potassium carbonate and 300 ml of acetonitrile were mixed and stirred under condition of reflux for 4 hours. Then the reaction mixture was cooled to room temperature. Ethyl acetate was added to it, and the solid was filtered off. The obtained organic layer was concentrated under reduced pressure. The residue was washed by hexane to obtain 46 g of 4-benzyloxy-3-methoxyacetophenone.
¹H-NMR (CDCl₃, TMS) δ (ppm): 7.28-7.57 (7H, m), 6.89 (1H, d, J=8.3 Hz), 5.23 (2H, s), 3.94 (3H, s), 2.54 (3H, s)

### Reference Example 10

18 g of ethyl diethylphosphonoacetate and 400 ml of tetrahydrofuran were mixed, added 3.2 g of sodium hydride (content 60 %) under ice cooling, and stirred for 10 minutes. Then the mixture of 20 g of 4-benzyloxy-3-methoxyacetophenone and 50 ml of tetrahydrofuran was added gradually to it under ice cooling, and stirred at room temperature for 2 hours. Water was added to the obtained mixture. The organic solvent was distilled off under reduced pressure, and then extracted with ethyl acetate. The oil layer was separated. It was washed with water and saturated aqueous solution of sodium chloride, dried by magnesium sulfate, concentrated under reduced pressure. The obtained residue was purified by silica gel column to obtain 17 g of ethyl 3-(3-methoxy-4-benzyloxyphenyl)-2-butenoate. 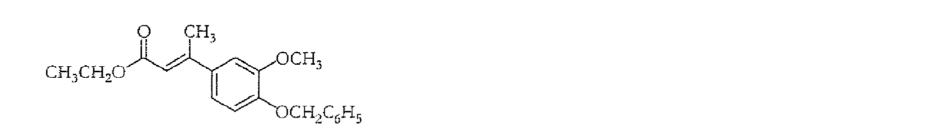 ¹H-NMR (CDCl₃, TMS) δ (ppm): 7.22-7.44 (5H, m), 7.00-7.02 (2H, m), 6.86 (1H, d, J=8.7 Hz), 6.09 (1H, q, J=1.2 Hz), 5.17 (2H, s), 4.21 (2H, q, J=7.0 Hz), 3.92 (3H, s), 2.55 (3H, d, J=1.2 Hz), 1.31 (3H, t, J=7.0 Hz)

### Reference Example 11

10.0 g of ethyl 3-{4-benzyloxy-3-methoxy-phenyl}-2-butenoate, 1.0 g of 10 % palladium charcoal, 15 ml of concentrated hydrochloric acid, 0.5 g of 10 % platinum charcoal and 100 ml of ethanol were stirred under hydrogen atmosphere. After stopping absorption of hydrogen gas, it was filtered, and the filtrate was concentrated under reduced pressure. The residue was separated with ethyl acetate to obtain 7.24 g of ethyl 2-(4-hydroxy-3-methoxyphenyl)butyrate. 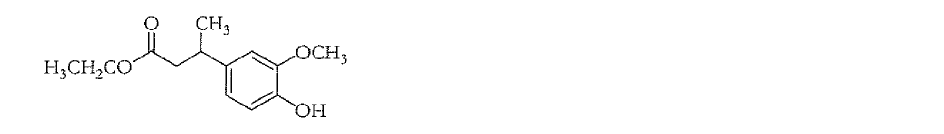 ¹H-NMR (CDCl₃, TMS) δ (ppm): 6.83 (1H, d, J=8.7 Hz), 6.70-6.75 (2H, m), 5.50 (1H, br.s), 4.07 (2H, q, J=7.0 Hz), 3.87 (3H, s), 3.15-3.26 (1H, m), 2.46-2.63 (2H, m), 1.27 (3H, d, J=6.7 Hz), 1.19 (3H, t, J=7.0 Hz)

### Reference Example 12

30 g of 4-benzyloxy-3-methoxybanzaldehyde, 12 g of sodium propionate and 24 g of propionyl anhydride were mixed, and stirred at 150 °C for 6 hours. Then the reaction mixture was cooled to room temperature. Water was added to it and extracted with ethyl acetate. The oil layer was separated. It was washed with 5 % hydrochloric acid and saturated aqueous solution of sodium chloride twice, dried by magnesium sulfate, and concentrated under reduced pressure. The obtained residue was washed by toluene and hexane to obtain 28 g of 3-(3-methoxy-4-benzyloxyphenyl)-2-methylacrylic acid. 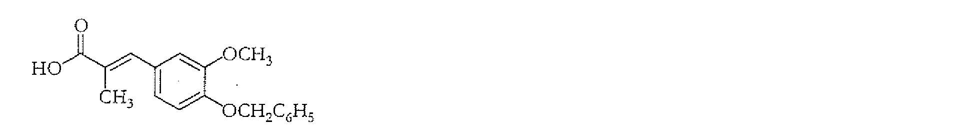 ¹H-NMR (CDCl₃, TMS) δ (ppm): 7.74 (1H, br.s), 7.29-7.45 (5H, m), 6.99-7.04 (2H, m), 6.91 (1H, d, J=8.9 Hz), 5.20 (2H, s), 3.91 (3H, s), 2.16 (3H, d, J=1.3 Hz)

### Reference Example 13

9 ml of acetyl chloride was mixed to 75 ml of ethanol, and stirred at room temperature for 10 minutes. 8.3 g of 3-(3-methoxy-4-benzyloxyphenyl)-2-methylacrylic acid was mixed to the said mixture, and stirred under the condition of reflux for 2 hours. The obtained reaction mixture was filtered, concentrated under reduced pressure. The residue, 1.0 g of 10 % palladium charcoal, 0.5 g of platinum charcoal, 15 ml of 36 % hydrochloric acid and 80 ml of ethanol were stirred under hydrogen atmosphere. After stopping absorption of hydrogen gas, it was filtered, and the filtrate was concentrated under reduced pressure. The residue was washed by hexane to obtain 5.4 g of ethyl 2-(4-hydroxy-3-methoxyphenyl)-2-methyl-proionate. 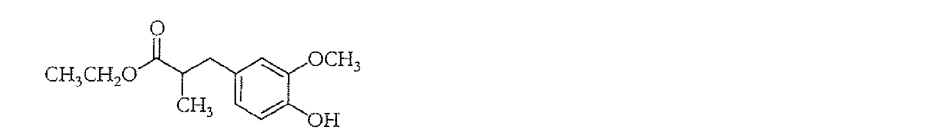 ¹H-NMR (CDCl₃, TMS) δ (ppm): 6.81 (1H, d, J=8.5 Hz), 6. 64-6. 56 (2H, m), 5.46 (1H, br.s), 4.09 (3H, q, J=7.1 Hz), 3.86 (3H, s), 2.94 (1H, dd, J=13.3 Hz, 6.8 Hz), 2.56-2.72 (2H, m), 1.20 (3H, t, J=7.1 Hz), 1.14 (3H, d, J=7.0 Hz)

### Reference Example 14

15.2 g of 3-hydroxy-4-methoxy-benzaldehyde, 18.0 g of benzyl bromide, 15.2 g of potassium carbonate and 200 ml of acetonitrile were mixed and stirred at 80 °C for 3 hours. Then the reaction mixture was cooled to room temperature, ethyl acetate was added to it and filtered. The filtrate was concentrated under reduced pressure. The residue was purified by silica gel column to obtain 23.3 g of (3-benzyloxy-4-methoxy) banzaldehyde.
¹H-NMR (CDCl₃, TMS) δ (ppm): 9.80 (1H, s), 7.30-7.48 (7H, m), 6.98 (1H, d, J=8.7 Hz), 5.118 (2H, s), 3.95 (3H, s)

### Reference Example 15

2. 1 g of sodium hydride and 150 ml of tetrahydrofuran were mixed at 0 to 5 °C, and 11.2 g of ethyl diethylphosphonoacetate was dropped slowly to it. After dropping, it was stirred for 30 minutes. Then tetrahydrofuran solution of 12.1 g of (3-benzyloxy-4-methoxy)benzaldehyde was dropped, and stirred at 0 to 5 °C for 15 minutes then at room temperature for 30 minutes . After that, water was added to it and separated to two layer with ethyl acetate. The organic layer was dried by magnesium sulfate, and concentrated under reduced pressure. The residue was washed by hexane to obtain 14.1 g of ethyl 3-(3-benzyloxy-4-methoxyphenyl)acrylate. 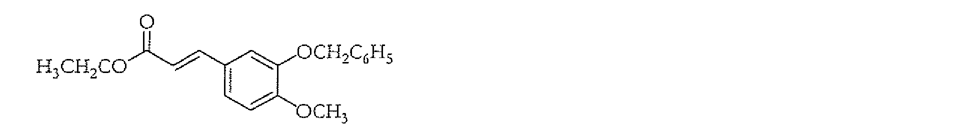 ¹H-NMR (CDCl₃, TMS) δ (ppm): 7.56 (1H, d, J=15,7 Hz), 7.42-7.47 (2H, m), 7.34-7.41 (2H, m), 7.27-7.34 (1H, m), 7.11 (1H, dd, J=8.2 Hz, 1.9 Hz), 7.07 (1H, d, J=1.9 Hz), 6.87 (1H, d, J=8.2 Hz), 6.22 (1H, d, J=15.6 Hz), 5.15 (2H, s), 4.24 (2H, q, J=7.0 Hz), 3.91 (3H, s), 1.32 (3H, t, J=7.0 Hz)

### Reference Example 16

8.0 g of ethyl 3- (3-benzyloxy-4-methoxyphenyl) acrylate, 0.8 g of 10 % palladium charcoal and 80 ml of ethanol were stirred under hydrogen atmosphere. After stopping absorption of hydrogen gas, it was filtered, and the filtrate was concentrated under reduced pressure. The residue was washed by hexane to obtain 5.33 g of ethyl 3-(3-hydroxy-4-methoxyphenyl)proionate. 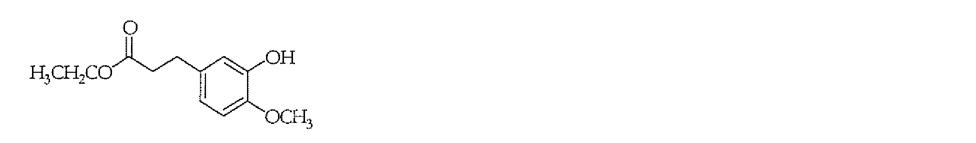 ¹H-NMR (CDCl₃, TMS) δ (ppm) : 6.74-6.78 (2H, m), 6.67 (1H, dd, J=8.1 Hz, 2.0 Hz), 5.55 (1H, br.s), 4.12 (2H, q, J=7.0 Hz), 3.86 (3H, s), 2.85 (2H, t, J=7.9 Hz), 2.57 (2H, t, J=7.9 Hz), 1.23 (3H, t, J=7.1 Hz)

### Reference Example 17

In the same way as in the Reference Production Example 12, 2.53 g of ethyl 3-{4-methoxy-3-(2-propynyloxy)phenyl}propionate was obtained from 2.24 g of ethyl 3-(3-hydroxy-4-methoxyphenyl)propionate. 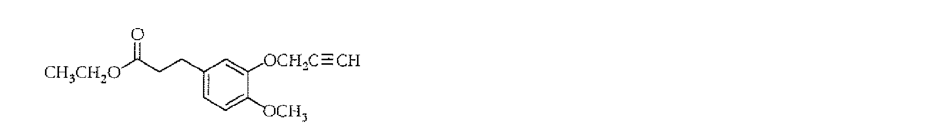 ¹H-NMR (CDCl₃, TMS) δ (ppm) : 6.79-5.89 (3H, m), 4.74 (2H, d, J=2.4 Hz), 4.12 (2H, q, J=7.1 Hz), 3.84 (3H, s), 2.89 (2H, t, J=7.6 Hz), 2.59 (2H, t, J=7.6 Hz), 2.49 (1H, t, J=2.4 Hz), 1.24 (3H, t, J=7.1 Hz)

### Reference Example 18

In the same way as in the Reference Production Example 13, 1.93 g of 3-{4-methoxy-3-(2-propynyloxy)phenyl}propionic acid was obtained from 2.53 g of ethyl 3-{4-methoxy-3-(2-propynyloxy)phenyl}propionate. 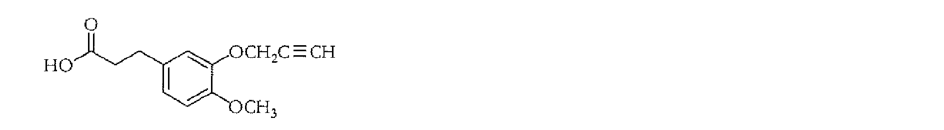 ¹H-NMR (CDCl₃, TMS) δ (ppm): 6.80-6.90 (3H, m), 4.75 (2H, d, J=2.4 Hz), 3.84 (3H, s), 2.91 (2H, t, J=7.6 Hz), 2.66 (2H, t, J=7.6 Hz), 2.49 (1H, t, J=2.4 Hz)

### Reference Example 19

1.59 g of 4-hydroxy-benzylamine hydrochloride, 2. 34 g of 3-{3-methoxy-4-(2-propynyloxy)phenyl}-propionic acid, 2.11 g of WSC and 25 ml of pyridine were mixed, and stirred at room temperature for 4 hours. Then water was added to the reaction mixture and extracted with ethyl acetate. The organic layer was washed successively with 5 % hydrochloric acid, water and saturated aqueous solution of sodium chloride, dried by magnesium sulfate, and concentrated under reduced pressure. The residue was purified by silica gel column to obtain 2.1 g of N-(4-hydroxy-benzyl)-3-{4-methoxy-3-(2-propynyloxy)phenyl}p ropanamide. 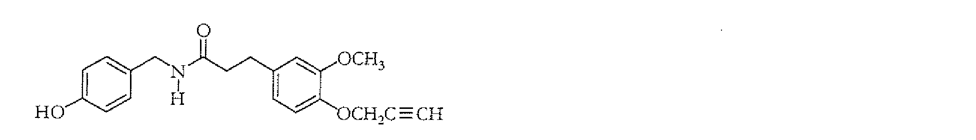 ¹H-NMR (CDCl₃, TMS) 5 (ppm): 6.91-7.02 (3H, m), 6.70-6.77 (4H, m), 5.54 (1H, br. s), 4.73 (2H, d, J=2.0 Hz), 4.31 (2H, d, J=5.6 Hz), 3.81 (3H, s), 2.94 (2H, t, J=7.6 Hz), 2.45-2.52 (3H, m)

### Reference Example 20

10 g of N-(4-chlorobenzyl)-3-{4-hydroxy-3-methoxyphenyl}propanamide, 5.6 g of benzyl bromide, 5.6 g of potassium carbonate and 150 ml of acetonitrile were mixed, and stirred under the condition of reflux for 4 hours. Then the reaction mixture was cooled to room temperature. Ethyl acetate was added to it, and the solid was filtered off. The obtained organic layer was concentrated under reduced pressure, the residue was washed by hexane to obtain 11 g of N-(4-chlorobenzyl)-3-{4-benzyloxy-3-methoxyphenyl}propanami de. 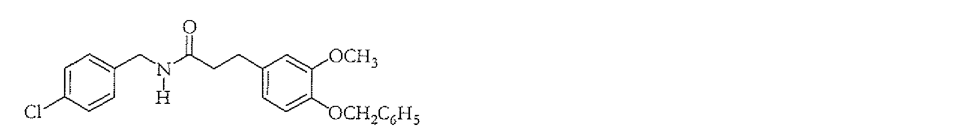 ¹H-NMR (CDCl₃, TMS) δ (ppm): 7.43-7.45 (2H, m), 7.23-7.38 (5H, m), 7.06 (2H, d, J=8.2 Hz), 6.78 (1H, d, J=8.0 Hz), 6.74 (1H, d, J=1.9 Hz), 6.64 (1H, dd, J=8.0 Hz, 1.9 Hz), 5.89 (1H, br.s), 5.12 (2H, s), 4.35 (2H, d, J=6.1 Hz), 3.84 (3H, s), 2.99 (2H, t, J=7.5 Hz), 2.49 (2H, t, J=7.5 Hz)

### Reference Example 21

3.7 g of N-(4-chlorobenzyl)-3-{4-benzyloxy-3-methoxyphenyl}propanami de, 50 ml of monochlorobenzene and 0.75 g 150 ml of sulfuryl chloride were mixed, and stirred at room temperature for 1 hour. Then toluene was added to the reaction mixture, and solid was filtered off. The obtained solid was washed with toluene and hexane, and purified by silica gel column to obtain 3.3 g of N-(4-chlorobenzyl)-3-{4-benzyloxy-2-chloro-5-methoxyphenyl} propanamide. 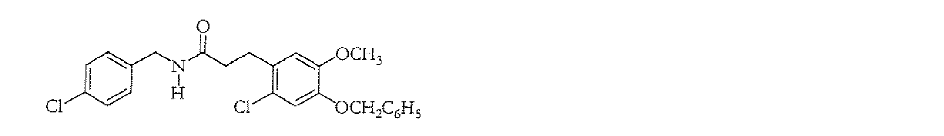 ¹H-NMR (CD₃SOCD₃, TMS) 5 (ppm) : 8.40 (1H, t, J=5.9 Hz3, 7.13-7.45 (9H, m), 7.07 (1H, s), 6.91 (1H, s), 5.08 (2H, s), 4.24 (2H, d, J=6.1 Hz), 3.70 (3H, s), 2.81 (2H, t, J=7.5 Hz), 2.44 (2H, t, J=7.5 Hz)

### Reference Example 22

3.0 g of N-(4-chlorobenzyl)-3-{benzyloxy-2-chloro-5-methoxyphenyl}pr opanamide, 50 ml of acetic acid and 1.48 g of 48 % hydrobromic acid were mixed and stirred at 80 °C for 3 hours. Then the reaction mixture was concentrated under reduced pressure and and purified by silica gel column to obtain 2.2 g of N-(4-chlorobenzyl)-3-{2-chloro-4-hydroxy-5-methoxyphnyl}pro panamide. 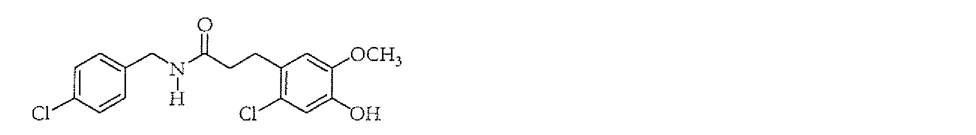 ¹H-NMR (CDCl₃, TMS) δ (ppm) : 7.20-7.25 (2H, m), 7.00-7.04 (2H, m), 6.91 (1H, s), 6.71 (1H, s), 5.69 (1H, br.s), 5.62 (1H, s), 4.34 (2H, d, J=5.8 Hz), 3.78 (3H, s), 3.01 (2H, t, J=7.2 Hz), 2.51 (2H, t, J=7.2 Hz)

### Reference Example 23

1.1 g of 3-fluoro-4-hydroxy-5-methoxybenzaldehyde, 1.2 g of benzyl bromide, 1.3 g of potassium carbonate and 15 ml of acetonitrile were mixed, and stirred under the condition of reflux for 4 hours. Then the reaction mixture was cooled to room temperature. Ethyl acetate was added to it, and the solid was filtered off. The obtained organic layer was concentrated under reduced pressure, the residue was washed by hexane to obtain 1.8 g of 4-benzyloxy-3-fluoro-5-methoxybenzaldehyde. Next, 1.5 g of ethyl diethylphosphonoacetate and 20 ml of tetrahydrofuran were mixed. 0.29 g of sodium hydride (content 55%) was mixed to the mixture under ice cooling for 10 minutes. The mixture of 1.8 g of 4-benzyloxy-3-fluoro-5-methoxybenzaldehyde and 5 ml of tetrahydrofuran was added slowly to it under ice cooling, and stirred at room temperature for 1 hour. Water was added to the obtained mixture . The organic solvent was distilled off under reducedpressure, and extracted with ethyl acetate. The oillayer was separated, washed with 5 % hydrochloric acid, saturated aqueous solution of sodium bicarbonate and saturated aqueous solution of sodium chloride, dried by magnesium sulfate, concentrated under reduced pressure. The obtained residue was washed by hexane to obtain 3.6 g of ethyl 3-(4-benzyloxy-3-fluoro-5-methoxyphenyl)acrylate. 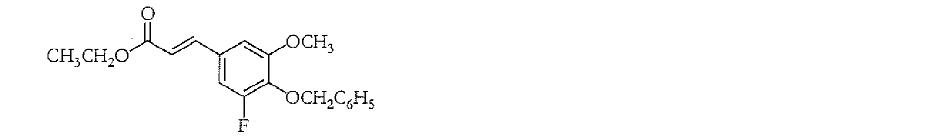 ¹H-NMR, (CDCl₃, TMS) δ (ppm): 7.54 (1H, d, J=16 Hz), 7.44-7.46 (2H, m), 7.29-7.40 (3H, m), 6.89 (1H, dd, J=11 Hz, 1.9 Hz), 6.83 (1H, br.s), 6.32 (1H, d, J=16 Hz), 5.14 (2H, s), 4.26 (2H, q, J=7.2 Hz), 3.88 (3H, s), 1.33 (3H, t, J=7.2 Hz)

### Reference Example 24

3. 6 g of ethyl 3- (4-benzyloxy-3-fluoro-5-methoxyphenyl) acrylate, 0.1 g of 5 % palladium charcoal about 0.01 g of 36 % hydrochloric acid, 50 ml of ethanol were stirred under hydrogen atmosphere. After stopping absorption of hydrogen gas, it was filtered, and the filtrate was concentrated under reduced pressure to obtain 2.2 g of ethyl 3-(3-fluoro-4-hydroxy-5-methoxyphenyl)proionate. 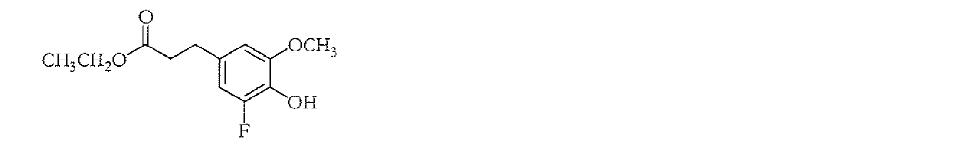 ¹H-NMR (CDCl₃, TMS) δ (ppm): 6.58 (1H, dd, J=11 Hz, 2.0 Hz), 6.51-6.52 (1H, m), 4.13 (2H, q, J=7.1 Hz), 3.89 (3H, s), 2.86 (2H, t, J=7.7 Hz), 2.58 (2H, t, J=7.7 Hz), 1.24 (3H, t, J=7.1 Hz)

### Reference Example 25

2.3 g of ethyl 3-(3-fluoro-4-hydroxy-5-methoxyphenyl)proionate, 0.63 ml of propargyl bromide, 1.23 g of potassium carbonate and 300 ml of acetonitrile were mixed and stirred under the condition of reflux for 1 hour. Then the reaction mixture was cooled to room temperature, and added ethyl acetate and filtered. The filtrate was concentrated under reduced pressure. The obtained residue, 0.54 g of lithium hydroxide, 40 ml of tetrahydrofuran and 20 ml of water were mixed, and stirred under the condition of reflux for 3 hours. Then water was added to the reaction mixture and concentrated under reduced pressure. The mixture was washed with methyl=tert-butylether, and aqueous layer was separated. 5 % hydrochloric acid was added to it, and extracted with chloroform for three times. The organic layer was dried by magnesium sulfate and concentrated under reduced pressure. The residue was washed by hexane to obtain 1.5 g of 3-{3-fluoro-5-methoxy-4-(2-propynyloxy)phenyl}proionic acid. 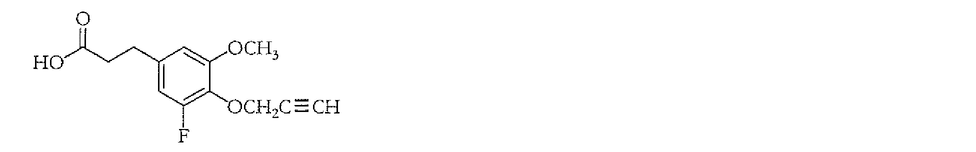 ¹H-NMR (CDCl₃, TMS) δ (ppm): 6.59 (1H, dd, J=11 Hz, 2.2 Hz), 6.55-6.56 (1H, m), 4.73 (2H, d, J=2.4 Hz), 3.56 (3H, s), 2.90 (2H, t, J=7.7 Hz), 2.67 (2H, t, J=7.7 Hz), 2.27 (1H, t, J=7.1 Hz)

### Reference Example 26

Tetrahydrofuran solution of 15.3 g of 2-naphthonitrile was dropped slowly to the mixture of 7.58 g of lithium aluminum hydride and 100 ml of tetrahydrofuran, and it was stirred at room temperature for 3 hours. Then the reaction mixture was cooled to 0 to 5 °C, and aqueous caustic soda was dropped slowly to it. After dropping, the mixture was filtered and filtrate was concentrated under reduced pressure. Ethyl acetate and water were added to the residue, and separated into two layer. The organic layer was dried by magnesium sulfate, and concentrated under reduced pressure. The residue was washed by hexane to obtain 12.5 g of C-naphethalene-2-yl-methylamine.
¹H-NMR (CDCl₃, TMS) δ (ppm): 7.80-7.83 (3H, m), 7.74 (1H, s), 7.41-7.49 (3H, m), 4.03 (2H, s), 1.62 (2H, br.s)

### Reference Example 27

The mixture of 31 g of aluminum chloride and 150 ml of methylene chloride was ice cooled, 30 g of ethyl oxalyl chloride was mixed to it, and stirred under ice cooling for 30 minutes. The obtained mixture was added slowly to the mixture of 22 g of indan and 200 ml of methylene chloride under ice cooling, and stirred at room temperature for 1 hour. The reaction mixture was mixed slowly to ice water, then organic layer was separated. The organic layer was washed with water, dried by magnesium sulfate, and concentrated under reduced pressure to obtain 37 g of crude product of ethyl indan-5-yl-oxo-acetate. 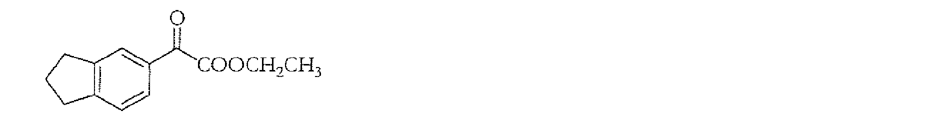 ¹H-NMR (CDCl₃, TMS) δ (ppm): 7.84 (1H, s), 7.78 (1H, d, J=7.8 Hz), 7.34 (1H, d, J=7.8 Hz), 4.44 (2H, q, J=7.1 Hz), 2.95-2.99 (4H, m), 2.09-2.17 (2H, m), 1.42 (3H, t, J=7.1 Hz)

### Reference Example 28

The mixture of 25 g of crude product of ethyl indan-5-yl-oxo-acetate, 7.0 g of sodium borohydride and 250 ml of ethanol was stirred at room temperature for 1 hour and at 60 °C for 2 hours. Water was added to the reaction mixture, and the organic solvent was distilled off, adjusted to PH=2 by 36 % hydrochloric acid, then extracted with chloroform. The organic layer was washed with water, dried by magnesium sulfate, and concentrated under reduced pressure. The residue was washed by hexane to obtain 11 g of indan-5-yl-ethan-1,2diol. 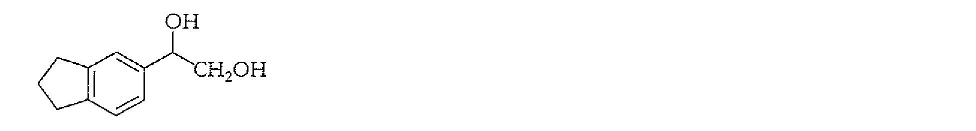 ¹H-NMR (CDCl₃, TMS) δ (ppm): 7.22 (1H, s), 7.20 (1H, d, J=7.7 Hz), 7.11 (1H, d, J=7.7 Hz), 4.78 (1H, dd, J=8.2 Hz, 3.6 Hz), 3.62-3.75 (2H, m), 2.87-2.91 (4H, m), 2.5 (1H, br.s), 2.3 (1H, br.s), 2.03-2.10 (2H, m)

### Reference Example 29

The mixture of 11 g of crude product of indan-5-yl-ethan-1,2-diol, 18 g of periodic acid, 100 ml of water and 100 ml of ethanol was stirred at room temperature for 12 hours. Water was added to the reaction mixture, and extracted with ethyl acetate. It was washed with water twice, then the organic solvent was distilled off. The residue was purified by silica gel column to obtain 8.1 g of indan-5-carbaldehyde. 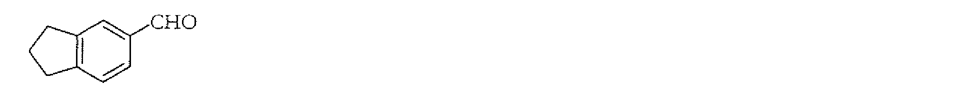 ¹H-NMR (CDCl₃, TMS) δ (ppm): 9.95 (1H, s), 7.73 (1H, s), 7.65 (1H, dd, J=7.7 Hz, 1.2 Hz), 7.36 (1H, d, J=7.7 Hz), 2.97 (4H, t, J=7.5 Hz), 2.08-2.17 (2H, m)

### Reference Example 30

The mixture of 8.1 g of indan-5-carbaldehyde, 4.3 g of hydroxylamine hydrochloride, 5.0 g of sodium acetate, 25 ml of water and 100 ml of ethanol was stirred at room temperature for 1 hour. Water was added to the reaction mixture, and extracted with methyl=tert-butylether. It was washed with water and saturated aqueous solution of sodium sulfate, then the organic solvent was distilled off under reduced pressure. The obtained residue was washed by hexane to obtain 6.3 g of indan-5-carbaldehydeoxime. 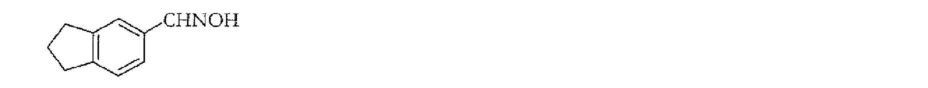 ¹H-NMR (CDCl₃, TMS) δ (ppm) : 8.11 (1H, s), 7.45 (1H, s), 7.30-7.35 (2H, m), 7.22 (1H, d, J=7.7 Hz), 2.91 (4H, t, J=7.5 Hz), 2.05-2.13 (2H, m)

### Reference Example 31

3.0 g of indan-5-carbaldehydeoxime, 0.8 g of 10 % palladium charcoal about 3.8 ml of 36 % hydrochloric acid, 90 ml of ethanol were stirred under hydrogen atmosphere. After stopping absorption of hydrogen gas, it was filtered, and the filtrate was concentrated under reduced pressure to obtain 3.2 g of indan-5-yl-methylamine hydrochloride. 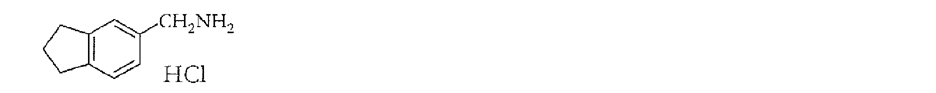 ¹H-NMR (CD₃SOCD₃, TMS) δ (ppm) : 8.37 (3H, br.s), 7.34 (1H, s), 7.21-7.34 (2H, m), 3.94 (2H, s), 2.85 (4H, t, J=7. 5 Hz), 1. 98-2. 05 (2H, m)

### Reference Example 32

In the same way as in the Reference Example 27, 55 g of crude product of ethyl 5,5,7,8-tetrahydro-naphthalene-2-yl-oxo-acetate was obtained from 58 g of Tetralin. 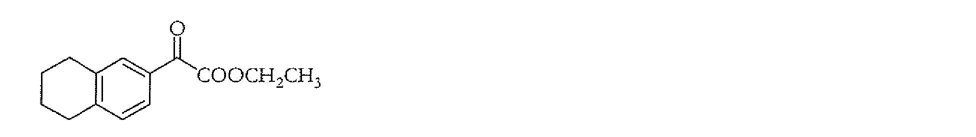 ¹H-NMR (CDCl₃, TMS) δ (ppm): 7.69-7.72 (2H, m), 7.17 (1H, d, J=7.8 Hz), 4.44 (2H, q, J=7.2 Hz), 2.75-2.83 (4H, m), 1.17-1.85 (4H, m), 1.42 (3H, t, J=7.2 Hz)

### Reference Example 33

In the same way as in the Reference Example 28, 17 g of 5,6,7,8-tetrahydro-naphethalene-2-yl-ethane-1,2diol was obtained from 30 g of crude product of ethyl 5,6,7,8-tetrahydro-naphthalene-2-yl-oxo-acetate. 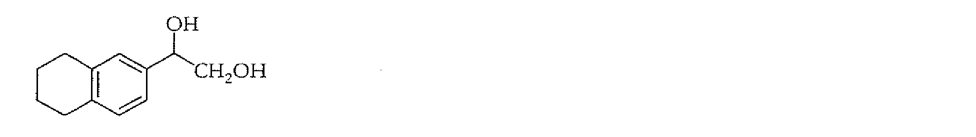 ¹H-NMR (CDCl₃, TMS) δ (ppm): 7.01-7.04 (3H, m), 4.76 (1H, dd, J=8.1 Hz, 3.7), 3.63-3.77 (2H, m), 2.75-2.76 (4H, m), 2.4 4 (1H, br.s), 2.0 (1H, br.s), 1.17-1.18 (4H, m)

### Reference Example 34

In the same way as in the Reference Example 29, 13 g of 5,6,7,8-tetrahydro-naphthalene-2-carbardehyde was obtained from 16 g of 5,6,7,8-tetrahydro-naphthalene-2-yl-ethane-1,2diol. 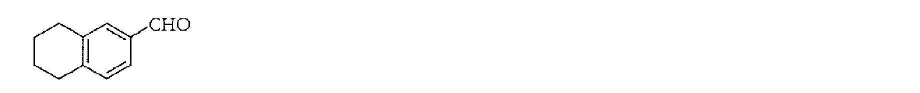 ¹H-NMR (CDCl₃, TMS) δ (ppm): 9.92 (1H, s), 7.57-7.59 (2H, m), 7.20 (1H, d, J=7.5 Hz), 2.82-2.85 (4H, m), 1.81-1.84 (4H, m)

### Reference Example 35

In the same way as in the Reference Example 30, 1.5 g of 5, 6,7, 8-tetrahydro-naphthalene-2-carbardehydeoxime was obtained from 2.6 g of 5,6,7,8-tetrahydro-naphthalene-2-carbardehyde. 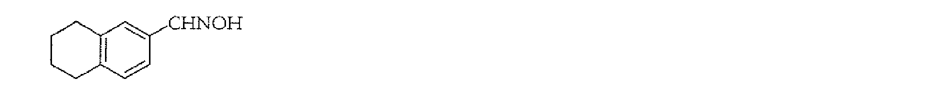 ¹H-NMR (CDCl₃, TMS) δ (ppm) : 8.08 (1H, s), 7. 33 (1H, s), 7.26-7.31 (2H, m), 7.07 (1H, d, J=7.6 Hz), 2.75-2.79 (4H, m), 1.78-1.82 (4H, m)

### Reference Example 36

In the same way as in the Reference Example 31, 1.5 g of 5,6,7,8-tetrahydro-naphthalene-2-yl-methylamine hydrochloride was obtained from 2.6 g of 5,6,7,8-tetrahydro-naphthalene-2-carbardehydeoxime. 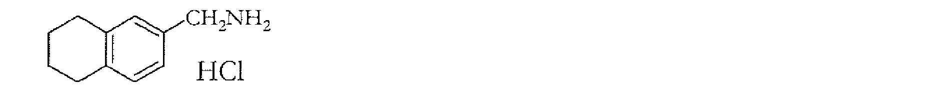 ¹H-NMR (CD₃SOCD₃, TMS) δ (ppm): 8.43 (3H, br.s), 7.17-7.19 (2H, m), 7.06-7.07 (1H, m), 3.89 (12H, d, J=5.1 Hz), 2.70 (4H, s), 1.72-1.73 (4H, m)

### Reference Example 37

In the same way as in the Reference Example 30, 4.69 g of mixture of isomer of 4-methoxy-3-methylbenzaldehydeoxime was obtained from 5.40 g of 4-methoxy-3-methylbanzaldehyde.
¹H-NMR (CDCl₃, TMS) δ (ppm) : 8.06 (1H, s), 7.81 (1H, br.s), 7.39 (1H, d, J=1.0 Hz), 7.34 (1H, dd, J=8.4 Hz, 2.0 Hz), 6.81 (1H, d, J=8.4 Hz), 3.84 (3H, s), 2.22 (3H, s)

### Reference Example 38

In the same way as in the Reference Example 31, 4.50 g of 4-methoxy-3-methyl-benzylamine hydrochloride was obtained from 4.69 g of mixture of isomer of 4-methoxy-3-methylbenzaldehydeoxime.
¹H-NMR (DMSO-d6, TMS) δ (ppm) : 8.26 (3H, br.s), 7.23-7.28 (2H, m), 6.94 (1H d, J=8.2 Hz), 3.88 (2H, s), 3.77 (3H, s), 2.13 (3H, s)

### Reference Example 39

In the same way as in the Reference Example 30, 5.00 g of mixture of isomer of 4-phenoxy-benzaldehydeoxime was obtained from 4.96 g of 4-phenoxy-banzaldehyde.
¹H-NMR (CDCl₃, TMS) δ (ppm): 8.11 (1H, s), 7.54 (2H, d, J=8.7 Hz), 7.32-7.40 (3H, m), 7.15 (1H, t, J=7.4 Hz), 7.04 (2H, d, J=7.7 Hz), 6.99 (2H, d, J=8.7 Hz)

### Reference Example 40

In the same way as in the Reference Example 31, 4.71 g of 4-phenoxy-benzylamine hydrochloride was obtained from 4.61 g of mixture of isomer of mixture of isomer of 4-phenoxy-benzaldehydeoxime.
¹H-NMR (DMSO-d6, TMS) δ (ppm) : 8.32 (3H, br.s), 7.48 (2H, d, J=8.7 Hz), 7.36-7.43 (2H, m), 7.15 (1H, t, J=7.4 Hz), 6.95-7.04 (4H, m), 3.97 (2H, s)

### Reference Example 41

9.5 g of potassium phthalimide, 10 g of 4-cyanobenzylbromide, 100 ml of dimethylformi were mixed, and stirred at 100 °C for 4 hours. Then the reaction mixture was cooled to room temperature, then water was added to it, and extracted with ethyl acetate. The oil layer was washed successively with 5 % hydrochloric acid, saturated aqueous solution of sodium bicarbonate and saturated aqueous solution of sodium chloride, dried by magnesium sulfate, concentrated underreducedpressure. The obtained residue was washed by hexane to obtain 13 g of N-(4-cyano-benzyl)phthalimide. 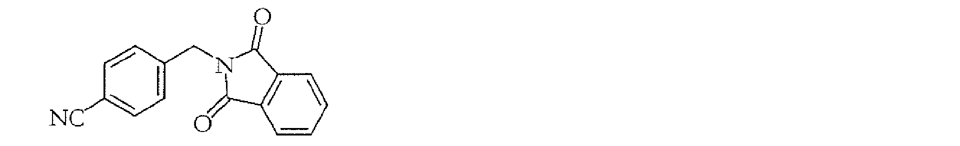 ¹H-NMR (CDCl₃, TMS) δ (ppm): 7.87 (2H, dd, J=5.4 Hz, 2.9 Hz), 7.74 (2H, dd, J=5.4 Hz, 2.9 Hz), 7.62 (2H, d, J=8.3 Hz), 7.52 (2H, d, J=8.3 Hz), 4. 89 (2H, s)

### Reference Example 42

0.90 g of hydrazine hydrate was dropped to mixture of 4.00 g of N-(4-cyano-benzyl)phthalimide and 15 ml of methanol, and stirred at 65 °C for 2 hours and half. Then the reaction mixture was cooled to room temperature, then water was added to it, and concentrated under reduced pressure. 1N hydrochloric acid was added to the residue, and it was filtered. Ethyl acetate was added to the filtrate, and it was washed successively with 25 % aqueous solution of caustic soda, saturated aqueous solution of sodium sulfate, dried by magnesium sulfate, and concentrated under reduced pressure to obtain 0.84 g of crude 4-cyanobenzylamine.
¹H-NMR (CDCl₃, TMS) δ (ppm): 7.61 (2H, d, J=8.2 Hz), 7. 44 (2H, d, J=7.9 Hz), 3.95 (2H, s), 1.55 (2H, br.s)

### Reference Example 43

In the same way as in the Reference Example 30, 6.4 g of mixture of isomer of 4-banzyloxy-benzaldehydeoxime was obtained from 6.39 g of 4-benzyloxy-banzaldehyde.
¹H-NMR (CDCl₃, TMS) δ (ppm): 8.08 (1H, s), 7.50 (2H, d, J=8.8 Hz), 7.30-7.47 (5H, m), 6.98 (2H, d, J=8.7 Hz), 5.09 (2H, s)

### Reference Example 44

In the same way as in the Reference Example 31, 4.0 g of 4-hydroxy-benzylamine hydrochloride was obtained from 6.4 g of mixture of isomer of mixture of isomer of 4-benzyloxy-benzaldehydeoxime.
¹H-NMR (DMSO-d6, TMS) δ (ppm) : 9.68 (1H, br.s), 8.31 (3H, br.s), 7.28 (2H, d, J=7.6Hz) 6.79 (2H, d, J=7.6Hz), 3.84-3.88 (2H, m)

### Reference Example 45

1.87 g of 2, 6-dimethylnaphthalene and 200 ml of 50 % aqueous acetic acid were mixed, and suspended at 80 °C. The mixed solution of 26.3 g of ammonium cerium(IV) nitrate and 400 ml of 50 % aqueous acetic acid was dropped to the said suspension over period for about 1 hour. Further it was stirred at 80 °C for 1 hour. Then the reaction solution was cooled to room temperature, and extracted with chloroform. The organic layer was washed successively with water, saturated aqueous solution of sodium bicarbonate, saturated aqueous solution of sodium chloride, concentrated under reduced pressure. The residue was purified by silica gel column to obtain 0.90 g of 6-methyl-2-naphthoaldehyde.
¹H-NMR (CDCl₃, TMS) δ (ppm) : 10.1 (1H, s), 8.29 (1H, s), 7.80-7.95 (3H, m), 7 . 68 (1H, s), 7.42 (1H, dd, J=8.4 Hz, 1.6 Hz), 2.56 (3H, s)

### Reference Example 46

In the same way as in the Reference Example 30, 1.56 g of mixture of isomer of 6-methyl-2-naphtoaldehydeoxime was obtained from 1.74 g of 6-methyl-2-naphtoaldehyde.
¹H-NMR (CDCl₃, TMS) δ (ppm): 8.27 (1H, s), 7.83 (1H, s), 7. 71-7, 81 (3H, m), 7.60 (1H, s), 7.41 (1H, s), 7.34 (1H, dd, J=8.5 Hz, 1.7 Hz), 2.51 (3H, s)

### Reference Example 47

In the same way as in the Reference Example 31, 1.60 g of C- (6-methyl-naphthlene-2-yl)-methylamine hydrochloride was obtained from 1.46 g of mixture of isomer of 6-methyl-2-naphtoaldehydeoxime.
¹H-NMR (DMSO-d6, TMS) δ (ppm): 8.64 (3H, br.s), 7.96 (1H, s), 7.86 (1H, d, J=8.7 Hz), 7.80 (1H, d, J=8.3 Hz), 7.71 (1H, s), 7.60-7.64 (1H, m), 7.37-7.43 (1H, m), 4. 10-4.20 (2H, m), 2.48 (3H, s)

### Reference Example 48

In the same way as in the Reference Example 45, 0.85 g of 7-methyl-2-naphthoaldehyde was obtained from 1.87 g of 2,7-dimethylnaphthalene.
¹H-NMR (CDCl₃, TMS) δ (ppm): 10.1 (1H, s), 8.25 (1H, s), 7.89 (2H, d, J=0.8 Hz), 7.81 (1H, d, J=8.3 Hz), 7.77 (1H, s), 7.48 (1H, dd, J=8.7 Hz, 1.5 Hz), 2.55 (3H, s)

### Reference Example 49

In the same way as in the Reference Example 30, 0.78 g of mixture of isomer of 7-methyl-2-naphtoaldehydeoxime was obtained from 1.32 g of 7-methyl-2-naphtoaldehyde.
¹H-NMR (CDCl₃, TMS) δ (ppm): 8.27 (1H, s), 7.70-7, 82 (4H, m), 7.61 (1H, s), 7.50 (1H, br.s), 7.34 (1H, dd, J=8.3 Hz, 1.7 Hz), 2.51 (3H, s)

### Reference Example 50

In the same way as in the Reference Example 31, 0.72 g of C-(7-methyl-naphthlene-2-yl)-methylamine hydrochloride was obtained from 0.78 g of mixture of isomer of 7-methyl-2-naphtoaldehydeoxime.
¹H-NMR (DMSO-d6, TMS) δ (ppm): 8.54 (3H, br.s), 7.89-7.93 (2H, m), 7.84 (1H, d, J=8.3 Hz), 7.66 (1H, s), 7.56 (1H, dd, J=8.5 Hz, 1.6 Hz), 7.39 (1H dd, J=8.5 Hz, 1.6 Hz), 4.16 (2H, s), 2.48 (3H, s)

### Reference Example 51

500 ml of tetrahydrofuran and 8.7 g of potassium hydride (mixture with liquid paraffin, content 35 %) were mixed. It was cooled to 0 °C, and mixed with 15 g of ethyl 4-chlorophenylacetate, then stirred at 0 °C for 30 minutes. 5.7 ml of 3-bromopropyn was mixed with it, and mixed at 0°C for 30 minutes and at room temperature for 8 hours. Water was added to the reaction mixture, and extracted with ethyl acetate. The oil layer was washed successively with 5 % hydrochloric acid, saturated aqueous solution of sodium bicarbonate and saturated aqueous solution of sodium chloride, dried by magnesium sulfate, and concentrated under reduced pressure. The obtained residue was was purified by silica gel column to obtain 13 g of ethyl 2-(4-chlorophenyl)-4-pentynic acid. 13 g of ethyl 2-(4-chlorophenyl)-4-pentynic acid, 27 ml of 20 % aqueous solution of caustic soda and about 60 ml of ethanol were mixed at 0 °C, and stirred at 70 °C for 1 hour. Water was added to the reaction mixture, then adjusted to PH=2 by 5 % hydrochloric acid, and extracted with ethyl acetate. The oil layer was washed successively with buffer solution of PH6.8, saturated aqueous solution of sodium chloride, dried by magnesium sulfate, and concentrated under reduced pressure. The obtained residue was washed by hexane to obtain 10 g of 2- (4-chlorophenyl)-4-pentynic acid.
¹H-NMR (CDCl₃, TMS) δ (ppm): 7.20-7.35 (4H, m), 3.80 (1H, t, J=7.6 Hz), 2.90 (1H, ddd, J=17 Hz, 7.6 Hz, 2.7 Hz), 2.64 (1H, ddd, J=17 Hz, 7.6 Hz, 2.7 Hz), 1.97 (1H, t, J=2.7 Hz)

### Reference Example 52

1.0 g of 2-(4-chlorophenyl)-4-pentynic acid, 1.2 ml of diphenylphosphoryl azide, 1 ml of triethylamine and 15 ml of tert-butanol were mixed, stirred under the condition of reflux for 3 hours. The reaction mixture was cooled, then water was added to it, and extracted with ethyl acetate. The oil layer was washed successively with 5 % hydrochloric acid, saturated aqueous solution of sodium bicarbonate, saturated aqueous solution of sodium chloride, dried by magnesium sulfate, and concentrated under reduced pressure. The obtained residue (tert-butyl {1-(4-chlorophenyl)-3-butynylamine}carbamylate), 1 ml of trifluoroacetic acid, 6 ml of acetic acid and 3 ml of water was mixed, and stirred at 70 °C for 3 hours. The reaction mixture was concentrated under reduced pressure to obtain 0.85 g of crude product of 1- (4-chlorophenyl) -3-butynylaminie acetic acid salt. The obtained 1-(4-chlorophenyl)-3-butynylaminie acetic acid salt was used as it is without purification. 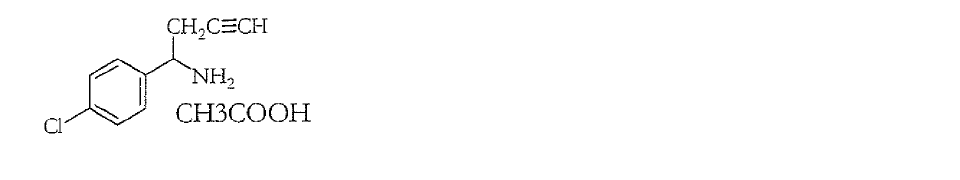 ¹H-NMR (CDCl₃, TMS) δ (ppm): 7.20-7.41 (4H, m), 4.8 (1H, br.s), 3.02-3.18 (1H, m), 2.62-2.76 (2H, m), 2.02 (1H, t, J=2.7 Hz), 1.43 (9H, s)

### Reference Example 53

10 g of 4-chloroacetophenone, 12 g of formamide and 1.0 g of formic acid were mixed, and stirred at 170 °C for 6 hours. The reaction mixture was cooled to room temperature, then water was added to it, and extracted with ethyl acetate. The oil layer was washed successively with water, and concentrated under reduced pressure. The obtained residue and 36 % hydrochloric acid were mixed, and stirred at 100 °C for 1 hour. Water was added to the reaction mixture, and it was washed by methyl=tert-butylether. The aqueous layer was adjusted to PH=10 by 20 % aqueous solution of caustic soda, then extracted with methyl-tert-butylether twice. The oil layer was washed successively with water and saturated aqueous solution of sodium chloride, dried by potassium carbonate, concentrated under reduced pressure. The obtained residue was washed by hexane to obtain 8.4 g of 1-(4-chlorophenyl)-ethylamine.
¹H-NMR (CDCl₃, TMS) δ (ppm): 7.27-7.30 (4H, m), 4. 10 (1H, q, J=6.5 Hz), 1.56 (2H, br.s), 1.36 (3H, d, J=6.5 Hz)

### Reference Example 54

In the same way as in the Reference Example 53, 6.6 g of 1-(3,4-dichlorophenyl)ethylamine was obtained from 9.5 g of 3,4-dichloroacetophenone. ¹H-NMR (CDCl₃, TMS) δ (ppm): 7.46 (1H, d, J=2.2 Hz), 7.38 (1H, d, J=8.2Hz), 7.18 (1H, dd, J=8.2Hz, 2.2 Hz), 4.19 (1H, q, J=6.5 Hz), 1.53 (2H, br.s), 1.35 (3H, d, J=6.5 Hz)

### Reference Example 55

In the same way as in the Reference Example 53, 5.3 g of 1-naphthalene-2-ylethylamine was obtained from 4.1 g of 2-acetylnaphtalene.
¹H-NMR (CDCl₃, TMS) δ (ppm): 7.76-7.84 (4H, m), 7.41-7.54 (3H, m), 4.28 (1H, q, J=6.6 Hz), 1.59 (2H, br.s), 1.46 (3H, d, J=6.5 Hz)

### Reference Example 56

3.0 g of 2-acetyl-5,6,7,8-tetrahydro-naphtalene, 3.1 g of formamide and 0.34 g of formic acid were mixed, and stirred at 170 °C for 6 hours. The reaction mixture was cooled to room temperature, then water was added to it, and extracted with ethyl acetate. The oil layer was washed successively with water, concentrated under reduced pressure. The obtained residue and 36 % hydrochloric acid were mixed, and stirred at 100 °C for 1 hour. Water was added to the reaction mixture, and the product was filtered off. The product was dried under reduced pressure to obtain 2.5 g of 1-(5,6,7,8-tetrahydronaphtalene-2yl)-ethylamine hydrochloride.
¹H-NMR (CD₃SOCD₃, TMS) δ (ppm): 8.51 (3H, br.s), 7.19-7.22 (2H, m), 7.08 (1H, d, J=8.0 Hz), 4.23-4.29 (1H, m), 2.70-2.71 (4H, m), 1.72-1.74 (4H, m), 1.48 (3H, d, J=6.8 Hz)

### Reference Example 57

4.75 g of 4-chloro-2-fluorobanzaldehyde and 30 ml of methanol were mixed. It was cooled to 0 °C, and 0.57 g of sodium borohydride was added to it, and stirred at 0 °C for 30 minutes and at room temperature for 1 hour. Water was added to the reaction mixture, and concentrated under reduced pressure. The obtained residue was extracted with chloroform. The organic layer was washed successively with 5 % hydrochloric acid, saturated aqueous solution of sodium chloride, dried by magnesium salfate, and concentrated under reduced pressure to obtain 4.57 g of (4-chloro-2-fluorophenyl)methanol.
¹H-NMR (CDCl₃, TMS) δ (ppm): 7.37 (1H, t, J=8.0 Hz), 7.12-7.17 (1H, m), 7.08 (1H, dd, J=9.7 Hz, 1.9 Hz), 4.73 (2H, s), 2.04 (1H, br.s)

### Reference Example 58

4.75 g of (4-chloro-2-fluoro-phenyl)methanol, 4.31 g of triethylamine and 60 ml of tetrahydrofuran were mixed. It was cooled to 0 °C, then 4.24 g of methanesulfonyl chloride was added to it, and stirred at 0 °C for 30 minutes, at room temperature for 2 hours. Water was added to the reaction mixture, and concentrated under reduced pressure. The obtained residue was extracted with chloroform. The organic layer was washed successively with 5 % hydrochloric acid, saturated aqueous solution of sodium chloride, dried by magnesium sulfate, concentrated under reduced pressure to obtain (4-chloro-2-fluorobenzyl) methanesulfonate.
¹H-NMR (CDCl₃, TMS) δ (ppm): 7.37-7.43 (1H, m), 7.14-7.22 (2H, m), 5.25 (2H, s), 3.01 (3H, s)

### Reference Example 59

2.64 g of potassium phthalimide, 3.4 g of (4-chloro-2-fluoro-benzyl) methanesulfonate and 80 ml of N, N-dimethylformamide were mixed, and stirred at room temperature for 2 hours. Then water was added to the reaction mixture, and extracted with chloroform. The organic layer was washed successively with 5 % hydrochloric acid, water, saturated aqueous solution of sodium chloride, dried by magnesium sulfate, concentrated under reduced pressure. The obtained residue was washed by hexane to obtain 2.92 g of N-(4-chloro-2-fluoro-benzyl)phthalimide. 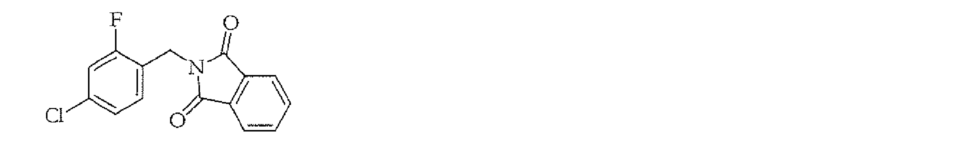 ¹H-NMR (CDCl₃, TMS) δ (ppm): 7.85-7.88 (2H, m), 7.71-7.75 (2H, m), 7.27-7.33 (1H, m), 7.05-7.11 (2H, m), 4.89 (2H, s)

### Reference Example 60

0.90 g of hydrazine hydrate was dropped to the mixed solution of 2.7 g of N-(4-chloro-2-fluoro-benzyl)phthalimide and 50 ml of methanol, and stirred at 65 °C for 2 hours and half. Then the reaction mixture was cooled to room temperature, water was added to it, and concentrated under reduced pressure. 1N-hydrocloric acid was added to the residue, and filtered. Chloroform was added to the filtrate, then 25 % aqueous solution of sodium hydroxide was added to it until the aqueous layer of the mixed solution turned to basic, and separated to two layer. The obtained organic layer was washed successively with saturated aqueous solution of sodium chloride, dried by magnesium sulfate, and concentrated under reduced pressure. 20 ml of acetonitrile and 2 ml of concentrated hydrochloric acid were added to the obtained residue. Then the residue which it was concentrated under reduced pressure was washed by hexane to obtain 1.13 g of 4-chloro-2-fluoro-benzylamine hydrochloride.
¹H-NMR (CD₃SOCD₃, TMS) δ (ppm) : 8.36 (3H, br. s), 7.51-7.68 (2H, m), 7.40 (1H, d, J=8.4 Hz), 4.04 (2H, s)

### Reference Example 61

In the same way as in the Reference Example 57, 3.98 g of (4-chloro-3-fluoro-phenyl)methanol was obtained from 3.96 g of . 4-chloro-3-fluorobenzaldehyde.
¹H-NMR (CDCl₃, TMS) δ (ppm): 7.33-7.39 (1H, m), 7.15-7.20 (1H, m), 7.05-7.09 (1H, m), 4.67 (2H, s), 1.83 (1H, br.s)

### Reference Example 62

In the same way as in the Reference Example 58, 5.77 g of (4-chloro-3-fluoro-benzyl) methanesulfonate was obtained from 3.98 g of (4-chloro-3-fluoro-phenyl)methanol.
¹H-NMR (CDCl₃, TMS) δ (ppm): 7.42-7.46 (1H, m), 7.20-7.24 (1H, m), 7.12-7.17 (1H, m), 5.19 (2H, s), 3.00 (3H, s)

### Reference Example 63

In the same way as in the Reference Example 59, 6.23 g of N-(4-chloro-3-fluoro-benzyl)phthalimide was obtained from 5.77 g of (4-chloro-3-fluoro-benzyl) methanesulfonate. 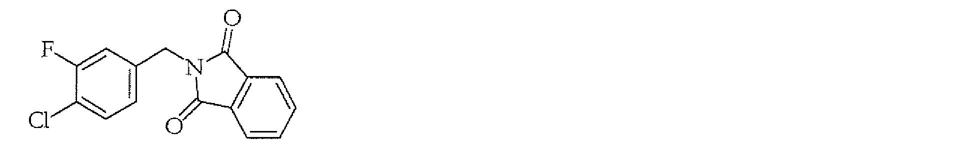 ¹H-NMR (CDCl₃, TMS) δ (ppm) : 7.84-7.88 (2H, m), 7.71-7.75 (2H, m), 7.30-7.36 (1H, m), 7.22 (1H, dd, J=9.5Hz, 1.9 Hz), 7.14-7.18 (1H, m), 4.80 (2H, s)

### Reference Example 64

In the same way as in the Reference Example 60, 1.2 g of 4-chloro-3-fluoro-benzylamine hydrochloride was obtained from 6.23 g of N-(4-chloro-3-fluoro-benzyl)phthalimide.
¹H-NMR (CD₃SOCD₃, TMS) δ (ppm): 8.35 (3H, br.s), 7.56-7.70 (2H, m), 7.33-7.49 (1H, m), 4.05 (2H, s)

### Reference Example 65

In the same way as in the Reference Example 45, 0.25 g of 6-fluoro-2-naphthoaldehyde was obtained from 1.0 g of 2-fluoro-6-methylnaphthalene.
¹H-NMR (CDCl₃, TMS) δ (ppm) : 10.1 (1H, s), 8.33 (1H, s), 7.95-8.05 (2H, m), 7.88 (1H, d, J=8.4 Hz), 7.52 (1H, dd, J=9.4 Hz, 2.4 Hz), 7.37 (1H, dt, J=8.6 Hz, 2.4 Hz)

### Reference Example 66

In the same way as in the Reference Example 30, 0.53 g of mixture of isomer of 6-fluoro-2-naphthoaldehydeoxime was obtained from 0.55 g of 6-fluoro-2-naphthoaldehyde.
¹H-NMR (CDCl₃, TMS) δ (ppm) : 8.26 (1H, s), 7.75-7.90 (4H, m), 7.42-7.47 (2H, m), 7.28 (1H, dt, J=8.8 Hz, 2.6 Hz)

### Reference Example 67

In the same way as in the Reference Example 31, 0.51 g of C-(6-fluoro-naphthalene-2-yl)-methylamine hydrochloride was obtained from 0.53 g of mixture of isomer of 6-fluoro-2-naphthoaldehydeoxime. 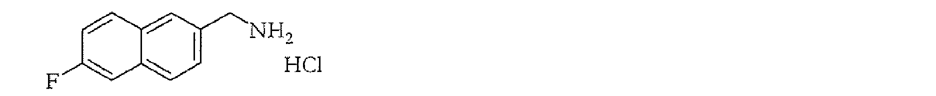 ¹H-NMR (CD₃SOCD₃, TMS) δ (ppm): 8.57 (3H, br.s), 7.94-8.10 (3H, m), 7.66-7.78 (2H, m), 7.48 (1H, dt, J=8.8 Hz, 2.2 Hz) , 4.17 (2H, s)

### Reference Example 68

10 g of 4-cyanoacetophenone, 6.4 g of ethylene glycol, about 0.1 g of p-toluenesulfonic acid and 150 ml of toluene were stirred under the condition of reflux with dehydration for 3 hours. Then 5 ml of ethylene glycol was mixed again, and stirred under the condition of reflux with dehydration for 3 hours. The reaction mixture was cooled to room temperature, then washed successively with water, saturated aqueous solution of sodium bicarbonate and saturated aqueous solution of sodium chloride, dried by magnesium sulfate, and concentrated under reduced pressure. The obtained residue was washed by hexane to obtain 11 g of 4-(2-methyl-[1,3]dioxolane-2-yl)benzonitrile. 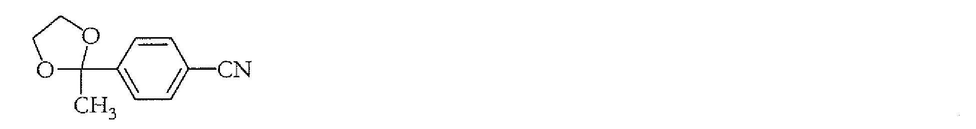 ¹H-NMR (CDCl₃, TMS) δ (ppm): 7.58-7.65 (4H, m), 4.02-4.15 (2H, m), 3.71-3.86 (2H, m), 1.63 (3H, s)

### Reference Example 69

4.4 g of lithium aluminium hydride was mixed with 150 ml of tetrahydrofuran, the mixture of 10 g of 4-(2-methyl-[1,3]dioxolane-2-yl)benzonitrile and 30 ml of tetrahydrofuran was slowly mixed with it. The mixture was stirred under the condition of reflux for 2 hours. It was cooled to room temperature, and mixed slowly with 9 ml of 20 % aqueous solution of sodium hydroxide and 11 ml of water, then stirred at room temperature for 1 hour. Then it was filtered to remove a solid. The obtained solution was concentrated under reduced pressure to obtain 11 g of 4- (2-methyl-[1,3] dioxolane-2-yl) benzylamine. 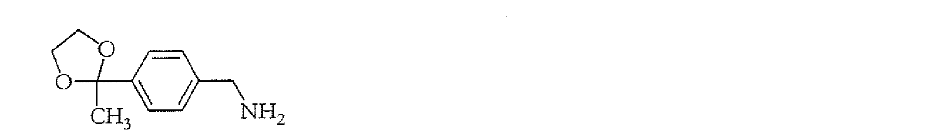 ¹H-NMR (CDCl₃, TMS) δ (ppm): 7.44 (2H, d, J=8.1 Hz), 7.28 (2H, d, J=8.1 Hz), 4.02-4.05 (2H, m), 3.86 (2H, s), 3.76-3.79 (2H, m), 1.65 (3H, s)1.46 (2H, br. s)

### Reference Example 70

In the same way as in the Reference Example 68, 13 g of 4-([1,3]dioxolane-2-yl)benzonitrile was obtained by using 11 g of 4-cyanobenzaldehyde, 8.6 g of ethylene glycol. 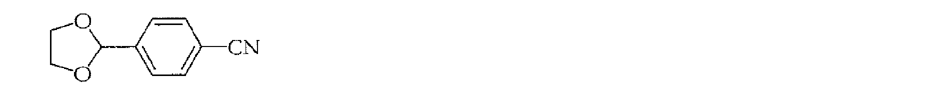 ¹H-NMR (CDCl₃, TMS) δ (ppm): 7.67 (2H, d, J=8.4 Hz), 7.59 (2H, d, J=8.4 Hz), 5.85 (1H, s), 4.09-4.11 (2H, m), 4.05-4.07 (2H, m)

### Reference Example 71

In the same way as in the Reference Example 69, 13 g of 4-([1,3]dioxolane-2-yl)benzylamine was obtained by using 5.5 g of lithium aluminum hydride and 13 g of 4-([1,3]dioxolane-2-yl)benzonitrile. 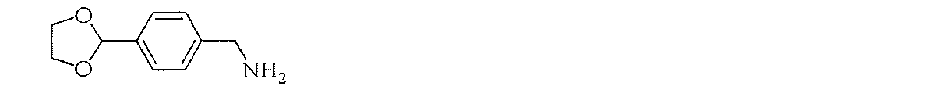 ¹H-NMR (CDCl₃, TMS) δ (ppm): 7. 44 (2H, d, J=8.0 Hz), 7.32 (2H, d, J=8.0 Hz), 5.80 (1H, s), 4.07-4.16 (2H, m), 4.01-4.05 (2H, m), 3.88 (2H, s) 1.43 (2H, br.s)

### Reference Example 72

25 g of 1-benzosuberone, 300 ml of ethanol and 3.0 g of sodium borohydride were mixed at room temperature for 3 hours. Saturated aqueous solution of ammonium chloride was added to the mixture, and concentrated under reduced pressure. Ethyl acetate was added to the residue, and extracted. The organic layer was washed with water and saturated aqueous solution of sodium chloride, dried by magnesium sulfate, concentrated under reduced pressure to obtain 25 g of 6,7,8,9-tetrahydro-5H-benzocycloheptene-5-ol. 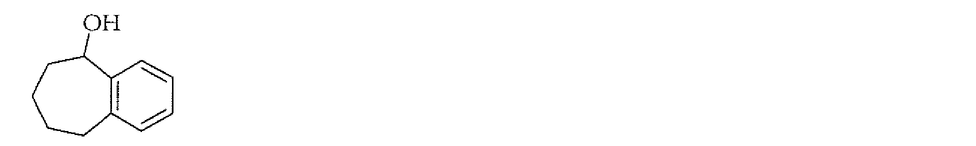 ¹H-NMR (CDCl₃, TMS) 5 (ppm): 7.43 (1H, d, J=7.1 Hz), 7.01-7.22 (3H, m), 4.93 (1H, d, J=5.5 Hz), 2.91 (1H, dd, J=14 Hz, 8.3 Hz), 2.71 (1H, ddd, J=14Hz, 11 Hz, 1.7 Hz), 2.02-2.07 (1H, m), 1.93-1.97 (1H, m), 1.75-1.86 (4H, m), 1.50-1.51 (1H, m)

### Reference Example 73

25 g of 6,7,8,9-tetrahydro-5H-benzocycloheptene-5-ol, 2.4 g of 10 % palladium charcoal, 200 ml of ethanol and 0.5 ml of 36 % hydrochloric acid were mixed under hydrogen current until the stopping of absorption of hydrogen gas. It was filtered, and filtrate was concentrated under reduced pressure. Hexane was added to the residue, and washed with water, saturated aqueous solution of sodium bicarbonate and saturated aqueous solution of sodium chloride, dried by magnesium sulfate, and concentrated under reduced pressure to obtain 22 g of 5,7,8,9-tetrahydro-5H-benzocycloheptene. 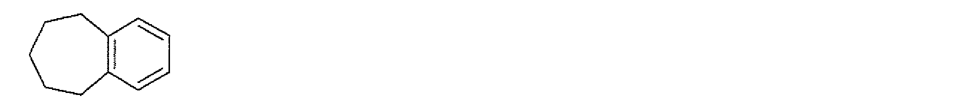 ¹H-NMR (CDCl₃, TMS) δ (ppm): 7.08 (4H, s), 2.77-2.80 (4H, m), 1.80-1.86 (2H, m), 1.63-1.69 (4H, m)

### Reference Example 74

In the same way as in the Reference Example 27, 12 g of crude product of ethyl 6,7,8,9-tetrahydro-5H-benzocycloheptene-2-yl-oxo-acetate was obtained by using 7.2 g of 6,7,8,9-tetrahydro-5H-benzocycloheptene, 6.2 g of ethyl oxalyl chloride and 6.3 g of aluminum chloride. 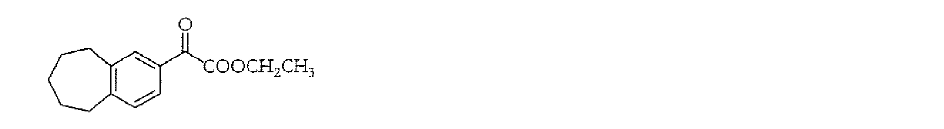 ¹H-NMR (CDCl₃, TMS) δ (ppm): 7.70-7.73 (1H, m), 7.21 (1H, d, J=4.6 Hz), 7.09 (1H, s), 4.44 (2H, q, J=7.0 Hz), 2.77-2.87 (4H, m), 1.80-1.88 (2H, m), 1.63-1.67 (4H, m), 1.42 (3H, t, J=7.0 Hz)

### Reference Example 75

In the same way as in the Reference Example 28, 5.1 g of crude product of 6,7,8,9-tetrahydro-5H-benzocycloheptene-2-yl-ethane-1,2diol was obtained by using 7.3 g of ethyl 6,7,8,9-tetrahydro-5H-benzocycloheptene-2-yl-oxo-acetate, 2.2 g of sodium borohydride. 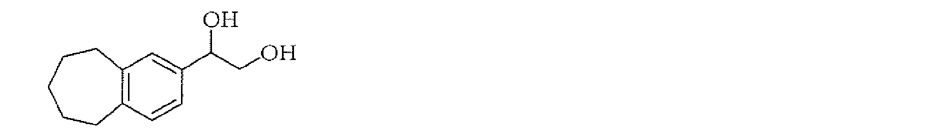 ¹H-NMR (CDCl₃, TMS) δ (ppm): 7.07-7.10 (3H, m), 4.76 (1H, dd, J=7.9 Hz, 3.6 Hz), 3.68-3.77 (2H, m), 2.76-2.80 (4H, m), 2.38 (1H, br.s), 2.02 (1H, br.s), 1.65-1.86 (2H, m), 1.63-1.64 (4H, m)

### Reference Example 76

In the same way as in the Reference Example 29, 5.1 g of crude product of 6,7,8,9-tetrahydro-5H-benzocycloheptene-2-carbaldehyde was obtained by using 5.0 g of 6,7,8,9-tetrahydro-5H-benzocycloheptene-2-y1-ethane-1,2diol, 2.2 g of sodium periodic acid. 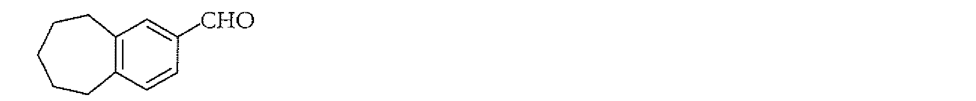 ¹H-NMR (CDCl₃, TMS) δ (ppm): 9.93 (1H, s), 7.58-7.60 (2H, m), 7.24-7.25(1H,m), 2.85-2.89 (4H, m), 1.83-1.89 (2H, m), 1.65-1.69 (4H, m)

### Reference Example 77

In the same way as in the Reference Example 30, 1.3 g of 6,7,8,9-tetrahydro-5H-benzocycloheptene-2-carbaldehydeoxime was obtained by using 1.0 g of 6,7,8,9-tetrahydro-5H-benzocycloheptene-2-carbaldehyde, 0.52 g of hydroxylamine hydrochloride. 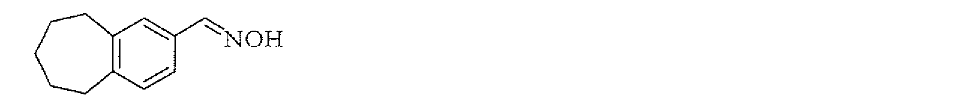 ¹H-NMR (CDCl₃, TMS) δ (ppm): 8.08 (1H, s), 7.27-7.33 (1H, m), 7.07-7.15 (2H, m), 3.33 (1H, s), 2.77-2.82 (4H, m), 1.80-1.84 (2H, m), 1.60-1.70 (4H, m)

### Reference Example 78

In the same way as in the Reference Example 31, 6,7,8,9-tetrahydro-5H-benzocycloheptene-2-methylamine hydrochloride was obtained by using 1.2 g of 6,7,8,9-tetrahydro-5H-benzocycloheptene-2-yl-carbaldehydeox ime. 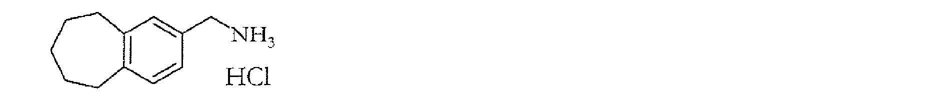 ¹H-NMR (CD₃SOCD₃, TMS) δ (ppm): 8.33 (3H, br.s), 7.12-7.22 (3H, m), 3.92 (2H, q, J=5.9 Hz), 2.75-2.77 (4H, m), 1.77-1.83 (2H, m), 1.50-1.60 (4H, m)

### Reference Example 79

According to Synlett, 2000, No.12, p1801 to 1803, 10 g of 4-bromobenzonitrile, trisdibenzylideneacetonepalladium(0), 0.98 g of 2-(di tert-butylphosphino)biphenyl and 200 ml of dimethylimidazoline were mixed at room temperature for 5 minutes hours. Then 16.9 ml of hexamethyldisilane and 2.0 g of water were added to it and washed with water for five times, dried by magnesium sulfate, concentrated under reduced pressure. The residue was purified by silica gel column to obtain 8.0 g of 4-trimethylsilylbenzonitrile. ¹H-NMR (CDCl₃, TMS) δ (ppm): 7.60 (4H, s), 0.29 (9H, s)

### Reference Example 80

In the same way as in the Reference Example 69, 0.65 g of 4-trimethylsilylbenzylamine was obtained by using 0.66 g of 4-trimethylsilylbenzonitrile, 0.29 g of lithium aluminum hydride.
¹H-NMR (CDCl₃, TMS) δ (ppm): 7.48-7.51 (2H, m), 7.31 (2H, d, J=8.0 Hz), 3.86 (2H, s), 1.43 (2H, br.s), 0.25 (9H, s)

### Reference Example 81

2.2 g of ethyl 2-fluoro-2-diethylphasphonoacetate and 50 ml of tetrahydrofuran were mixed, 040 g of sodium hydride (content 55 %) was added to it under ice cooling, and stirred for 10 minutes. Then the mixture of 2.0 g of 4-benzyloxy-3-methoxybenzaldehyde and 5 ml of tetrahydrofuran was slowly added to it, and stirred at room temperature for 3 hours. Water was added to the obtained mixture, the organic solvent was distilled off under reduced pressure, and extracted with ethyl acetate. The oil layer was separated, then it was washed with water, 5 % hydrochloric acid, saturated aqueous solution of sodium bicarbonate and saturated aqueous solution of sodium chloride, dried by magnesium sulfate, concentrated under reduced pressure. The obtained residue was purified by silica gel column to obtain 2.8 g of crude product of ethyl 3-(3-methoxy-4-benzyloxyphenyl)-2-fluoroacrylate(mixture of cis-form and trans-form). 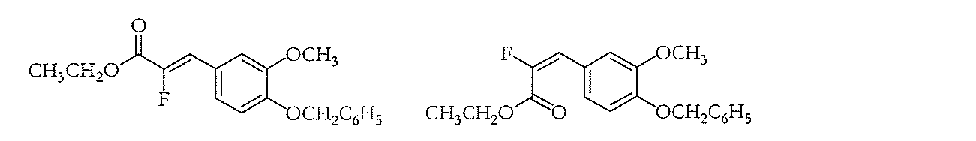 ¹H-NMR (CDCl₃, TMS) δ (ppm) : 7.27-7.44 (6H, m), 7.13 (0.3H, dd, J=8.6 Hz, 1.7 Hz,), 7.01 (0. 6H, dd, J=8.3 Hz, 1.9 Hz,), 6.79-6.90 (2H, m), 5.20 (0.6H, s), 5.18 (1.4H, s), 4.25-4.36 (2H, m), 3.91 (3H, s), 1.27-1.36 (3H, m)

### Reference Example 82

In the same way as in the Reference Example 11, 2.0 g of crude product of ethyl 3-(3-methoxy-4-hydroxyphenyl)-2-fluoropropionate was obtained by using 2.6 g of crude product of ethyl 3-(3-methoxy-4-benzyloxyphenyl)-2-fluoroacrylate. 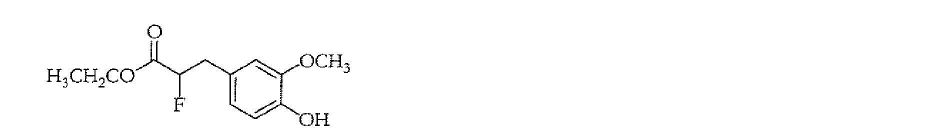 ¹H-NMR (CDCl₃, TMS) δ (ppm): 6. 85 (1H, d, J=8.0 Hz), 6.70-6.75 (2H, m), 5.52 (1H, br.s), 5.04 (1H, ddd, J=49 Hz, 7.6 Hz, 4.2 Hz), 4.22 (2H, q, J=7.1 Hz), 3.88 (3H, s), 3.06-3.18 (2H, m), 1.27 (3H, t, J=7.1 Hz)

### Reference Example 83

1.0 g of 4-iodobenzylamine, 1.1 g of di tert butyl dicarbonete, 0.9 ml of triethylamine, about 10 ml of dimethylaminopyridine and 2 ml of tetrahydrofuran were mixed at room temperature for 4 hours. Then water was added to it, and extracted with ethyl acetate. The oil layer was separated, washed with 5 % hydrochloric acid twice, saturated aqueous solution of sodium bicarbonate and saturated aqueous solution of sodium chloride, dried by magnesium sulfate, and concentrated under reduced pressure to obtain 1.5g of crude product of tert butyl (4-iodobenzyl)carbamate. 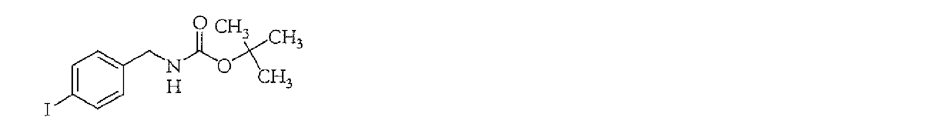 ¹H-NMR (CDCl₃, TMS) δ (ppm): 7.64 (2H, d, J=8.2 Hz), 7.03 (2H, d, J=8.2 Hz), 4.83 (1H, br.s), 4.25 (2H, d, J=5. 6 Hz), 1.45 (9H, s)

### Reference Example 84

1.5 g of tert butyl (4-iodobenzyl)carbamate, 1.3 ml of trimethylsilyl acetylene, 97 mg of bis(triphenylphosphine)palladium(II) dichloride, 1.3 ml of triethylamine, 26 mg of copper iodide (I) and 30 ml of dimethylformamide were mixed at 50°C for 4 hours. Then water was added to it, and extracted with ethyl acetate. The oil layer was separated, washed with 5 % hydrochloric acid, saturated aqueous solution of sodium bicarbonete and saturated aqueous solution of sodium cloride, dried by magnesium sulfate, and concentrated under reduced pressure. The residue was purified by silica gel column chromathography to obtain 0.55 g of tert butyl {(4-trimethylsilyletynyl)benzyl}carbamate. 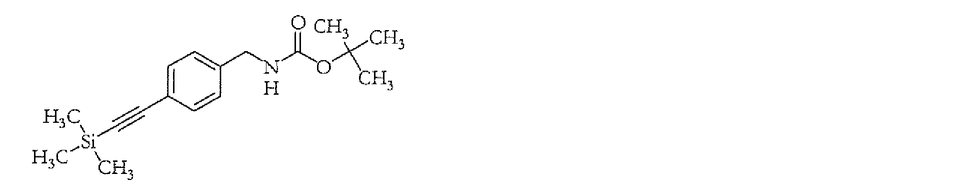 ¹H-NMR (CDCl₃, TMS) δ (ppm): 7.42 (2H, d, J=8.1 Hz), 7.20 (2H, d, J=8.1 Hz), 4.82 (1H, br.s), 4.30 (2H, d, J=5.1 Hz), 1.46 (9H, s), 0.24 (9H, s)

### Reference Example 85

0.55 g of tert butyl {(4-trimethylsilyletynyl)benzyl}carbamate, 0.42 ml of trifluoroacetic acid, 20 ml of acetic acid and 10 ml of water were mixed at 60°C for 2 hours. Then it was concentrated under reduced pressure to obtain 0.52 g of crude product of (4-trimethylsilyletynyl)benzylamine acetic acid salt. 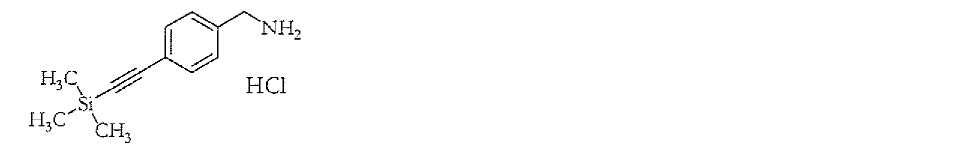 ¹H-NMR (CD₃SOCD₃, TMS) δ (ppm): 8.15 (3H, br.s), 7.52 (2H, d, J=8.4 Hz), 7.44 (2H, d, J=8.2 Hz), 4.05 (2H, s), 0.23 (9H, s)

### Reference Example 86

In the same way as in the Reference Example 69, 0.51 g of 4-cyclopropylbenzylamine was obtained by using 0.58 g of 4-cyclopropylcyanobenzen which was produced according to the method described Tetrahedron Letters 43, 6987 to 6999, and 0.27g of lithium aluminum hydride . 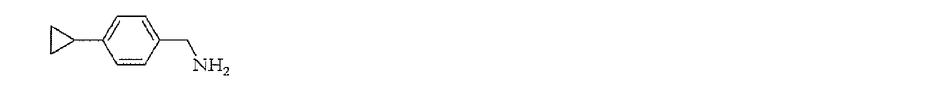 ¹H-NMR (CDCl₃, TMS) δ (ppm) : 7.19 (2H, d, J=8.1 Hz), 7.04 (2H, d, J=8.1 Hz), 3.81 (2H, s), 1.85-1.91 (1H, m), 1.45 (2H, br.s), 0.92-0.96 (2H, m), 0.65-0.69 (2H, m)

Next formulation examples are shown. Parts represent parts by weight.

### Formulation Example 1

Fifty parts of each of the compounds of the present invention 1 to 129, 3 parts of calcium ligninsulfonate, 2 parts of magnesium laurylsulfate and 45 parts of synthetic hydrated silica are pulverized and mixed well to give wettable powders of each compound.

### Formulation Example 2

Twenty parts of each of the compounds of the present invention 1 to 129 and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol are added to give a flowable of each compound.

### Formulation Example 3

Two parts of each of the compounds of the present invention 1 to 129, 88 parts of kaolin clay and 10 parts of talc are pulverized and mixed well to give a dust of each compound.

### Formulation Example 4

Five parts of each of the compounds of the present invention 1 to 129, 14 parts of polyoxyethylenestyryl phenyl ether, 6 parts of calcium dodecylbenzenesulfonate and 75 parts of xylene are mixed well to give an emulsifiable concentrate of each compound.

### Formulation Example 5

Two parts of each of the compounds of the present invention 1 to 129, 1 part of synthetic hydrated silica, 2 parts of calcium ligninsulfonate, 30 parts of bentonite and 65 parts of kaolin clay are pulverized and mixed well, and water is added thereto and kneeded, granulated and dried to give a granule of each compound.

### Formulation Example 6

Ten parts of each of the compounds of the present invention 1 to 129, 35 parts of white carbon containing 50% by weight of ammonium polyoxyethylenealkyl ether sulfate and 55 parts of water are mixed and wet pulverized finely to give a formulation of each compound.

Next, it is shown that the compound of the present invention are useful for controlling plant diseases in test examples.

Additionally, the control effect was evaluated by visually observing the area of a lesion on a sample plant in investigation and comparing the area of a lesion on a non-treatment plant and the area of a lesion on a treated plant with the compound of the present invention.

And the compound (A) shown by the formula described below which is described Journal of Chemical Society, Perkin Transactions I, 6, p.661 (1976) is also used for the test.

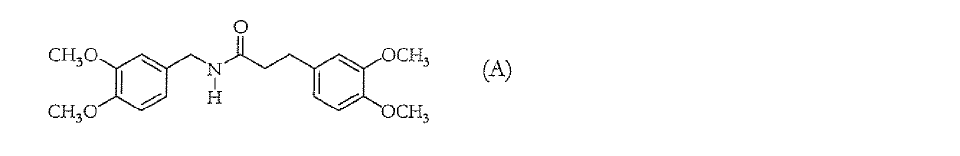

### Test Example 1

Sand loam was compacted in a plastic pot, and a tomato (variety: Ponterosa) was seeded and grown in a green house for 20 days. Each of the compounds of the present invention 1 to 27, 29 to 35, 37 to 79, 82 to 93, 95, 96, 98 to 112, 115 to 118 and 120 to 123; and the compound (A) was formulated according to Formulation Example 6, then, diluted with water to provide prescribed concentration (500 ppm), and these diluted solutions were sprayed onto stems and leaves so as to give sufficient adhesion on the surface of the tomato leaves. After spraying, the liquid on the stem was air-dried, and a suspension of zoosporangia of Phytophthora infestans (about 10,000 zoosporangia were contained in 1ml of the suspension) was inoculated by spraying (the amount of the sprayed suspension was about 2 ml for one plant). After inoculation, the plant was first grown for one day at 23°C under 90 % or more humidity, then further grown for 4 days in the green house, which was 24°C in daytime and 20°C in night-time. Then, the areas of a lesion of the plants were observed.

As a result, the lesion areas on the plants treated with the compounds of the present invention 1 to 27, 29 to 35, 37 to 79, 82 to 93, 95, 96, 98 to 112 and 115 to 118 and 120 to 123 were not more than 10% of the lesion area on a non-treatment plant. The lesion areas on the plant treated with the compound (A) was 76 to 100 % of the lesion area on a non-treatment plant.

### Test Example 2

Sand loam was compacted in a plastic pot, and a tomato (variety: Ponterosa) was seeded and grown in a green house for 20 days. Each of the compounds of the present invention 94, 113, 119 and 125 to 129 was formulated according to Formulation Example 6, then, diluted with water to provide prescribed concentration (200 ppm), and these diluted solutions were sprayed onto stems and leaves so as to give sufficient adhesion on the surface of the tomato leaves. After spraying, the liquid on the stem was air-dried, and a suspension of zoosporangia of Phytophthora infestans (about 10,000 zoosporangia were contained in 1ml of the suspension) was inoculated by spraying (the amount of the sprayed suspension was about 2 ml for one plant) . After inoculation, the plant was first grown for one day at 23°C under 90 % or more humidity, then further grown for 4 days in the green house, which was 24°C in daytime and 20°C in night-time. Then, the areas of a lesion of the plants were observed.

As a result, the lesion areas on the plants treated with the compounds of the present invention 94, 113, 119 and 125 to 129 were not more than 10% of the lesion area on a non-treatment plant.

### Test Example 3

Sand loam was compacted in a plastic pot, and a grape (variety: Berry A) was seeded and grown in a green house for 40 days. Each of the compounds of the present invention 1 to 4, 9 to 11, 16 to 19, 21, 23, 34 to 36, 40, 41, 45, 46, 49, 50, 55, 61, 63, 68, 70 to 73, 77 to 79, 81, 83, 84, 86 to 92, 95 to 97, 102, 103, 105 to 112, 116 and 120 to 129 was formulated according to Formulation Example 6, then, diluted with water to provide prescribed concentration (200ppm), and these diluted solutions were sprayed onto stems and leaves so as to give sufficient adhesion on the surface of grape leaves. After spraying, the liquid on the stem was air-dried, and a suspension of zoosporangia of Plasmopara viticola (about 10,000 zoosporangia were contained in 1ml of the suspension) was inoculated by spraying(the amount of the sprayed suspension was about 2 ml for one plant). After inoculation, the plant was first grown for one day at 23°C under 90 % or more humidity, then further grown for 6 days in the green house, which was 24°C in daytime and 20°C in night-time. Then, the areas of a lesion of the plants were obserbed.

As a result, the lesion areas on the plants treated with the compounds of the present invention 1 to 4, 9 to 11, 16 to 19, 21, 23, 34 to 36, 40, 41, 45, 46, 49, 50, 55, 61, 63, 68, 70 to 73, 77 to 79, 81, 83, 84, 86 to 92, 95 to 97, 102, 103, 105 to 112, 116 and 120 to 129 were not more than 10% of the lesion area on a non-treatment plant.

### Test Example 4

Sand loam was compacted in a plastic pot, and a grape (variety: Berry A) was seeded and grown in a green house for 40 days. Each of the compounds of the present invention 6, 13 to 15, 20, 27 to 33, 37 to 39, 48, 53, 54, 62, 64, 66, 67, 69, 74 to 76, 93, 98 to 100, 115 and 118 was formulated according to Formulation Example 6, then, diluted with water to provide prescribed concentration(50ppm), and these diluted solutions were sprayed onto stems and leaves so as to give sufficient adhesion on the surface of grape leaves. After spraying, the liquid on the stem was air-dried, and a suspension of zoosporangia of Plasmopara viticola (about 10,000 zoosporangia were contained in 1ml of the suspension) was inoculated by spraying (the amount of the sprayed suspension was about 2 ml for one plant). After inoculation, the plant was first grown for one day at 23°C under 90 % or more humidity, then further grown for 6 days in the green house, which was 24°C in daytime and 20°C in night-time. Then, the areas of a lesion of the plants were obserbed.

As a result, the lesion areas on the plants treated with the compounds of the present invention 6, 13 to 15, 20, 27 to 33, 37 to 39, 48, 53, 54, 62, 64, 66, 67, 69, 74 to 76, 93, 98 to 100, 115 and 118 were not more than 10% of the lesion area on a non-treatment plant.

### Test Example 5

Sand loam was compacted in a plastic pot, and a grape (variety: Berry A) was seeded and grown in a green house for 40 days. Each of the compounds of the present invention 5, 7, 8, 12, 44, 56, 59 and 85 was formulated according to Formulation Example 6, then, diluted with water to provide prescribed concentration (12.5ppm), and these diluted solutions were sprayed onto stems and leaves so as to give sufficient adhesion on the surface of grape leaves. After spraying, the liquid on the stem was air-dried, and a suspension of zoosporangia of Plasmopara viticola (about 10,000 zoosporangia were contained in 1ml of the suspension) was inoculated by spraying(the amount of the sprayed suspension was about 2 ml for one plant). After inoculation, the plant was first grown for one day at 23°C under 90 % or more humidity, then further grown for 6 days in the green house, which was 24°C in daytime and 20°C in night-time. Then, the areas of a lesion of the plants were obserbed.

As a result, the lesion areas on the plants treated with the compounds of the present invention 5, 7, 8, 12, 44, 56, 59 and 85 were not more than 10% of the lesion area on a non-treatment plant.

### Industrial Applicability

Plant diseases can be controlled by using the compound of the present compounds.

## Claims

1. An amide compound represented by the formula (1): 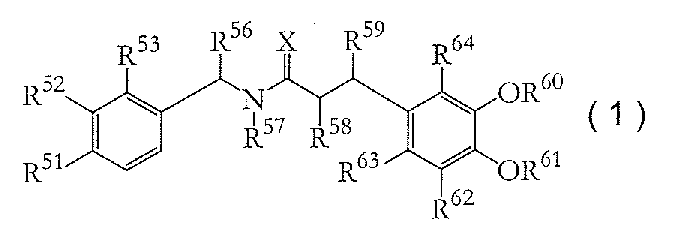 wherein, in the formula,
R⁵¹ represents a halogen atom, a C1-C6 alkyl group, a C3-C6 cycloalkyl group, a C1-C6 haloalkyl group, a C2-C6 alkenyl group, a C2-C6 haloalkenyl group, a C2-C6 alkynyl group, a C2-C6 haloalkynyl group, a C1-C6 alkoxy group, a C3-C6 alkenyloxy group, a C3-C6 alkynyloxy group, a C1-C6 haloalkoxy group, a (C1-C6 alkoxy)C1-C6 alkyl group, a phenoxy C1-C6 alkyl group, a C1-C6 hydroxyalkyl group, a (C1-C6alkyl) sulfonyloxy C1-C6 alkyl group, a C1-C6 alkylthio group, a C1-C6 haloalkylthio group, a C1-C6 alkylamino group, a di (C1-C6alkyl) amino group, a formyl group, a (C1-C6 alkyl) carbonyl group, a (C1-C6 alkoxy) carbonyl group, a (C1-C6 alkoxy) imino C1-C6 alkyl group, benzyloxyimino C1-C6 alkyl group, a di(C1-C6 alkylamino) imino C1-C6 alkyl group, a tri(C1-C6 alkyl) silyl group, a phenyl group, a phenoxy group, a cyano group or a nitro group; R⁵² represents a hydrogen atom, a halogen atom, a C1-C6 alkyl group, a C1-C6 haloalkyl group, a C2-C6 alkenyl group, a C2-C6 alkynyl group, a cyano group or a nitro group; or both of R⁵¹ and R⁵² are combined together to represent a C3-C6 alkylene group or a group of -CR⁶⁵=CR⁶⁶-CR⁶⁷=CR⁶⁸-(R⁶⁵, R⁶⁶, R⁶⁷ and R⁶⁸ independently represent a hydrogen atom, a halogen atom, a C1-C3 alkyl group, a C1-C3 alkoxy group or a C1-C3 haloalkyl group);
R⁵³ represents a hydrogen atom, a halogen atom, a C1-C3 alkyl group or a C1-C3 haloalkyl group;
R⁵⁶ represents a hydrogen atom, a C1-C4 alkyl group, a C2-C4 alkenyl group or a C2-C4 alkynyl group;
R⁵⁷ represents a hydrogen atom, a C1-C4 alkyl group, a C2-C4 alkenyl group or a C2-C4 alkynyl group;
R ⁵⁸ and R⁵⁹ independently represent a hydrogen atom, a halogen atom or a C1-C3 alkyl group;
R⁶⁰ represents a C1-C4 alkyl group, a C1-C4 haloalkyl group, a C3-C4 alkenyl group or a C3-C6 alkynyl group;
R⁶¹ represents a C1-C4 alkyl group, a C1-C4 haloalkyl group, a C3-C4 alkenyl group or a C3-C6 alkynyl group or a C2-C4 cyanoalkyl group;
each of R⁶², R⁶³ and R⁶⁴ represents a hydrogen atom, a halogen atom or a C1-C2 alkyl group;
X represents an oxygen atom or a sulfur atom.

2. The amide compound according to claim 1, wherein R⁵¹ is a halogen atom, a C1-C6 alkyl group, a C3-C6 cycloalkyl group, a C1-C6 haloalkyl group, aC2-C6alkenyl group, a C2-C6 haloalkenyl group, a C2-C6 alkynyl group, a C2-C6 haloalkynyl group, a C1-C6 alkoxy group, a C3-C6 alkenyloxy group, a C3-C6 alkynyloxy group, a C1-C6 haloalkoxy group, a (C1-C6alkoxy) C1-C6 alkyl group, a phenoxy C1-C6 alkyl group, a C1-C6 hydroxyalkyl group, a (C1-C6 alkyl) sulfonyloxy C1-C6 alkyl group, a C1-C6 alkylthio group, a C1-C6 haloalkylthio group, a C1-C6 alkylamino group, a di (C1-C6 alkyl) amino group, a formyl group, a (C1-C6 alkyl) carbonyl group, a (C1-C6 alkoxy) carbonyl group, a (C1-C6 alkoxy) imino C1-C6 alkyl group, a benzyloxyimino C1-C6 alkyl group, a di (C1-C6 alkylamino) imino C1-C6 alkyl group, tri (C1-C6 alkyl) silyl group, a phenyl group, a phenoxy group, a cyano group or a nitro group; R⁵² is a hydrogen atom, a halogen atom, a C1-C6 alkyl group, a C1-C6 haloalkyl group, a C2-C6 alkenyl group, a C2-C6 alkynyl group, a cyano group or a nitro group.

3. The amide compound according to claim 1, wherein the group which R⁵¹ and R⁵² are combined together is a group of -CR⁶⁵=CR⁶⁶-CR⁶⁷=CR⁶⁸- (R⁶⁵, R⁶⁶, R⁶⁷ and R⁶⁸ is independently a hydrogen atom, a halogen atom, a C1-C3 alkyl group, a C1-C3 alkoxy group or a C1-C3 haloalkyl group).

4. The amide compound according to any one of claim 1 to 3, wherein R⁵³ is a hydrogen atom.

5. The amide compound according to any one of claim 1 to 4, wherein R⁶², R⁶³ and R⁶⁴ are hydrogen atoms.

6. The amide compound according to anyone of claim 1 to 5, wherein R⁵⁸ and R⁵⁹ is independently a hydrogen atom, a fluorine atom or a methyl group.

7. The amide compound according to any one of claim 1 to 5, wherein R⁵⁸ and R⁵⁹ are hydrogen atoms.

8. The amide compound according to any one of claim 1 to 7, wherein R⁵⁶ is a hydrogen atom.

9. The amide compound according to claim 1, wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di (C1-C4alkyl) amino group or a cyano group; R⁵² is a hydrogen atom, a halogen atom, a C1-C4 alkyl group, a C1-C4 haloalkyl group, a C2-C4 alkenyl group or a C2-C4 alkynyl group; or both of R⁵¹ and R⁵² are combined together to be a C3-C5 alkylene group or a group of -CH=CH-CH=CH-;
R⁵⁷ is a hydrogen atom or a C1-C3 alkyl group;
R⁶⁰ is a C1-C4 alkyl group, a C1-C4 haloalkyl group, a C3-C4 alkenyl group or a C3-C4 alkynyl group;
R⁶¹ is a C1-C4 alkyl group, a C1-C4 haloalkyl group, a C3-C4 alkenyl group or a C3-C4 alkynyl group.

10. The amide compound according to claim 9, wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group, a C1-C4 alkylamino group, a di(C1-C4 alkyl) amino group or a cyano group; R⁵² is a hydrogen atom, a halogen atom, a C1-C4 alkyl group, a C1-C4 haloalkyl group, a C2-C4 alkenyl group or a C2-C4 alkynyl group.

11. The amide compound according to claim 9, wherein the group which R⁵¹ and R⁵² are combined together is a C3-C5 alkylene group or a group of -CH=CH-CH=CH-.

12. The amide compound according to any one of claim 9 to 11, wherein R⁵³ is a hydrogen atom.

13. The amide compound according to any one of claim 9 to 12, wherein R⁶², R⁶³ and R⁶⁴ are hydrogen atoms.

14. The amide compound according to any one of claim 9 to 13, wherein R⁵⁸ and R⁵⁹ are hydrogen atoms.

15. The amide compound according to any one of claim 9 to 14, wherein R⁵⁶ is a hydrogen atom.

16. The amide compound according to any one of claim 1 to 15, wherein R⁵⁷ is a hydrogen atom.

17. The amide compound according to any one of claim 1 to 16, wherein X is an oxygen atom.

18. The amide compound according to any one of claim 1 to 16, wherein X is a sulfur atom.

19. The amide compound according to any one of claim 1 to 18, wherein R⁵¹ is a halogen atom, a C1-C4 alkyl group, a C1-C4 haloalkyl group, a C2-C4 alkenyl group, a C2-C4 alkynyl group, a C1-C4 alkoxy group, a C1-C4 haloalkoxy group or a cyano group; R⁵² is a hydrogen atom, a halogen atom, a C1-C4 alkyl group, a C1-C4 haloalkyl group, a C2-C4 alkenyl group or a C2-C4 alkynyl group; or both of R⁵¹ and R⁵² are combined together to be a C3-C5 alkylene group or a group of -CH=CH-CH=CH-.

20. The amide compound according to any one of claim 1 to 18, wherein R⁵² is a hydrogen atom, a halogen atom, a C1-C4 alkyl group or a C1-C4 haloalkyl group.

21. The amide compound according to any one of claim 1 to 20, wherein R⁵¹ is a halogen atom, a C1-C4 alkyl group or a C1-C4 haloalkyl group.

22. The amide compound according to any one of claim 1 to 20, wherein R⁵² is a hydrogen atom.

23. The amide compound according to any one of claim 1 to 18, wherein both of R⁵¹ and R⁵² may be combined together to be a C3-C6 alkylene group or a group of -CH=CH-CH=CH-.

24. The amide compound according to any one of claim 1 to 23, wherein R⁶⁰ is a C1-C4 alkyl group.

25. The amide compound according to any one of claim 1 to 23, wherein R⁶⁰ is a C1-C2 alkyl group.

26. The amide compound according to any one of claim 1 to 25, wherein R⁶¹ is a C3-C4 alkynyl group.

27. A plant diseases controlling composition comprising the amide compound according to any one of claim 1 to 26 as an active ingredient.

28. A method for controlling plant diseases comprising a step applying an effective amount of the amide compound according to any one of claim 1 to 26 to plants or soils growing the plant.

29. A use of the amide compound according to any one of claim 1 to 26 as an active ingredient of a plant disease controlling composition.

30. A compound represented by the formula (3): 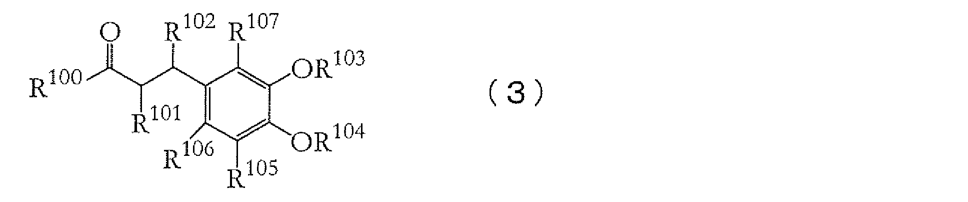 wherein, in the formula,
R¹⁰⁰ represents a methoxy group, an ethoxy group, a propoxy group, an isopropoxy group, a butyloxy group, an isopropyloxy group, a tert-buthyloxy group, an OH group or a chlorine atom; R¹⁰¹ and R¹⁰² independently represent a hydrogen atom, a halogen atom or a C1-C3 alkyl group; R¹⁰³ represents a C1-C4 alkyl group; R¹⁰⁴ represents a C3-C6 alkynyl group; R¹⁰⁵, R¹⁰⁶ and R¹⁰⁷ independently represent a hydrogen atom, a halogen atom or a C1-C2 alkyl group.

31. The compound according to claim 30, wherein each of R¹⁰¹ and R¹⁰² is a hydrogen atom, a fluorine atom or a methyl group; R¹⁰⁵, R¹⁰⁶ and R¹⁰⁷ are hydrogen atoms.

32. The compound according to claim 30, wherein R¹⁰¹, R¹⁰², R¹⁰⁵, R¹⁰⁶ and R¹⁰⁷ are hydrogen atoms.

33. The compound according to any one of claim 30 to 32, wherein R¹⁰³ is a methyl group or an ethyl group.

34. The compound according to any one of claim 30 to 33, wherein R¹⁰⁴ is a 2-propynyl group.

35. An amide compound represented by the formula (4): 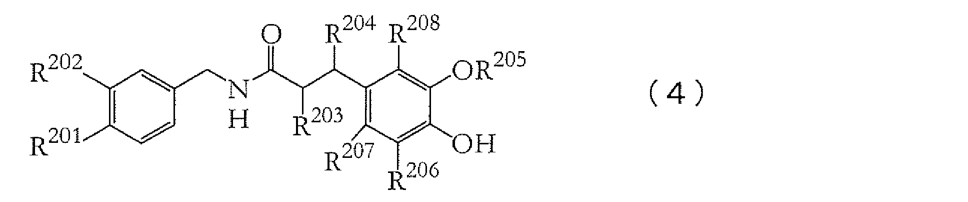 wherein, in the formula,
R²⁰¹ represents a halogen atom, a C1-C4 alkyl group, a C1-C4 haloalkyl group, a C1-C4 alkoxy group, a C1-C4 haloalkoxy group, a di(C1-C4alkyl)amino group or a cyano group; R²⁰² represents a hydrogen atom, a halogen atom, a C1-C4 alkyl group or a C1-C4 haloalkyl group; or both of R²⁰¹ and R²⁰² are combined together to represent a C3-C5 alkylene group or a group or -CH=CH-CH=CH-; R²⁰³ and R²⁰⁴ independently represent a hydrogen atom, a halogen atom or a C1-C3 alkyl group; R²⁰⁵ represents a C1-C4 alkyl group, R²⁰⁶, R²⁰⁷ and R²⁰⁸ independently represent a hydrogen atom, a halogen atom or a C1-C2 alkyl group.

36. The amide compound according to claim 35, wherein each of R²⁰³ and R²⁰⁴ is a hydrogen atom, a fluorine atom or a methyl group; R²⁰⁶, R²⁰⁷ and R²⁰⁸ are hydrogen atoms.

37. The amide compound according to claim 35, wherein R²⁰³, R²⁰⁴, R²⁰⁶, R²⁰⁷ and R²⁰⁸ are hydrogen atoms.

38. The compound according to any one of claim 35 to 37, wherein R²⁰⁵ is a methyl group or an ethyl group.
